# EUROPEAN PATENT SPECIFICATION

(11) **EP 1 847 610 B1**
(45) Date of publication and mention of the grant of the patent: **28.11.2012**
(21) Application number: 07013621.3
(22) Date of filing: 16.08.1999
(51) Int. Cl.: C12N 15/54, C12N 9/10, C12N 5/10, C12N 15/82

(54) **Maize cellulose synthases and uses thereof**
Maiscellulosesynthasen und Verwendungen davon
Synthases de cellulose de maïs et utilisations associées

(30) Priority: 17.08.1998 US 96822 P
(43) Date of publication of application: 24.10.2007
(62) Divisional of application: 99942280.1
(73) Proprietor: PIONEER HI-BRED INTERNATIONAL, INC., Des Moines, Iowa 50309 (US)
(72) Inventor: Dhugga, Kanwarpal S., Johnston Iowa 50131 (US); Bowen, Benjamin A., Berkeley California 94705 (US); Helentjaris, Timothy G., Tucson, AZ 85745-8609 (US); Wang, Xun, Beijing 101312 (CN)
(74) Representative: Bentham, Andrew

(56) References cited:
- WO-A-00/04166
- WO-A-98/00549
- WO-A-98/18949
- US-A- 5 723 764
- ARIOLI ET AL.: "Molecular analysis of cellulose biosynthesis in Arabidopsis" EMBL SEQUENCE DATABASE, 1 June 1998 (1998-06-01), XP002125688 HEIDELBERG DE -& ARIOLI ET AL.: "Molecular analysis of cellulose biosynthesis in Arabidopsis" EMBL SEQUENCE DATABASE, 20 January 1998 (1998-01-20), XP002140698 HEIDELBERG DE -& ARIOLI ET AL.: "Molecular analysis of cellulose biosynthesis in Arabidopsis" EMBL SEQUENCE DATABASE, 1 June 1998 (1998-06-01), XP002140697 HEIDELBERG DE -& ARIOLI ET AL.: "Molecular analysis of cellulose biosynthesis in Arabidopsis" EMBL SEQUENCE DATABASE, 3 February 1998 (1998-02-03), XP002125689 HEIDELBERG DE -& ARIOLI ET AL.: "Molecular analysis of cellulose biosynthesis in Arabidopsis" EMBL SEQUENCE DATABASE, 1 June 1998 (1998-06-01), XP002140699 HEIDELBERG DE -& ARIOLI ET AL.: "Molecular analysis of cellulose biosynthesis in Arabidopsis" EMBL SEQUENCE DATABASE, 3 February
- WU ET AL.: "Cellulose synthase" EMBL SEQUENCE DATABASE, 1 August 1998 (1998-08-01), XP002140701 HEIDELBERG DE
- PEAR J R ET AL: "HIGHER PLANTS CONTAIN HOMOLOGS OF THE BACTERIAL CELA GENES ENCODINGTHE CATALYTIC SUBUNIT OF CELLULOSE SYNTHASE" PROCEEDINGS OF THE NATIONAL ACADEMY OF SCIENCES OF USA,US,NATIONAL ACADEMY OF SCIENCE. WASHINGTON, vol. 93, October 1996 (1996-10), page 12637-12642, XP002061424 ISSN: 0027-8424
- AMOR Y ET AL: "EVIDENCE FOR A CYCLIC DIGUANYLIC ACID-DEPENDENT CELLULOSE SYNTHASE IN PLANTS" PLANT CELL,US,AMERICAN SOCIETY OF PLANT PHYSIOLOGISTS, ROCKVILLE, MD, vol. 3, September 1991 (1991-09), page 989-995, XP002061420 ISSN: 1040-4651

## Description

### TECHNICAL FIELD

The present invention relates generally to plant molecular biology. More specifically, it relates to nucleic acids and methods for modulating their expression in plants.

### BACKGROUND OF THE INVENTION

Polysaccharides constitute the bulk of the plant cell walls and have been traditionally classified into three categories: cellulose, hemicellulose, and pectin. Fry, S. C. (1988), The growing plant cell wall: Chemical and metabolic analysis. New York: Longman Scientific & Technical. Whereas cellulose is made at the plasma membrane and directly laid down into the cell wall, hemicellulosic and pectic polymers are first made in the Golgi apparatus and then exported to the cell wall by exocytosis. Ray, P. M., et al., (1976), Ber. Deutsch. Bot. Ges. Bd. 89, 121-146. The variety of chemical linkages in the pectic and hemicellulosic polysaccharides indicates that there must be tens of polysaccharide synthases in the Golgi apparatus. Darvill et al., (1980). The primary cell walls of flowering plants. In The Plant Cell (N. E. Tolbert, ed.), Vol. 1 in Series: The biochemistry of plants: A comprehensive treatise, eds. P.K. Stumpf and E.E. Conn (New York: Academic Press), pp. 91-162.

Cellulose, by virtue of its ability to form semicrystalline microfibrils, has a very high tensile strength which approaches that of some metals. Niklas, K. J. (1992). Plant Biomechanics: An engineering approach to plant form and function, The University of Chicago Press, pp. 607. Bending strength of the culm of normal and brittle-culm mutants of barley has been found to be directly correlated with the concentration of cellulose in the cell wall. Kokubo, et al., (1989), Plant Physiology 91, 876-882; Kokubo, et al., (1991) Plant Physiology 97, 509-514.

Even though sugar and polysaccharide compositions of the plant cell walls have been well characterized, very limited progress has been made toward identification of the enzymes involved in polysaccharides formation, the reason being their labile nature and recalcitrance to solubilization by available detergents. Sporadic claims for the identification of cellulose synthase from plant sources have been made over the years. Callaghan, T., and Benziman, M. (1984), Nature 311, 165-167; Okuda, et al., (1993), Plant Physiol. 101, 1131-1142. However, these claims have been met with skepticism. Callaghan, T., and Benziman, M. (1985), Nature 314,383-384; Delmer, et al., (1993), Plant Physiol. 103,307-308. It was only recently that a putative gene for plant cellulose synthase (CelA) was cloned from the developing cotton fibers based on homology to the bacterial gene. Pear, et al., Proc. Natl. Acad. Sci. (USA) 93,12637-12642; Saxena, et al., (1990), Plant Molecular Biology 15, 673-684; see also, WO 9818949.

As brittle snap is a major problem in corn breeding, what is needed in the art are compositions and methods for manipulating cellulose concentration in the cell wall and thereby altering plant stalk quality for improved standability or silage. The present invention provides these and other advantages.

### SUMMARY OF THE INVENTION

Generally, it is the object of the present invention to provide nucleic acids and proteins relating to cellulose synthases. It is an object of the present invention to provide:
1) nucleic acids and proteins relating to maize cellulose synthases; 2) transgenic plants comprising the nucleic acids of the present invention; 3) methods for modulating, in a transgenic plant, the expression of the nucleic acids of the present invention.

Therefore, in one aspect, the present invention provides an isolated cellulose synthase nucleic acid encoding a polypeptide having cellulose synthase activity and comprising:
(a) a polynucleotide of SEQ ID NO: 57; or
(b) a polynucleotide encoding a polypeptide of SEQ ID NO: 58.

The isolated nucleic acid can be DNA or RNA.

In another aspect, the present invention relates to recombinant expression cassettes, comprising a nucleic acid of the present invention operably linked to a promoter.

In another aspect, the present invention is directed to a host cell transfected with the recombinant expression cassette.

In a further aspect, the present invention relates to an isolated protein having cellulose synthase activity and comprising:
(a) a polypeptide of SEQ ID NO: 58; and
(b) a polypeptide encoded by an isolated nucleic acid of the present invention.

In another aspect, the present invention relates to a recombinant expression cassette comprising a nucleic acid, wherein the nucleic acid is operably linked to a promoter. In some embodiments, the present invention relates to a host cell transfected with this recombinant expression cassette. In some embodiments, the present invention relates to a protein of the present invention which is produced from this host cell.

In yet another aspect, the present invention relates to a transgenic plant comprising a recombinant expression cassette comprising a plant promoter operably linked to any of the isolated nucleic acids of the present invention. In some embodiments, the transgenic plant is *Zea mays.* The present invention also provides transgenic seed from the transgenic plant.

In a further aspect, the present invention relates to a method of modulating expression of the genes encoding the proteins of the present invention in a plant cell capable of plant regeneration, comprising the steps of (a) transforming a plant cell with a recombinant expression cassette comprising a polynucleotide of the present invention operably linked to a promoter; (b) growing the plant cell under plant growing conditions; and (c) inducing expression of the polynucleotide for a time sufficient to modulate expression of the genes in the plant. In some embodiments, the plant is maize. Expression of the genes encoding the proteins of the present invention can be increased or decreased relative to a non-transformed control plant.

### Definitions

Units, prefixes, and symbols may be denoted in their SI accepted form. Unless otherwise indicated, nucleic acids are written left to right in 5' to 3' orientation; amino acid sequences are written left to right in amino to carboxy orientation, respectively. Numeric ranges are inclusive of the numbers defining the range and include each integer within the defined range. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. Unless otherwise provided for, software, electrical, and electronics terms as used herein are as defined in The New IEEE Standard Dictinary of Electrical and electronics Terms (5th edition, 1993). The terms defined below are more fully defined by reference to the specification as a whole.

By "amplified" is meant the construction of multiple copies of a nucleic acid sequence or multiple copies complementary to the nucleic acid sequence using at least one of the nucleic acid sequences as a template. Amplification systems include the polymerase chain reaction (PCR) system, ligase chain reaction (LCR) system, nucleic acid sequence based amplification (NASBA, Cangene, Mississauga, Ontario), *Q-Beta* Replicase systems, transcription-based amplification system (TAS), and strand displacement amplification (SDA). See, e.g., Diagnostic Molecular Microbiology: Principles and Applications, D. H. Persing et al., Ed., American Society for Microbiology, Washington, D.C. (1993). The product of amplification is termed an amplicon.

The term "antibody" includes reference to antigen binding forms of antibodies (e.g., Fab, F(ab)₂). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized *de novo* either chemically or by utilizing recombinant DNA methodology. Thus, the term antibody, as used herein, also includes antibody fragments such as single chain Fv, chimeric antibodies (i.e., comprising constant and variable regions from different species), humanized antibodies (i.e., comprising a complementarity determining region (CDR) from a non-human source) and heteroconjugate antibodies (e.g., bispecific antibodies).

The term "antigen" includes reference to a substance to which an antibody can be generated and/or to which the antibody is specifically immunoreactive. The specific immunoreactive sites within the antigen are known as epitopes or antigenic determinants. These epitopes can be a linear array of monomers in a polymeric composition - such as amino acids in a protein - or consist of or comprise a more complex secondary or tertiary structure. Those of skill will recognize that all immunogens (i.e., substances capable of eliciting an immune response) are antigens; however some antigens, such as haptens, are not immunogens but may be made immunogenic by coupling to a carrier molecule. An antibody immunologically reactive with a particular antigen can be generated *in vivo* or by recombinant methods such as selection of libraries of recombinant antibodies in phage or similar vectors. *See, e.g.,* Huse et al., Science 246: 1275-1281 (1989); and Ward, et al., Nature 341: 544-546 (1989); and Vaughan et al., Nature Biotech. 14: 309-314 (1996).

As used herein, "antisense orientation" includes reference to a duplex polynucleotide sequence which is operably linked to a promoter in an orientation where the antisense strand is transcribed. The antisense strand is sufficiently complementary to an endogenous transcription product such that translation of the endogenous transcription product is often inhibited.

As used herein, "chromosomal region" includes reference to a length of a chromosome which may be measured by reference to the linear segment of DNA which it comprises. The chromosomal region can be defined by reference to two unique DNA sequences, i.e., markers.

The term "conservatively modified variants" applies to both amino acid and nucleic acid sequences. With respect to particular nucleic acid sequences, conservatively modified variants refers to those nucleic acids which encode identical or conservatively modified variants of the amino acid sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. Every nucleic acid sequence herein which encodes a polypeptide also describes every possible silent variation of the nucleic acid. One of ordinary skill will recognize that each codon in a nucleic acid (except AUG; which is ordinarily the only codon for methionine; and UGG , which is ordinarily the only codon for for tryptophan) can be modified to yield a functionally identical molecule. Accordingly, each silent variation of a nucleic acid which encodes a polypeptide of the present invention is implicit in each described polypeptide sequence and incorporated herein by reference.

As to amino acid sequences, one of skill will recognize that individual substitutions, deletions or additions to a nucleic acid, peptide, polypeptide, or protein sequence which alters, adds or deletes a single amino acid or a small-percentage of amino acids in the encoded sequence is a "conservatively modified variant" where the alteration results in the substitution of an amino acid with a chemically similar amino acid. Thus, any number of amino acid residues selected from the group of integers consisting of from 1 to 15 can be so altered. Thus, for example, 1, 2, 3, 4, 5, 7, or 10 alterations can be made. Conservatively modified variants typically provide similar biological activity as the unmodified polypeptide sequence from which they are derived. For example, substrate specificity, enzyme activity, or ligand/receptor binding is generally at least 30%, 40%, 50%, 60%, 70%, 80%, or 90% of the native protein for it's native substrate. Conservative substitution tables providing functionally similar amino acids are well known in the art.

The following six groups each contain amino acids that are conservative substitutions for one another:
1) Alanine (A). Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W). See also, Creighton (1984) Proteins W.H. Freeman and Company.

By "encoding" or "encoded", with respect to a specified nucleic acid, is meant comprising the information for translation into the specified protein. A nucleic acid encoding a protein may comprise non-translated sequences (e.g., introns) within translated regions of the nucleic acid, or may lack such intervening non-translated sequences (e.g., as in cDNA). The information by which a protein is encoded is specified by the use of codons. Typically, the amino acid sequence is encoded by the nucleic acid using the "universal" genetic code. However, variants of the universal code, such as are present in some plant, animal, and fungal mitochondria, the bacterium *Mycoplasma capricolum* (Proc. Natl. Acad. Sci. (USA), 82: 2306-2309 (1985)), or the ciliate *Macronucleus,* may be used when the nucleic acid is expressed using these organisms.

When the nucleic acid is prepared or altered synthetically, advantage can be taken of known codon preferences of the intended host where the nucleic acid is to be expressed. For example, although nucleic acid sequences of the present invention may be expressed in both monocotyledonous and dicotyledonous plant species, sequences can be modified to account for the specific codon preferences and GC content preferences of monocotyledons or dicotyledons as these preferences have been shown to differ (Murray et al. Nucl. Acids Res. 17: 477-498 (1989)). Thus, the maize preferred codon for a particular amino acid may be derived from known gene sequences from maize. Maize codon usage for 28 genes from maize plants are listed in Table 4 of Murray *et al., above.*

As used herein "full-length sequence" in reference to a specified polynucleotide or its encoded protein means having the entire amino acid sequence of, a native (non-synthetic), endogenous, catalytically active form of the specified protein. Methods to determine whether a sequence is full-length are well known in the art including such exemplary techniques as northern or western blots, primer extension, S1 protection, and ribonuclease protection. See, e.g., Plant Molecular Biology: A Laboratory Manual, Clark, Ed., Springer-Verlag, Berlin (1997). Comparison to known full-length homologous (orthologous and/or paralogous) sequences can also be used to identify full-length sequences of the present invention. Additionally, consensus sequences typically present at the 5' and 3' untranslated regions of mRNA aid in the identification of a polynucleotide as full-length. For example, the consensus sequence ANNNNAUGG, where the underlined codon represents the N-terminal methionine, aids in determining whether the polynucleotide has a complete 5' end. Consensus sequences at the 3' end, such as polyadenylation sequences, aid in determining whether the polynucleotide has a complete 3' end.

The term "gene activity" refers to one or more steps involved in gene expression, including transcription, translation, and the functioning of the protein encoded by the gene.

As used herein, "heterologous" in reference to a nucleic acid is a nucleic acid that originates from a foreign species, or, if from the same species, is substantially modified from its native form in composition and/or genomic locus by deliberate human intervention. For example, a promoter operably linked to a heterologous structural gene is from a species different from that from which the structural gene was derived, or, if from the same species, one or both are substantially modified from their original form. A heterologous protein may originate from a foreign species or, if from the same species, is substantially modified from its original form by deliberate human intervention.

By "host cell" is meant a cell which contains a vector and supports the replication and/or expression of the expression vector. Host cells may be prokaryotic cells such as *E. coli,* or eukaryotic cells such as yeast, insect, amphibian, or mammalian cells. Preferably, host cells are monocotyledonous or dicotyledonous plant cells. A particularly preferred monocotyledonous host cell is a maize host cell.

The term "hybridization complex" includes reference to a duplex nucleic acid structure formed by two single-stranded nucleic acid sequences selectively hybridized with each other.

By "immunologically reactive conditions" or "immunoreactive conditions" is meant conditions which allow an antibody, generated to a particular epitope, to bind to that epitope to a detectably greater degree (e.g., at least 2-fold over background) than the antibody binds to substantially all other epitopes in a reaction mixture comprising the particular epitope. Immunologically reactive conditions are dependent upon the format of the antibody binding reaction and typically are those utilized in immunoassay protocols. See Harlow and Lane, Antibodies, A Laboratory Manual, Cold Spring Harbor Publications, New York (1988), for a description of immunoassay formats and conditions.

The term "introduced" in the context of inserting a nucleic acid into a cell, means "transfection" or "transformation" or "transduction" and includes reference to the incorporation of a nucleic acid into a eukaryotic or prokaryotic cell where the nucleic acid may be incorporated into the genome of the cell (e.g., chromosome, plasmid, plastid or mitochondrial DNA), converted into an autonomous replicon, or transiently expressed (e.g., transfected mRNA).

The terms "isolated" refers to material, such as a nucleic acid or a protein, which is: (1) substantially or essentially free from components which normally accompany or interact with it as found in its naturally occurring environment. The isolated material optionally comprises material not found with the material in its natural environment; or (2) if the material is in its natural environment, the material has been synthetically (non-naturally) altered by deliberate human intervention to a composition and/or placed at a locus in the cell (e.g., genome or subcellular organelle) not native to a material found in that environment. The alteration to yield the synthetic material can be performed on the material within or removed-from its natural state. For example, a naturally occurring nucleic acid becomes an isolated nucleic acid if it is altered, or if it is transcribed from DNA which has been altered, by non-natural, synthetic (i.e., "man-made") methods performed within the cell from which it originates. See, e.g., Compounds and Methods for Site Directed Mutagenesis in Eukaryotic Cells, Kmiec, U.S. Patent No. 5,565,350; *In Vivo* Homologous Sequence Targeting in Eukaryotic Cells; Zarling et al., PCT/US93/03868. Likewise, a naturally occurring nucleic acid (e.g., a promoter) becomes isolated if it is introduced by non-naturally occurring means to a locus of the genome not native to that nucleic acid. Nucleic acids which are "isolated" as defined herein, are also referred to as "heterologous" nucleic acids.

Unless otherwise stated, the term "cellulose synthase nucleic acid" is a nucleic acid of the present invention and means a nucleic acid comprising a polynucleotide of the present invention (a "cellulose synthase polynucleotide") encoding a cellulose synthase polypeptide. A "cellulose synthase gene" is a gene of the present invention and refers to a non-heterologous genomic form of a full-length cellulose synthase polynucleotide.

As used herein, "localized within the chromosomal region defined by and including" with respect to particular markers includes reference to a contiguous length of a chromosome delimited by and including the stated markers.

As used herein, "marker" includes reference to a locus on a chromosome that serves to identify a unique position on the chromosome. A "polymorphic marker" includes reference to a marker which appears in multiple forms (alleles) such that different forms of the marker, when they are present in a homologous pair, allow transmission of each of the chromosomes in that pair to be followed. A genotype may be defined by use of one or a plurality of markers.

As used herein, "nucleic acid" includes reference to a deoxyribonucleotide or ribonucleotide polymer in either single- or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids).

By "nucleic acid library" is meant a collection of isolated DNA or RNA molecules which comprise and substantially represent the entire transcribed fraction of a genome of a specified organism. Construction of exemplary nucleic acid libraries, such as genomic and cDNA libraries, is taught in standard molecular biology references such as Berger and Kimmel, Guide to Molecular Cloning Techniques, Methods in Enzymology, Vol. 152, Academic Press, Inc., San Diego, CA (Berger); Sambrook et al., Molecular Cloning - A Laboratory Manual, 2nd ed., Vol. 1-3 (1989); and Current Protocols in Molecular Biology, F.M. Ausubel et al., Eds., Current Protocols, a joint venture between Greene Publishing Associates, Inc. and John Wiley & Sons, Inc. (1994 Supplement).

As used herein "operably linked" includes reference to a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame.

As used herein, the term "plant" includes reference to whole plants, plant parts or organs (e.g., leaves, stems, roots, etc.), plant cells, seeds and progeny of same. Plant cell, as used herein includes, without limitation, cells obtained from or found in: seeds, suspension cultures, embryos, meristematic regions, callus tissue, leaves, roots, shoots, gametophytes, sporophytes, pollen, and microspores. Plant cells can also be understood to include modified cells, such as protoplasts, obtained from the aforementioned tissues. The class of plants which can be used in the methods of the invention is generally as broad as the class of higher plants amenable to transformation techniques, including both monocotyledonous and dicotyledonous plants. Particularly preferred plants include maize, soybean, sunflower, sorghum, canola, wheat, alfalfa, cotton, rice, barley and millet.

As used herein, "polynucleotide" includes reference to a deoxyribopolynucleotide, ribopolynucleotide, or analogs thereof that have the essential nature of a natural ribonucleotide in that they hybridize, under stringent hybridization conditions, to substantially the same nucleotide sequence as naturally occurring nucleotides and/or allow translation into the same amino acid(s) as the naturally occurring nucleotide(s). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including *among other things,* simple and complex cells.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulfation, gamma-carboxylation of glutamic acid residues, hydroxylation and ADP-ribosylation. Exemplary modifications are described in most basic texts, such as, Proteins - Structure and Molecular Properties, 2nd ed., T. E. Creighton, W. H. Freeman and Company, New York (1993). Many detailed reviews are available on this subject, such as, for example, those provided by Wold, F., Post-translational Protein Modifications: Perspectives and Prospects, pp. 1-12 in Posttranslational Covalent Modification of Proteins, B. C. Johnson, Ed., Academic Press, New York (1983); Seifter et al., Meth. Enzymol. 182: 626-646 (1990) and Rattan et al., Protein Synthesis: PosttranslationalModificationsand Aging, Ann. N. Y. Acad. Sci. 663: 48-62 (1992). It will be appreciated, as is well known and as noted above, that polypeptides are not always entirely linear. For instance, polypeptides may be branched as a result of ubiquitination, and they may be circular, with or without branching, generally as a result of posttranslation events, including natural processing event and events brought about by human manipulation which do not occur naturally, Circular, branched and branched circular polypeptides may be synthesized by non-translation natural process and by entirely synthetic methods, as well. Modifications can occur anywhere in a polypeptide, including the peptide backbone, the amino acid side-chains and the amino or carboxyl-termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in *E*. *coli* or other cells, prior to proteolytic processing, almost invariably will be N-formylmethionine. During post-translational modification of the peptide, a methionine residue at the NH₂-terminus may be deleted. Accordingly, this invention contemplates the use of both the methionine-containing and the methionine-less amino terminal variants of the protein of the invention. In general, as used herein, the term polypeptide encompasses all such modifications, particularly those that are present in polypeptides synthesized by expressing a polynucleotide in a host cell.

As used herein "promoter" includes reference to a region of DNA upstream from the start of transcription and involved in recognition and binding of RNA polymerase and other proteins to initiate transcription. A "plant promoter" is a promoter capable of initiating transcription in plant cells. Exemplary plant promoters include, but are not limited to, those that are obtained from plants, plant viruses, and bacteria which comprise genes expressed in plant cells such *Agrobacterium* or *Rhizobium.* Examples of promoters under developmental control include promoters that preferentially initiate transcription in certain tissues, such as leaves, roots, or seeds. Such promoters are referred to as "tissue preferred". Promoters which initiate transcription only in certain tissue are referred to as "tissue specific". A "cell type" specific promoter primarily drives expression in certain cell types in one or more organs, for example, vascular cells in roots or leaves. An "inducible" promoter is a promoter which is under environmental control. Examples of environmental conditions that may effect transcription by inducible promoters include anaerobic conditions or the presence of light. Tissue specific, tissue preferred, cell type specific, and inducible promoters constitute the class of "non-constitutive" promoters. A "constitutive" promoter is a promoter which is active under most environmental conditions.

The term "cellulose synthase polypeptide" is a polypeptide of the present invention and refers to one or more amino acid sequences, in glycosylated or non-glycosylated form. The term is also inclusive of fragments, variants, homologs, alleles or precursors (e.g., preproproteins or proproteins) thereof. A "cellulose synthase protein" is a protein of the present invention and comprises a cellulose synthase polypeptide.

As used herein "recombinant" includes reference to a cell or vector, that has been modified by the introduction of a heterologous nucleic acid or that the cell is derived from a cell so modified. Thus, for example, recombinant cells express genes that are not found in identical form within the native (non-recombinant) form of the cell or express native genes that are otherwise abnormally expressed, under-expressed or not expressed at all as a result of deliberate human intervention. The term " recombinant" as used herein does not encompass the alteration of the cell or vector by naturally occurring events (e.g., spontaneous mutation, natural transformation/transduction/transposition) such as those occurring without deliberate human intervention.

As used herein, a "recombinant expression cassette" is a nucleic acid construct, generated recombinantly or synthetically, with a series of specified nucleic acid elements which permit transcription of a particular nucleic acid in a host cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid to be transcribed, and a promoter.

The term "residue" or "amino acid residue" or "amino acid" are used interchangeably herein to refer to an amino acid that is incorporated into a protein, polypeptide, or peptide (collectively "protein"). The amino acid may be a naturally occurring amino acid and, unless otherwise limited, may encompass known analogs of natural amino acids that can function in a similar manner as naturally occurring amino acids.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, preferably 90% sequence identity, and most preferably 100% sequence identity (i.e., complementary) with each other.

The term "specifically reactive", includes reference to a binding reaction between an antibody and a protein having an epitope recognized by the antigen binding site of the antibody. This binding reaction is determinative of the presence of a protein having the recognized epitope amongst the presence of a heterogeneous population of proteins and other biologics. Thus, under designated immunoassay conditions, the specified antibodies bind to an analyte having the recognized epitope to a substantially greater degree (e.g., at least 2-fold over background) than to substantially all other analytes lacking the epitope which are present in the sample.

The terms "stringent conditions" or "stringent hybridization conditions" includes reference to conditions under which a probe will hybridize to its target sequence, to a detectably greater degree than other sequences (e.g., at least 2-fold over background). Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (*e.g.,* 10 to 50 nucleotides) and at least about 60°C for long probes (*e.g*., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

Specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 138:267-284 (1984): Tₘ = 81.5 °C + 16.6 (log M) + 0.41 (%.GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1 % of mismatching; thus, Tₘ hybridization and/or wash conditions can be adjusted to hybridize to sequences of the desired identity. For example, if sequences with ≥90% identity are sought, the Tₘ can be decreased 10 °C. Generally, stringent conditions are selected to be about 5 °C lower than the thermal melting point (Tₘ) for the specific sequence and its complement at a defined ionic strength and pH. However, severely stringent conditions can utilize a hybridization and/or wash at 1, 2, 3, or 4 °C lower than the thermal melting point (Tₘ); moderately stringent conditions can utilize a hybridization and/or wash at 6, 7, 8, 9, or 10 °C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology-Hybridization with Nucleic Acid Probes, Part 1, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995).

As used herein, "Transgenic plant" includes reference to a plant which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. "Transgenic" is used herein to include any cell, cell line, callus, tissue, plant part or plant, the genotype of which has been altered by the presence of heterologous nucleic acid including those transgenics initially so altered as well as those created by sexual crosses or asexual propagation from the initial transgenic. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

As used herein, "vector" includes reference to a nucleic acid used in transfection of a host cell and into which can be inserted a polynucleotide. Vectors are often replicons. Expression vectors permit transcription of a nucleic acid inserted therein.

The following terms are used to describe the sequence relationships between two or more nucleic acids or polynucleotides: (a) "reference sequence", (b) "comparison window" , (c) "sequence identity" , (d) "percentage of sequence identity" , and (e) "substantial identity".
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" means includes reference to a contiguous and specified segment of a polynucleotide sequence, wherein the polynucleotide sequence may be compared to a reference sequence and wherein the portion of the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well-known in the art. Optimal alignment of sequences for comparison may be conducted by the local homology algorithm of Smith and Waterman, Adv. Appl. Math. 2: 482 (1981); by the homology alignment algorithm of Needleman and Wunsch, J. Mol. Biol. 48: 443 (1970); by the search for similarity method of Pearson and Lipman, Proc. Natl. Acad. Sci. 85: 2444 (1988); by computerized implementations of these algorithms, including, but not limited to: CLUSTAL in the PC/Gene program by Intelligenetics, Mountain View, California, GAP, BESTFIT, BLAST, FASTA, and TFASTA in the Wisconsin Genetics Software Package, Genetics Computer Group (GCG), 575 Science Dr., Madison, Wisconsin, USA; the CLUSTAL program is well described by Higgins and Sharp, Gene 73: 237-244 (1988); Higgins and Sharp, CABIOS 5: 151-153 (1989); Corpet, et al., Nucleic Acids Research 16: 10881-90 (1988); Huang, et al., Computer Applications in the Biosciences 8: 155-65 (1992), and Pearson, et al., Methods in Molecular Biology 24: 307-331 (1994). The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, et al., Eds., Greene Publishing and Wiley-Interscience, New York (1995).

Unless otherwise stated, sequence identity/similarity values provided herein refer to the value obtained using the BLAST 2.0 suite of programs using default parameters. Altschul et al., Nucleic Acids Res. 25:3389-3402 (1997). Software for performing BLAST analyses is publicly available, e.g., through the National Center for Biotechnology Information (http://www.ncbi.ntm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra).* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

In addition to calculating percent sequence identity, the BLAST algorithm also performs a statistical analysis of the similarity between two sequences (*see, e.g.,* Karlin & Altschul, Proc. Nat'l. Acad Sci. USA 90:5873-5787 (1993)). One measure of similarity provided by the BLAST algorithm is the smallest sum probability (P(N)), which provides an indication of the probability by which a match between two nucleotide or amino acid sequences would occur by chance.

BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, Comput. Chem., 17:149-163 (1993)) and XNU (Claverie and States, Comput. Chem., 17:191-201 (1993)) low-complexity filters can be employed alone or in combination.
(c) As used herein, "sequence identity" or "identify" in the context of two nucleic acid or polypeptide sequences includes reference to the residues in the two sequences which are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are said to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g.,* according to the algorithm of Meyers and Miller, Computer Applic. Biol. Sci., 4: 11-17 (1988) *e.g.,* as implemented in the program PC/GENE (Intelligenetics, Mountain View, California, USA).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.
(e) (i) The term "substantial identity" of polynucleotide sequences means that a polynucleotide comprises a sequence that has at least 70% sequence identity, preferably at least 80%, more preferably at least 90% and most preferably at least 95%, compared to a reference sequence using one of the alignment programs described using standard parameters. One of skill will recognize that these values can be appropriately adjusted to determine corresponding identity of proteins encoded by two nucleotide sequences by taking into account codon degeneracy, amino acid similarity, reading frame positioning and the like. Substantial identity of amino acid sequences for these purposes normally means sequence identity of at least 60%, more preferably at least 70%, 80%, 90%, and most preferably at least 95%.

Another indication that nucleotide sequences are substantially identical is if two molecules hybridize to each other under stringent conditions. However, nucleic acids which do not hybridize to each other under stringent conditions are still substantially identical if the polypeptides which they encode are substantially identical. This may occur, *e.g.,* when a copy of a nucleic acid-is created using the maximum codon degeneracy permitted by the genetic code. One indication that two nucleic acid sequences are substantially identical is that the polypeptide which the first nucleic acid encodes is immunologically cross reactive with the polypeptide encoded by the second nucleic acid.
(e) (ii) The terms "substantial identity" in the context of a peptide indicates that a peptide comprises a sequence with at least 70% sequence identity to a reference sequence, preferably 80%, more preferably 85%, most preferably at least 90% or 95% sequence identity to the reference sequence over a specified comparison window. Preferably, optimal alignment is conducted using the homology alignment algorithm of Needleman and Wunsch, J. Mol. Biol. 48: 443 (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution. Peptides which are "substantially similar" share sequences as noted above except that residue positions which are not identical may differ by conservative amino acid changes.

### DETAILED DESCRIPTION OF THE INVENTION

### Overview

The present invention provides, *among other things,* compositions and methods for modulating (i.e., increasing or decreasing) the level of polypeptides of the present invention in plants. In particular, the polypeptides of the present invention can be expressed at developmental stages, in tissues, and/or in quantities which are uncharacteristic of non-recombinantly engineered plants. Thus, the present invention provides utility in such exemplary applications as improvement of stalk quality for improved stand or silage. Further, the present invention provides for an increased concentration of cellulose in the pericarp; hardening the kernel and thus improving its handling ability.

Isolated nucleic acids comprising polynucleotide of sufficient length and complementarity to a gene of the present invention may be used as probes or amplification primers in the detection, quantitation, or isolation of gene transcripts. For example, isolated nucleic acids of the present invention can be used as probes in detecting deficiencies in the level of mRNA in screenings for desired transgenic plants, for detecting mutations in the gene (e.g., substitutions, deletions, or additions), for monitoring upregulation of expression or changes in enzyme activity in screening assays of compounds, for detection of any number of allelic variants (polymorphisms) of the gene, or for use as molecular markers in plant breeding programs. The isolated nucleic acids of the present invention can also be used for recombinant expression of their encoded polypeptides, or for use as immunogens in the preparation and/or screening of antibodies. The isolated nucleic acids of the present invention can also be employed for use in sense or antisense suppression of one or more genes of the present invention in a host cell, tissue, or plant. Attachment of chemical agents which bind, intercalate, cleave and/or crosslink to the isolated nucleic acids of the present invention can also be used to modulate transcription or translation.

The present invention also provides isolated proteins comprising a polypeptide of the present invention (e.g., preproenzyme, proenzyme, or enzymes). The present invention also provides proteins comprising at least one epitope from a polypeptide of the present invention. The proteins of the present invention can be employed in assays for enzyme agonists or antagonists of enzyme function, or for use as immunogens or antigens to obtain antibodies specifically immunoreactive with a protein of the present invention. Such antibodies can be used in assays for expression levels, for identifying and/or isolating nucleic acids of the present invention from expression libraries, or for purification of polypeptides of the present invention.

The isolated nucleic acids and proteins of the present invention can be used over a broad range of plant types, particularly monocots such as the species of the Family *Graminiae* including *Sorghum bicolor* and *Zea mays.* The isolated nucleic acid and proteins of the present invention can also be used in species from the genera: *Cucurbita, Rosa, Viris, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Brawaalia, Glycine, Pisum, Phaseolus, Lolium, Oryza, Avena, Hordeum, Secale, Triticum, Bambusa, Dendrocalamus,* and *Melocanna.*

### Nucleic Acids

The present invention provides, *among other things,* isolated nucleic acids of RNA, DNA, and analogs and/or chimeras thereof, comprising a polynucleotide of the present invention.

A polynucleotide of the present invention is inclusive of:
(a) a polynucleotide encoding a polypeptide of SEQ ID NO 58, and conservatively modified and polymorphic variants thereof, including exemplary polynucleotides of SEQ ID NO:

### A. Polynucleotides Encoding A Polypeptide of the Present Invention or Conservatively Modified or Polymorphic Variants Thereof

As indicated in (a), above, the present invention provides isolated nucleic acids comprising a polynucleotide of the present invention, wherein the polynucleotide encodes a polypeptide of the present invention, or conservatively modified or polymorphic variants thereof. Those of skill in the art will recognize that the degeneracy of the genetic code allows for a plurality of polynucleotides to encode for the identical amino acid sequence. Such "silent variations" can be used, for example, to selectively hybridize and detect allelic variants of polynucleotides of the present invention. Accordingly, the present invention includes polynucleotides of SEQ ID NO: 57, and silent variations of polynucleotides encoding a polypeptide of SEQ ID NO 58. The present invention further provides isolated nucleic acids comprising polynucleotides encoding conservatively modified variants of a polypeptide of SEQ ID NO: 58. Additionally, the present invention further provides isolated nucleic acids comprising polynucleotides encoding one or more polymorphic (allelic) variants of polypeptides/polynucleotides. Polymorphic variants are frequently used to follow segregation of chromosomal regions in, for example, marker assisted selection methods for crop improvement.

### B. Polynucleotides Amplified from a Zea mays Nucleic Acid Library

Polynucleotides of the present invention, may be amplified from a *Zea mays* nucleic acid library as may additional sequences. *Zea mays* lines B73, PHRE1, A632, BMS-P2#10, W23, and Mol7 are known and publicly available. Other publicly known and available maize lines can be obtained from the Maize Genetics Cooperation (Urbana, IL). The nucleic acid library may be a cDNA library, a genomic library, or a library generally constructed from nuclear transcripts at any stage of intron processing cDNA libraries can be normalized to increase the representation of relatively rare cDNAs. In optional embodiments, the cDNA library is constructed using a full-length cDNA synthesis method. Examples of such methods include Oligo-Capping (Maruyama, K. and Sugano, S. Gene 138: 171-174, 1994), Biotinylated CAP Trapper (Carninci, P., Kvan, C., et al. Genomics 37: 327-336, 1996), and CAP Retention Procedure (Edery, E., Chu, L.L., et al. Molecular and Cellular Biology 15: 3363-3371, 1995). cDNA synthesis is often catalyzed at 50-55°C to prevent formation of RNA secondary structure. Examples of reverse transcriptases that are relatively stable at these temperatures are SuperScript II Reverse Transcriptase (Life Technologies, Inc.), AMV Reverse Transcriptase (Boehringer Mannheim) and RetroAmp Reverse Transcriptase (Epicentre). Rapidly growing tissues, or rapidly dividing cells are preferably used as mRNA sources such as from the elongating internode of corn plants. Polynucleotides may be amplified using the following primer pairs:
SEQ ID NOS: 3 and 4 which yield an amplicon comprising a sequence having substantial identity to SEQ ID NO: 1;
SEQ ID NOS: 7 and 8 which yield an amplicon comprising a sequence having substantial identity to SEQ ID NO: 5; and
SEQ ID NOS: 11 and 12 which yield an amplicon comprising a sequence having substantial identity to SEQ ID NO: 9.
SEQ ID NOS: 15 and 16 which yield an amplicon comprising a sequence having substantial identity to SEQ ID NO: 13.
SEQ ID NOS: 19 and 20 which yield an amplicon comprising a sequence having substantial identity to SEQ ID NO: 17;
SEQ ID NOS: 23 and 24 which yield an amplicon comprising a sequence having substantial identity to SEQ ID NO: 21; and
SEQ ID NOS: 27 and 28 which yield an amplicon comprising a sequence having substantial identity to SEQ ID NO: 25.
SEQ ID NOS: 31 and 32 which yield an amplicon comprising a sequence having substantial identity to SEQ ID NO: 29.
SEQ ID NOS: 35 and 36 which yield an amplicon comprising a sequence having substantial identity to SEQ ID NO: 33;
SEQ ID NOS: 39 and 40 which yield an amplicon comprising a sequence having substantial identity to SEQ ID NO: 37; and
SEQ ID NOS: 43 and 44 which yield an amplicon comprising a sequence having substantial identity to SEQ ID NO: 41.
SEQ ID NOS: 47 and 48 which yield an amplicon comprising a sequence having substantial identity to SEQ ID NO: 45.
SEQ ID NOS: 51 and 52 which yield an amplicon comprising a sequence having substantial identity to SEQ ID NO: 49;
SEQ ID NOS: 55 and 56 which yield an amplicon comprising a sequence having substantial identity to SEQ ID NO: 53; and
SEQ ID NOS: 59 and 60 which yield an amplicon comprising a sequence having substantial identity to SEQ ID NO: 57 of the invention.

The present invention also relates to subsequences of the polynucleotides of the present invention. A variety of subsequences can be obtained using primers which selectively hybridize under stringent conditions to at least two sites within a polynucleotide of the present invention, or to two sites within the nucleic acid which flank and comprise a polynucleotide of the present invention, or to a site within a polynucleotide of the present invention and a site within the nucleic acid which comprises it. Primers are chosen to selectively hybridize, under stringent hybridization conditions, to a polynucleotide of the present invention. Generally, the primers are complementary to a subsequence of the target nucleic acid which they amplify. As those skilled in the art will appreciate, the sites to which the primer pairs will selectively hybridize are chosen such that a single contiguous nucleic acid can be formed under the desired amplification conditions.

The primers may be constructed so that they selectively hybridize under stringent conditions to a sequence (or its complement) within the target nucleic acid which comprises the codon encoding the carboxy or amino terminal amino acid residue (i.e., the 3' terminal coding region and 5' terminal coding region, respectively) of the polynucleotides of the present invention. Optionally, the primers will be constructed to selectively hybridize entirely within the coding region of the target polynucleotide of the present invention such that the product of amplification of a cDNA target will consist of the coding region of that cDNA. The primer length in nucleotides is selected from the group of integers consisting of from at least 15 to 50. Thus, the primers can be at least 15,18, 20, 25, 30, 40, or 50 nucleotides in length. Those of skill will recognize that a lengthened primer sequence can be employed to increase specificity of binding (i.e., annealing) to a target sequence. A non-annealing sequence at the 5'end of a primer (a "tail") can be added, for example, to introduce a cloning site at the terminal ends of the amplicon.

The amplification products can be translated using expression systems well known to those of skill in the art and as discussed, *infra.* The resulting translation products can be confirmed as polypeptides of the present invention by, for example, assaying for the appropriate catalytic activity (e.g., specific activity and/or substrate specificity), or verifying the presence of one or more linear epitopes which are specific to a polypeptide of the present invention. Methods for protein synthesis from PCR derived templates are known in the art and available commercially. See, e.g., Amersham Life Sciences, Inc, Catalog '97, p.354.

Methods for obtaining 5' and/or 3' ends of a vector insert are well known in the art. See, e.g., RACE (Rapid Amplification of Complementary Ends) as described in Frohman, M. A., in PCR Protocols: A Guide to Methods and Applications, M. A. Innis, D. H. Gelfand, J. J. Sninsky, T. J. White, Eds. (Academic Press, Inc., San Diego, 1990), pp. 28-38.); see also, U.S. Pat. No. 5,470,722, and Current Protocols in Molecular Biology, Unit 15.6, Ausubel, et al., Eds., Greene Publishing and Wiley-Interscience, New York (1995); Frohman and Martin, Techniques 1:165 (1989).

### C. Polynucleotides Which Selectively Hybridize to a Polynucleotide of (A) or (B).

The present invention also relates to isolated nucleic acids comprising polynucleotides of the present invention, wherein the polynucleotides selectively hybridize, under selective hybridization conditions, to a polynucleotide of paragraphs (A) or (B) as discussed, above. Such polynucleotides can be used for isolating, detecting, and/or quantifying nucleic acids comprising the polynucleotides of the invention of (A) or (B). For example, such polynucleotides can be used to identify, isolate, or amplify partial or full-length clones in a deposited library. Such polynucleotides may be genomic or cDNA sequences isolated or otherwise complementary to a cDNA from a dicot or monocot nucleic acid library. Exemplary species of monocots and dicots include, but are not limited to: corn, canola, soybean, cotton, wheat, sorghum, sunflower, oats, sugar cane, millet, barley, and rice. Optionally, the cDNA library comprises at least 80% full-length sequences, preferably at least 85% or 90% full-length sequences, and more preferably at least 95% full-length sequences. The cDNA libraries can be normalized to increase the representation of rare sequences. Low stringency hybridization conditions are typically, but not exclusively, employed with sequences having a reduced sequence identity relative to complementary sequences. Moderate and high stringency conditions can optionally be employed for sequences of greater identity. Low stringency conditions allow selective hybridization of sequences having about 70% sequence identity and can be employed to identify orthologous or paralogous sequences.

### E. Polynucleolides Encoding a Protein which has a Subsequence from a Prototype Polypeptide and is Cross-Reactive to the Prototype Polypeptide

The present invention also relates to isolated nucleic acids comprising polynucleotides of the present invention, wherein the polynucleotides encode a protein having a subsequence of contiguous amino acids from a prototype polypeptide of the present invention such as are provided in (a), above. The length of contiguous amino acids from the prototype polypeptide is selected from the group of integers consisting of from at least 10 to the number of amino acids within the prototype sequence. Thus, for example, such a polynucleotide can encode a polypeptide having a subsequence having at least 10, 15, 20, 25, 30, 35, 40, 45, or 50, contiguous amino acids from the prototype polypeptide. Further, the number of such subsequences encoded by a polynucleotide of the instant embodiment can be any integer selected from the group consisting of from 1 to 20, such as 2, 3, 4, or 5. The subsequences can be separated by any integer of nucleotides from 1 to the number of nucleotides in the sequence such as at least 5, 10, 15, 25, 50, 100, or 200 nucleotides.

Such proteins when presented as an immunogen, elicit the production of polyclonal antibodies which specifically bind to a prototype polypeptide such as but not limited to, a polypeptide encoded by the polynucleotide of (a) or (b), above. Generally, however, a protein encoded by such a polynucleotide does not bind to antisera raised against the prototype polypeptide when the antisera has been fully immunosorbed with the prototype polypeptide. Methods of making and assaying for antibody binding specificity/affinity are well known in the art. Exemplary immunoassay formats include ELISA, competitive immunoassays, radioimmunoassays, Western blots, indirect immunofluorescent assays and the like.

In a preferred assay method, fully immunosorbed and pooled antisera which is elicited to the prototype polypeptide can be used in a competitive binding assay to test the protein. The concentration of the prototype polypeptide required to inhibit 50% of the binding of the antisera to the prototype polypeptide is determined. If the amount of the protein required to inhibit binding is less than twice the amount of the prototype protein, then the protein is said to specifically bind to the antisera elicited to the immunogen. Accordingly, the proteins of the present invention embrace allelic variants, conservatively modified variants, and minor recombinant modifications to a prototype polypeptide.

Such polynucleotide optionally encodes a protein having a molecular weight as the non-glycosylated protein within 20% of the molecular weight of the full-length non-glycosylated polypeptides of the present invention. Molecular weight can be readily determined by SDS-PAGE under reducing conditions. Preferably, the molecular weight is within 15 % of a full length polypeptide of the present invention, more preferably within 10% or 5%, and most preferably within 3 % , 2 %, or 1% of a full length polypeptide of the present invention. Molecular weight determination of a protein can be conveniently performed by SDS-PAGE under denaturing conditions.

Optionally, such polynucleotides will encode a protein having a specific activity at least 50%, 60%, 80%, or 90% of the native, endogenous (i.e., non-isolated), full-length polypeptide of the present invention. Further, the proteins encoded by such polynucleotides will optionally have a substantially similar affinity constant (Kₘ ) and/or catalytic activity (i.e., the microscopic rate constant, k_{cat}) as the native endogenous, full-length protein. Those of skill in the art will recognize that k_{car}/Kₘ value determines the specificity for competing substrates and is often referred to as the specificity constant. Such proteins can have a k_{car}/Kₘ value at least 10% of a non-isolated full-length polypeptide of the present invention as determined using the endogenous substrate of that polypeptide. Optionally, the k_{car}/Kₘ value will be at least 20%,30%,40%,50%, and most preferably at least 60%, 70%, 80%, 90%, or 95% the k_{car}/Kₘ value of the non-isolated, full-length polypeptide of the present invention. Determination of k_{car}, Kₘ, and k_{car}/Kₘ can be determined by any number of means well known to those of skill in the art. For example, the initial rates (i.e., the first 5% or less of the reaction) can be determined using rapid mixing and sampling techniques (e.g., continuous-flow, stopped-flow, or rapid quenching techniques), flash photolysis, or relaxation methods (e.g., temperature jumps) in conjunction with such exemplary methods of measuring as spectrophotometry, spectrofluorimetry, nuclear magnetic resonance, or radioactive procedures. Kinetic values are conveniently obtained using a Lineweaver-Burk or Eadie-Hofstee plot.

### F. Polynucleotides Complementary to the Polynucleotides of (A)-(E)

The present invention relates to isolated nucleic acids comprising polynucleotides complementary to the polynucleotides of paragraphs A-E. above. As those of skill in the art will recognize, complementary sequences base-pair throughout the entirety of their length with the polynucleotides of (A)-(E) (i.e., have 100% sequence identity over their entire length). Complementary bases associate through hydrogen bonding in double stranded nucleic acids. For example, the following base pairs are complementary: guanine and cytosine; adenine and thymine; and adenine and uracil.

### G. polynucleotides Which are Subsequences of the Polynucleotides of (A)-(F)

The present invention also relates to isolated nucleic acids comprising polynucleotides which comprise at least 15 contiguous bases from the polynucleotides of (A) through (F) as discussed above. The length of the polynucleotide is given as an integer selected from the group consisting of from at least 15 to the length of the nucleic acid sequence from which the polynucleotide is a subsequence of. Thus, for example, such polynucleotides may comprise at least 15, 20, 25, 30, 40, 50, 60, 75, or 100 contiguous nucleotides in length from the polynucleotides of (A)-(F). Optionally, the number of such subsequences can be any integer selected from the group consisting of from 1 to 20, such as 2, 3, 4, or 5. The subsequences can be separated by any integer of nucleotides from 1 to the number of nucleotides in the sequence such as at least 5, 10, 15, 25, 50, 100, or 200 nucleotides.

Such sequences can comprise structural characteristics of the sequence from which it is derived. Alternatively, the subsequences can lack certain structural characteristics of the larger sequence from which it is derived. For example, a subsequence from a polynucleotide encoding a polypeptide having at least one linear epitope in common with a prototype polypeptide sequence as provided in (a), above, may encode an epitope in common with the prototype sequence. Alternatively, the subsequence may not encode an epitope in common with the prototype sequence but can be used to isolate the larger sequence by, for example, nucleic acid hybridization with the sequence from which it's derived. Subsequences can be used to modulate or detect gene expression by introducing into the subsequences compounds which bind, intercalate, cleave and/or crosslink to nucleic acids. Exemplary compounds include acridine, psoralen, phenanthroline, naphthoquinone, daunomycin or chloroethylantinoaryl conjugates.

### Construction of Nucleic Acids

The isolated nucleic acids of the present invention can be made using (a) standard recombinant methods, (b) synthetic techniques, or combinations thereof. In some embodiments, the polynucleotides of the present invention will be cloned, amplified, or otherwise constructed from a monocot. In preferred embodiments the monocot is *Zea mays.*

The nucleic acids may conveniently comprise sequences in addition to a polynucleotide of the present invention. For example, a multi-cloning site comprising one or more endonuclease restriction sites may be inserted into the nucleic acid to aid in isolation of the polynucleotide. Also, translatable sequences may be inserted to aid in the isolation of the translated polynucleotide of the present invention. For example, a hexa-histidine marker sequence provides a convenient means to purify the proteins of the present invention. A polynucleotide of the present invention can be attached to a vector, adapter, or linker for cloning and/or expression of a polynucleotide of the present invention. Additional sequences may be added to such cloning and/or expression sequences to optimize their function in cloning and/or expression, to aid in isolation of the polynucleotide, or to improve the introduction of the polynucleotide into a cell. Typically, the length of a nucleic acid of the present invention less the length of its polynucleotide of the present invention is less than 20 kilobase pairs, often less than 15 kb, and frequently less than 10 kb. Use of cloning vectors, expression vectors, adapters, -and-linkers is well known and extensivley described in the art. For a description of various nucleic acids see, for example, Stratagene Cloning Systems, Catalogs 1995, 1996, 1997 (La Jolla, CA); and, Amersham Life Sciences, Inc, Catalog '97 (Arlington Heights, IL).

### A. Recombinant Methods for Constructing Nucleic Acids

The isolated nucleic acid compositions of this invention, such as RNA, cDNA, genomic DNA, or a hybrid thereof, can be obtained from plant biological sources using any number of cloning methodologies known to those of skill in the art. In some embodiments, oligonucleotide probes which selectively hybridize, under stringent conditions, to the polynucleotides of the present invention are used to identify the desired sequence in a cDNA or genomic DNA library. While isolation of RNA, and construction of cDNA and genomic libraries is well known to those of ordinary skill in the art, the following highlights some of the methods employed.

### A1. mRNA Isolation and Purification

Total RNA from plant cells comprises such nucleic acids as mitochondrial RNA, chloroplastic RNA, rRNA, tRNA, hnRNA and mRNA. Total RNA preparation typically involves lysis of cells and removal of proteins, followed by precipitation of nucleic acids. Extraction of total RNA from plant cells can be accomplished by a variety of means. Frequently, extraction buffers include a strong detergent such as SDS and an organic denaturant such as guanidinium isothiocyanate, guanidine hydrochloride or phenol. Following total RNA isolation, poly(A)⁺ mRNA is typically purified from the remainder RNA using oligo(dT) cellulose. Exemplary total RNA and mRNA isolation protocols are described in Plant Molecular Biology: A Laboratory Manual, Clark, Ed., Springer-Verlag, Berlin (1997); and, Current Protocols in Molecular Biology, Ausubel, et al., Eds., Greene Publishing and Wiley-Interscience, New York (1995). Total RNA and mRNA isolation kits are commercially available from vendors such as Stratagene (La Jolla, CA), Clonetech (Palo Alto, CA), Pharmacia (Piscataway, NJ), and 5'-3' (Paoli, PA). See also, U.S. Patent Nos. 5,614,391; and, 5,459,253. The mRNA can be fractionated into populations with size ranges of about 0.5, 1.0, 1.5, 2.0, 2.5 or 3.0 kb. The cDNA synthesized for each of these fractions can be size selected to the same size range as its mRNA prior to vector insertion. This method helps eliminate truncated cDNA formed by incompletely-reverse transcribed mRNA.

### A2. Construction of a cDNA Library

Construction of a cDNA library generally entails five steps. First, first strand cDNA synthesis is initiated from a poly(A)⁺ mRNA template using a poly(dT) primer or random hexanucleotides. Second, the resultant RNA-DNA hybrid is converted into double stranded cDNA, typically by a combination of RNAse H and DNA polymerase I (or Klenow fragment). Third, the termini of the double stranded cDNA are ligated to adaptors. Ligation of the adaptors will produce cohesive ends for cloning. Fourth, size selection of the double stranded cDNA eliminates excess adaptors and primer fragments, and eliminates partial cDNA molecules due to degradation of mRNAs or the failure of reverse transcriptase to synthesize complete first strands. Fifth, the cDNAs are ligated into cloning vectors and packaged. cDNA synthesis protocols are well known to the skilled artisan and are described in such standard references as: Plant Molecular Biology: A Laboratory Manual, Clark, Ed., Springer-Verlag, Berlin (1997); and, Current Protocols in Molecular Biology, Ausubel, et al., Eds., Greene Publishing and Wiley-Interscience, New York (1995). cDNA synthesis kits are available from a variety of commercial vendors such as Stratagene or Pharmacia.

A number of cDNA synthesis protocols have been described which provide substantially pure full-length cDNA libraries. Substantially pure full-length cDNA libraries are constructed to comprise at least 90%, and more preferably at least 93% or 95% full-length inserts amongst clones containing inserts. The length of insert in such libraries can be from 0 to 8, 9, 10, 11, 12, 13, or more kilobase pairs. Vectors to accommodate inserts of these sizes are known in the art and available commercially. See, e.g., Stratagene's lambda ZAP Express (cDNA cloning vector with 0 to 12 kb cloning capacity).

An exemplary method of constructing a greater than 95% pure full-length cDNA library is described by Carninci et al., Genomics, 37:327-336 (1996). In that protocol, the cap-structure of eukaryotic mRNA is chemically labeled with biotin. By using streptavidin-coated magnetic beads, only the full-length first-strand cDNA/mRNA hybrids are selectively recovered after RNase I treatment. The method provides a high yield library with an unbiased representation of the starting mRNA population. Other methods for producing full-length libraries are known in the art. See, e.g., Edery et al., Mol. Cell Biol. 15(6):3363-3371 (1995); and, PCT Application WO 96/34981.

### A3. Normalized or Subtracted cDNA Libraries

A non-normalized cDNA library represents the mRNA population of the tissue it was made from. Since unique clones are out-numbered by clones derived from highly expressed genes their isolation can be laborious. Normalization of a cDNA library is the process of creating a library in which each clone is more equally represented.

A number of approaches to normalize cDNA libraries are known in the art. One approach is based on hybridization to genomic DNA. The frequency of each hybridized cDNA in the resulting normalized library would be proportional to that of each corresponding gene in the genomic DNA. Another approach is based on kinetics. If cDNA reannealing follows second-order kinetics, rarer species anneal less rapidly and the remaining single-stranded fraction of cDNA becomes progressively more normalized during the course of the hybridization. Specific loss of any species of cDNA, regardless of its abundance, does not occur at any Cot value. Construction of normalized libraries is described in Ko, Nucl. Acids. Res., 18(19):5705-5711 (1990); Patanjali et al., Proc. Natl. Acad. U.S.A., 88:1943-1947 (1991); U.S. Patents 5,482,685, and 5,637,685. In an exemplary method described by Soares et al., normalization resulted in reduction of the abundance of clones from a range of four orders of magnitude to a narrow range of only 1 order of magnitude. Proc. Natl. Acad. Sci. USA, 91:9228-9232 (1994).

Subtracted cDNA libraries are another means to increase the proportion of less abundant cDNA species. In this procedure, cDNA prepared from one pool of mRNA is depleted of sequences present in a second pool of mRNA by hybridization. The cDNA:mRNA hybrids are removed and the remaining un-hybridized cDNA pool is enriched for sequences unique to that pool. See, Foote et al. in, Plant Molecular Biology: A Laboratory Manual, Clark, Ed., Springer-Verlag, Berlin (1997); Kho and Zarbl, Technique, 3(2):58-63 (1991); Sive and St. John, Nucl. Acids Res., 16(22):10937 (1988); Current Protocols in Molecular Biology, Ausubel, et al., Eds., Greene Publishing and Wiley-Interscience, New York (1995); and, Swaroop et al., Nucl. Acids Res., 19)8):1954 (1991). cDNA subtraction kits are commercially available. See, e.g., PCR-Select (Clontech).

### A4. Construction of a Genomic Library

To construct genomic libraries, large segments of genomic DNA are generated by random fragmentation, e.g. using restriction endonucleases, and are ligated with vector DNA to form concatemers that can be packaged into the appropriate vector. Methodologies to accomplish these ends, and sequencing methods to verify the sequence of nucleic acids are well known in the art. Examples of appropriate molecular biological techniques and instructions sufficient to direct persons of skill through many construction, cloning, and screening methodologies are found in Sambrook, et al., Molecular Cloning: A Laboratory Manual, 2nd Ed., Cold Spring Harbor Laboratory Vols. 1-3 (1989), Methods in Enzymology, Vol. 152: Guide to Molecular Cloning Techniques, Berger and Kimmel, Eds., San Diego: Academic Press, Inc. (1987), Current Protocols in Molecular Biology, Ausubel, et al., Eds., Greene Publishing and Wiley-Interscience, New York (1995); Plant Molecular Biology: A Laboratory Manual, Clark, Ed., Springer-Verlag, Berlin (1997). Kits for construction of genomic libraries are also commercially available.

### A5. Nucleic Acid Screening and Isolation Methods

The cDNA or genomic library can be screened using a probe based upon the sequence of a polynucleotide of the present invention such as those disclosed herein. Probes may be used to hybridize with genomic DNA or cDNA sequences to isolate homologous genes in the same or different plant species. Those of skill in the an will appreciate that various degrees of stringency of hybridization can be employed in the assay; and either the hybridization or the wash medium can be stringent. As the conditions for hybridization become more stringent, there must be a greater degree of complementarity between the probe and the target for duplex formation to occur. The degree of stringency can be controlled by temperature, ionic strength, pH and the presence of a partially denaturing solvent such as formamide. For example, the stringency of hybridization is conveniently varied by changing the polarity of the reactant solution through manipulation of the concentration of formamide within the range of 0% to 50%. The degree of complementarity (sequence identity) required for detectable binding will vary in accordance with the stringency of the hybridization medium and/or wash medium. The degree of complementarity will optimally be 100 percent; however, it should be understood that minor sequence variations in the probes and primers may be compensated for by reducing-the stringency-of the hybridization and/or wash medium.

The nucleic acids of interest can also be amplified from nucleic acid samples using amplification techniques. For instance, polymerase chain reaction (PCR) technology can be used to amplify the sequences of polynucleotides of the present invention and related genes directly from genomic DNA or cDNA libraries. PCR and other *in vitro* amplification methods may also be useful, for example, to clone nucleic acid sequences that code for proteins to be expressed, to make nucleic acids to use as probes for detecting the presence of the desired mRNA in samples, for nucleic acid sequencing, or for other purposes. Examples of techniques sufficient to direct persons of skill through *in vitro* amplification methods are found in Berger, Sambrook, and Ausubel, as well as Mullis et al., U.S. Patent No. 4,683,202 (1987); and, PCR Protocols A Guide to Methods and Applications, Innis et al., Eds., Academic Press Inc., San Diego, CA (1990). Commercially available kits for genomic PCR amplification are known in the art. See, e.g., Advantage-GC Genomic PCR Kit (Clontech). The T4 gene 32 protein (Boehringer Mannheim) can be used to improve yield of long PCR products.

PCR-based screening methods have also been described. Wilfinger et al. describe a PCR-based method in which the longest cDNA is identified in the first step so that incomplete clones can be eliminated from study. BioTechniques, 22(3): 481-486 (1997). In that method, a primer pair is synthesized with one primer annealing to the 5' end of the sense strand of the desired cDNA and the other primer to the vector. Clones are pooled to allow large-scale screening. By this procedure, the longest possible clone is identified amongst candidate clones. Further, the PCR product is used solely as a diagnostic for the presence of the desired cDNA and does not utilize the PCR product itself. Such methods are particularly effective in combination with a full-length cDNA construction methodology, above.

### B. Synthetic Methods for Constructing Nucleic Acids

The isolated nucleic acids of the present invention can also be prepared by direct chemical synthesis by methods such as the phosphotriester method of Narang et al., Meth. Enzymol. 68: 90-99 (1979); the phosphodiester method of Brown et al., Meth. Enzymol. 68: 109-151 (1979); the diethylphosphoramidite method of Beaucage et al., Tetra. Lett. 22: 1859-1862 (1981); the solid phase phosphoramidite triester method described by Beaucage and Caruthers. Tetra. Letts. 22(20): 1859-1862 (1981), *e.g.,* using an automated synthesizer, e.g., as described in Needham-VanDevanter et al., Nucleic Acids Res., 12: 6159-6168 (1984); and, the solid support method of U.S. Patent No. 4,458,066. Chemical synthesis generally produces a single stranded oligonucleotide. This may be converted into double stranded DNA by hybridization with a complementary sequence, or by polymerization with a DNA polymerase using the single strand as a template. One of skill will recognize that while chemical synthesis of DNA is limited to sequences of about 100 bases, longer sequences may be obtained by the ligation of shorter sequences.

### Recombinant Expression Cassettes

The present invention further provides recombinant expression cassettes comprising a nucleic acid of the present invention. A nucleic acid sequence coding for the desired polynucleotide of the present invention, for example a cDNA or a genomic sequence encoding a full length polypeptide of the present invention, can be used to construct a recombinant expression cassette which can be introduced into the desired host cell. A recombinant expression cassette will typically comprise a polynucleotide of the present invention operably linked to transcriptional initiation regulatory sequences which will direct the transcription of the polynucleotide in the intended host cell, such as tissues of a transformed plant.

For example, plant expression vectors may include (1) a cloned plant gene under the transcriptional control of 5' and 3' regulatory sequences and (2) a dominant selectable marker. Such plant expression vectors may also contain, if desired, a promoter regulatory region (e.g., one conferring inducible or constitutive, environmentally- or developmentally-regulated, or cell- or tissue-specific/selective expression), a transcription initiation start site, a ribosome binding site, an RNA processing signal, a transcription termination site, and/or a polyadenylation signal.

A plant promoter fragment can be employed which will direct expression of a polynucleotide of the present invention in all tissues of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'- promoter derived from T-DNA of *Agrobacterium tumefaciens,* the ubiquitin 1 promoter, the Smas promoter, the cinnamyl alcohol dehydrogenase promoter (U.S. Patent No. 5,683,439), the *Nos* promoter, the pEmu promoter, the rubisco promoter, the GRP1-8 promoter, the actin promoter, the F3.7 promoter, and other transcription initiation regions from various plant genes known to those of skill.

Alternatively, the plant promoter can direct expression of a polynucleotide of the present invention in a specific tissue or may be otherwise under more precise environmental or developmental control. Such promoters are referred to here as "inducible" promoters. Environmental conditions that may effect transcription by inducible promoters include pathogen attack, anaerobic conditions, or the presence of light. Examples of inducible promoters are the Adh1 promoter which is inducible by hypoxia or cold stress, the Hsp70 promoter which is inducible by heat stress, and the PPDK promoter which is inducible by light.

Examples of promoters under developmental control include promoters that initiate transcription only, or preferentially, in certain tissues, such as leaves, roots, fruit, seeds, or flowers. The operation of a promoter may also vary depending on its location in the genome. Thus, an inducible promoter may become fully or partially constitutive in certain locations.

Both heterologous and non-heterologous (i.e., endogenous) promoters can be employed to direct expression of the nucleic acids of the present invention. These promoters can also be used, for example, in recombinant expression cassettes to drive expression of antisense nucleic acids to reduce, increase, or alter concentration and/or composition of the proteins of the present invention in a desired tissue. Thus, in some embodiments, the nucleic acid construct will comprise a promoter functional in a plant cell, such as in *Zea mays,* operably linked to a polynucleotide of the present invention. Promoters useful in these embodiments include the endogenous promoters driving expression of a polypeptide of the present invention.

In some embodiments, isolated nucleic acids which serve as promoter or enhancer elements can be introduced in the appropriate position (generally upstream) of a non-heterologous form of a polynucleotide of the present invention so as to up or down regulate expression of a polynucleotide of the present invention. For example, endogenous promoters can be altered *in vivo* by mutation, deletion, and/or substitution (see, Kmiec, U.S. Patent 5,565,350; Zarling et al., PCT/US93/03868), or isolated promoters can be introduced into a plant cell in the proper orientation and distance from a gene of the present invention so as to control the expression of the gene. Gene expression can be modulated under conditions suitable for plant growth so as to alter the total concentration and/or alter the composition of the polypeptides of the present invention in plant cell. Thus, the present invention provides compositions, and methods for making, heterologous promoters and/or enhancers operably linked to a native, endogenous (i.e., non-heterologous) form of a polynucleotide of the present invention.

Methods for identifying promoters with a particular expression pattern, in terms of, e.g., tissue type, cell type, stage of development, and/or environmental conditions, are well known in the art. See, e.g., The Maize Handbook, Chapters 114-115, Freeling and Walbot, Eds., Springer, New York (1994); Corn and Corn Improvement, 3rd edition, Chapter 6, Sprague and Dudley, Eds., American Society of Agronomy, Madison, Wisconsin (1988). A typical step in promoter isolation methods is identification of gene products that are expressed with some degree of specificity in the target tissue. Amongst the range of methodologies are: differential hybridization to cDNA libraries; subtractive hybridization; differential display; differential 2-D protein gel electrophoresis; DNA probe arrays; and isolation of proteins known to be expressed with some specificity in the target tissue. Such methods are well known to those of skill in the art. Commercially available products for identifying promoters are known in the art such as Clontech's (Palo Alto, CA) Universal GenomeWalker Kit.

For the protein-based methods, it is helpful to obtain the amino acid sequence for at least a portion of the identified protein, and then to use the protein sequence as the basis for preparing a nucleic acid that can be used as a probe to identify either genomic DNA directly, or preferably, to identify a cDNA clone from a library prepared from the target tissue. Once such a cDNA clone has been identified, that sequence can be used to identify the sequence at the 5' end of the transcript of the indicated gene. For differential hybridization, subtractive hybridization and differential display, the nucleic acid sequence identified as enriched in the target tissue is used to identify the sequence at the 5' end of the transcript of the indicated gene. Once such sequences are identified, starting either from protein sequences or nucleic acid sequences, any of these sequences identified as being from the gene transcript can be used to screen a genomic library prepared from the target organism. Methods for identifying and confirming the transcriptional start site are well known in the art.

In the process of isolating promoters expressed under particular environmental conditions or stresses, or in specific tissues, or at particular developmental stages, a number of genes are identified that are expressed under the desired circumstances, in the desired tissue, or at the desired stage. Further analysis will reveal expression of each particular gene in one or more other tissues of the plant. One can identify a promoter with activity in the desired tissue or condition but that do not have activity in any other common tissue.

To identify the promoter sequence, the 5' portions of the clones described here are analyzed for sequences characteristic of promoter sequences. For instance, promoter sequence elements include the TATA box consensus sequence (TATAAT), which is usually an AT-rich stretch of 5-10 bp located approximately 20 to 40 base pairs upstream of the transcription start site. Identification of the TATA box is well known in the art. For example, one way to predict the location of this element is to identify the transcription start site using standard RNA-mapping techniques such as primer extension, S1 analysis, and/or RNase protection. To confirm the presence of the AT-rich sequence, a structure-function analysis can be performed involving mutagenesis of the putative region and quantification of the mutation's effect on expression of a linked downstream reporter gene. See, e.g., The Maize Handbook, Chapter 114, Freeling and Walbot, Eds., Springer, New York, (1994).

In plants, further upstream from the TATA box, at positions -80 to -100, there is typically a promoter element (i.e., the CAAT box) with a series of adenines surrounding the trinucleotide G (or T) N G. J. Messing et al., in Genetic Engineering in Plants, Kosage, Meredith and Hollaender, Eds., pp. 221-227 1983. In maize, there is no well conserved CAAT box but there are several short, conserved protein-binding motifs upstream of the TATA box. These include motifs for the trans-acting transcription factors involved in light regulation, anaerobic induction, hormonal regulation, or anthocyanin biosynthesis, as appropriate for each gene.

Once promoter and/or gene sequences are known, a region of suitable size is selected from the genomic DNA that is 5' to the transcriptional start, or the translational start site, and such sequences are then linked to a coding sequence. If the transcriptional start site is used as the point of fusion, any of a number of possible 5' untranslated regions can be used in between the transcriptional start site and the partial coding sequence. If the translational start site at the 3' end of the specific promoter is used, then it is linked-directly to the methionine start codon of a coding sequence.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added can be derived from, for example, the nopaline synthase or octopine synthase genes, or alternatively from another plant gene, or less preferably from any other eukaryotic gene.

An intron sequence can be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold. Buchman and Berg, Mol. Cell Biol. 8: 4395-4405 (1988); Callis et al., Genes Dev. 1: 1183-1200 (1987). Such intron enhancement of gene expression is typically greatest when placed near the 5' end of the transcription unit. Use of maize introns Adh1-S intron 1, 2, and 6, the Bronze-1 intron are known in the art. See generally, The Maize Handbook, Chapter 116, Freeling and Walbot, Eds., Springer, New York (1994).

The vector comprising the sequences from a polynucleotide of the present invention will typically comprise a marker gene which confers a selectable phenotype on plant cells. Usually, the selectable marker gene will encode antibiotic resistance, with suitable genes including genes coding for resistance to the antibiotic spectinomycin (e.g., the aada gene), the streptomycin phosphotransferase (SPT) gene coding for streptomycin resistance, the neomycin phosphotransferase (NPTII) gene encoding kanamycin or geneticin resistance, the hygromycin phosphotransferase (HPT) gene coding for hygromycin resistance, genes coding for resistance to herbicides which act to inhibit the action of acetolactate synthase (ALS), in particular the sulfonylurea-type herbicides (e.g., the acetolactate synthase (ALS) gene containing mutations leading to such resistance in particular the S4 and/or Hra mutations), genes coding for resistance to herbicides which act to inhibit action of glutamine synthase, such as phosphinothricin or basta (e.g., the *bar* gene), or other such genes known in the an. The *bar* gene encodes resistance to the herbicide basta, the *nptII* gene encodes resistance to the antibiotics kanamycin and geneticin, and the ALS gene encodes resistance to the herbicide chlorsulfuron.

Typical vectors useful for expression of genes in higher plants are well known in the art and include vectors derived from the tumor-inducing (Ti) plasmid of *Agrobacterium tumefaciens* described by Rogers et al., Meth. In Enzymol., 153:253-277 (1987). These vectors are plant integrating vectors in that on transformation, the vectors integrate a portion of vector DNA into the genome of the host plant. Exemplary A. *tumefaciens* vectors useful herein are plasmids pKYLX6 and pKYLX7 of Schardl et al., Gene, 61:1-11 (1987) and Berger et al., Proc. Natl. Acad. Sci. U.S.A., 86:8402-8406 (1989). Another useful vector herein is plasmid pBI101.2 that is available from Clontech Laboratories, Inc. (Palo Alto, CA).

A polynucleotide of the present invention can be expressed in either sense or anti-sense orientation as desired. It will be appreciated that control of gene expression in either sense or anti-sense orientation can have a direct impact on the observable plant characteristics. Antisense technology can be conveniently used to gene expression in plants. To accomplish this, a nucleic acid segment from the desired gene is cloned and operably linked to a promoter such that the anti-sense strand of RNA will be transcribed. The construct is then transformed into plants and the antisense strand of RNA is produced. In plant cells, it has been shown that antisense RNA inhibits gene expression by preventing the accumulation of mRNA which encodes the enzyme of interest, see, e.g., Sheehy et al., Proc. Nat'l. Acad. Sci. (USA) 85: 8805-8809 (1988); and Hiatt et al., U.S. Patent No. 4,801,340.

Another method of suppression is sense suppression. Introduction of nucleic acid configured in the sense orientation has been shown to be an effective means by which to block the transcription of target genes. For an example of the use of this method to modulate expression of endogenous genes see, Napoli et al., The Plant Cell 2: 279-289 (1990) and U.S. Patent No. 5,034,323.

Catalytic RNA molecules or ribozymes can also be used to inhibit expression of plant genes. It is possible to design ribozymes that specifically pair with virtually any target RNA and cleave the phosphodiester backbone at a specific location, thereby functionally inactivating the target RNA. In carrying out this cleavage, the ribozyme is not itself altered, and is thus capable of recycling and cleaving other molecules, making it a true enzyme. The inclusion of ribozyme sequences within antisense RNAs confers RNA-cleaving activity upon them, thereby increasing the activity of the constructs. The design and use of target RNA-specific ribozymes is described in Haseloff et al., Nature 334: 585-591 (1988).

A variety of cross-linking agents, alkylating agents and radical generating species as pendant groups on polynucleotides of the present invention can be used to bind, label, detect, and/or cleave nucleic acids. For example, Vlassov, V. V., et al., Nucleic Acids Res (1986) 14:4065-4076, describe covalent bonding of a single-stranded DNA fragment with alkylating derivatives of nucleotides complementary to target sequences. A report of similar work by the same group is that by Knorre, D. G., et al., Biochimie (1985) 67:785-789. Iverson and Dervan also showed sequence-specific cleavage of single-stranded DNA mediated by incorporation of a modified nucleotide which was capable of activating cleavage *(*J Am Chem Soc (1987) 109:1241-1243). Meyer, R. B., et al., J Am Chem Soc (1989) 111:8517-8519, effect covalent crosslinking to a target nucleotide using an alkylating agent complementary to the single-stranded target nucleotide sequence. A photoactivated crosslinking to single-stranded oligonucleotides mediated by psoralen was disclosed by Lee, B. L., et al., Biochemistry (1988) 27:3197-3203. Use of crosslinking in triple-helix forming probes was also disclosed by Home, et al., J Am Chem Soc (1990) 112:2435-2437. Use of N4, N4-ethanocytosine as an alkylating agent to crosslink to single-stranded oligonucleotides has also been described by Webb and Matteucci, J Am Chem Soc (1986) 108:2764-2765; Nucleic Acids Res (1986) 14:7661-7674; Feteritz et al., J. Am. Chem. Soc. 113:4000 (1991). Various compounds to bind, detect, label, and/or cleave nucleic acids are known in the art. See, for example, U.S. Patent Nos. 5,543,507; 5.672,593; 5,484,908; 5,256,648; and, 5,681941.

The invention provides on isolated protein having canular synthase activity and selected from: Proteins (a) a polypeptide of SEQ ID NO:58; and (b) a polypeptide encoded by a nucleic acid of the invention.

As those of skill will appreciate, the present invention includes catalytically active polypeptides of the present invention (i.e., enzymes). Catalytically active polypeptides have a specific activity of at least 20%, 30%, or 40%, and preferably at least 50%, 60%, or 70%, and most preferably at least 80%, 90%, or 95% that of the native (non-synthetic), endogenous polypeptide. Further, the substrate specificity (k_{car}/Kₘ) is optionally substantially similar to the native (non-synthetic), endogenous polypeptide. Typically, the Kₘ will be at least 30%, 40%, or 50%, that of the native (non-synthetic), endogenous polypeptide; and more preferably at least 60%, 70%, 80%, or 90%. Methods of assaying and quantifying measures of enzymatic activity and substrate specificity (k_{car}/Kₘ), are well known to those of skill in the art.

Generally, the proteins of the present invention will, when presented as an immunogen, elicit production of an antibody specifically reactive to a polypeptide of the present invention. Further, the proteins of the present invention will not bind to antisera raised against a polypeptide of the present invention which has been fully immunosorbed with the same polypeptide. Immunoassays for determining binding are well known to those of skill in the art. A preferred immunoassay is a competitive immunoassay as discussed, *infra.* Thus, the proteins of the present invention can be employed as immunogens for constructing antibodies immunoreactive to a protein of the present invention for such exemplary utilities as immunoassays or protein purification techniques.

### Expression of Proteins in Host Cells

Using the nucleic acids of the present invention, one may express a protein of the present invention in a recombinantly engineered cell such as bacteria, yeast, insect, mammalian, or preferably plant cells. The cells produce the protein in a non-natural condition (e.g., in quantity, composition, location, and/or time), because they have been genetically altered through human intervention to do so.

It is expected that those of skill in the art are knowledgeable in the numerous expression systems available for expression of a nucleic acid encoding a protein of the present invention. No attempt to describe in detail the various methods known for the expression of proteins in prokaryotes or eukaryotes will be made.

In brief summary, the-expression of isolated nucleic acids encoding a protein of the present invention will typically be achieved by operably linking, for example, the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences, and promoters useful for regulation of the expression of the DNA encoding a protein of the present invention. To obtain high level expression of a cloned gene, it is desirable to construct expression vectors which contain, at the minimum, a strong promoter to direct transcription, a ribosome binding site for translational initiation, and a transcription/translation terminator. One of skill would recognize that modifications can be made to a protein of the present invention without diminishing its biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids (*e.g.*, poly His) placed on either terminus to create conveniently located purification sequences. Restriction sites or termination codons can also be introduced.

### A. Expression in Prokaryotes

Prokaryotic cells may be used as hosts for expression. Prokaryotes most frequently are represented by various strains of *E. coli;* however, other microbial strains may also be used. Commonly used prokaryotic control sequences which are defined herein to include promoters for transcription initiation, optionally with an operator, along with ribosome binding site sequences, include such commonly used promoters as the beta lactamase (penicillinase) and lactose (lac) promoter systems (Chang et al., Nature 198:1056 (1977)), the tryptophan (trp) promoter system (Goeddel et al., Nucleic Acids Res. 8:4057 (1980)) and the lambda derived P L promoter and N-gene ribosome binding site (Shimatake et al., Nature 292:128 (1981)). The inclusion of selection markers in DNA vectors transfected in *E. coli* is also useful. Examples of such markers include genes specifying resistance to ampicillin, tetracycline, or chloramphenicol.

The vector is selected to allow introduction into the appropriate host cell. Bacterial vectors are typically of plasmid or phage origin. Appropriate bacterial cells are infected with phage vector particles or transfected with naked phage vector DNA. If a plasmid vector is used, the bacterial cells are transfected with the plasmid vector DNA. Expression systems for expressing a protein of the present invention are available using *Bacillus sp.* and *Salmonella* (Palva, et al., Gene 22: 229-235 (1983); Mosbach, et al., Nature 302: 543-545 (1983)).

### B. Expression in Eukaryotes

A variety of eukaryotic expression systems such as yeast, insect cell lines, plant and mammalian cells, are known to those of skill in the art. As explained briefly below, a of the present invention can be expressed in these eukaryotic systems. In some embodiments, transformed/transfected plant cells, as discussed *infra,* are employed as expression systems for production of the proteins of the instant invention.

Synthesis of heterologous proteins in yeast is well known. Sherman, F., et al., Methods in Yeast Genetics, Cold Spring Harbor Laboratory (1982) is a well recognized work describing the various methods available to produce the protein in yeast. Two widely utilized yeast for production of eukaryotic proteins are *Saccharomyces cerevisiae* and *Pichia pastoris.* Vectors, strains, and protocols for expression in *Saccharomyces* and *Pichia* are known in the art and available from commercial suppliers (e.g., Invitrogen). Suitable vectors usually have expression control sequences, such as promoters, including 3-phosphoglycerate kinase or alcohol oxidase, and an origin of replication, termination sequences and the like as desired.

A protein of the present invention, once expressed, can be isolated from yeast by lysing the cells and applying standard protein isolation techniques to the lysates. The monitoring of the purification process can be accomplished by using Western blot techniques or radioimmunoassay of other standard immunoassay techniques.

The sequences encoding proteins of the present invention can also be ligated to various expression vectors for use in transfecting cell cultures of, for instance, mammalian, insect, or plant origin. Illustrative of cell cultures useful for the production of the peptides are mammalian cells. Mammalian cell systems often will be in the form of monolayers of cells although mammalian cell suspensions may also be used. A number of suitable host cell lines capable of expressing intact proteins have been developed in the art, and include the HEK293, BHK21, and CHO cell lines. Expression vectors for these cells can include expression control sequences, such as an origin of replication, a promoter (*e.g.*, the CMV promoter, a HSV *tk* promoter or *pgk* (phosphoglycerate kinase) promoter), an enhancer (Queen et al., Immunol Rev. 89: 49 (1986)), and necessary processing information sites, such as ribosome binding sites, RNA splice sites, polyadenylation sites (*e.g.*, an SV40 large T Ag poly A addition site), and transcriptional terminator sequences. Other animal cells useful for production of proteins of the present invention are available, for instance, from the American Type Culture Collection Catalogue of Cell Lines and Hybridomas (7th edition, 1992).

Appropriate vectors for expressing proteins of the present invention in insect cells are usually derived from the SF9 baculovirus. Suitable insect cell lines include mosquito larvae, silkworm, armyworm, moth and *Drosophila* cell lines such as a Schneider cell line (See Schneider, J. Embryol. Exp. Morphol. 27: 353-365 (1987).

As with yeast, when higher animal or plant host cells are employed, polyadenlyation or transcription terminator sequences are typically incorporated into the vector. An example of a terminator sequence is the polyadenlyation sequence from the bovine growth hormone gene. Sequences for accurate splicing of the transcript may also be included. An example of a splicing sequence is the VPI intron from SV40 (Sprague, et al., J. Virol. 45: 773-781 (1983)). Additionally, gene sequences to control replication in the host cell may be incorporated into the vector such as those found in bovine papilloma virus type-vectors. Saveria-Campo, M., Bovine Papilloma Virus DNA a Eukaryotic Cloning Vector in DNA Cloning Vol. II a Practical Approach, D.M. Glover, Ed., IRL Press, Arlington, Virginia pp. 213-238 (1985),

### Transfection/Transformation of Cells

The method of transformation/transfection is not critical to the instant invention; various methods of transformation or transfection are currently available. As newer methods are available to transform crops or other host cells they may be directly applied. Accordingly, a wide variety of methods have been developed to insert a DNA sequence into the genome of a host cell to obtain the transcription and/or translation of the sequence to effect phenotypic changes in the organism. Thus, any method which provides for efficient transformation/transfection may be employed.

### A. Plant Transformation

A DNA sequence coding for the desired polynucleotide of the present invention, for example a cDNA or a genomic sequence encoding a full length protein, will be used to construct a recombinant expression cassette which can be introduced into the desired plant.

Isolated nucleic acid acids of the present invention can be introduced into plants according techniques known in the art. Generally, recombinant expression cassettes as described above and suitable for transformation of plant cells are prepared. Techniques for transforming a wide variety of higher plant species are well known and described in the technical, scientific, and patent literature. See, for example, Weising et al., Ann. Rev. Genet. 22: 421-477 (1988). For example, the DNA construct may be introduced directly into the genomic DNA of the plant cell using techniques such as electroporation, PEG poration, particle bombardment, silicon fiber delivery, or microinjection of plant cell protoplasts or embryogenic callus. See e.g., Tomes, et al., Direct DNA Transfer into Intact Plant Cells via Microprojectile Bombardment, pp. 197-213 in Plant Cell, Tissue and Organ Culture, Fundamental Methods, (eds. O.L. Gamborg and G.C. Phillips, Springer-Verlag Berlin Heidelberg New York, 1995). Alternatively, the DNA constructs may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. See, U.S. Patent No. 5,591,616.

The introduction of DNA constructs using polyethylene glycol precipitation is described in Paszkowski et al.. Embo J. 3: 2717-2722 (1984). Electroporation techniques are described in Fromm et al., Proc. Natl. Acad. Sci. 82: 5824 (1985). Ballistic transformation techniques are described in Klein et al., Nature 327: 70-73 (1987).

*Agrobacterium* tumefaciens-meditated transformation techniques are well described in the scientific literature. See, for example Horsch et al., Science 233: 496-498 (1984), and Fraley et al., Proc. Natl. Acad. Sci. 80: 4803 (1983). Although *Agrobacterium* is useful primarily in dicots, certain monocots can be transformed by *Agrobacterium.* For instance, *Agrobacterium* transformation of maize is described in U.S. Patent No. 5,550,318.

Other methods of transfection or transformation include (1) *Agrobacterium rhizogenes*-mediated transformation (see, e.g., Lichtenstein and Fuller In: Genetic Engineering, vol. 6, PWJ Rigby, Ed., London, Academic Press, 1987; and Lichtenstein, C. P., and Draper, J,. In: DNA Cloning, Vol. II, D. M. Glover, Ed., Oxford, IRI Press, 1985),Application PCT/US87/02512 (WO 88/02405 published Apr. 7, 1988) describes the use of *A.rhizogenes* strain A4 and its Ri plasmid along with *A. tumefaciens* vectors pARC8 or pARC16 (2) liposome-mediated DNA uptake (see, e.g., Freeman et al., Plant Cell Physiol. 25: 1353, 1984), (3) the vortexing method (see, e.g., Kindle, Proc. Natl. Acad. Sci., USA 87: 1228, (1990).

DNA can also be introduced into plants by direct DNA transfer into pollen as described by Zhou et al., Methods in Enzymology, 101:433 (1983); D. Hess, Intern Rev. Cytol., 107:367 (1987); Luo et al., Plane Mol. Biol. Reporter, 6:165 (1988). Expression of polypeptide coding genes can be obtained by injection of the DNA into reproductive organs of a plant as described by Pena et al., Nature, 325.:274 (1987). DNA can also be injected directly into the cells of immature embryos and the rehydration of desiccated embryos as described by Neuhaus et al. , Theor. Appl. Genet., 75:30 (1987); and Benbrook et al., in Proceedings Bio Expo 1986, Butterworth, Stoneham, Mass., pp. 27-54 (1986). A variety of plant viruses that can be employed as vectors are known in the art and include cauliflower mosaic virus (CaMV), geminivirus, brome mosaic virus, and tobacco mosaic virus.

### B. Transfection of Prokaryotes, Lower Eukaryotes, and Animal Cells

Animal and lower eukaryotic (e.g., yeast) host cells are competent or rendered competent for transfection by various means. There are several well-known methods of introducing DNA into animal cells. These include: calcium phosphate precipitation, fusion of the recipient cells with bacterial protoplasts containing the DNA, treatment of the recipient cells with liposomes containing the DNA, DEAE dextran, electroporation, biolistics, and micro-injection of the DNA directly into the cells. The transfected cells are cultured by means well known in the art. Kuchler, R.J., Biochemical Methods in Cell Culture and Virology, Dowden, Hutchinson and Ross, Inc. (1977).

### Synthesis of Proteins

The proteins of the present invention can be constructed using non-cellular synthetic methods. Solid phase synthesis of proteins of less than about 50 amino acids in length may be accomplished by attaching the C-terminal amino acid of the sequence to an insoluble support followed by sequential addition of the remaining amino acids in the sequence. Techniques for solid phase synthesis are described by Barany and Merrifield, Solid-Phase Peptide Synthesis, pp. 3-284 in The Peptides: Analysis, Synthesis, Biology. Vol. 2: Special Methods in Peptide Synthesis, Part A.*;* Merrifield, et al., J. Am. Chem. Soc. 85: 2149-2156 (1963), and Stewart et al.. Solid Phase Peptide Synthesis, 2nd ed., Pierce Chem. Co., Rockford, III. (1984). Proteins of greater length may be synthesized by condensation of the amino and carboxy termini of shorter fragments. Methods of forming peptide bonds by activation of a carboxy terminal end (e.g., by the use of the coupling reagent N,N'-dicycylohexylcarbodiimide)) is known to those of skill.

### Purification of Proteins

The proteins of the present invention may be purified by standard techniques well known to those of skill in the art. Recombinantly produced proteins of the present invention can be directly expressed or expressed as a fusion protein. The recombinant protein is purified by a combination of cell lysis (e.g., sonication, French press) and affinity chromatography. For fusion products, subsequent digestion of the fusion protein with an appropriate proteolytic enzyme releases the desired recombinant protein.

The proteins of this invention, recombinant or synthetic, may be purified to substantial purity by standard techniques well known in the art, including detergent solubilization, selective precipitation with such substances as ammonium sulfate, column chromatography, immunopurification methods, and others. *See,* for instance, R. Scopes, Protein Purification: Principles and Practice, Springer-Verlag: New York (1982); Deutscher, Guide to Protein Purification, Academic Press (1990). For example, antibodies may be raised to the proteins as described herein. Purification from *E. coli* can be achieved following procedures described in U.S. Patent No. 4,511,503. The protein may then be isolated from cells expressing the protein and further purified by standard protein chemistry techniques as described herein. Detection of the expressed protein is achieved by methods known in the art and include, for example, radioimmunoassays, Western blotting techniques or immunoprecipitation.

### Transgenic Plant Regeneration

Transformed plant cells which are derived by any of the above transformation techniques can be cultured to regenerate a whole plant which possesses the transformed genotype. Such regeneration-techniques often rely on manipulation of certain phytohormones in a tissue culture growth medium, typically relying on a biocide and/or herbicide marker which has been introduced together with a polynucleotide of the present invention. For transformation and regeneration of maize see, Gordon-Kamm et al., The Plant Cell, 2:603-618 (1990).

Plants cells transformed with a plant expression vector can be regenerated, e.g., from single cells, callus tissue or leaf discs according to standard plant tissue culture techniques. It is well known in the art that various cells, tissues, and organs from almost any plant can be successfully cultured to regenerate an entire plant. Plant regeneration from cultured protoplasts is described in Evans et al. , Protoplasts Isolation and Culture, Handbook of Plant Cell Culture, Macmillilan Publishing Company, New York, pp. 124-176 (1983); and Binding, Regeneration of Plants, Plant Protoplasts, CRC Press, Boca Raton, pp. 21-73 (1985).

The regeneration of plants containing the foreign gene introduced by *Agrobacterium* from leaf explants can be achieved as described by Horsch et al., Science, 227:1229-1231 (1985). In this procedure, transformants are grown in the presence of a selection agent and in a medium that induces the regeneration of shoots in the plant species being transformed as described by Fraley et al., Proc. Natl. Acad. Sci. U.S.A., 80:4803 (1983). This procedure typically produces shoots within two to four weeks and these transformant shoots are then transferred to an appropriate root-inducing medium containing the selective agent and an antibiotic to prevent bacterial growth. Transgenic plants of the present invention may be fertile or sterile.

Regeneration can also be obtained from plant callus, explants, organs, or parts thereof. Such regeneration techniques are described generally in Klee et al., Ann. Rev. of Plant Phys. 38: 467-486 (1987). The regeneration of plants from either single plant protoplasts or various explants is well known in the art. See, for example, Methods for Plant Molecular Biology, A. Weissbach and H. Weissbach, eds., Academic Press, Inc., San Diego, Calif. (1988). This regeneration and growth process includes the steps of selection of transformant cells and shoots, rooting the transformant shoots and growth of the plantlets in soil. For maize cell culture and regeneration see generally, The Maize Handbook, Freeling and Walbot, Eds., Springer, New York (1994); Corn and Corn Improvement, 3rd edition, Sprague and Dudley Eds., American Society of Agronomy, Madison, Wisconsin (1988).

One of skill--will recognize that after the recombinant expression cassette is stably incorporated in transgenic plants and confirmed to be operable, it can be introduced into other plants by sexual crossing. Any of a number of standard breeding techniques can be used, depending upon the species to be crossed.

In vegetatively propagated crops, mature transgenic plants can be propagated by the taking of cuttings or by tissue culture techniques to produce multiple identical plants. Selection of desirable transgenics is made and new varieties are obtained and propagated vegetatively for commercial use. In seed propagated crops, mature transgenic plants can be self crossed to produce a homozygous inbred plant. The inbred plant produces seed containing the newly introduced heterologous nucleic acid. These seeds can be grown to produce plants that would produce the selected phenotype.

Parts obtained from the regenerated plant, such as flowers, seeds, leaves, branches, fruit, and the like are included in the invention, provided that these parts comprise cells comprising the isolated nucleic acid of the present invention. Progeny and variants, and mutants of the regenerated plants are also included within the scope of the invention, provided that these parts comprise the introduced nucleic acid sequences.

Transgenic plants expressing the selectable marker can be screened for transmission of the nucleic acid of the present invention by, for example, standard immunoblot and DNA detection techniques. Transgenic lines are also typically evaluated on levels of expression of the heterologous nucleic acid. Expression at the RNA level can be determined initially to identify and quantitate expression-positive plants. Standard techniques for RNA analysis can be employed and include PCR amplification assays using oligonucleotide primers designed to amplify only the heterologous RNA templates and solution hybridization assays using heterologous nucleic acid-specific probes. The RNA-positive plants can then analyzed for protein expression by Western immunoblot analysis using the specifically reactive antibodies of the present invention. In addition, *in situ* hybridization and immunocytochemistry according to standard protocols can be done using heterologous nucleic acid specific polynucleotide probes and antibodies, respectively, to localize sites of expression within transgenic tissue. Generally, a number of transgenic lines are usually screened for the incorporated nucleic acid to identify and select plants with the most appropriate expression profiles.

A preferred embodiment is a transgenic plant that is homozygous for the added heterologous nucleic acid; i.e., a transgenic plant that contains two added nucleic acid sequences, one gene at the same locus on each chromosome of a chromosome pair. A homozygous transgenic plant can be obtained by sexually mating (selfing) a heterozygous transgenic plant that contains a single added heterologous nucleic acid, germinating some of the seed produced and analyzing the resulting plants produced for altered expression of a polynucleotide of the present invention relative to a control plant (i.e., native, non-transgenic). Back-crossing to a parental plant and out-crossing with a non- transgenic plant are also contemplated.

### Modulating Polypeptide Levels and/or Composition

The present invention further provides a method for modulating (i.e., increasing or decreasing) the concentration or composition of the polypeptides of the present invention in a plant or part thereof. Modulation can be effected by increasing or decreasing the concentration and/or the composition (i.e., the ratio of the polypeptides of the present invention) in a plant. The method comprises transforming a plant cell with a recombinant expression cassette comprising a polynucleotide of the present invention as described above to obtain a transformed plant cell, growing the transformed plant cell under plant forming conditions, and inducing expression of a polynucleotide of the present invention in the plant for a time sufficient to modulate concentration and/or composition in the plant or plant part.

In some embodiments, the content and/or composition of polypeptides of the present invention in a plant may be modulated by altering, *in vivo* or in *vitro,* the promoter of a non-isolated gene of the present invention to up- or down-regulate gene expression. In some embodiments, the coding regions.of native genes of the present invention can be altered via substitution, addition, insertion, or deletion to decrease activity of the encoded enzyme. See, e.g., Kmiec, U.S. Patent 5,565,350; Zarling et al., PCT/US93/03868. And in some embodiments, an isolated nucleic acid (e.g., a vector) comprising a promoter sequence is transfected into a plant cell. Subsequently, a plant cell comprising the promoter operably linked to a polynucleotide of the present invention is selected for by means known to those of skill in the art such as, but not limited to, Southern blot, DNA sequencing, or PCR analysis using primers specific to the promoter and to the gene and detecting amplicons produced therefrom. A plant or plant part altered or modified by the foregoing embodiments is grown under plant forming conditions for a time sufficient to modulate the concentration and/or composition of polypeptides of the present invention in the plant. Plant forming conditions are well known in the art and discussed briefly, *above.*

In general, concentration or composition is increased or decreased by at least 5%, 10%, 20%, 30%, 40%, 50%, 60%, 70%, 80%, or 90% relative to a native control plant, plant part, or cell lacking the aforementioned recombinant expression cassette. Modulation in the present invention may occur during and/or subsequent to growth of the plant to the desired stage of development. Modulating nucleic acid expression temporally and/or in particular tissues can be controlled by employing the appropriate promoter operably linked to a polynucleotide of the present invention in, for example, sense or antisense orientation as discussed in greater detail, above. Induction of expression of a polynucleotide of the present invention can also be controlled by exogenous administration of an effective amount of inducing compound. Inducible promoters and inducing compounds which activate expression from these promoters are well known in the art. In preferred embodiments, the polypeptides of the present invention are modulated in monocots, particularly maize.

### Molecular Markers

The present invention provides a method of genotyping a plant comprising a polynucleotide of the present invention. Preferably, the plant is a monocot, such as maize or sorghum. Genotyping provides a means of distinguishing homologs of a chromosome pair and can be used to differentiate segregants in a plant population. Molecular marker methods can be used for phylogenetic studies, characterizing genetic relationships among crop varieties, identifying crosses or somatic hybrids, localizing chromosomal segments affecting monogenic traits, map based cloning, and the study of quantitative inheritance. See, e.g., Plant Molecular Biology: A Laboratory Manual, Chapter 7, Clark, Ed., Springer-Verlag, Berlin (1997). For molecular marker methods, see generally, The DNA Revolution by Andrew H. Paterson 1996 (Chapter 2) in: Genome Mapping in Plants (ed. Andrew H. Paterson) by Academic Press/R. G. Landis Company, Austin, Texas, pp.7-21.

The particular method of genotyping in the present invention may employ any number of molecular marker analytic techniques such as, but not limited to, restriction fragment length polymorphisms (RFLPs) RFLPs are the product of allelic differences between DNA restriction fragments caused by nucleotide sequence variability. As is well known to those of skill in the art, RFLPs are typically detected by extraction of genomic DNA and digestion with a restriction enzyme. Generally, the resulting fragments are separated according to size and hybridized with a probe; single copy probes are preferred. Restriction fragments from homologous chromosomes are revealed. Differences in fragment size among alleles represent an RFLP. Thus, the present invention further provides a means to follow segregation of a gene or nucleic acid of the present invention as well as chromosomal sequences genetically linked to these genes or nucleic acids using such techniques as RFLP analysis. Linked chromosomal sequences are within 50 centiMorgans (cM), often within 40 or 30 cM, preferably within 20 or 10 cM, more preferably within 5, 3, 2, or 1 cM of a gene of the present invention.

In the present invention, the nucleic acid probes employed for molecular marker mapping of plant nuclear genomes selectively hybridize, under selective hybridization conditions, to a gene encoding a polynucleotide of the present invention. In preferred embodiments, the probes are selected from polynucleotides of the present invention. Typically, these probes are cDNA probes or *Pst 1* genomic clones. The length of the probes is discussed in greater detail, above, but are typically at least 15 bases in length, more preferably at least 20, 25, 30, 35, 40, or 50 bases in length. Generally, however, the probes are less than about I kilobase in length. Preferably, the probes are single copy probes that hybridize to a unique locus in a haploid chromosome complement. Some exemplary restriction enzymes employed in RFLP mapping are *EcoRI, EcoRv,* and SstI. As used herein the term "restriction enzyme" includes reference to a composition that recognizes and, alone or in conjunction with another composition, cleaves at a specific nucleotide sequence.

The method of detecting an RFLP comprises the steps of (a) digesting genomic DNA of a plant with a restriction enzyme; (b) hybridizing a nucleic acid probe, under selective hybridization conditions, to a sequence of a polynucleotide of the present of said genomic DNA; (c) detecting therefrom a RFLP. Other methods of differentiating polymorphic (allelic) variants of polynucleotides of the present invention can be had by utilizing molecular marker techniques well known to those of skill in the art including such techniques as: 1) single stranded conformation analysis (SSCP); 2) denaturing gradient gel electrophoresis (DGGE); 3) RNase protection assays; 4) allele-specific oligonucleotides (ASOs); 5) the use of proteins which recognize nucleotide mismatches, such as the *E. coli* mutS protein; and 6) allele-specific PCR. Other approaches based on the detection of mismatches between the two complementary DNA strands include clamped denaturing gel electrophoresis (CDGE); heteroduplex analysis (HA); and chemical mismatch cleavage (CMC). Exemplary polymorphic variants are provided in Table I, above. Thus, the present invention further provides a method of genotyping comprising the steps of contacting, under stringent hybridization conditions, a sample suspected of comprising a polynucleotide of the present invention with a nucleic acid probe. Generally, the sample is a plant sample; preferably, a sample suspected of comprising a maize polynucleotide of the present invention (e.g., gene, mRNA). The nucleic acid probe selectively hybridizes, under stringent conditions, to a subsequence of a polynucleotide of the present invention comprising a polymorphic marker. Selective hybridization of the nucleic acid probe to the polymorphic marker nucleic acid sequence yields a hybridization complex. Detection of the hybridization complex indicates the presence of that polymorphic marker in the sample. In preferred embodiments, the nucleic acid probe comprises a polynucleotide of the present invention.

### UTR's and Codon Preference

In general, translational efficiency has been found to be regulated by specific sequence elements in the 5' non-coding or untranslated region (5' UTR) of the RNA. Positive sequence motifs include translational initiation consensus sequences (Kozak, Nucleic Acids Res.15:8125 (1987)) and the 7-methylguanosine cap structure (Drummond et al., Nucleic Acids Res. 13:7375 (1985)). Negative elements include stable intramolecular 5' UTR stem-loop structures (Muesing et al., Cell 48:691 (1987)) and AUG sequences or short open reading frames preceded by an appropriate AUG in the 5' UTR (Kozak, above, Rao et al., Mol, and Cell. Biol. 8:284 (1988)). Accordingly, the present invention provides 5' and/or 3' UTR regions for modulation of translation of heterologous coding sequences.

Further, the polypeptide-encoding segments of the polynucleotides of the present invention can be modified to alter codon usage. Altered codon usage can be employed to alter translational efficiency and/or to optimize the coding sequence for expression in a desired host or to optimize the codon usage in a heterologous sequence for expression in maize. Codon usage in the coding regions of the polynucleotides of the present invention can be analyzed statistically using commercially available software packages such as "Codon Preference" available from the University of Wisconsin Genetics Computer Group (see Devereaux et al., Nucleic Acids Res. 12: 387-395 (1984)) or Mac Vector 4.1 (Eastman Kodak Co., New Haven, Conn.). Thus, the present invention provides a codon usage frequency characteristic of the coding region of at least one of the polynucleotides of the present invention. The number of polynucleotides that can be used to determine a codon usage frequency can be any integer from 1 to the number of polynucleotides of the present invention as provided herein. Optionally, the polynucleotides will be full-length sequences. An exemplary number of sequences for statistical analysis can be at least 1, 5, 10, 20, 50, or 100.

### Sequence Shuffling

The present invention relates to methods for sequence shuffling using polynucleotides of the present invention, and compositions resulting therefrom. Sequence shuffling is described in PCT publication No. 96/19256. See also, Zhang, J.-H., et al. Proc. Natl. Acad. Sci. USA 94:4504-4509 (1997). Generally, sequence shuffling provides a means for generating libraries of polynucleotides having a desired characteristic which can be selected or screened for. Libraries of recombinant polynucleotides are generated from a population of related sequence polynucleotide which comprise sequence regions which have substantial sequence identity and can be homologously recombined in vitro or in vivo. The population of sequence-recombined polynucleotides comprises a subpopulation of polynucleotides which possess desired or advantageous characteristics and which can be selected by a suitable selection or screening method. The characteristics can be any property or attribute capable of being selected for or detected in a screening system, and may include properties of: an encoded protein, a transcriptional element, a sequence controlling transcription, RNA processing, RNA stability, chromatin conformation, translation, or other expression property of a gene or transgene, a replicative element, a protein-binding element, or the like, such as any feature which confers a selectable or detectable property. The selected characteristic may be a decreased Kₘ and/or increased Kₑₓₜ over the wild-type protein as provided herein. In other embodiments, a protein or polynculeotide generated from sequence shuffling will have a ligand binding affinity greater than the non-shuffled wild-type polynucleotide. The increase in such properties can be at least 110%, 120%, 130%, 140% or at least 150% of the wild-type value.

### Generic and Consensus Sequences

The present invention also relates to polynuclotides and polypeptides having: (a) a generic sequence of at least two homologous polynucleotides or polypeptides, respectively, of the present invention; and, (b) a consensus sequence of at least three homologous polynucleotides or polypeptides, respectively, of the present invention. A polynucleotide having an amino acid or nucleic acid consensus sequence can be used to generate antibodies or produce nucleic acid probes or primers to screen for homologs in other species, genera, families, orders, classes, phyla, or kingdoms. For example, a polynucleotide having a consensus sequences from a gene family of *Zea mays* can be used to generate antibody or nucleic acid probes or primers to other *Gramineae* species such as wheat, rice, or sorghum. Alternatively, a polynucleotide having a consensus sequence generated from orthologous genes can be used to identify or isolate orthologs of other taxa. Typically, a polynucleotide having a consensus sequence will be at least 9, 10, 15, 20, 25, 30, or 40 amino acids in length, or 20, 30, 40, 50, 100, or 150 nucleotides in length. As those of skill in the art are aware, a conservative amino acid substitution can be used for amino acids which differ amongst aligned sequence but are from the same conservative substitution group as discussed above. Optionally, no more than 1 or 2 conservative amino acids are substituted for each 10 amino acid length of consensus sequence.

Similar sequences used for generation of a consensus or generic sequence include any number and combination of allelic variants of the same gene, orthologous, or paralogous sequences as provided herein. Optionally, similar sequences used in generating a consensus or generic sequence are identified using the BLAST algorithm's smallest sum probability (P(N)). Various suppliers of sequence-analysis software are listed in chapter 7 of Current Protocols in Molecular Biology, F.M. Ausubel et al., Eds., Current Protocols, a joint venture between Greene Publishing Associates, Inc. and John Wiley & Sons, Inc. (Supplement 30). A polynucleotide sequence is considered similar to a reference sequence if the smallest sum probability in a comparison of the test nucleic acid to the reference nucleic acid is less than about 0.1, more preferably less than about 0.01, or 0.001, and most preferably less than about 0.0001, or 0.00001. Similar polynucleotides can be aligned and a consensus or generic sequence generated using multiple sequence alignment software available from a number of commercial suppliers such as the Genetics Computer Group's (Madison, WI) PILEUP software, Vector NTI's (North Bethesda, MD) ALIGNX, or Genecode's (Ann Arbor, MI) SEQUENCHER. Conveniently, default parameters of such software can be used to generate consensus or generic sequences.

### Detection of Nucleic Acids

The present invention further relates to methods for detecting a polynucleotide of the present invention in a nucleic acid sample suspected of comprising a polynucleotide of the present invention, such as a plant cell lysate, particularly a lysate of corn. In some embodiments, a gene of the present invention or portion thereof can be amplified prior to the step of contacting the nucleic acid sample with a polynucleotide of the present invention. The nucleic acid sample is contacted with the polynucleotide to form a hybridization complex. The polynucleotide hybridizes under stringent conditions to a gene encoding a polypeptide of the present invention. Formation of the hybridization complex is used to detect a gene encoding a polypeptide of the present invention in the nucleic acid sample. Those of skill will appreciate that an isolated nucleic acid comprising a polynucleotide of the present invention should lack cross-hybridizing sequences in common with non-target genes that would yield a false positive result.

Detection of the hybridization complex can be achieved using any number of well known methods. For example, the nucleic acid sample, or a portion thereof, may be assayed by hybridization formats including but not limited to, solution phase, solid phase, mixed phase, or *in situ* hybridization assays. Briefly, in solution (or liquid) phase hybridizations, both the target nucleic acid and the probe or primer are free to interact in the reaction mixture. In solid phase hybridization assays, probes or primers are typically linked to a solid support where they are available for hybridization with target nucleic in solution. In mixed phase, nucleic acid intermediates in solution hybridize to target nucleic acids in solution as well as to a nucleic acid linked to a solid support. In *in situ* hybridization, the target nucleic acid is liberated from its cellular surroundings in such as to be available for hybridization within the cell while preserving the cellular morphology for subsequent interpretation and analysis. The following articles provide an overview of the various hybridization assay formats: Singer et al., Biotechniques 4(3): 230-250 (1986); Haase et al., Methods in Virology, Vol. VII, pp. 189-226 (1984); Wilkinson, The theory and practice of in situ hybridization in: In situ Hybridization, D.G. Wilkinson, Ed., IRL Press, Oxford University Press, Oxford; and Nucleic Acid Hybridization: A Practical Approach, Hames, B.D. and Higgins, S.J., Eds., IRL Press (1987).

### Nucleic Acid Labels and Detection Methods

The means by which nucleic acids of the present invention are labeled is not a critical aspect of the present invention and can be accomplished by any number of methods currently known or later developed. Detectable labels suitable for use in the present invention include any composition detectable by spectroscopic, radioisotopic, photochemical, biochemical, immunochemical, electrical, optical or chemical means. Useful labels in the present invention include biotin for staining with labeled streptavidin conjugate, magnetic beads, fluorescent dyes (*e.g.*, fluorescein, texas red, rhodamine, green fluorescent protein, and the like), radiolabels *(e.g.*, ³H, ¹²⁵I, ³⁵S, ¹⁴C, or ³²P), enzymes (*e.g.*, horse radish peroxidase, alkaline phosphatase and others commonly used in an ELISA), and colorimetric labels such as colloidal gold or colored glass or plastic (*e.g.*, polystyrene, polypropylene, latex, etc.) beads.

Nucleic acids of the present invention can be labeled by any one of several methods typically used to detect the presence of hybridized nucleic acids. One common method of detection is the use of autoradiography using probes labeled with ³H, ¹²⁵I, ³⁵S, ¹⁴C, or ³²P, or the like. The choice of radio-active isotope depends on research preferences due to ease of synthesis, stability, and half lives of the selected isotopes. Other labels include ligands which bind to antibodies labeled with fluorophores, chemiluminescent agents, and enzymes. Alternatively, probes can be conjugated directly with labels such as fluorophores, chemiluminescent agents or enzymes. The choice of label depends on sensitivity required, ease of conjugation with the probe, stability requirements, and available instrumentation. Labeling the nucleic acids of the present invention is readily achieved such as by the use of labeled PCR primers.

In some embodiments, the label is simultaneously incorporated during the amplification step in the preparation of the nucleic acids. Thus, for example, polymerase chain reaction (PCR) with labeled primers or labeled nucleotides will provide a labeled amplification product. In another embodiment, transcription amplification using a labeled nucleotide (*e.g.*, fluorescein-labeled UTP and/or CTP) incorporates a label into the transcribed nucleic acids.

Non-radioactive probes are often labeled by indirect means. For example, a ligand molecule is covalently bound to the probe. The ligand then binds to an anti-ligand molecule which is either inherently detectable or covalently bound to a detectable signal system, such as an enzyme, a fluorophore, or a chemiluminescent compound. Enzymes of interest as labels will primarily be hydrolases, such as phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescers include luciferin, and 2,3-dihydrophthalazinediones, *e.g.*, luminol. Ligands and anti-ligands may be varied widely. Where a ligand has a natural anti-ligand, namely ligands such as biotin, thyroxine, and cortisol, it can be used in conjunction with its labeled, naturally occurring anti-ligands. Alternatively, any haptenic or antigenic compound can be used in combination with an antibody.

Probes can also be labeled by direct conjugation with a label. For example, cloned DNA probes have been coupled directly to horseradish peroxidase or alkaline phosphatase, (Renz. M., and Kurz, K., A Colorimetric Method for DNA Hybridization, Nucl. Acids Res. 12: 3435-3444 (1984)) and synthetic oligonucleotides have been coupled directly with alkaline phosphatase (Jablonski, E., et al., Preparation of Oligodeoxynucleotide-Alkaline Phosphatase Conjugates and Their Use as Hybridization Probes, Nuc. Acids. Res. 14: 6115-6128 (1986); and Li P., et al., Enzyme-linked Synthetic Oligonucleotide probes: Non-Radioactiye Detection of Enteroloxigenic Escherichia Coli in Faeca Specimens, Nucl. Acids Res. 15: 5275-5287 (1987)).

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

### Antibodies to Proteins

Antibodies can be raised to a protein of the present invention, including individual, allelic, strain, or species variants, and fragments thereof, both in their naturally occurring (full-length) forms and in recombinant forms. Additionally, antibodies are raised to these proteins in either their native configurations or in non-native configurations. Anti-idiotypic antibodies can also be generated. Many methods of making antibodies are known to persons of skill. The following discussion is presented as a general overview of the techniques available; however, one of skill will recognize that many variations upon the following methods are known.

A number of immunogens are used to produce antibodies specifically reactive with a protein of the present invention. An isolated recombinant, synthetic, or native polynucleotide of the present invention are the preferred immunogens (antigen) for the production of monoclonal or polyclonal antibodies. Those of skill will readily understand that the proteins of the present invention are typically denatured, and optionally reduced, prior to formation of antibodies for screening expression libraries or other assays in which a putative protein of the present invention is expressed or denatured in a non-native secondary, tertiary, or quartenary structure. Non-isolated polypeptides of the present invention can be used either in pure or impure form.

The protein of the present invention is then injected into an animal capable of producing antibodies. Either monoclonal or polyclonal antibodies can be generated for subsequent use in immunoassays to measure the presence and quantity of the protein of the present invention. Methods of producing polyclonal antibodies are known to those of skill in the art. In brief, an immunogen (antigen), preferably a purified protein, a protein coupled to an appropriate carrier (*e.g.*, GST, keyhole limpet hemanocyanin, *etc.*), or a protein incorporated into an immunization vector such as a recombinant vaccinia virus (*see,* U.S. Patent No. 4,722,848) is mixed with an adjuvant and animals are immunized with the mixture. The animal's immune response to the immunogen preparation is monitored by taking test bleeds and determining the titer of reactivity to the protein of interest. When appropriately high titers of antibody to the immunogen are obtained, blood is collected from the animal and antisera are prepared. Further fractionation of the antisera to enrich for antibodies reactive to the protein is performed where desired (See, *e.g.*, Coligan, Current Protocols in Immunology, Wiley/Greene, NY (1991); and Harlow and Lane, Antibodies: A Laboratory Manual, Cold Spring Harbor Press, NY (1989)).

Antibodies, including binding fragments and single chain recombinant versions thereof, against predetermined fragments of a protein of the present invention are raised by immunizing animals, *e.g.*, with conjugates of the fragments with carrier proteins as described above. Typically, the immunogen of interest is a protein of at least about 5 amino acids, more typically the protein is 10 amino acids in length, preferably, 15 amino acids in length and more preferably the protein is 20 amino acids in length or greater. The peptides are typically coupled to a carrier protein (*e.g.*, as a fusion protein), or are recombinantly expressed in an immunization vector. Antigenic determinants on peptides to which antibodies bind are typically 3 to 10 amino acids in length.

Monoclonal antibodies are prepared from cells secreting the desired antibody. Monoclonals antibodies are screened for binding to a protein from which the immunogen was derived. Specific monoclonal and polyclonal antibodies will usually have an antibody binding site with an affinity constant for its cognate monovalent antigen at least between 10⁶-10⁷, usually at least 10⁸, preferably at least 10⁹, more preferably at least 10¹⁰, and most preferably at least 10¹¹ liters/mole,

In some instances, it is desirable to prepare monoclonal antibodies from various mammalian hosts, such as mice, rodents, primates, humans, *etc.* Description of techniques for preparing such monoclonal antibodies are found in, *e.g.,* Basic and Clinical Immunology. 4th ed., Stites et al., Eds., Lange Medical Publications, Los Altos, CA, and references cited therein; Harlow and Lane, *Supra*; Goding, Monoclonal Antibodies: Principles and Practice, 2nd ed., Academic Press, New York, NY (1986); and Kohler and Milstein, Nature 256: 495-497 (1975). Summarized briefly, this method proceeds by injecting an animal with an immunogen comprising a protein of the present invention. The animal is then sacrificed and cells taken from its spleen, which are fused with myeloma cells. The result is a hybrid cell or "hybridoma" that is capable of reproducing *in vitro.* The population of hybridomas is then screened to isolate individual clones, each of which secrete a single antibody species to the immunogen. In this manner, the individual antibody species obtained are the products of immortalized and cloned single B cells from the immune animal generated in response to a specific site recognized on the immunogenic substance.

Other suitable techniques involve selection of libraries of recombinant antibodies in phage or similar vectors (*see, e.g.,* Huse et al., Science 246: 1275-1281.(1989); and Ward, et al., Nature 341: 544-546 (1989); and Vaughan et al., Nature Biotechnology, 14: 309-314 (1996)). Alternatively, high avidity human monoclonal antibodies can be obtained from transgenic mice comprising fragments of the unrearranged human heavy and light chain Ig loci (i.e., minilocus transgenic mice). Fishwild et al., Nature Biotech., 14: 845-851 (1996). Also, recombinant immunoglobulins may be produced. See, Cabilly, U.S. Patent No. 4,816,567; and Queen et al., Proc. Nat'l Acad. Sci. 86: 10029-10033 (1989).

The antibodies of this invention are also used for affinity chromatography in isolating proteins of the present invention. Columns are prepared, *e.g.*, with the antibodies linked to a solid support, *e.g.*, particles, such as agarose, Sephadex, or the like, where a cell lysate is passed through the column, washed, and treated with increasing concentrations of a mild denaturant, whereby purified protein are released.

The antibodies can be used to screen expression libraries for particular expression products such as normal or abnormal protein. Usually the antibodies in such a procedure are labeled with a moiety allowing easy detection of presence of antigen by antibody binding.

Antibodies raised against a protein of the present invention can also be used to raise anti-idiotypic antibodies. These are useful for detecting or diagnosing various pathological conditions related to the presence of the respective antigens.

Frequently, the proteins and antibodies of the present invention will be labeled by joining, either covalently or non-covalently, a substance which provides for a detectable signal. A wide variety of labels and conjugation techniques are known and are reported extensively in both the scientific and patent literature. Suitable labels include radionucleotides, enzymes, substrates, cofactors, inhibitors, fluorescent moieties, chemiluminescent moieties, magnetic particles, and the like.

### Protein Immunoassays

Means of detecting the proteins of the present invention are not critical aspects of the present invention. In a preferred embodiment, the proteins are detected and/or quantified using any of a number of well recognized immunological binding assays (*see, e.g.*, U.S. Patents 4,366,241; 4,376,110; 4,517,288; and 4,837,168). For a review of the general immunoassays, see also Methods in Cell Biology, Vol. 37: Antibodies in Cell Biology, Asai, Ed., Academic Press, Inc. New York (1993); Basic and Clinical Immunology 7th Edition, Stites & Terr, Eds. (1991). Moreover, the immunoassays of the present invention can be performed in any of several configurations, *e.g*., those reviewed in Enzyme Immunoassays, Maggio, Ed., CRC Press, Boca Raton, Florida (1980); Tijan, Practice and Theory of Enzyme Immunoassays, Laboratory Techniques in Biochemistry and Molecular Biology, Elsevier Science Publishers B.V., Amsterdam (1985); Harlow and Lane, above; Immunoassay: A Practical Guide, Chan, Ed., Academic Press, Orlando, FL (1987); Principles and Practice of Immunoassaysm, Price and Newman Eds., Stockton Press, NY (1991); and Non-isotopic Immunoassays, Ngo, Ed., Plenum Press, NY (1988). Immunological binding assays (or immunoassays) typically utilize a "capture agent" to specifically bind to and often immobilize the analyte (in this case, a protein of the present invention). The capture agent is a moiety that specifically binds to the analyte. In a preferred embodiment, the capture agent is an antibody that specifically binds a protein(s) of the present invention. The antibody may be produced by any of a number of means known to those of skill in the art as described herein.

Immunoassays also often utilize a labeling agent to specifically bind to and label the binding complex formed by the capture agent and the analyte. The labeling agent may itself be one of the moieties comprising the antibody/analyte complex. Thus, the labeling agent may be a labeled protein of the present invention or a labeled antibody specifically reactive to a protein of the present invention. Alternatively, the labeling agent may be a third moiety, such as another antibody, that specifically binds to the antibody/protein complex.

In a preferred embodiment, the labeling agent is a second antibody bearing a label. Alternatively, the second antibody may lack a label, but it may, in turn, be bound by a labeled third antibody specific to antibodies of the species from which the second antibody is derived. The second can be modified with a detectable moiety, such as biotin, to which a third labeled molecule can specifically bind, such as enzyme-labeled streptavidin.

Other proteins capable of specifically binding immunoglobulin constant regions, such as protein A or protein G may also be used as the label agent. These proteins are normal constituents of the cell walls of streptococcal bacteria. They exhibit a strong non-immunogenic reactivity with immunoglobulin constant regions from a variety of species (See, generally Kronval, et al., J. Immunol. 111: 1401-1406 (1973), and Akerstrom, et al., J. Immunol. 135: 2589-2542 (1985)).

Throughout the assays, incubation and/or washing steps may be required after each combination of reagents. Incubation steps can vary from about 5 seconds to several hours, preferably from about 5 minutes to about 24 hours. However, the incubation time will depend upon the assay format, analyte, volume of solution, concentrations, and the like. Usually, the assays will be carried out at ambient temperature, although they can be conducted over a range of temperatures, such as 10°C to 40°C.

While the details of the immunoassays of the present invention may vary with the particular format employed, the method of detecting a protein of the present invention in a biological sample generally comprises the steps of contacting the biological sample with an antibody which specifically reacts, under immunologically reactive conditions, to a protein of the present invention. The antibody is allowed to bind to the protein under immunologically reactive conditions, and the presence of the bound antibody is detected directly or indirectly.

### A. Non-Competitive Assay Formats

Immunoassays for detecting proteins of the present invention include competitive and noncompetitive formats. Noncompetitive immunoassays are assays in which the amount of captured analyte (i.e., a protein of the present invention) is directly measured. In one preferred "sandwich" assay, for example, the capture agent (e.g., an antibody specifically reactive, under immunoreactive conditions, to a protein of the present invention) can be bound directly to a solid substrate where they are immobilized. These immobilized antibodies then capture the protein present in the test sample. The protein thus immobilized is then bound by a labeling agent, such as a second antibody bearing a label. Alternatively, the second antibody may lack a label, but it may, in turn, be bound by a labeled third antibody specific to antibodies of the species from which the second antibody is derived. The second can be modified with a detectable moiety, such as biotin, to which a third labeled molecule can specifically bind, such as enzyme-labeled streptavidin.

### B. Competitive Assay Formats

In competitive assays, the amount of analyte present in the sample is measured indirectly by measuring the amount of an added (exogenous) analyte (e.g., a protein of the present invention) displaced (or competed away) from a capture agent (e.g., an antibody specifically reactive, under immunoreactive conditions, to the protein) by the analyte present in the sample. In one competitive assay, a known amount of analyte is added to the sample and the sample is then contacted with a capture agent that specifically binds a protein of the present invention. The amount of protein bound to the capture agent is inversely proportional to the concentration of analyte present in the sample.

In a particularly preferred embodiment, the antibody is immobilized on a solid substrate. The amount of protein bound to the antibody may be determined either by measuring the amount of protein present in a protein/antibody complex, or alternatively by measuring the amount of remaining uncomplexed protein. The amount of protein may be detected by providing a labeled protein.

A hapten inhibition assay is another preferred competitive assay. In this assay a known analyte, (such as a protein of the present invention) is immobilized on a solid substrate. A known amount of antibody specifically reactive, under immunoreactive conditions, to the protein is added to the sample, and the sample is then contacted with the immobilized protein. In this case, the amount of antibody bound to the immobilized protein is inversely proportional to the amount of protein present in the sample. Again, the amount of immobilized antibody may be detected by detecting either the immobilized fraction of antibody or the fraction of the antibody that remains in solution. Detection may be direct where the antibody is labeled or indirect by the subsequent addition of a labeled moiety that specifically binds to the antibody as described above.

### C. Generation of pooled antisera for use in immunoassays

A protein that specifically binds to or that is specifically immunoreactive with an antibody generated against a defined immunogen is determined in an immunoassay. The immunoassay uses a polyclonal antiserum which is raised to a polypeptide of the present invention (i.e., the immunogenic polypeptide). This antiserum is selected to have low crossreactivity against other proteins and any such crossreactivity is removed by immunoabsorbtion prior to use in the immunoassay (*e.g.*, by immunosorbtion of the antisera with a protein of different substrate specificity (e.g., a different enzyme) and/or a protein with the same substrate specificity but of a different form).

In order to produce antisera for use in an immunoassay, a polypeptide of the present invention is isolated as described herein. For example, recombinant protein can be produced in a mammalian or other eukaryotic cell line. An inbred strain of mice is immunized with the protein using a standard adjuvant, such as Freund's adjuvant, and a standard mouse immunization protocol (see Harlow and Lane, above). Alternatively, a synthetic polypeptide derived from the sequences disclosed herein and conjugated to a carrier protein is used as an immunogen. Polyclonal sera are collected and titered against the immunogenic polypeptide in an immunoassay, for example, a solid phase immunoassay with the immunogen immobilized on a solid support. Polyclonal antisera with a titer of 10⁴ or greater are selected and tested for their cross reactivity against polypeptides of different forms or substrate specificity, using a competitive binding immunoassay such as the one described in Harlow and Lane, above, at pages 570-573. Preferably, two or more distinct forms of polypeptides are used in this determination. These distinct types of polypeptides are used as competitors to identify antibodies which are specifically bound by the polypeptide being assayed for. The competitive polypeptides can be produced as recombinant proteins and isolated using standard molecular biology and protein chemistry techniques as described herein.

Immunoassays in the competitive binding format are used for crossreactivity determinations. For example, the immunogenic polypeptide is immobilized to a solid support. Proteins added to the assay compete with the binding of the antisera to the immobilized antigen. The ability of the above proteins to compete with the binding of the antisera to the immobilized protein is compared to the immunogenic polypeptide. The percent crossreactivity for the above proteins is calculated, using standard calculations. Those antisera with less than 10% crossreactivity with a distinct form of a polypeptide are selected and pooled. The cross-reacting antibodies are then removed from the pooled antisera by immunoabsorbtion with a distinct form of a polypeptide.

The immunoabsorbed and pooled antisera are then used in a competitive binding immunoassay as described herein to compare a second "target" polypeptide to the immunogenic polypeptide. In order to make this comparison, the two polypeptides are each assayed at a wide range of concentrations and the amount of each polypeptide required to inhibit 50% of the binding of the antisera to the immobilized protein is determined using standard techniques. If the amount of the target polypeptide required is less than twice the amount of the immunogenic polypeptide that is required, then the target polypeptide is said to specifically bind to an antibody generated to the immunogenic protein. As a final determination of specificity, the pooled antisera is fully immunosorbed with the immunogenic polypeptide until no binding to the polypeptide used in the immunosorbtion is detectable. The fully immunosorbed antisera is then tested for reactivity with the test polypeptide. If no reactivity is observed, then the test polypeptide is specifically bound by the antisera elicited by the immunogenic protein.

### D. Other Assay Formats

In a particularly preferred embodiment, Western blot (immunoblot) analysis is used to detect and quantify the presence of protein of the present invention in the sample. The technique generally comprises separating sample proteins by gel electrophoresis on the basis of molecular weight, transferring the separated proteins to a suitable solid support, (such as a nitrocellulose filter, a nylon filter, or derivatized nylon filter), and incubating the sample with the antibodies that specifically bind a protein of the present invention. The antibodies specifically bind to the protein on the solid support. These antibodies may be directly labeled or alternatively may be subsequently detected using labeled antibodies (*e.g*., labeled sheep anti-mouse antibodies) that specifically bind to the antibodies.

### E. Quantification of Proteins.

The proteins of the present invention may be detected and quantified by any of a number of means well known to those of skill in the art. These include analytic biochemical methods such as electrophoresis, capillary electrophoresis, high performance liquid chromatography (HPLC), thin layer chromatography (TLC), hyperdiffusion chromatography, and the like, and various immunological methods such as fluid or gel precipitin reactions, immunodiffusion (single or double), immunoelectrophoresis, radioimmunoassays (RIAs), enzyme-linked immunosorbent assays (ELISAs), immunofluorescent assays, and the like.

### F. Reduction of Non-Specific Binding

One of skill will appreciate that it is often desirable to reduce non-specific binding in immunoassays and during analyte purification. Where the assay involves an antigen, antibody, or other capture agent immobilized on a solid substrate, it is desirable to minimize the amount of non-specific binding to the substrate. Means of reducing such non-specific binding are well known to those of skill in the art. Typically, this involves coating the substrate with a proteinaceous composition. In particular, protein compositions such as bovine serum albumin (BSA), nonfat powdered milk, and gelatin are widely used.

### G. Immunoassay Labels

The labeling agent can be, *e.g*., a monoclonal antibody, a polyclonal antibody, a binding protein or complex, or a polymer such as an affinity matrix, carbohydrate or lipid. Detectable labels suitable for use in the present invention include any composition detectable by spectroscopic, radioisotopic, photochemical, biochemical, immunochemical, electrical, optical or chemical means. Detection may proceed by any known method, such as immunoblotting, western analysis, gel-mobility shift assays, fluorescent *in situ* hybridization analysis (FISH), tracking of radioactive or bioluminescent markers, nuclear magnetic resonance, electron paramagnetic resonance, stopped-flow spectroscopy, column chromatography, capillary electrophoresis, or other methods which track a molecule based upon an alteration in size and/or charge. The particular label or detectable group used in the assay is not a critical aspect of the invention. The detectable group can be any material having a detectable physical or chemical property. Such detectable labels have been well-developed in the field of immunoassays and, in general, any label useful in such methods can be applied to the present invention. Thus, a label is any composition detectable by spectroscopic, photochemical, biochemical, immunochemical, electrical, optical or chemical means. Useful labels in the present invention include magnetic beads, fluorescent dyes, radiolabels, enzymes, and colorimetric labels or colored glass or plastic beads, as discussed for nucleic acid labels, above.

The label may be coupled directly or indirectly to the desired component of the assay according to methods well known in the art. As indicated above, a wide variety of labels may be used, with the choice of label depending on the sensitivity required, ease of conjugation of the compound, stability requirements, available instrumentation, and disposal provisions.

Non-radioactive labels are often attached by indirect means. Generally, a ligand molecule (*e.g*., biotin) is covalently bound to the molecule. The ligand then binds to an anti-ligand (*e.g*., streptavidin) molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, *e.g*., by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, and 2,3-dihydrophthalazinediones, e.g., luminol. For a review of various labeling or signal producing systems which may be used, see, U.S. Patent No. 4,391,904, which is incorporated herein by reference.

Means of detecting labels are well known to those of skill in the art. Thus, for example, where the label is a radioactive label, means for detection include a scintillation counter or photographic film as in autoradiography. Where the label is a fluorescent label, it may be detected by exciting the fluorochrome with the appropriate wavelength of light and detecting the resulting fluorescence, *e.g*., by microscopy, visual inspection, via photographic film, by the use of electronic detectors such as charge coupled devices (CCDs) or photomultipliers and the like. Similarly, enzymatic labels may be detected by providing appropriate substrates for the enzyme and detecting the resulting reaction product. Finally, simple colorimetric labels may be detected simply by observing the color associated with the label. Thus, in various dipstick assays, conjugated gold often appears pink, while various conjugated beads appear the color of the bead.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

### Assays for Compounds that Modulate Enzymatic Activity or Expression

The present invention also relates to means for identifying compounds that bind to (e.g., substrates), and/or increase or decrease (i.e., modulate) the enzymatic activity of, catalytically active polypeptides of the present invention. The method comprises contacting a polypeptide of the present invention with a compound whose ability to bind to or modulate enzyme activity is to be determined. The polypeptide employed will have at least 20%, preferably at least 30% or 40%, more preferably at least 50% or 60%, and most preferably at least 70% or 80% of the specific activity of the native, full-length polypeptide of the present invention (e.g., enzyme). Generally, the polypeptide will be present in a range sufficient to determine the effect of the compound, typically about 1 nM to 10 µM. Likewise, the compound will be present in a concentration of from about 1 nM to 10 µM. Those of skill will understand that such factors as enzyme concentration, ligand concentrations (i.e., substrates, products, inhibitors, activators), pH, ionic strength, and temperature will be controlled so as to obtain useful kinetic data and determine the presence of absence of a compound that binds or modulates polypeptide activity. Methods of measuring enzyme kinetics is well known in the art. See, e.g., Segel, Biochemical Calculations, 2nd ed., John Wiley and Sons, New York (1976).

Although the present invention has been described in some detail by way of illustration and example for purposes of clarity of understanding, it will be obvious that certain changes and modifications may be practiced within the scope of the appended claims.

### Example 1

This example describes the construction cDNA libraries.

### Total RNA Isolation

Total RNA was isolated from corn tissues with TRIzol Reagent (Life Technology Inc. Gaithersburg, MD) using a modification of the guanidine isothiocyanate/acid-phenol procedure described by Chomczynski and Sacchi (Chomczynski, P., and Sacchi, N. Anal. Biochem. 162, 156 (1987)). In brief, plant tissue samples were pulverized in liquid nitrogen before the addition of the TRIzol Reagent, and then were further homogenized with a mortar and pestle. Addition of chloroform followed by centrifugation was conducted for separation of an aqueous phase and an organic phase. The total RNA was recovered by precipitation with isopropyl alcohol from the aqueous phase.

### Poly(A)+ RNA Isolation

The selection of poly(A)+ RNA from total RNA was performed using PolyATact system (Promega Corporation. Madison, WI). In brief, biotinylated oligo(dT) primers were used to hybridize to the 3' poly(A) tails on mRNA. The hybrids were captured using streptavidin coupled to paramagnetic particles and a magnetic separation stand. The mRNA was washed at high stringent condition and eluted by RNase-free deionized water.

### cDNA Library Construction

cDNA synthesis was performed and unidirectional cDNA libraries were constructed using the SuperScript Plasmid System (Life Technology Inc. Gaithersburg, MD). The first stand of cDNA was synthesized by priming an oligo(dT) primer containing a Not I site. The reaction was catalyzed by SuperScript Reverse Transcriptase II at 45°C. The second strand of cDNA was labeled with alpha-³¹P-dCTP and a portion of the reaction was analyzed by agarose gel electrophoresis to determine cDNA sizes. cDNA molecules smaller than 500 base pairs and unligated adapters were removed by Sephacryl-S400 chromatography. The selected cDNA molecules were ligated into pSPORT1 vector in between of Not 1 and Sal I sites.

### Example 2

This example describes cDNA sequencing and library subtraction.

### Sequencing Template Preparation

Individual colonies were picked and DNA was prepared either by PCR with M13 forward primers and M13 3 reverse primers, or by plasmid isolation. All the cDNA clones were sequenced using M13 reverse primers.

### Q-bot Subtraction Procedure

cDNA libraries subjected to the subtraction procedure were plated out on 22 x 22 cm² agar plate at density of about 3,000 colonies per plate. The plates were incubated in a 37°C incubator for 12-24 hours. Colonies were picked into 384-well plates by a robot colony picker, Q-bot (GENETIX Limited). These plates were incubated overnight at 37°C.

Once sufficient colonies were picked, they were pinned onto 22 x 22 cm² nylon membranes using Q-bot. Each membrane contained 9,216 colonies or 36,864 colonies. These membranes were placed onto agar plate with appropriate antibiotic. The plates were incubated at 37°C for overnight.

After colonies were recovered on the second day, these filters were placed on filter paper prewetted with denaturing solution for four minutes, then were incubated on top of a boiling water bath for additional four minutes. The filters were then placed on filter paper prewetted with neutralizing solution for four minutes. After excess solution was removed by placing the filters on dry filter papers for one minute, the colony side of the filters were place into Proteinase K solution, incubated at 37°C for 40-50 minutes. The filters were placed on dry filter papers to dry overnight. DNA was then crosslinked to nylon membrane by UV light treatment.

Colony hybridization was conducted as described by Sambrook,J., Fritsch, E.F. and Maniatis, T., (in Molecular Cloning: A laboratory Manual, 2nd Edition). The following probes were used in colony hybridization:
1. First strand cDNA from the same tissue as the library was made from to remove the most redundant clones.
2. 48-192 most redundant cDNA clones from the same library based on previous sequencing data.
3. 192 most redundant cDNA clones in the entire corn partial sequence database.
4. A Sal-A20 oligo nucleotide: TCG ACC CAC GCG TCC GAA AAA AAA AAA AAA AAA AAA, removes clones containing a poly A tail but no cDNA.
5. cDNA clones derived from rRNA.

The image of the autoradiography was scanned into computer and the signal intensity and cold colony addresses of each colony was analyzed. Re-arraying of cold-colonies from 384 well plates to 96 well plates was conducted using Q-bot.

### Example 3

This example describes identification of the gene from a computer homology search. Gene identities were determined by conducting BLAST (Basic Local Alignment Search Tool; Altschul, S. F., et al., (1993) J. Mol. Biol. 215:403-410; see also www.ncbi.nlm.nih.gov/BLAST/) searches under default parameters for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences were analyzed for similarity to all publicly available DNA sequences contained in the "nr" database using the BLASTN algorithm. The DNA sequences were translated in all reading frames and compared for similarity to all publicly available protein sequences contained in the "nr" database using the BLASTX algorithm (Gish, W. and States, D. J. (1993) Nature Genetics 3:266-272) provided by the NCBI. In some cases, the sequencing data from two or more clones containing overlapping segments of DNA were used to construct contiguous DNA sequences.

The above examples are provided to illustrate the invention but not to limit its scope. Other variants of the invention will be readily apparent to one of ordinary skill in the art and are encompassed by the appended claims.

### SEQUENCE LISTING

<110> Pioneer Hi-Bred International, Inc.
<120> Maize Cellulose Synthases and Uses
   Thereof
<130> 0864-PCT
<150> 60/096, 822
   <151> August 17, 1998
<160> 60
<170> PastSEQ for Windows Version 3.0
<210> 1
   <211> 3568
   <212> DNA
   <213> Zea mays
<220>
   <221> CDS
   <222> (63)...(3237)
<400> 1
   gtcgacccac gcgtccggag ctcgtcgtca tccgccgcga tggcgagcca gggccgaagc      60
   cc atg gac cag cgg aac ggc cag gtg tgc cag att tgc ggc gac gac        107
      Met Asp Gln Arg Asn Gly Gln Val Cys Gln Ile Cys Gly Asp Asp
      1               5                   10                  15
   gtg ggg cgc aac ccc gac ggg gag cct ttc gtg gcc tgc aac gag tgc       155
   Val Gly Arg Asn Pro Asp Gly Glu Pro Phe Val Ala Cys Asn Glu Cys
                   20                  25                  30
   gcc ttc ccc atc tgc cgg gac tgc tac gag tac gag cgc cgc gag ggc       203
   Ala Phe Pro Ile Cys Arg Asp Cys Tyr Glu Tyr Glu Arg Arg Glu Gly
               35                  40                  45
   acg cag aac tgc ccc cag tgc aag acc cgc ttc aag cgc ttc aag ggg       251
   Thr Gln Asn Cys Pro Gln Cys Lys Thr Arg Phe Lys Arg Phe Lys Gly
           50                  55                  60
   tgc gcg cgc gtg ccc ggg gac gag gag gag gac ggc gtc gac gac ctg       299
   Cys Ala Arg Val Pro Gly Asp Glu Glu Glu Asp Gly Val Asp Asp Leu
       65                  70                  75
   gag aac gag ttc aac tgg agc gac aag cac gac tcc cag tac ctc gcc       347
   Glu Asn Glu Phe Asn Trp Ser Asp Lys His Asp Ser Gln Tyr Leu Ala
   80                  85                  90                  95
   gag tcc atg ctc cac gcc cac atg agc tac ggc cgc ggc gcc gac ctc       395
   Glu Ser Met Leu His Ala His Met Ser Tyr Gly Arg Gly Ala Asp Leu
                   100                 105                 110
   gac ggc gtg ccg cag cca ttc cac ccc atc ccc aat gtt ccc ctc ctc       443
   Asp Gly Val Pro Gln Pro Phe His Pro Ile Pro Asn Val Pro Leu Leu
               115                 120                 125
   acc aac gga cag atg gtc gat gac atc ccg ccg gac cag cac gcc ctt       491
   Thr Asn Gly Gln Met Val Asp Asp Ile Pro Pro Asp Gln His Ala Leu
           130                 135                 140
   gtg ccc tcg ttc gtg ggt ggc ggg ggg aag agg att cac cct ctc ccg       539
   Val Pro Ser Phe Val Gly Gly Gly Gly Lys Arg Ile His Pro Leu Pro
       145                 150                 155
   tac gcg gat ccc aac ctt cct gtg caa ccg agg tct atg gac cct tcc       587
   Tyr Ala Asp Pro Asn Leu Pro Val Gln Pro Arg Ser Met Asp Pro Ser
   160                 165                 170                 175
   aag gat ctc gcc gca tat ggc tac ggg agc gta gca tgg aag gag agg       635
   Lys Asp Leu Ala Ala Tyr Gly Tyr Gly Ser Val Ala Trp Lys Glu Arg
                   180                 185                 190
   atg gag agc tgg aag cag aag cag gag agg atg cac cag acg agg aac       683
   Met Glu Ser Trp Lys Gln Lys Gln Glu Arg Met His Gln Thr Arg Asn
               195                 200                 205
   gat ggc ggc ggc gat gat ggt gat gat gca gat cta cca cta atg gat       731
   Asp Gly Gly Gly Asp Asp Gly Asp Asp Ala Asp Leu Pro Leu Met Asp
           210                 215                 220
   gaa gct aga cag cca ttg tcc aga aag atc ccg ctt cct tca agc caa       779
   Glu Ala Arg Gln Pro Leu Ser Arg Lys Ile Pro Leu Pro Ser Ser Gln
       225                 230                 235
   atc aac ccc tat agg atg att ata ata att cgg cta gtg gtt ttg tgt       827
   Ile Asn Pro Tyr Arg Met Ile Ile Ile Ile Arg Leu Val Val Leu Cys
   240                 245                 250                 255
   ttc ttc ttc cac tac cga gtg atg cat ccg gtg cct gat gca ttt gct       875
   Phe Phe Phe His Tyr Arg Val Met His Pro Val Pro Asp Ala Phe Ala
                   260                 265                 270
   tta tgg ctc ata tct gtg atc tgt gaa att tgg ttt gcc atg tct tgg       923
   Leu Trp Leu Ile Ser Val Ile Cys Glu Ile Trp Phe Ala Met Ser Trp
               275                 280                 285
   att ctt gac cag ttt cca aag tgg ttt cct atc gag agg gaa acc tat       971
   Ile Leu Asp Gln Phe Pro Lys Trp Phe Pro Ile Glu Arg Glu Thr Tyr
           290                 295                 300
   ctt gac cgg ctg agt tta agg ttt gac aag gaa ggg cat cct tct caa      1019
   Leu Asp Arg Leu Ser Leu Arg Phe Asp Lys Glu Gly His Pro Ser Gln
       305                 310                 315
   ctc gcc cct gtt gat ttc ttt gtc agt acg gtt gat ccc ttg aag gaa      1067
   Leu Ala Pro Val Asp Phe Phe Val Ser Thr Val Asp Pro Leu Lys Glu
   320                 325                 330                 335
   cct cca ttg gtc act gct aat act gtt cta tct atc ctt tcg gtg gat      1115
   Pro Pro Leu Val Thr Ala Asn Thr Val Leu Ser Ile Leu Ser Val Asp
                   340                 345                 350
   tat cca gtt gat aag gtt tca tgc tac gtt tct gat gat ggt gct gcc      1163
   Tyr Pro Val Asp Lys Val Ser Cys Tyr Val Ser Asp Asp Gly Ala Ala
                   355                 360                 365
   atg ctg aca ttt gaa gca ttg tct gaa aca tct gaa ttt gca aag aaa      1211
   Met Leu Thr Phe Glu Ala Leu Ser Glu Thr Ser Glu Phe Ala Lys Lys
               370                 375                 380
   tgg gtt cct ttc tgc aaa aga tat agc ctt gag cct cgt gct cca gag      1259
   Trp Val Pro Phe Cys Lys Arg Tyr Ser Leu Glu Pro Arg Ala Pro Glu
       385                 390                 395
   tgg tac ttc caa cag aag ata gac tac ctg aaa gac aag gtg gcg cca      1307
   Trp Tyr Phe Gln Gln Lys Ile Asp Tyr Leu Lys Asp Lys Val Ala Pro
   400                 405                 410                 415
   aac ttt gtt aga gaa cgg aga gca atg aag aga gag tat gag gaa ttc      1355
   Asn Phe Val Arg Glu Arg Arg Ala Met Lys Arg Glu Tyr Glu Glu Phe
                   420                 425                 430
   aag gtc aga atc aat gcc ttg gtt gct aaa gcc caa aag gtt cct gag      1403
   Lys Val Arg Ile Asn Ala Leu Val Ala Lys Ala Gln Lys Val Pro Glu
                   435                 440                 445
   gaa gga tgg aca atg cag gat gga act cca tgg ccc gga aat aat gtc      1451
   Glu Gly Trp Thr Met Gln Asp Gly Thr Pro Trp Pro Gly Asn Asn Val
               450                 455                 460
   cgt gat cat cct gga atg att cag gtt ttc ctt ggt caa agt ggt ggc      1499
   Arg Asp His Pro Gly Met Ile Gln Val Phe Leu Gly Gln Ser Gly Gly
       465                 470                 475
   cat gat gtg gaa gga aat gag ctg cct cga ttg gtt tat gtt tca aga      1547
   His Asp Val Glu Gly Asn Glu Leu Pro Arg Leu Val Tyr Val Ser Arg
   480                 485                 490                 495
   gaa aaa cgg cca ggc tac aac cat cac aag aag gct ggt gct atg aat      1595
   Glu Lys Arg Pro Gly Tyr Asn His His Lys Lys Ala Gly Ala Met Asn
                   500                 505                 510
   gca ttg gtc cga gtc tct gct gta cta act aat gct cct tat ttg ctg      1643
   Ala Leu Val Arg Val Ser Ala Val Leu Thr Asn Ala Pro Tyr Leu Leu
                   515                 520                 525
   aac ttg gat tgt gat cac tat atc aat aat agt aag gct ata aag gaa      1691
   Asn Leu Asp Cys Asp His Tyr Ile Asn Asn Ser Lys Ala Ile Lys Glu
               530                 535                 540
   gca atg tgt ttt atg atg gat cct ttg ctt gga aag aaa gtt tgc tat      1739
   Ala Met Cys Phe Met Met Asp Pro Leu Leu Gly Lys Lys Val Cys Tyr
       545                 550                 555
   gtg cag ttt cct caa aga ttt gat ggg att gat cgc cat gat cga tat      1787
   Val Gln Phe Pro Gln Arg Phe Asp Gly Ile Asp Arg His Asp Arg Tyr
   560                 565                 570                 575
   gct aac aga aat gtt gtc ttt ttc gat atc aac atg aaa ggt ttg gat      1835
   Ala Asn Arg Asn Val Val Phe Phe Asp Ile Asn Met Lys Gly Leu Asp
                   580                 585                 590
   ggt atc cag ggc cca att tat gtg ggt act gga tgt gtc ttc aga agg      1883
   Gly Ile Gln Gly Pro Ile Tyr Val Gly Thr Gly Cys Val Phe Arg Arg
               595                 600                 605
   cag gca tta tat ggc tac gat gct ccc aaa aca aag aag cca cca tca      1931
   Gln Ala Leu Tyr Gly Tyr Asp Ala Pro Lys Thr Lys Lys Pro Pro Ser
           610                 615                 620
   aga act tgc aac tgc tgg cca aag tgg tgc att tgc tgt tgc tgt ttt      1979
   Arg Thr Cys Asn Cys Trp Pro Lys Trp Cys Ile Cys Cys Cys Cys Phe
       625                 630                 635
   ggt aac agg aag acc aag aag aag acc aag acc tct aaa cct aaa ttt      2027
   Gly Asn Arg Lys Thr Lys Lys Lys Thr Lys Thr Ser Lys Pro Lys Phe
   640                 645                 650                 655
   gag aag ata aag aaa ctt ttt aag aaa aag gaa aat caa gcc cct gca      2075
   Glu Lys Ile Lys Lys Leu Phe Lys Lys Lys Glu Asn Gln Ala Pro Ala
                   660                 665                 670
   tat gct ctt ggt gaa att gat gaa gcc gct cca gga gct gaa aat gaa      2123
   Tyr Ala Leu Gly Glu Ile Asp Glu Ala Ala Pro Gly Ala Glu Asn Glu
               675                 680                 685
   aag gct agt att gta aat caa cag aag ttg gaa aag aaa ttt ggc cag      2171
   Lys Ala Ser Ile Val Asn Gln Gln Lys Leu Glu Lys Lys Phe Gly Gln
           690                 695                 700
   tct tca gtt ttt gtt gca tcc aca ctt ctt gag aat ggt gga acc ctg      2219
   Ser Ser Val Phe Val Ala Ser Thr Leu Leu Glu Asn Gly Gly Thr Leu
       705                 710                 715
   aag agt gcc agt cca gct tct ctt ctg aag gaa gct ata cat gtc atc      2267
   Lys Ser Ala Ser Pro Ala Ser Leu Leu Lys Glu Ala Ile His Val Ile
   720                 725                 730                 735
   agt tgt gga tat gaa gac aaa aca ggc tgg gga aaa gat att ggt tgg      2315
   Ser Cys Gly Tyr Glu Asp Lys Thr Gly Trp Gly Lys Asp Ile Gly Trp
                   740                 745                 750
   att tat gga tca gtc aca gaa gat att ctt act ggg ttt aag atg cac      2363
   Ile Tyr Gly Ser Val Thr Glu Asp Ile Leu Thr Gly Phe Lys Met His
               755                 760                 765
   tgc cat ggt tgg cgg tca att tac tgc ata cct aaa cgg gcc gcc ttc      2411
   Cys His Gly Trp Arg Ser Ile Tyr Cys Ile Pro Lys Arg Ala Ala Phe
           770                 775                 780
   aaa ggt tcc gca cct ctc aat ctt tcc gat cgt ttt cac cag gtt ctt      2459
   Lys Gly Ser Ala Pro Leu Asn Leu Ser Asp Arg Phe His Gln Val Leu
       785                 790                 795
   cgg tgg gct ctt ggt tca att gaa att ttg ttc agc aac cac tgc cct      2507
   Arg Trp Ala Leu Gly Ser Ile Glu Ile Leu Phe Ser Asn His Cys Pro
   800                 805                 810                 815
   ctc tgg tat ggg tat ggt ggt gga cta aag ttc ctg gaa agg ttt tcg      2555
   Leu Trp Tyr Gly Tyr Gly Gly Gly Leu Lys Phe Leu Glu Arg Phe Ser
                   820                 825                 830
   tac att aac tcc atc gta tac cct tgg aca tct atc ccg ctc ttg gcc      2603
   Tyr Ile Asn Ser Ile Val Tyr Pro Trp Thr Ser Ile Pro Leu Leu Ala
               835                 840                 845
   tat tgc aca ttg cct gcc atc tgc ttg ctg aca ggg aaa ttt atc acg      2651
   Tyr Cys Thr Leu Pro Ala Ile Cys Leu Leu Thr Gly Lys Phe Ile Thr
           850                 855                 860
   cca gag ctt aac aat gtt gcc agc ctc tgg ttc atg tca ctt ttc atc      2699
   Pro Glu Leu Asn Asn Val Ala Ser Leu Trp Phe Met Ser Leu Phe Ile
       865                 870                 875
   tgc att ttt gct acg agc atc ctg gaa atg aga tgg agt ggt gta ggc      2747
   Cys Ile Phe Ala Thr Ser Ile Leu Glu Met Arg Trp Ser Gly Val Gly
   880                 885                 890                 895
   atc gat gac tgg tgg aga aac gag cag ttt tgg gtc att gga ggc gtg      2795
   Ile Asp Asp Trp Trp Arg Asn Glu Gln Phe Trp Val Ile Gly Gly Val
                   900                 905                 910
   tct tca cat ctc ttt gct gtg ttc cag gga ctc ctc aag gtc ata gct      2843
   Ser Ser His Leu Phe Ala Val Phe Gln Gly Leu Leu Lys Val Ile Ala
               915                 920                 925
   ggt gta gac acg agc ttc act gtg aca tcc aag ggc gga gac gac gag      2891
   Gly Val Asp Thr Ser Phe Thr Val Thr Ser Lys Gly Gly Asp Asp Glu
           930                 935                 940
   gag ttc tca gag ctg tac aca ttc aaa tgg acg acc ctt ctg ata cct      2939
   Glu Phe Ser Glu Leu Tyr Thr Phe Lys Trp Thr Thr Leu Leu Ile Pro
       945                 950                 955
   ccg aca acc ctg ctc cta ctg aac ttc att gga gtg gta gct ggc atc      2987
   Pro Thr Thr Leu Leu Leu Leu Asn Phe Ile Gly Val Val Ala Gly Ile
   960                 965                 970                 975
   tcc aat gcg atc aac aac gga tat gaa tca tgg ggc ccc ctg ttc ggg      3035
   Ser Asn Ala Ile Asn Asn Gly Tyr Glu Ser Trp Gly Pro Leu Phe Gly
                   980                 985                 990
   aag ctc ttc ttt gca ttt tgg gtg atc gtc cat ctt tac ccg ttc ctc      3083
   Lys Leu Phe Phe Ala Phe Trp Val Ile Val His Leu Tyr Pro Phe Leu
               995                 1000                1005
   aag ggt ctg gtt ggg agg cag aac agg acg cca acg att gtc att gtc      3131
   Lys Gly Leu Val Gly Arg Gln Asn Arg Thr Pro Thr Ile Val Ile Val
           1010                1015                1020
   tgg tcc atc ctc ctg gct tcg atc ttc tcg ctg ctt tgg gtc cgg atc      3179
   Trp Ser Ile Leu Leu Ala Ser Ile Phe Ser Leu Leu Trp Val Arg Ile
       1025                1030                1035
   gac ccg ttc ctt gcg aag gat gat ggt ccc ctg ttg gag gag tgt ggt      3227
   Asp Pro Phe Leu Ala Lys Asp Asp Gly Pro Leu Leu Glu Glu Cys Gly
   1040                1045                1050                1055
   ctg gat tgc a actaggaggt cagcacgtgg acttccccgt cagtgtgtgg         3277
   Leu Asp Cys
   tcgaagaagt atttttgcag atgttttgtg cccatatttc tttactcaat ttttgtccct 3337
   ctgtagattg aaacaagggg tgaaggggaa aaaaagtact tgtatttctt ttgttccatg 3397
   gtggtggtgg tggtgggcgg ctcagcctcg tgagtgcaat attgggcaaa ccggaggttg 3457
   cggcaacctt gtgcagttcg tccacgaatn tactagggat gatcgcgacc aatcaatcaa 3517
   tcgatgaccg agttcaattg ttcaaaaaaa aaaaaaaaaa agggcggccg c          3568
<210> 2
   <211> 1059
   <212> PRT
   <213> Zea mays
<400> 2 <210> 3 <211> 25 <212> DNA <213> Zea mays <400> 3
   atggaccagc ggaacggcca ggtgt
25 <210> 4 <211> 25 <212> DNA <213> Zea mays <400> 4
ctagttgcaa tccagaccac actcc
25 <210> 5 <211> 3773
<212> DNA
   <213> Zea mays
<220>
   <221> CDS
   <222> (338)...(3566)
<400> 5
<210> 6
   <211> 1077
   <212> PRT
   <213> Zea mays
<400> 6 <210> 7 <211> 25 <212> DNA <213> Zea mays <400> 7
   atggagggcg acgcggacgg cgtga
25 <210> 8 <211> 25 <212> DNA <213> Zea mays <400> 8
ctagcagttg atgccacacg tctgg
25 <210> 9 <211> 3780 <212> DNA <213> Zea mays <220> <221> CDS <222> (201)...(3423) <400> 9
<210> 10
   <211> 1075
   <212> PRT
   <213> Zea mays
<400> 10
   Met Ala Ala Asn Lys Gly Met Val Ala Gly Ser His Asn Arg Asn Glu
   1               5                   10                  15
   Phe Val Met Ile Arg His Asp Gly Asp Val Pro Gly Ser Ala Lys Pro
               20                  25                  30
   Thr Lys Ser Ala Asn Gly Gln Val Cys Gln Ile Cys Gly Asp Ser Val
   35 40 45
   Gly Val Ser Ala Thr Gly Asp Val Phe Val Ala Cys Asn Glu Cys Ala
   50 55 60
   Phe Pro Val Cys Arg Pro Cys Tyr Glu Tyr Glu Arg Lys Glu Gly Asn
   65 70 75 80
   Gln Cys Cys Pro Gln Cys Lys Thr Arg Tyr Lys Arg Gln Lys Gly Ser
   85 90 95
   Pro Arg Val His Gly Asp Glu Asp Glu Glu Asp Val Asp Asp Leu Asp
   100 105 110
   Asn Glu Phe Asn Tyr Lys Gln Gly Ser Gly Lys Gly Pro Glu Trp Gln
   115 120 125
   Leu Gln Gly Asp Asp Ala Asp Leu Ser Ser Ser Ala Arg His Glu Pro
   130 135 140
   His His Arg Ile Pro Arg Leu Thr Ser Gly Gln Gln Ile Ser Gly Glu
   145 150 155 160
   Ile Pro Asp Ala Ser Pro Asp Arg His Ser Ile Arg Ser Pro Thr Ser
   165 170 175
   Ser Tyr Val Asp Pro Ser Val Pro Val Pro Val Arg Ile Val Asp Pro
   180 185 190
   Ser Lys Asp Leu Asn Ser Tyr Gly Leu Asn Ser Val Asp Trp Lys Glu
   195 200 205
   Arg Val Glu Ser Trp Arg Val Lys Gln Asp Lys Asn Met Met Gln Val
   210 215 220
   Thr Asn Lys Tyr Pro Glu Ala Arg Gly Gly Asp Met Glu Gly Thr Gly
   225 230 235 240
   Ser Asn Gly Glu Xaa Met Gln Met Val Asp Asp Ala Arg Leu Pro Leu
   245 250 255
   Ser Arg Ile Val Pro Ile Ser Ser Asn Gln Leu Asn Leu Tyr Arg Val
   260 265 270
   Val Ile Ile Leu Arg Leu Ile Ile Leu Cys Phe Phe Phe Gln Tyr Arg
   275 280 285
   Val Ser His Pro Val Arg Asp Ala Tyr Gly Leu Trp Leu Val Ser Val
   290 295 300
   Ile Cys Glu Val Trp Phe Ala Leu Ser Trp Leu Leu Asp Gln Phe Pro
   305 310 315 320
   Lys Trp Tyr Pro Ile Asn Arg Glu Thr Tyr Leu Asp Arg Leu Ala Leu
   325 330 335
   Arg Tyr Asp Arg Glu Gly Glu Pro Ser Gln Leu Ala Pro Ile Asp Val
   340 345 350
   Phe Val Ser Thr Val Asp Pro Leu Lys Glu Pro Pro Leu Ile Thr Ala
   355 360 365
   Asn Thr Val Leu Ser Ile Leu Ser Val Asp Tyr Pro Val Asp Lys Val
   370 375 380
   Ser Cys Tyr Val Ser Asp Asp Gly Ser Ala Met Leu Thr Phe Glu Ser
   385 390 395 400
   Leu Ser Glu Thr Ala Glu Phe Ala Arg Lys Trp Val Pro Phe Cys Lys
   405 410 415
   Lys His Asn Ile Glu Pro Arg Ala Pro Glu Phe Tyr Phe Ala Gln Lys
   420 425 430
   Ile Asp Tyr Leu Lys Asp Lys Ile Gln Pro Ser Phe Val Lys Glu Arg
   435 440 445
   Arg Ala Met Lys Arg Glu Tyr Glu Glu Phe Lys Val Arg Ile Asn Ala
   450 455 460
   Leu Val Ala Lys Ala Gln Lys Val Pro Glu Glu Gly Trp Thr Met Ala
   465 470 475 480
   Asp Gly Thr Ala Trp Pro Gly Asn Asn Pro Arg Asp His Pro Gly Met
   485 490 495
   Ile Gln Val Phe Leu Gly His Ser Gly Gly Leu Asp Thr Asp Gly Asn
                       500                 505                 510
   Glu Leu Pro Arg Leu Val Tyr Val Ser Arg Glu Lys Arg Pro Gly Phe
               515                 520                 525
   Gln His His Lys Lys Ala Gly Ala Met Asn Ala Leu Ile Arg Val Ser
       530                 535                 540
   Ala Val Leu Thr Asn Gly Ala Tyr Leu Leu Asn Val Asp Cys Asp His
   545                 550                 555                 560
   Tyr Phe Asn Ser Ser Lys Ala Leu Arg Glu Ala Met Cys Phe Met Met
                   565                 570                 575
   Asp Pro Ala Leu Gly Arg Lys Thr Cys Tyr Val Gln Phe Pro Gln Arg
               580                 585                 590
   Phe Asp Gly Ile Asp Leu His Asp Arg Tyr Ala Asn Arg Asn Ile Val
           595                 600                 605
   Phe Phe Asp Ile Asn Met Lys Gly Leu Asp Gly Ile Gln Gly Pro Val
       610                 615                 620
   Tyr Val Gly Thr Gly Cys Cys Phe Asn Arg Gln Ala Leu Tyr Gly Tyr
   625                 630                 635                 640
   Asp Pro Val Leu Thr Glu Ala Asp Leu Glu Pro Asn Ile Val Ile Lys
                   645                 650                 655
   Ser Cys Cys Gly Arg Arg Lys Lys Lys Asn Lys Ser Tyr Met Asp Ser
               660                 665                 670
   Gln Ser Arg Ile Met Lys Arg Thr Glu Ser Ser Ala Pro Ile Phe Asn
           675                 680                 685
   Met Glu Asp Ile Glu Glu Gly Ile Glu Gly Tyr Glu Asp Glu Arg Ser
       690                 695                 700
   Val Leu Met Ser Gln Arg Lys Leu Glu Lys Arg Phe Gly Gln Ser Pro
   705                 710                 715                 720
   Ile Phe Ile Ala Ser Thr Phe Met Thr Gln Gly Gly Ile Pro Pro Ser
                   725                 730                 735
   Thr Asn Pro Ala Ser Leu Leu Lys Glu Ala Ile His Val Ile Ser Cys
               740                 745                 750
   Gly Tyr Glu Asp Lys Thr Glu Trp Gly Lys Glu Ile Gly Trp Ile Tyr
           755                 760                 765
   Gly Ser Val Thr Glu Asp Ile Leu Thr Gly Phe Lys Met His Ala Arg
       770                 775                 780
   Gly Trp Gln Ser Ile Tyr Cys Met Pro Pro Arg Pro Cys Phe Lys Gly
   785                 790                 795                 800
   Ser Ala Pro Ile Asn Leu Ser Asp Arg Leu Asn Gln Val Leu Arg Trp
                   805                 810                 815
   Ala Leu Gly Ser Val Glu Ile Leu Leu Ser Arg His Cys Pro Ile Trp
               820                 825                 830
   Tyr Gly Tyr Asn Gly Arg Leu Lys Leu Leu Glu Arg Leu Ala Tyr Ile
           835                 840                 845
   Asn Thr Ile Val Tyr Pro Ile Thr Ser Ile Pro Leu Ile Ala Tyr Cys
       850                 855                 860
   Val Leu Pro Ala Ile Cys Leu Leu Thr Asn Lys Phe Ile Ile Pro Glu
   865                 870                 875                 880
   Ile Ser Asn Tyr Ala Gly Met Phe Phe Ile Leu Leu Phe Ala Ser Ile
                   885                 890                 895
   Phe Ala Thr Gly Ile Leu Glu Leu Arg Trp Ser Gly Val Gly Ile Glu
               900                 905                 910
   Asp Trp Trp Arg Asn Glu Gln Phe Trp Val Ile Gly Gly Thr Ser Ala
           915                 920                 925
   His Leu Phe Ala Val Phe Gln Gly Leu Leu Lys Val Leu Ala Gly Ile
       930                 935                 940
   Asp Thr Asn Phe Thr Val Thr Ser Lys Ala Ser Asp Glu Asp Gly Asp
   945                 950                 955                 960
   Phe Ala Glu Leu Tyr Val Phe Lys Trp Thr Ser Leu Leu Ile Pro Pro
                       965                 970                 975
   Thr Thr Val Leu Val Ile Asn Leu Val Gly Met Val Ala Gly Ile Ser
                   980                 985                 990
   Tyr Ala Ile Asn Ser Gly Tyr Gln Ser Trp Gly Pro Leu Phe Gly Lys
               995                 1000                1005
   Leu Phe Phe Ser Ile Trp Val Ile Leu His Leu Tyr Pro Phe Leu Lys
       1010                1015                1020
   Gly Leu Met Gly Arg Gln Asn Arg Thr Pro Thr Ile Val Ile Val Trp
   1025                1030                1035                1040
   Ser Ile Leu Leu Ala Ser Ile Phe Ser Leu Leu Trp Val Lys Ile Asp
                   1045                1050                1055
   Pro Phe Ile Ser Pro Thr Gln Lys Ala Ala Ala Leu Gly Gln Cys Gly
               1060                1065                1070
   Val Asn Cys
           1075
<210> 11
   <211> 25
   <212> DNA
   <213> Zea mays
<400> 11
   atggcggcca acaaggggat ggtgg
   25
<210> 12
   <211> 25
   <212> DNA
   <213> Zea mays
<400> 12
   tcagcagttg acgccacatt gcccc
   25
<210> 13
   <211> 3725
   <212> DNA
   <213> Zea mays
<220>
   <221> CDS
   <222> (179)...(3398)
<400> 13
   gcagcagcag caccaccact gcgcggcatt gcagcgagca agcgggaggg atctggggca    60
   tggtggcggt cgctgccgct gccgctcgga tctagagggc cgcacgggct gattgccctc   120
   cgccggcctc gtcggtgtcg gtggagtgtg aatcggtgtg tgtaggagga gcgcggag     178
   atg gcg gcc aac aag ggg atg gtg gca ggc tct cac aac cgc aac gag     226
   Met Ala Ala Asn Lys Gly Met Val Ala Gly Ser His Asn Arg Asn Glu
    1               5                  10                  15
   ttc gtc atg atc cgc cac gac ggc gac gcg cct gtc ccg gct aag ccc     274
   Phe Val Met Ile Arg His Asp Gly Asp Ala Pro Val Pro Ala Lys Pro
               20                  25                  30
   acg aag agt gcg aat ggg cag gtc tgc cag att tgt ggc gac act gtt     322
   Thr Lys Ser Ala Asn Gly Gln Val Cys Gln Ile Cys Gly Asp Thr Val
           35                  40                  45
   ggc gtt tca gcc act ggt gat gtc ttt gtt gcc tgc aat gag tgt gcc      370
   Gly Val Ser Ala Thr Gly Asp Val Phe Val Ala Cys Asn Glu Cys Ala
        50                  55                  60
   ttc cct gtc tgc cgc cct tgc tat gag tac gag cgc aag gaa ggg aac      418
   Phe Pro Val Cys Arg Pro Cys Tyr Glu Tyr Glu Arg Lys Glu Gly Asn
    65                  70                  75                  80
   caa tgc tgc cct cag tgc aag act aga tac aag aga cag aaa ggt agc      466
   Gln Cys Cys Pro Gln Cys Lys Thr Arg Tyr Lys Arg Gln Lys Gly Ser
                    85                  90                  95
   cct cga gtt cat ggt gat gat gag gag gaa gat gtt gat gac ctg gac      514
   Pro Arg Val His Gly Asp Asp Glu Glu Glu Asp Val Asp Asp Leu Asp
               100                 105                 110
   aat gaa ttc aac tat aag caa ggc aat ggg aag ggc cca gag tgg cag      562
   Asn Glu Phe Asn Tyr Lys Gln Gly Asn Gly Lys Gly Pro Glu Trp Gln
           115                 120                 125
   ctt caa gga gat gac gct gat ctg tct tca tct gct cgc cat gac cca      610
   Leu Gln Gly Asp Asp Ala Asp Leu Ser Ser Ser Ala Arg His Asp Pro
       130                 135                 140
   cac cat cgg att cca cgc ctt aca agt gga caa cag ata tct gga gag      658
   His His Arg Ile Pro Arg Leu Thr Ser Gly Gln Gln Ile Ser Gly Glu
   145                 150                 155                 160
   atc cct gat gca tcc cct gac cgt cat tct atc cgc agt cca aca tcg      706
   Ile Pro Asp Ala Ser Pro Asp Arg His Ser Ile Arg Ser Pro Thr Ser
                   165                 170                 175
   agc tat gtt gat cca agc gtt cca gtt cct gtg agg att gtg gac ccc      754
   Ser Tyr Val Asp Pro Ser Val Pro Val Pro Val Arg Ile Val Asp Pro
               180                 185                 190
   tcg aag gac ttg aat tcc tat ggg ctt aat agt gtt gac tgg aag gaa      802
   Ser Lys Asp Leu Asn Ser Tyr Gly Leu Asn Ser Val Asp Trp Lys Glu
           195                 200                 205
   aga gtt gag agc tgg agg gtt aaa cag gac aaa aat atg ttg caa gtg      850
   Arg Val Glu Ser Trp Arg Val Lys Gln Asp Lys Asn Met Leu Gln Val
       210                 215                 220
   act aat aaa tat cca gag gct aga gga gac atg gag ggg act ggc tca      898
   Thr Asn Lys Tyr Pro Glu Ala Arg Gly Asp Met Glu Gly Thr Gly Ser
   225                 230                 235                 240
   aat gga gaa gat atg caa atg gtt gat gat gca cgc cta cct ttg agc      946
   Asn Gly Glu Asp Met Gln Met Val Asp Asp Ala Arg Leu Pro Leu Ser
                   245                 250                 255
   cgc att gtg cca att tcc tca aac cag ctc aac ctt tac cgg ata gta      994
   Arg Ile Val Pro Ile Ser Ser Asn Gln Leu Asn Leu Tyr Arg Ile Val
               260                 265                 270
   atc att ctc cgt ctt atc atc ctg tgc ttc ttc ttc caa tat cgt atc     1042
   Ile Ile Leu Arg Leu Ile Ile Leu Cys Phe Phe Phe Gln Tyr Arg Ile
           275                 280                 285
   agt cat cca gtg cgt aat gct tat gga ttg tgg cta gta tct gtt atc      1090
   Ser His Pro Val Arg Asn Ala Tyr Gly Leu Trp Leu Val Ser Val Ile
       290                 295                 300
   tgt gag gtc tgg ttt gcc ttg tcc tgg ctt cta gat cag ttc cca aaa      1138
   Cys Glu Val Trp Phe Ala Leu Ser Trp Leu Leu Asp Gln Phe Pro Lys
   305                 310                 315                 320
   tgg tat cca atc aac cgt gag aca tat ctc gac agg ctt gca ttg agg      1186
   Trp Tyr Pro Ile Asn Arg Glu Thr Tyr Leu Asp Arg Leu Ala Leu Arg
                   325                 330                 335
   tat gat aga gag gga gag cca tca cag ctg gct ccc att gat gtc ttt      1234
   Tyr Asp Arg Glu Gly Glu Pro Ser Gln Leu Ala Pro Ile Asp Val Phe
               340                 345                 350
   gtc agt aca gtg gat cca ttg aag gaa cct cca ctg atc aca gcc aac      1282
   Val Ser Thr Val Asp Pro Leu Lys Glu Pro Pro Leu Ile Thr Ala Asn
           355                 360                 365
   act gtt ttg tcc att ctt gct gtg gat tac cct gtt gac aaa gtg tca      1330
   Thr Val Leu Ser Ile Leu Ala Val Asp Tyr Pro Val Asp Lys Val Ser
       370                 375                 380
   tgc tat gtt tct gat gat ggc tca gct atg ctg act ttt gag tct ctc      1378
   Cys Tyr Val Ser Asp Asp Gly Ser Ala Met Leu Thr Phe Glu Ser Leu
   385                 390                 395                 400
   tct gaa act gcc gaa ttt gct aga aag tgg gtt ccc ttt tgt aag aag      1426
   Ser Glu Thr Ala Glu Phe Ala Arg Lys Trp Val Pro Phe Cys Lys Lys
                   405                 410                 415
   cac aat att gaa cca aga gct cca gaa ttt tac ttt gct caa aaa ata      1474
   His Asn Ile Glu Pro Arg Ala Pro Glu Phe Tyr Phe Ala Gln Lys Ile
               420                 425                 430
   gat tac ctg aag gac aaa att caa cct tca ttt gtt aag gaa aga cga      1522
   Asp Tyr Leu Lys Asp Lys Ile Gln Pro Ser Phe Val Lys Glu Arg Arg
           435                 440                 445
   gca atg aag aga gag tat gaa gaa ttc aaa ata aga atc aat gcc ctt      1570
   Ala Met Lys Arg Glu Tyr Glu Glu Phe Lys Ile Arg Ile Asn Ala Leu
       450                 455                 460
   gtt gcc aaa gca cag aaa gtg cct gaa gag ggg tgg acc atg gct gat      1618
   Val Ala Lys Ala Gln Lys Val Pro Glu Glu Gly Trp Thr Met Ala Asp
   465                 470                 475                 480
   gga act gct tgg cct ggg aat aac cct agg gac cat cct ggc atg att      1666
   Gly Thr Ala Trp Pro Gly Asn Asn Pro Arg Asp His Pro Gly Met Ile
                   485                 490                 495
   cag gtg ttc ttg ggg cac agt ggt ggg ctt gac act gat gga aat gaa      1714
   Gln Val Phe Leu Gly His Ser Gly Gly Leu Asp Thr Asp Gly Asn Glu
               500                 505                 510
   tta cca cgt ctt gtc tat gtc tct cgt gaa aag aga cca ggc ttt cag      1762
   Leu Pro Arg Leu Val Tyr Val Ser Arg Glu Lys Arg Pro Gly Phe Gln
           515                 520                 525
   cat cac aag aag gct ggt gca atg aat gca ctg att cgt gta tct gct      1810
   His His Lys Lys Ala Gly Ala Met Asn Ala Leu Ile Arg Val Ser Ala
       530                 535                 540
   gtg ctg aca aat ggt gcc tat ctt ctc aat gtg gat tgt gac cat tac      1858
   Val Leu Thr Asn Gly Ala Tyr Leu Leu Asn Val Asp Cys Asp His Tyr
   545                 550                 555                 560
   ttc aat agc agc aaa gct ctt aga gaa gca atg tgc ttc atg atg gat      1906
   Phe Asn Ser Ser Lys Ala Leu Arg Glu Ala Met Cys Phe Met Met Asp
                   565                 570                 575
   cca gct cta gga agg aaa act tgt tat gta caa ttt cca caa aga ttt      1954
   Pro Ala Leu Gly Arg Lys Thr Cys Tyr Val Gln Phe Pro Gln Arg Phe
               580                 585                 590
   gat ggc att gac ttg cac gat cga tat gct aat agg aac ata gtc ttc      2002
   Asp Gly Ile Asp Leu His Asp Arg Tyr Ala Asn Arg Asn Ile Val Phe
           595                 600                 605
   ttt gat atc aac atg aaa ggt cta gat ggc att cag ggt cca gtc tat      2050
   Phe Asp Ile Asn Met Lys Gly Leu Asp Gly Ile Gln Gly Pro Val Tyr
       610                 615                 620
   gtg gga aca gga tgc tgt ttc aat agg cag gct ttg tat gga tat gat      2098
   Val Gly Thr Gly Cys Cys Phe Asn Arg Gln Ala Leu Tyr Gly Tyr Asp
   625                 630                 635                 640
   cct gtt ttg act gaa gct gat ctg gaa cct aac att gtt gtt aag agc      2146
   Pro Val Leu Thr Glu Ala Asp Leu Glu Pro Asn Ile Val Val Lys Ser
                   645                 650                 655
   tgc tgt ggt aga agg aag aga aag aac aag agt tat atg gat agt caa      2194
   Cys Cys Gly Arg Arg Lys Arg Lys Asn Lys Ser Tyr Met Asp Ser Gln
               660                 665                 670
   agc cgt att atg aag aga aca gaa tct tca gct ccc atc ttt aac atg      2242
   Ser Arg Ile Met Lys Arg Thr Glu Ser Ser Ala Pro Ile Phe Asn Met
           675                 680                 685
   gaa gac atc gag gag ggt att gaa ggt tat gag gat gaa agg tca gtg      2290
   Glu Asp Ile Glu Glu Gly Ile Glu Gly Tyr Glu Asp Glu Arg Ser Val
       690                 695                 700
   ctt atg tcc cag agg aaa ttg gag aaa cgc ttt ggt cag tct cca atc      2338
   Leu Met Ser Gln Arg Lys Leu Glu Lys Arg Phe Gly Gln Ser Pro Ile
   705                 710                 715                 720
   ttc att gca tcc acc ttt atg act caa ggt ggc ata cca cct tca aca      2386
   Phe Ile Ala Ser Thr Phe Met Thr Gln Gly Gly Ile Pro Pro Ser Thr
                   725                 730                 735
   aac cca gct tct cta ctg aag gaa gct atc cat gtt atc agc tgt ggg      2434
   Asn Pro Ala Ser Leu Leu Lys Glu Ala Ile His Val Ile Ser Cys Gly
               740                 745                 750
   tac gag gac aaa act gaa tgg gga aaa gag att ggc tgg atc tat ggt      2482
   Tyr Glu Asp Lys Thr Glu Trp Gly Lys Glu Ile Gly Trp Ile Tyr Gly
           755                 760                 765
   tca gtt aca gag gat att ctg act ggg ttt aaa atg cat gca aga ggc      2530
   Ser Val Thr Glu Asp Ile Leu Thr Gly Phe Lys Met His Ala Arg Gly
       770                 775                 780
   tgg caa tca atc tac tgc atg cca cca cga cct tgt ttc aag ggt tct      2578
   Trp Gln Ser Ile Tyr Cys Met Pro Pro Arg Pro Cys Phe Lys Gly Ser
   785                 790                 795                 800
   gca cca atc aat ctt tct gat cgt ctt aat cag gtg ctc cgt tgg gct      2626
   Ala Pro Ile Asn Leu Ser Asp Arg Leu Asn Gln Val Leu Arg Trp Ala
                   805                 810                 815
   ctt ggg tca gtg gaa att ctg ctt agc aga cat tgt cct ata tgg tat      2674
   Leu Gly Ser Val Glu Ile Leu Leu Ser Arg His Cys Pro Ile Trp Tyr
               820                 825                 830
   ggc tac aat ggg cga ttg aag ctt ttg gag agg ctg gct tac att aac      2722
   Gly Tyr Asn Gly Arg Leu Lys Leu Leu Glu Arg Leu Ala Tyr Ile Asn
           835                 840                 845
   acc att gtt tat cca atc aca tct gtt ccg ctt atc gcc tat tgt gtg      2770
   Thr Ile Val Tyr Pro Ile Thr Ser Val Pro Leu Ile Ala Tyr Cys Val
       850                 855                 860
   ctt cct gct atc tgt ctt ctt acc aat aaa ttt atc att cct gag att      2818
   Leu Pro Ala Ile Cys Leu Leu Thr Asn Lys Phe Ile Ile Pro Glu Ile
   865                 870                 875                 880
   agt aat tat gct gga atg ttc ttc att ctt ctt ttt gcc tcc att ttc      2866
   Ser Asn Tyr Ala Gly Met Phe Phe Ile Leu Leu Phe Ala Ser Ile Phe
                   885                 890                 895
   gca act ggt ata ttg gag ctc aga tgg agt ggt gtt ggc att gaa gat      2914
   Ala Thr Gly Ile Leu Glu Leu Arg Trp Ser Gly Val Gly Ile Glu Asp
               900                 905                 910
   tgg tgg aga aat gag cag ttt tgg gtt att ggt ggc acc tct gcc cat      2962
   Trp Trp Arg Asn Glu Gln Phe Trp Val Ile Gly Gly Thr Ser Ala His
           915                 920                 925
   ctc ttc gcg gtg ttc cag ggt ctg ctg aaa gtg ttg gct ggg att gat      3010
   Leu Phe Ala Val Phe Gln Gly Leu Leu Lys Val Leu Ala Gly Ile Asp
       930                 935                 940
   acc aac ttc aca gtt acc tca aag gca tct gat gag gat ggc gac ttt      3058
   Thr Asn Phe Thr Val Thr Ser Lys Ala Ser Asp Glu Asp Gly Asp Phe
   945                 950                 955                 960
   gct gag cta tat gtg ttc aag tgg acc agt ttg ctc atc cct ccg acc      3106
   Ala Glu Leu Tyr Val Phe Lys Trp Thr Ser Leu Leu Ile Pro Pro Thr
                   965                 970                 975
   act gtt ctt gtc att aac ctg gtc gga atg gtg gca gga att tcg tat     3154
   Thr Val Leu Val Ile Asn Leu Val Gly Met Val Ala Gly Ile Ser Tyr
               980                 985                 990
   gcc att aac agc ggc tac caa tcc tgg ggt ccg ctc ttt gga aag ctg     3202
   Ala Ile Asn Ser Gly Tyr Gln Ser Trp Gly Pro Leu Phe Gly Lys Leu
           995                 1000                1005
   ttc ttc tcg atc tgg gtg atc ctc cat ctc tac ccc ttc ctc aag ggt     3250
   Phe Phe Ser Ile Trp Val Ile Leu His Leu Tyr Pro Phe Leu Lys Gly
       1010                1015                1020
   ctc atg ggc agg cag aac cgc acg cca aca atc gtc atc gtt tgg tcc     3298
   Leu Met Gly Arg Gln Asn Arg Thr Pro Thr Ile Val Ile Val Trp Ser
   1025                1030                1035                1040
   atc ctc ctt gcg tct atc ttc tcc ttg ctg tgg gtg aag atc gat cct     3346
   Ile Leu Leu Ala Ser Ile Phe Ser Leu Leu Trp Val Lys Ile Asp Pro
                   1045                1050                1055
   ttc atc tcc ccg aca cag aaa gct gcc gcc ttg ggg caa tgt ggt gtg     3394
   Phe Ile Ser Pro Thr Gln Lys Ala Ala Ala Leu Gly Gln Cys Gly Val
               1060                1065                1070
   aac t gctgatccag attgtgactc ttatctgaag aggctcagcc aaagatctgc       3448
   Asn
   cccctcgtgt aaatacctga gggggctaga tgggaatttt ttgttgtaga tgaggatgga  3508
   tctgcatcca agttatgcct ctgtttatta gcttcttcgg tgccggtgct gctgcagaca  3568
   atcatggagc ctttctacct tgcttgtagt gctggccagc agcgtaaatt gtgaattctg  3628
   cattttttta tacgtggtgt ttattgtttt agagtaaatt atcatttgtt tgaggtaact   3688
   attcacacga actatatggc aatgctgtta tttaaaa                            3725
<210> 14
   <211> 1074
   <212> PRT
   <213> Zea mays
<400> 14
   Met Ala Ala Asn Lys Gly Met Val Ala Gly Ser His Asn Arg Asn Glu
   1               5                   10                  15
   Phe Val Met Ile Arg His Asp Gly Asp Ala Pro Val Pro Ala Lys Pro
               20                  25                  30
   Thr Lys Ser Ala Asn Gly Gln Val Cys Gln Ile Cys Gly Asp Thr Val
           35                  40                  45
   Gly Val Ser Ala Thr Gly Asp Val Phe Val Ala Cys Asn Glu Cys Ala
       50                  55                  60
   Phe Pro Val Cys Arg Pro Cys Tyr Glu Tyr Glu Arg Lys Glu Gly Asn
   65                  70                  75                  80
   Gln Cys Cys Pro Gln Cys Lys Thr Arg Tyr Lys Arg Gln Lys Gly Ser
                   85                  90                  95
   Pro Arg Val His Gly Asp Asp Glu Glu Glu Asp Val Asp Asp Leu Asp
               100                 105                 110
   Asn Glu Phe Asn Tyr Lys Gln Gly Asn Gly Lys Gly Pro Glu Trp Gln
           115                 120                 125
   Leu Gln Gly Asp Asp Ala Asp Leu Ser Ser Ser Ala Arg His Asp Pro
           130                 135                 140
   His His Arg Ile Pro Arg Leu Thr Ser Gly Gln Gln Ile Ser Gly Glu
   145                 150                 155                 160
   Ile Pro Asp Ala Ser Pro Asp Arg His Ser Ile Arg Ser Pro Thr Ser
                   165                 170                 175
   Ser Tyr Val Asp Pro Ser Val Pro Val Pro Val Arg Ile Val Asp Pro
               180                 185                 190
   Ser Lys Asp Leu Asn Ser Tyr Gly Leu Asn Ser Val Asp Trp Lys Glu
           195                 200                 205
   Arg Val Glu Ser Trp Arg Val Lys Gln Asp Lys Asn Met Leu Gln Val
       210                 215                 220
   Thr Asn Lys Tyr Pro Glu Ala Arg Gly Asp Met Glu Gly Thr Gly Ser
   225                 230                 235                 240
   Asn Gly Glu Asp Met Gln Met Val Asp Asp Ala Arg Leu Pro Leu Ser
                   245                 250                 255
   Arg Ile Val Pro Ile Ser Ser Asn Gln Leu Asn Leu Tyr Arg Ile Val
               260                 265                 270
   Ile Ile Leu Arg Leu Ile Ile Leu Cys Phe Phe Phe Gln Tyr Arg Ile
           275                 280                 285
   Ser His Pro Val Arg Asn Ala Tyr Gly Leu Trp Leu Val Ser Val Ile
       290                 295                 300
   Cys Glu Val Trp Phe Ala Leu Ser Trp Leu Leu Asp Gln Phe Pro Lys
   305                 310                 315                 320
   Trp Tyr Pro Ile Asn Arg Glu Thr Tyr Leu Asp Arg Leu Ala Leu Arg
                   325                 330                 335
   Tyr Asp Arg Glu Gly Glu Pro Ser Gln Leu Ala Pro Ile Asp Val Phe
               340                 345                 350
   Val Ser Thr Val Asp Pro Leu Lys Glu Pro Pro Leu Ile Thr Ala Asn
           355                 360                 365
   Thr Val Leu Ser Ile Leu Ala Val Asp Tyr Pro Val Asp Lys Val Ser
       370                 375                 380
   Cys Tyr Val Ser Asp Asp Gly Ser Ala Met Leu Thr Phe Glu Ser Leu
   385                 390                 395                 400
   Ser Glu Thr Ala Glu Phe Ala Arg Lys Trp Val Pro Phe Cys Lys Lys
                   405                 410                 415
   His Asn Ile Glu Pro Arg Ala Pro Glu Phe Tyr Phe Ala Gln Lys Ile
               420                 425                 430
   Asp Tyr Leu Lys Asp Lys Ile Gln Pro Ser Phe Val Lys Glu Arg Arg
           435                 440                 445
   Ala Met Lys Arg Glu Tyr Glu Glu Phe Lys Ile Arg Ile Asn Ala Leu
       450                 455                 460
   Val Ala Lys Ala Gln Lys Val Pro Glu Glu Gly Trp Thr Met Ala Asp
   465                 470                 475                 480
   Gly Thr Ala Trp Pro Gly Asn Asn Pro Arg Asp His Pro Gly Met Ile
                   485                 490                 495
   Gln Val Phe Leu Gly His Ser Gly Gly Leu Asp Thr Asp Gly Asn Glu
               500                 505                 510
   Leu Pro Arg Leu Val Tyr Val Ser Arg Glu Lys Arg Pro Gly Phe Gln
           515                 520                 525
   His His Lys Lys Ala Gly Ala Met Asn Ala Leu Ile Arg Val Ser Ala
       530                 535                 540
   Val Leu Thr Asn Gly Ala Tyr Leu Leu Asn Val Asp Cys Asp His Tyr
   545                 550                 555                 560
   Phe Asn Ser Ser Lys Ala Leu Arg Glu Ala Met Cys Phe Met Met Asp
                   565                 570                 575
   Pro Ala Leu Gly Arg Lys Thr Cys Tyr Val Gln Phe Pro Gln Arg Phe
               580                 585                 590
   Asp Gly Ile Asp Leu His Asp Arg Tyr Ala Asn Arg Asn Ile Val Phe
   595 600 605
   Phe Asp Ile Asn Met Lys Gly Leu Asp Gly Ile Gln Gly Pro Val Tyr
   610 615 620
   Val Gly Thr Gly Cys Cys Phe Asn Arg Gln Ala Leu Tyr Gly Tyr Asp
   625 630 635 640
   Pro Val Leu Thr Glu Ala Asp Leu Glu Pro Asn Ile Val Val Lys Ser
   645 650 655
   Cys Cys Gly Arg Arg Lys Arg Lys Asn Lys Ser Tyr Met Asp Ser Gln
   660 665 670
   Ser Arg Ile Met Lys Arg Thr Glu Ser Ser Ala Pro Ile Phe Asn Met
   675 680 685
   Glu Asp Ile Glu Glu Gly Ile Glu Gly Tyr Glu Asp Glu Arg Ser Val
   690 695 700
   Leu Met Ser Gln Arg Lys Leu Glu Lys Arg Phe Gly Gln Ser Pro Ile
   705 710 715 720
   Phe Ile Ala Ser Thr Phe Met Thr Gln Gly Gly Ile Pro Pro Ser Thr
   725 730 735
   Asn Pro Ala Ser Leu Leu Lys Glu Ala Ile His Val Ile Ser Cys Gly
   740 745 750
   Tyr Glu Asp Lys Thr Glu Trp Gly Lys Glu Ile Gly Trp Ile Tyr Gly
   755 760 765
   Ser Val Thr Glu Asp Ile Leu Thr Gly Phe Lys Met His Ala Arg Gly
   770 775 780
   Trp Gln Ser Ile Tyr Cys Met Pro Pro Arg Pro Cys Phe Lys Gly Ser
   785 790 795 800
   Ala Pro Ile Asn Leu Ser Asp Arg Leu Asn Gln Val Leu Arg Trp Ala
   805 810 815
   Leu Gly Ser Val Glu Ile Leu Leu Ser Arg His Cys Pro Ile Trp Tyr
   820 825 830
   Gly Tyr Asn Gly Arg Leu Lys Leu Leu Glu Arg Leu Ala Tyr Ile Asn
   835 840 845
   Thr Ile Val Tyr Pro Ile Thr Ser Val Pro Leu Ile Ala Tyr Cys Val
   850 855 860
   Leu Pro Ala Ile Cys Leu Leu Thr Asn Lys Phe Ile Ile Pro Glu Ile
   865 870 875 880
   Ser Asn Tyr Ala Gly Met Phe Phe Ile Leu Leu Phe Ala Ser Ile Phe
   885 890 895
   Ala Thr Gly Ile Leu Glu Leu Arg Trp Ser Gly Val Gly Ile Glu Asp
   900 905 910
   Trp Trp Arg Asn Glu Gln Phe Trp Val Ile Gly Gly Thr Ser Ala His
   915 920 925
   Leu Phe Ala Val Phe Gln Gly Leu Leu Lys Val Leu Ala Gly Ile Asp
   930 935 940
   Thr Asn Phe Thr Val Thr Ser Lys Ala Ser Asp Glu Asp Gly Asp Phe
   945 950 955 960
   Ala Glu Leu Tyr Val Phe Lys Trp Thr Ser Leu Leu Ile Pro Pro Thr
   965 970 975
   Thr Val Leu Val Ile Asn Leu Val Gly Met Val Ala Gly Ile Ser Tyr
   980 985 990
   Ala Ile Asn Ser Gly Tyr Gln Ser Trp Gly Pro Leu Phe Gly Lys Leu
   995 1000 1005
   Phe Phe Ser Ile Trp Val Ile Leu His Leu Tyr Pro Phe Leu Lys Gly
   1010 1015 1020
   Leu Met Gly Arg Gln Asn Arg Thr Pro Thr Ile Val Ile Val Trp Ser
   1025 1030 1035 1040
   Ile Leu Leu Ala Ser Ile Phe Ser Leu Leu Trp Val Lys Ile Asp Pro
   1045 1050 1055
   Phe Ile Ser Pro Thr Gln Lys Ala Ala Ala Leu Gly Gln Cys Gly Val
   1060 1065 1070
   Asn Cys
<210> 15
   <211> 25
   <212> DNA
   <213> Zea mays
<400> 15
   atggcggcca acaaggggat ggtgg
   25
<210> 16
   <211> 25
   <212> DNA
   <213> Zea mays
<400> 16
   tcagcagttc acaccacatt gcccc
   25
<210> 17
   <211> 3969
   <212> DNA
   <213> Zea mays
<220>
   <221> CDS
   <222> (144)...(3399)
<400> 17
   cttctccctc gtcggtgcgg cgtggcgcgg ctcggcgttc ggtgagaaac cactcggggg    60
   atgaggatct gctgctagag tgagaggagc tacggtcagt atcctctgcc ttcgtcggcg   120
   gcggaagtgg aggggaggaa gcg atg gag gcg agc gcc ggg ctg gtg gcc ggc   173
                             Met Glu Ala Ser Ala Gly Leu Val Ala Gly
                              1               5                  10
   tcc cac aac cgc aac gag ctc gtc gtc atc cgc cgc gac ggc gat ccc     221
   Ser His Asn Arg Asn Glu Leu Val Val Ile Arg Arg Asp Gly Asp Pro
                   15                  20                  25
   ggg ccg aag ccg ccg cgg gag cag aac ggg cag gtg tgc cag att tgc     269
   Gly Pro Lys Pro Pro Arg Glu Gln Asn Gly Gln Val Cys Gln Ile Cys
               30                  35                  40
   ggc gac gac gtc ggc ctt gcc ccc ggc ggg gac ccc ttc gtg gcg tgc     317
   Gly Asp Asp Val Gly Leu Ala Pro Gly Gly Asp Pro Phe Val Ala Cys
           45                  50                  55
   aac gag tgc gcc ttc ccc gtc tgc cgg gac tgc tac gaa tac gag cgc     365
   Asn Glu Cys Ala Phe Pro Val Cys Arg Asp Cys Tyr Glu Tyr Glu Arg
       60                  65                  70
   cgg gag ggc acg cag aac tgc ccc cag tgc aag act cga tac aag cgc     413
   Arg Glu Gly Thr Gln Asn Cys Pro Gln Cys Lys Thr Arg Tyr Lys Arg
   75                  80                  85                  90
   ctc aag ggc tgc caa cgt gtg acc ggt gac gag gag gag gac ggc gtc      461
   Leu Lys Gly Cys Gln Arg Val Thr Gly Asp Glu Glu Glu Asp Gly Val
                    95                  100                 105
   gat gac ctg gac aac gag ttc aac tgg gac ggc cat gac tcg cag tct      509
   Asp Asp Leu Asp Asn Glu Phe Asn Trp Asp Gly His Asp Ser Gln Ser
               110                 115                 120
   gtg gcc gag tcc atg ctc tac ggc cac atg agc tac ggc cgt gga ggt      557
   Val Ala Glu Ser Met Leu Tyr Gly His Met Ser Tyr Gly Arg Gly Gly
           125                 130                 135
   gac cct aat ggc gcg cca caa gct ttc cag ctc aac ccc aat gtt cca      605
   Asp Pro Asn Gly Ala Pro Gln Ala Phe Gln Leu Asn Pro Asn Val Pro
       140                 145                 150
   ctc ctc acc aac ggg caa atg gtg gat gac atc cca ccg gag cag cac      653
   Leu Leu Thr Asn Gly Gln Met Val Asp Asp Ile Pro Pro Glu Gln His
   155                 160                 165                 170
   gcg ctg gtg cct tct ttc atg ggt ggt ggg gga aag agg ata cat ccc      701
   Ala Leu Val Pro Ser Phe Met Gly Gly Gly Gly Lys Arg Ile His Pro
                   175                 180                 185
   ctt cct tat gcg gat ccc agc tta cct gtg caa ccc agg tct atg gac      749
   Leu Pro Tyr Ala Asp Pro Ser Leu Pro Val Gln Pro Arg Ser Met Asp
               190                 195                 200
   cca tcc aag gat ctt gct gca tat ggg tat ggt agt gtt gct tgg aag      797
   Pro Ser Lys Asp Leu Ala Ala Tyr Gly Tyr Gly Ser Val Ala Trp Lys
           205                 210                 215
   gaa cgg atg gag aat tgg aag cag aga caa gag agg atg cac cag acg      845
   Glu Arg Met Glu Asn Trp Lys Gln Arg Gln Glu Arg Met His Gln Thr
       220                 225                 230
   ggg aat gat ggt ggt ggt gat gat ggt gac gat gct gat cta cca cta      893
   Gly Asn Asp Gly Gly Gly Asp Asp Gly Asp Asp Ala Asp Leu Pro Leu
   235                 240                 245                 250
   atg gat gaa gca aga caa caa ctg tcc agg aaa att cca ctt cca tca      941
   Met Asp Glu Ala Arg Gln Gln Leu Ser Arg Lys Ile Pro Leu Pro Ser
                   255                 260                 265
   agc cag att aat cca tat agg atg att atc att att cgg ctt gtg gtt      989
   Ser Gln Ile Asn Pro Tyr Arg Met Ile Ile Ile Ile Arg Leu Val Val
               270                 275                 280
   ttg ggg ttc ttc ttc cac tac cga gtg atg cat ccg gtg aat gat gca     1037
   Leu Gly Phe Phe Phe His Tyr Arg Val Met His Pro Val Asn Asp Ala
           285                 290                 295
   ttt gct ttg tgg ctc ata tct gtt atc tgt gaa atc tgg ttt gcc atg     1085
   Phe Ala Leu Trp Leu Ile Ser Val Ile Cys Glu Ile Trp Phe Ala Met
       300                 305                 310
   tct tgg att ctt gat caa ttc cca aag tgg ttc cct att gag aga gag     1133
   Ser Trp Ile Leu Asp Gln Phe Pro Lys Trp Phe Pro Ile Glu Arg Glu
   315                 320                 325                 330
   act tac cta gac cgg ctg tca ctg agg ttc gac aag gaa ggc cag cca      1181
   Thr Tyr Leu Asp Arg Leu Ser Leu Arg Phe Asp Lys Glu Gly Gln Pro
                   335                 340                 345
   tct caa ctt gct cca att gat ttc ttt gtc agt acg gtt gat ccc tta      1229
   Ser Gln Leu Ala Pro Ile Asp Phe Phe Val Ser Thr Val Asp Pro Leu
               350                 355                 360
   aag gaa cct cct ttg gtc aca aca aat act gtt cta tct atc ctt tcg      1277
   Lys Glu Pro Pro Leu Val Thr Thr Asn Thr Val Leu Ser Ile Leu Ser
           365                 370                 375
   gtg gat tat cct gtt gat aag gtt tct tgc tat gtt tct gat gat ggt      1325
   Val Asp Tyr Pro Val Asp Lys Val Ser Cys Tyr Val Ser Asp Asp Gly
       380                 385                 390
   gct gca atg cta acg ttt gaa gca tta tct gaa aca tct gaa ttt gca      1373
   Ala Ala Met Leu Thr Phe Glu Ala Leu Ser Glu Thr Ser Glu Phe Ala
   395                 400                 405                 410
   aag aaa tgg gtt cct ttc tgc aaa cgg tac aat att gaa cct cgc gct      1421
   Lys Lys Trp Val Pro Phe Cys Lys Arg Tyr Asn Ile Glu Pro Arg Ala
                   415                 420                 425
   cca gag tgg tac ttc caa cag aag ata gac tac ttg aaa gac aag gtg      1469
   Pro Glu Trp Tyr Phe Gln Gln Lys Ile Asp Tyr Leu Lys Asp Lys Val
               430                 435                 440
   gca gca aac ttt gtt agg gag agg aga gca atg aag aga gag tat gag      1517
   Ala Ala Asn Phe Val Arg Glu Arg Arg Ala Met Lys Arg Glu Tyr Glu
           445                 450                 455
   gaa ttc aag gtg aga atc aat gcc tta gtt gcc aaa gcc cag aaa gtt      1565
   Glu Phe Lys Val Arg Ile Asn Ala Leu Val Ala Lys Ala Gln Lys Val
       460                 465                 470
   cct gaa gaa gga tgg aca atg caa gat gga acc ccc tgg cct gga aac      1613
   Pro Glu Glu Gly Trp Thr Met Gln Asp Gly Thr Pro Trp Pro Gly Asn
   475                 480                 485                 490
   aat gtt cgt gat cat cct gga atg att cag gtc ttc ctt ggc caa agc      1661
   Asn Val Arg Asp His Pro Gly Met Ile Gln Val Phe Leu Gly Gln Ser
                   495                 500                 505
   gga ggc ctt gac tgt gag gga aat gaa ctg cca cga ttg gtt tat gtt      1709
   Gly Gly Leu Asp Cys Glu Gly Asn Glu Leu Pro Arg Leu Val Tyr Val
               510                 515                 520
   tct aga gag aaa cga cca ggc tat aac cat cat aag aaa gct ggt gct      1757
   Ser Arg Glu Lys Arg Pro Gly Tyr Asn His His Lys Lys Ala Gly Ala
           525                 530                 535
   atg aat gca ttg gtc cga gtc tct gct gta cta aca aat gct cca tat      1805
   Met Asn Ala Leu Val Arg Val Ser Ala Val Leu Thr Asn Ala Pro Tyr
       540                 545                 550
   ttg tta aac ttg gat tgt gat cac tac atc aac aac agc aag gct ata      1853
   Leu Leu Asn Leu Asp Cys Asp His Tyr Ile Asn Asn Ser Lys Ala Ile
   555                 560                 565                 570
   aag gaa gca atg tgt ttt atg atg gac cct tta cta gga aag aag gtt      1901
   Lys Glu Ala Met Cys Phe Met Met Asp Pro Leu Leu Gly Lys Lys Val
                   575                 580                 585
   tgc tat gta cag ttc cct caa aga ttt gat ggg att gat cgc cat gac      1949
   Cys Tyr Val Gln Phe Pro Gln Arg Phe Asp Gly Ile Asp Arg His Asp
               590                 595                 600
   cga tat gct aac cgg aat gtt gtc ttt ttt gat atc aac atg aaa ggt      1997
   Arg Tyr Ala Asn Arg Asn Val Val Phe Phe Asp Ile Asn Met Lys Gly
           605                 610                 615
   ttg gat ggt att cag ggt cca att tat gtt ggt act gga tgt gta ttt      2045
   Leu Asp Gly Ile Gln Gly Pro Ile Tyr Val Gly Thr Gly Cys Val Phe
       620                 625                 630
   aga agg cag gca tta tat ggt tat gat gcc ccc aaa aca aag aag cca      2093
   Arg Arg Gln Ala Leu Tyr Gly Tyr Asp Ala Pro Lys Thr Lys Lys Pro
   635                 640                 645                 650
   cca tca agg act tgc aac tgc tgg ccc aag tgg tgc ttt tgc tgt tgc      2141
   Pro Ser Arg Thr Cys Asn Cys Trp Pro Lys Trp Cys Phe Cys Cys Cys
                   655                 660                 665
   tgc ttt ggc aat agg aag caa aag aag act acc aaa ccc aaa aca gag      2189
   Cys Phe Gly Asn Arg Lys Gln Lys Lys Thr Thr Lys Pro Lys Thr Glu
               670                 675                 680
   aag aaa aag tta tta ttt ttc aag aaa gaa gag aac caa tcc cct gca      2237
   Lys Lys Lys Leu Leu Phe Phe Lys Lys Glu Glu Asn Gln Ser Pro Ala
           685                 690                 695
   tat gct ctt ggt gaa att gac gaa gct gct cca gga gct gag aat gaa      2285
   Tyr Ala Leu Gly Glu Ile Asp Glu Ala Ala Pro Gly Ala Glu Asn Glu
       700                 705                 710
   aag gcc ggt att gta aat caa caa aaa tta gaa aag aaa ttt ggc caa      2333
   Lys Ala Gly Ile Val Asn Gln Gln Lys Leu Glu Lys Lys Phe Gly Gln
   715                 720                 725                 730
   tct tct gtt ttt gtt aca tcc aca ctt ctc gag aat ggt gga acc ttg      2381
   Ser Ser Val Phe Val Thr Ser Thr Leu Leu Glu Asn Gly Gly Thr Leu
                   735                 740                 745
   aag agt gca agt cct gct tct ctt ttg aaa gaa gct ata cat gtc att      2429
   Lys Ser Ala Ser Pro Ala Ser Leu Leu Lys Glu Ala Ile His Val Ile
               750                 755                 760
   agt tgt ggt tat gaa gac aag aca gac tgg gga aaa gag att ggc tgg      2477
   Ser Cys Gly Tyr Glu Asp Lys Thr Asp Trp Gly Lys Glu Ile Gly Trp
           765                 770                 775
   atc tat gga tca gtt aca gaa gat att cta act ggt ttc aag atg cat      2525
   Ile Tyr Gly Ser Val Thr Glu Asp Ile Leu Thr Gly Phe Lys Met His
           780                 785                 790
   tgt cat ggt tgg cgg tca att tac tgc ata cct aaa cgg gtt gca ttc      2573
   Cys His Gly Trp Arg Ser Ile Tyr Cys Ile Pro Lys Arg Val Ala Phe
   795                 800                 805                 810
   aaa ggt tct gca cct ctg aat ctt tca gat cgt ctt cac cag gtg ctt      2621
   Lys Gly Ser Ala Pro Leu Asn Leu Ser Asp Arg Leu His Gln Val Leu
                   815                 820                 825
   cgg tgg gct ctt ggg tct att gag atc ttc ttc agc aat cat tgc cct      2669
   Arg Trp Ala Leu Gly Ser Ile Glu Ile Phe Phe Ser Asn His Cys Pro
               830                 835                 840
   ctt tgg tat ggg tat ggt ggc ggt ctg aaa ttt ttg gaa aga ttt tcc      2717
   Leu Trp Tyr Gly Tyr Gly Gly Gly Leu Lys Phe Leu Glu Arg Phe Ser
           845                 850                 855
   tac atc aac tcc atc gtg tat cct tgg aca tct att ccc ctc ttg gct      2765
   Tyr Ile Asn Ser Ile Val Tyr Pro Trp Thr Ser Ile Pro Leu Leu Ala
       860                 865                 870
   tac tgt aca ttg cct gcc atc tgt tta ttg aca ggg aaa ttt atc act      2813
   Tyr Cys Thr Leu Pro Ala Ile Cys Leu Leu Thr Gly Lys Phe Ile Thr
   875                 880                 885                 890
   cca gag ctg aat aat gtt gcc agc ctg tgg ttc atg tca ctt ttt atc      2861
   Pro Glu Leu Asn Asn Val Ala Ser Leu Trp Phe Met Ser Leu Phe Ile
                   895                 900                 905
   tgc att ttt gct acg agc atc cta gaa atg aga tgg agt ggt gtt gga      2909
   Cys Ile Phe Ala Thr Ser Ile Leu Glu Met Arg Trp Ser Gly Val Gly
               910                 915                 920
   att gat gac tgg tgg agg aat gag cag ttc tgg gtc att gga ggt gtg      2957
   Ile Asp Asp Trp Trp Arg Asn Glu Gln Phe Trp Val Ile Gly Gly Val
           925                 930                 935
   tcc tca cac ctc ttt gct gtg ttc cag gga ctt ctc aag gtc ata gct      3005
   Ser Ser His Leu Phe Ala Val Phe Gln Gly Leu Leu Lys Val Ile Ala
       940                 945                 950
   ggt gtt gat aca agc ttc acc gtg aca tca aag ggt gga gat gat gag      3053
   Gly Val Asp Thr Ser Phe Thr Val Thr Ser Lys Gly Gly Asp Asp Glu
   955                 960                 965                 970
   gag ttc tca gag cta tat aca ttc aaa tgg act acc tta ttg ata cct      3101
   Glu Phe Ser Glu Leu Tyr Thr Phe Lys Trp Thr Thr Leu Leu Ile Pro
                   975                 980                 985
   cct acc acc ttg ctt cta ttg aac ttc att ggt gtg gtc gct ggc gtt      3149
   Pro Thr Thr Leu Leu Leu Leu Asn Phe Ile Gly Val Val Ala Gly Val
               990                 995                 1000
   tca aat gcg atc aat aac gga tat gag tca tgg ggc ccc ctc ttt ggg      3197
   Ser Asn Ala Ile Asn Asn Gly Tyr Glu Ser Trp Gly Pro Leu Phe Gly
           1005                1010                1015
   aag cta ttc ttt gca ttt tgg gtg att gtc cat ctt tat ccc ttt ctc      3245
   Lys Leu Phe Phe Ala Phe Trp Val Ile Val His Leu Tyr Pro Phe Leu
       1020                1025                1030
   aaa ggt ttg gtt gga agg caa aac agg aca cca acg att gtc atc gtc      3293
   Lys Gly Leu Val Gly Arg Gln Asn Arg Thr Pro Thr Ile Val Ile Val
   1035                1040                1045                1050
   tgg tcc att ctg ctg gct tca atc ttc tcg ctc ctt tgg gtt cgg att      3341
   Trp Ser Ile Leu Leu Ala Ser Ile Phe Ser Leu Leu Trp Val Arg Ile
                   1055                1060                1065
   gat cct ttc ctt gcg aag gat gat ggt ccg ctt ctt gag gag tgt ggt      3389
   Asp Pro Phe Leu Ala Lys Asp Asp Gly Pro Leu Leu Glu Glu Cys Gly
               1070                1075                1080
   ttg gat tgc a actaggatgt cagtgcatca gctcccccaa tctgcatatg            3439
   Leu Asp Cys
           1085
   cttgaagtat attttctggt gtttgtcccc atattcagtg tctgtagata agagacatga     3499
   aatgtcccaa gtttcttttg atccatggtg aacctactta atatctgaga gatatactgg     3559
   gggaaaatgg aggctgcggc aatccttgtg cagttgggcc gtggaataca gcatatgcaa    3619
   gtgtttgatt gtgcagcatt ctttattact tggtcgcaat atagatgggc tgagccgaac    3679
   agcaaggtat tttgattctg cactgctccc gtgtacaaac ttggttctca ataaggcagg    3739
   caggaatgca tctgccagtg gaacagagca acctgcacat tatttatgta tgcctgttca    3799
   ttggagggct tgttcattac atgttcgtct atactagaaa aaacagaata ttagcattaa    3859
   tctatagtta attaaagtat gtaaatgcgc ctgttttttg ttgtgtactg taatcatctg     3919
   agttggtttt gtgaaaaaaa aaaaaaaaaa aaaaaaaaaa aaaaaaaaaa               3969
<210> 18
   <211> 1086
   <212> PRT
   <213> Zea mays
<400> 18 <211> 25 <212> DNA <213> Zea mays <400> 19
   atggaggcga gcgccgggct ggtgg
25 <210> 20 <211> 25 <212> DNA <213> Zea mays <400> 20
ctagttgcaa tccaaaccac actcc
25 <210> 21 <211> 3725 <212> DNA <213> Zea mays <220> <221> CDS <222> (179)...(3398) <400> 21
<210> 22
   <211> 1074
   <212> PRT
   <213> Zea mays
<400> 22
   Met Ala Ala Asn Lys Gly Met Val Ala Gly Ser His Asn Arg Asn Glu
    1               5                  10                  15
   Phe Val Met Ile Arg His Asp Gly Asp Ala Pro Val Pro Ala Lys Pro
               20                  25                  30
   Thr Lys Ser Ala Asn Gly Gln Val Cys Gln Ile Cys Gly Asp Thr Val
           35                  40                  45
   Gly Val Ser Ala Thr Gly Asp Val Phe Val Ala Cys Asn Glu Cys Ala
       50                  55                  60
   Phe Pro Val Cys Arg Pro Cys Tyr Glu Tyr Glu Arg Lys Glu Gly Asn
   65                  70                  75                  80
   Gln Cys Cys Pro Gln Cys Lys Thr Arg Tyr Lys Arg Gln Lys Gly Ser
                   85                  90                  95
   Pro Arg Val His Gly Asp Asp Glu Glu Glu Asp Val Asp Asp Leu Asp
               100                 105                 110
   Asn Glu Phe Asn Tyr Lys Gln Gly Asn Gly Lys Gly Pro Glu Trp Gln
           115                 120                 125
   Leu Gln Gly Asp Asp Ala Asp Leu Ser Ser Ser Ala Arg His Asp Pro
       130                 135                 140
   His His Arg Ile Pro Arg Leu Thr Ser Gly Gln Gln Ile Ser Gly Glu
   145                 150                 155                 160
   Ile Pro Asp Ala Ser Pro Asp Arg His Ser Ile Arg Ser Pro Thr Ser
                   165                 170                 175
   Ser Tyr Val Asp Pro Ser Val Pro Val Pro Val Arg Ile Val Asp Pro
               180                 185                 190
   Ser Lys Asp Leu Asn Ser Tyr Gly Leu Asn Ser Val Asp Trp Lys Glu
           195                 200                 205
   Arg Val Glu Ser Trp Arg Val Lys Gln Asp Lys Asn Met Leu Gln Val
       210                 215                 220
   Thr Asn Lys Tyr Pro Glu Ala Arg Gly Asp Met Glu Gly Thr Gly Ser
   225                 230                 235                 240
   Asn Gly Glu Asp Met Gln Met Val Asp Asp Ala Arg Leu Pro Leu Ser
                   245                 250                 255
   Arg Ile Val Pro Ile Ser Ser Asn Gln Leu Asn Leu Tyr Arg Ile Val
               260                 265                 270
   Ile Ile Leu Arg Leu Ile Ile Leu Cys Phe Phe Phe Gln Tyr Arg Ile
           275                 280                 285
   Ser His Pro Val Arg Asn Ala Tyr Gly Leu Trp Leu Val Ser Val Ile
       290                 295                 300
   Cys Glu Val Trp Phe Ala Leu Ser Trp Leu Leu Asp Gln Phe Pro Lys
   305                 310                 315                 320
   Trp Tyr Pro Ile Asn Arg Glu Thr Tyr Leu Asp Arg Leu Ala Leu Arg
                   325                 330                 335
   Tyr Asp Arg Glu Gly Glu Pro Ser Gln Leu Ala Pro Ile Asp Val Phe
               340                 345                 350
   Val Ser Thr Val Asp Pro Leu Lys Glu Pro Pro Leu Ile Thr Ala Asn
           355                 360                 365
   Thr Val Leu Ser Ile Leu Ala Val Asp Tyr Pro Val Asp Lys Val Ser
       370                 375                 380
   Cys Tyr Val Ser Asp Asp Gly Ser Ala Met Leu Thr Phe Glu Ser Leu
   385                 390                 395                 400
   Ser Glu Thr Ala Glu Phe Ala Arg Lys Trp Val Pro Phe Cys Lys Lys
                   405                 410                 415
   His Asn Ile Glu Pro Arg Ala Pro Glu Phe Tyr Phe Ala Gln Lys Ile
               420                 425                 430
   Asp Tyr Leu Lys Asp Lys Ile Gln Pro Ser Phe Val Lys Glu Arg Arg
           435                 440                 445
   Ala Met Lys Arg Glu Tyr Glu Glu Phe Lys Ile Arg Ile Asn Ala Leu
       450                 455                 460
   Val Ala Lys Ala Gln Lys Val Pro Glu Glu Gly Trp Thr Met Ala Asp
   465                 470                 475                 480
   Gly Thr Ala Trp Pro Gly Asn Asn Pro Arg Asp His Pro Gly Met Ile
                   485                 490                 495
   Gln Val Phe Leu Gly His Ser Gly Gly Leu Asp Thr Asp Gly Asn Glu
               500                 505                 510
   Leu Pro Arg Leu Val Tyr Val Ser Arg Glu Lys Arg Pro Gly Phe Gln
           515                 520                 525
   His His Lys Lys Ala Gly Ala Met Asn Ala Leu Ile Arg Val Ser Ala
       530                 535                 540
   Val Leu Thr Asn Gly Ala Tyr Leu Leu Asn Val Asp Cys Asp His Tyr
   545                 550                 555                 560
   Phe Asn Ser Ser Lys Ala Leu Arg Glu Ala Met Cys Phe Met Met Asp
                   565                 570                 575
   Pro Ala Leu Gly Arg Lys Thr Cys Tyr Val Gln Phe Pro Gln Arg Phe
               580                 585                 590
   Asp Gly Ile Asp Leu His Asp Arg Tyr Ala Asn Arg Asn Ile Val Phe
           595                 600                 605
   Phe Asp Ile Asn Met Lys Gly Leu Asp Gly Ile Gln Gly Pro Val Tyr
       610                 615                 620
   Val Gly Thr Gly Cys Cys Phe Asn Arg Gln Ala Leu Tyr Gly Tyr Asp
   625                 630                 635                 640
   Pro Val Leu Thr Glu Ala Asp Leu Glu Pro Asn Ile Val Val Lys Ser
                   645                 650                 655
   Cys Cys Gly Arg Arg Lys Arg Lys Asn Lys Ser Tyr Met Asp Ser Gln
               660                 665                 670
   Ser Arg Ile Met Lys Arg Thr Glu Ser Ser Ala Pro Ile Phe Asn Met
           675                 680                 685
   Glu Asp Ile Glu Glu Gly Ile Glu Gly Tyr Glu Asp Glu Arg Ser Val
       690                 695                 700
   Leu Met Ser Gln Arg Lys Leu Glu Lys Arg Phe Gly Gln Ser Pro Ile
   705                 710                 715                 720
   Phe Ile Ala Ser Thr Phe Met Thr Gln Gly Gly Ile Pro Pro Ser Thr
                   725                 730                 735
   Asn Pro Ala Ser Leu Leu Lys Glu Ala Ile His Val Ile Ser Cys Gly
   740 745 750
   Tyr Glu Asp Lys Thr Glu Trp Gly Lys Glu Ile Gly Trp Ile Tyr Gly
   755 760 765
   Ser Val Thr Glu Asp Ile Leu Thr Gly Phe Lys Met His Ala Arg Gly
   770 775 780
   Trp Gln Ser Ile Tyr Cys Met Pro Pro Arg Pro Cys Phe Lys Gly Ser
   785 790 795 800
   Ala Pro Ile Asn Leu Ser Asp Arg Leu Asn Gln Val Leu Arg Trp Ala
   805 810 815
   Leu Gly Ser Val Glu Ile Leu Leu Ser Arg His Cys Pro Ile Trp Tyr
   820 825 830
   Gly Tyr Asn Gly Arg Leu Lys Leu Leu Glu Arg Leu Ala Tyr Ile Asn
   835 840 845
   Thr Ile Val Tyr Pro Ile Thr Ser Val Pro Leu Ile Ala Tyr Cys Val
   850 855 860
   Leu Pro Ala Ile Cys Leu Leu Thr Asn Lys Phe Ile Ile Pro Glu Ile
   865 870 875 880
   Ser Asn Tyr Ala Gly Met Phe Phe Ile Leu Leu Phe Ala Ser Ile Phe
   885 890 895
   Ala Thr Gly Ile Leu Glu Leu Arg Trp Ser Gly Val Gly Ile Glu Asp
   900 905 910
   Trp Trp Arg Asn Glu Gln Phe Trp Val Ile Gly Gly Thr Ser Ala His
   915 920 925
   Leu Phe Ala Val Phe Gln Gly Leu Leu Lys Val Leu Ala Gly Ile Asp
   930 935 940
   Thr Asn Phe Thr Val Thr Ser Lys Ala Ser Asp Glu Asp Gly Asp Phe
   945 950 955 960
   Ala Glu Leu Tyr Val Phe Lys Trp Thr Ser Leu Leu Ile Pro Pro Thr
   965 970 975
   Thr Val Leu Val Ile Asn Leu Val Gly Met Val Ala Gly Ile Ser Tyr
   980 985 990
   Ala Ile Asn Ser Gly Tyr Gln Ser Trp Gly Pro Leu Phe Gly Lys Leu
   995 1000 1005
   Phe Phe Ser Ile Trp Val Ile Leu His Leu Tyr Pro Phe Leu Lys Gly
   1010 1015 1020
   Leu Met Gly Arg Gln Asn Arg Thr Pro Thr Ile Val Ile Val Trp Ser
   1025 1030 1035 1040
   Ile Leu Leu Ala Ser Ile Phe Ser Leu Leu Trp Val Lys Ile Asp Pro
   1045 1050 1055
   Phe Ile Ser Pro Thr Gln Lys Ala Ala Ala Leu Gly Gln Cys Gly Val
   1060 1065 1070
   Asn Cys
<210> 23
   <211> 25
   <212> DNA
   <213> Zea mays
<400> 23
   atggcggcca acaaggggat ggtgg
   25
<210> 24
   <211> 25
   <212> DNA
   <213> Zea mays
<400> 24
   tcagcagttc acaccacatt gcccc
   25
<210> 25
   <211> 3813
   <212> DNA
   <213> Zea mays
<220>
   <221> CDS
   <222> (215)...(3494)
<400> 25
   ccacagctca tataccaaga gccggagcag cttagcgcag cccagagcgg cgccgcgcca    60
   agcacaaccc ccacccgcca cagccgcgtg cgcatgtgag cggtcgccgc ggccgggaga   120
   ccagaggagg ggaggactac gtgcatttcg ctgtgccgcc gccgcggggt tcgtgcgcga   180
   gcgagatccg gcggggcggg gcgggggccc tgag atg gag gct agc gcg ggg ctg   235
                                         Met Glu Ala Ser Ala Gly Leu
                                          1               5
   gtg gcc ggc tcg cat aac cgg aac gag ctg gtg gtg atc cgc cgc gac     283
   Val Ala Gly Ser His Asn Arg Asn Glu Leu Val Val Ile Arg Arg Asp
           10                  15                  20
   cgc gag tcg gga gcc gcg ggc ggc ggc gcg gcg cgc cgg gcg gag gcg     331
   Arg Glu Ser Gly Ala Ala Gly Gly Gly Ala Ala Arg Arg Ala Glu Ala
       25                  30                  35
   ccg tgc cag ata tgc ggc gac gag gtc ggg gtg ggc ttc gac ggg gag     379
   Pro Cys Gln Ile Cys Gly Asp Glu Val Gly Val Gly Phe Asp Gly Glu
   40                  45                  50                  55
   ccc ttc gtg gcg tgc aac gag tgc gcc ttc ccc gtc tgc cgc gcc tgc     427
   Pro Phe Val Ala Cys Asn Glu Cys Ala Phe Pro Val Cys Arg Ala Cys
                   60                  65                  70
   tac gag tac gag cgc cgc gag ggc tcg caa gcg tgc ccg cag tgc agg     475
   Tyr Glu Tyr Glu Arg Arg Glu Gly Ser Gln Ala Cys Pro Gln Cys Arg
               75                  80                  85
   acc cgc tac aag cgc ctc aag ggc tgc ccg cgg gtg gcc ggc gac gag     523
   Thr Arg Tyr Lys Arg Leu Lys Gly Cys Pro Arg Val Ala Gly Asp Glu
           90                  95                  100
   gag gag gac ggc gtc gac gac ctg gag ggc gag ttc ggc ctg cag gac     571
   Glu Glu Asp Gly Val Asp Asp Leu Glu Gly Glu Phe Gly Leu Gln Asp
       105                 110                 115
   ggc gcc gcc cac gag gac gac ccg cag tac gtc gcc gag tcc atg ctc     619
   Gly Ala Ala His Glu Asp Asp Pro Gln Tyr Val Ala Glu Ser Met Leu
   120                 125                 130                 135
   agg gcg cag atg agc tac ggc cgc ggc ggc gac gcg cac ccc ggc ttc     667
   Arg Ala Gln Met Ser Tyr Gly Arg Gly Gly Asp Ala His Pro Gly Phe
                   140                 145                 150
   agc ccc gtc ccc aac gtg ccg ctc ctc acc aac ggc cag atg gtt gat      715
   Ser Pro Val Pro Asn Val Pro Leu Leu Thr Asn Gly Gln Met Val Asp
               155                 160                 165
   gac atc ccg ccg gag cag cac gcg ctc gtg ccg tcc tac atg agc ggc      763
   Asp Ile Pro Pro Glu Gln His Ala Leu Val Pro Ser Tyr Met Ser Gly
           170                 175                 180
   ggc ggc ggc ggg ggc aag agg atc cac ccg ctc cct ttc gca gat ccc      811
   Gly Gly Gly Gly Gly Lys Arg Ile His Pro Leu Pro Phe Ala Asp Pro
       185                 190                 195
   aac ctt cca gtg caa ccg aga tcc atg gac ccg tcc aag gat ctg gcc      859
   Asn Leu Pro Val Gln Pro Arg Ser Met Asp Pro Ser Lys Asp Leu Ala
   200                 205                 210                 215
   gcc tac gga tat ggc agc gtg gcc tgg aag gag aga atg gag ggc tgg      907
   Ala Tyr Gly Tyr Gly Ser Val Ala Trp Lys Glu Arg Met Glu Gly Trp
                   220                 225                 230
   aag cag aag cag gag cgc ctg cag cat gtc agg agc gag ggt ggc ggt      955
   Lys Gln Lys Gln Glu Arg Leu Gln His Val Arg Ser Glu Gly Gly Gly
               235                 240                 245
   gat tgg gat ggc gac gat gca gat ctg cca cta atg gat gaa gct agg     1003
   Asp Trp Asp Gly Asp Asp Ala Asp Leu Pro Leu Met Asp Glu Ala Arg
           250                 255                 260
   cag cca ttg tcc aga aaa gtc cct ata tca tca agc cga att aat ccc     1051
   Gln Pro Leu Ser Arg Lys Val Pro Ile Ser Ser Ser Arg Ile Asn Pro
       265                 270                 275
   tac agg atg att atc gtt atc cgg ttg gtg gtt ttg ggt ttc ttc ttc     1099
   Tyr Arg Met Ile Ile Val Ile Arg Leu Val Val Leu Gly Phe Phe Phe
   280                 285                 290                 295
   cac tac cga gtg atg cat ccg gcg aaa gat gca ttt gca ttg tgg ctc     1147
   His Tyr Arg Val Met His Pro Ala Lys Asp Ala Phe Ala Leu Trp Leu
                   300                 305                 310
   ata tct gta atc tgt gaa atc tgg ttt gcg atg tcc tgg att ctt gat     1195
   Ile Ser Val Ile Cys Glu Ile Trp Phe Ala Met Ser Trp Ile Leu Asp
               315                 320                 325
   cag ttc cca aag tgg ctt cca atc gag aga gag act tac ctg gac cgt     1243
   Gln Phe Pro Lys Trp Leu Pro Ile Glu Arg Glu Thr Tyr Leu Asp Arg
           330                 335                 340
   ttg tca cta agg ttt gac aag gaa ggt caa ccc tct cag ctt gct cca     1291
   Leu Ser Leu Arg Phe Asp Lys Glu Gly Gln Pro Ser Gln Leu Ala Pro
       345                 350                 355
   atc gac ttc ttt gtc agt acg gtt gat ccc aca aag gaa cct ccc ttg     1339
   Ile Asp Phe Phe Val Ser Thr Val Asp Pro Thr Lys Glu Pro Pro Leu
   360                 365                 370                 375
   gtc aca gcg aac act gtc ctt tcc atc ctt tct gtg gat tat ccg gtt     1387
   Val Thr Ala Asn Thr Val Leu Ser Ile Leu Ser Val Asp Tyr Pro Val
                       380                 385                 390
   gag aag gtc tcc tgc tat gtt tct gat gat ggt gct gca atg ctt acg      1435
   Glu Lys Val Ser Cys Tyr Val Ser Asp Asp Gly Ala Ala Met Leu Thr
                   395                 400                 405
   ttt gaa gca ttg tct gaa aca tct gaa ttt gca aag aaa tgg gtt cct      1483
   Phe Glu Ala Leu Ser Glu Thr Ser Glu Phe Ala Lys Lys Trp Val Pro
               410                 415                 420
   ttc agc aaa aag ttt aat atc gag cct cgt gct cct gag tgg tac ttc      1531
   Phe Ser Lys Lys Phe Asn Ile Glu Pro Arg Ala Pro Glu Trp Tyr Phe
           425                 430                 435
   caa cag aag ata gac tac ctg aaa gac aag gtt gct gct tca ttt gtt      1579
   Gln Gln Lys Ile Asp Tyr Leu Lys Asp Lys Val Ala Ala Ser Phe Val
   440                 445                 450                 455
   agg gag agg agg gcg atg aag aga gaa tac gag gaa ttc aag gta agg      1627
   Arg Glu Arg Arg Ala Met Lys Arg Glu Tyr Glu Glu Phe Lys Val Arg
                   460                 465                 470
   atc aat gcc ttg gtt gca aaa gcc caa aag gtt cct gag gaa gga tgg      1675
   Ile Asn Ala Leu Val Ala Lys Ala Gln Lys Val Pro Glu Glu Gly Trp
               475                 480                 485
   aca atg caa gat gga agc ccc tgg cct gga aac aac gta cgc gat cat      1723
   Thr Met Gln Asp Gly Ser Pro Trp Pro Gly Asn Asn Val Arg Asp His
           490                 495                 500
   cct gga atg att cag gta ttc ctt ggc caa agt ggc ggt cgt gat gtg      1771
   Pro Gly Met Ile Gln Val Phe Leu Gly Gln Ser Gly Gly Arg Asp Val
       505                 510                 515
   gaa gga aat gag ttg cct cgc ctg gtt tat gtc tcg aga gaa aag agg      1819
   Glu Gly Asn Glu Leu Pro Arg Leu Val Tyr Val Ser Arg Glu Lys Arg
   520                 525                 530                 535
   cca ggt tat aac cat cac aag aag gct ggt gcc atg aat gca ctg gtc      1867
   Pro Gly Tyr Asn His His Lys Lys Ala Gly Ala Met Asn Ala Leu Val
                   540                 545                 550
   cgt gtc tct gct gtc tta tca aat gct gca tac cta ttg aac ttg gac      1915
   Arg Val Ser Ala Val Leu Ser Asn Ala Ala Tyr Leu Leu Asn Leu Asp
               555                 560                 565
   tgt gat cac tac atc aac aat agc aag gcc ata aaa gag gct atg tgt      1963
   Cys Asp His Tyr Ile Asn Asn Ser Lys Ala Ile Lys Glu Ala Met Cys
           570                 575                 580
   ttc atg atg gat cct ttg gtg ggg aag aaa gtg tgc tat gta cag ttc      2011
   Phe Met Met Asp Pro Leu Val Gly Lys Lys Val Cys Tyr Val Gln Phe
       585                 590                 595
   cct cag agg ttt gat ggt att gac aaa aat gat cga tac gct aac agg      2059
   Pro Gln Arg Phe Asp Gly Ile Asp Lys Asn Asp Arg Tyr Ala Asn Arg
   600                 605                 610                 615
   aac gtt gtc ttt ttt gac atc aac atg aaa ggt ttg gac ggt att caa      2107
   Asn Val Val Phe Phe Asp Ile Asn Met Lys Gly Leu Asp Gly Ile Gln
                   620                 625                 630
   gga ccc att tat gtg ggt act gga tgt gtt ttc aga cgg cag gca ctg      2155
   Gly Pro Ile Tyr Val Gly Thr Gly Cys Val Phe Arg Arg Gln Ala Leu
               635                 640                 645
   tat ggt tat gat gct cct aaa acg aag aag cca cca tca aga act tgc      2203
   Tyr Gly Tyr Asp Ala Pro Lys Thr Lys Lys Pro Pro Ser Arg Thr Cys
           650                 655                 660
   aac tgc tgg ccc aag tgg tgc ctc tct tgc tgc tgc agc agg aac aag      2251
   Asn Cys Trp Pro Lys Trp Cys Leu Ser Cys Cys Cys Ser Arg Asn Lys
       665                 670                 675
   aat aaa aag aag act aca aaa cca aag acg gag aag aag aaa aga tta      2299
   Asn Lys Lys Lys Thr Thr Lys Pro Lys Thr Glu Lys Lys Lys Arg Leu
   680                 685                 690                 695
   ttt ttc aag aaa gca gaa aac cca tct cct gca tat gct ttg ggt gaa      2347
   Phe Phe Lys Lys Ala Glu Asn Pro Ser Pro Ala Tyr Ala Leu Gly Glu
                   700                 705                 710
   att gat gaa ggt gct cca ggt gct gat atc gag aag gcc gga atc gta      2395
   Ile Asp Glu Gly Ala Pro Gly Ala Asp Ile Glu Lys Ala Gly Ile Val
               715                 720                 725
   aat caa cag aaa cta gag aag aaa ttt ggg cag tct tct gtt ttt gtc      2443
   Asn Gln Gln Lys Leu Glu Lys Lys Phe Gly Gln Ser Ser Val Phe Val
           730                 735                 740
   gca tca aca ctt ctt gag aac gga ggg acc ctg aag agc gca agt cca      2491
   Ala Ser Thr Leu Leu Glu Asn Gly Gly Thr Leu Lys Ser Ala Ser Pro
       745                 750                 755
   gct tct ctt ctg aag gaa gct ata cat gtt atc agc tgc ggc tac gaa      2539
   Ala Ser Leu Leu Lys Glu Ala Ile His Val Ile Ser Cys Gly Tyr Glu
   760                 765                 770                 775
   gac aag acc gac tgg gga aaa gag att ggc tgg att tac gga tcg atc      2587
   Asp Lys Thr Asp Trp Gly Lys Glu Ile Gly Trp Ile Tyr Gly Ser Ile
                   780                 785                 790
   aca gag gat atc ttg act gga ttt aag atg cac tgc cat ggc tgg cgg      2635
   Thr Glu Asp Ile Leu Thr Gly Phe Lys Met His Cys His Gly Trp Arg
               795                 800                 805
   tct att tac tgc atc ccg aag cgg cct gca ttc aaa ggt tct gcg cct      2683
   Ser Ile Tyr Cys Ile Pro Lys Arg Pro Ala Phe Lys Gly Ser Ala Pro
           810                 815                 820
   ctg aac ctt tcc gac cgt ctt cac cag gtc ctt cgc tgg gcc ctt ggg      2731
   Leu Asn Leu Ser Asp Arg Leu His Gln Val Leu Arg Trp Ala Leu Gly
       825                 830                 835
   tcc gtc gaa att ttc ttc agc aag cac tgc cca ctt tgg tac gga tac      2779
   Ser Val Glu Ile Phe Phe Ser Lys His Cys Pro Leu Trp Tyr Gly Tyr
   840                 845                 850                 855
   ggc ggc ggg cta aaa ttc ctg gaa agg ttt tct tat atc aac tcc atc      2827
   Gly Gly Gly Leu Lys Phe Leu Glu Arg Phe Ser Tyr Ile Asn Ser Ile
                   860                 865                 870
   gtt tat ccc tgg acg tcc att cct ctc ctg gct tac tgt acc ttg cct      2875
   Val Tyr Pro Trp Thr Ser Ile Pro Leu Leu Ala Tyr Cys Thr Leu Pro
               875                 880                 885
   gcc atc tgc ctg ctc acg ggg aag ttt atc aca cca gag ctt acc aat      2923
   Ala Ile Cys Leu Leu Thr Gly Lys Phe Ile Thr Pro Glu Leu Thr Asn
           890                 895                 900
   gtc gcc agt atc tgg ttc atg gca ctt ttc atc tgc atc tcc gtg acc      2971
   Val Ala Ser Ile Trp Phe Met Ala Leu Phe Ile Cys Ile Ser Val Thr
       905                 910                 915
   ggc atc ctg gaa atg agg tgg agt ggc gtg gcc atc gac gac tgg tgg      3019
   Gly Ile Leu Glu Met Arg Trp Ser Gly Val Ala Ile Asp Asp Trp Trp
   920                 925                 930                 935
   agg aac gag cag ttc tgg gtc atc gga ggc gtt tcg gcg cat ctg ttc      3067
   Arg Asn Glu Gln Phe Trp Val Ile Gly Gly Val Ser Ala His Leu Phe
                   940                 945                 950
   gcg gtg ttc cag ggc ctg ctg aag gtg ttc gcc ggc atc gac acg agc      3115
   Ala Val Phe Gln Gly Leu Leu Lys Val Phe Ala Gly Ile Asp Thr Ser
               955                 960                 965
   ttc acc gtg acg tcg aag gcc ggg gac gac gag gag ttc tcg gag ctg      3163
   Phe Thr Val Thr Ser Lys Ala Gly Asp Asp Glu Glu Phe Ser Glu Leu
           970                 975                 980
   tac acg ttc aag tgg acc acc ctg ctg ata ccc ccg acc acg ctc ctc      3211
   Tyr Thr Phe Lys Trp Thr Thr Leu Leu Ile Pro Pro Thr Thr Leu Leu
       985                 990                 995
   ctg ctg aac ttc atc ggg gtg gtg gcc ggg atc tcg aac gcg atc aac      3259
   Leu Leu Asn Phe Ile Gly Val Val Ala Gly Ile Ser Asn Ala Ile Asn
   1000                1005                1010                1015
   aac ggg tac gag tcg tgg ggc ccc ctg ttc ggg aag ctc ttc ttc gcc      3307
   Asn Gly Tyr Glu Ser Trp Gly Pro Leu Phe Gly Lys Leu Phe Phe Ala
                   1020                1025                1030
   ttc tgg gtg atc gtc cac ctg tac ccg ttc ctc aag ggt ctg gtg ggg      3355
   Phe Trp Val Ile Val His Leu Tyr Pro Phe Leu Lys Gly Leu Val Gly
               1035                1040                1045
   agg cag aac agg acg ccg acg atc gtc atc gtc tgg tcc atc ctg ctg      3403
   Arg Gln Asn Arg Thr Pro Thr Ile Val Ile Val Trp Ser Ile Leu Leu
           1050                1055                1060
   gcc tcg atc ttc tcg ctc ctg tgg gtc cgc gtc gac ccg ttc ctc gcc      3451
   Ala Ser Ile Phe Ser Leu Leu Trp Val Arg Val Asp Pro Phe Leu Ala
       1065                1070                1075
   aag agc aac ggc ccg ctc ctg gag gag tgt ggc ctg gac tgc a          3494
   Lys Ser Asn Gly Pro Leu Leu Glu Glu Cys Gly Leu Asp Cys
   1080                1085                1090
   actgaagtgg gggccccctg tcactcgaag ttctgtcacg ggcgaattac gcctgatttt   3554
   ttgttgttgt tgttgttgga attctttgct gtagatagaa accacatgtc cacggcatct   3614
   ctgctgtgtc cattggagca ggagagaggt gcctgctgct gtttgttgag taaattaaaa   3674
   gttttaaagt tatacagtga tgcacattcc agtgcccagt gtattccctt tttacagtct   3734
   gtatattagc gacaaaggac atattggtta ggagtttgat tcttttgtaa aaaaaaaaaa   3794
   aaaaaaaaaa aaaaaaaaa                                                 3813
<210> 26
   <211> 1094
   <212> PRT
   <213> Zea mays
<400> 26
   Met Glu Ala Ser Ala Gly Leu Val Ala Gly Ser His Asn Arg Asn Glu
   1               5                   10                  15
   Leu Val Val Ile Arg Arg Asp Arg Glu Ser Gly Ala Ala Gly Gly Gly
               20                  25                  30
   Ala Ala Arg Arg Ala Glu Ala Pro Cys Gln Ile Cys Gly Asp Glu Val
           35                  40                  45
   Gly Val Gly Phe Asp Gly Glu Pro Phe Val Ala Cys Asn Glu Cys Ala
       50                  55                  60
   Phe Pro Val Cys Arg Ala Cys Tyr Glu Tyr Glu Arg Arg Glu Gly Ser
   65                  70                  75                  80
   Gln Ala Cys Pro Gln Cys Arg Thr Arg Tyr Lys Arg Leu Lys Gly Cys
                   85                  90                  95
   Pro Arg Val Ala Gly Asp Glu Glu Glu Asp Gly Val Asp Asp Leu Glu
               100                 105                 110
   Gly Glu Phe Gly Leu Gln Asp Gly Ala Ala His Glu Asp Asp Pro Gln
           115                 120                 125
   Tyr Val Ala Glu Ser Met Leu Arg Ala Gln Met Ser Tyr Gly Arg Gly
       130                 135                 140
   Gly Asp Ala His Pro Gly Phe Ser Pro Val Pro Asn Val Pro Leu Leu
   145                 150                 155                 160
   Thr Asn Gly Gln Met Val Asp Asp Ile Pro Pro Glu Gln His Ala Leu
                   165                 170                 175
   Val Pro Ser Tyr Met Ser Gly Gly Gly Gly Gly Gly Lys Arg Ile His
               180                 185                 190
   Pro Leu Pro Phe Ala Asp Pro Asn Leu Pro Val Gln Pro Arg Ser Met
           195                 200                 205
   Asp Pro Ser Lys Asp Leu Ala Ala Tyr Gly Tyr Gly Ser Val Ala Trp
       210                 215                 220
   Lys Glu Arg Met Glu Gly Trp Lys Gln Lys Gln Glu Arg Leu Gln His
   225                 230                 235                 240
   Val Arg Ser Glu Gly Gly Gly Asp Trp Asp Gly Asp Asp Ala Asp Leu
                   245                 250                 255
   Pro Leu Met Asp Glu Ala Arg Gln Pro Leu Ser Arg Lys Val Pro Ile
               260                 265                 270
   Ser Ser Ser Arg Ile Asn Pro Tyr Arg Met Ile Ile Val Ile Arg Leu
           275                 280                 285
   Val Val Leu Gly Phe Phe Phe His Tyr Arg Val Met His Pro Ala Lys
       290                 295                 300
   Asp Ala Phe Ala Leu Trp Leu Ile Ser Val Ile Cys Glu Ile Trp Phe
   305                 310                 315                 320
   Ala Met Ser Trp Ile Leu Asp Gln Phe Pro Lys Trp Leu Pro Ile Glu
                           325                       330                       335
   Arg Glu Thr Tyr Leu Asp Arg Leu Ser Leu Arg Phe Asp Lys Glu Gly
                       340                     345                     350
   Gln Pro Ser Gln Leu Ala Pro Ile Asp Phe Phe Val Ser Thr Val Asp
               355                     360                     365
   Pro Thr Lys Glu Pro Pro Leu Val Thr Ala Asn Thr Val Leu Ser Ile
       370                     375                     380
   Leu Ser Val Asp Tyr Pro Val Glu Lys Val Ser Cys Tyr Val Ser Asp
   385                     390                     395                     400
   Asp Gly Ala Ala Met Leu Thr Phe Glu Ala Leu Ser Glu Thr Ser Glu
                       405                     410                     415
   Phe Ala Lys Lys Trp Val Pro Phe Ser Lys Lys Phe Asn Ile Glu Pro
                       420                     425                     430
   Arg Ala Pro Glu Trp Tyr Phe Gln Gln Lys Ile Asp Tyr Leu Lys Asp
               435                     440                     445
   Lys Val Ala Ala Ser Phe Val Arg Glu Arg Arg Ala Met Lys Arg Glu
       450                     455                     460
   Tyr Glu Glu Phe Lys Val Arg Ile Asn Ala Leu Val Ala Lys Ala Gln
   465                     470                     475                     480
   Lys Val Pro Glu Glu Gly Trp Thr Met Gln Asp Gly Ser Pro Trp Pro
                       485                     490                     495
   Gly Asn Asn Val Arg Asp His Pro Gly Met Ile Gln Val Phe Leu Gly
                       500                     505                     510
   Gln Ser Gly Gly Arg Asp Val Glu Gly Asn Glu Leu Pro Arg Leu Val
               515                     520                     525
   Tyr Val Ser Arg Glu Lys Arg Pro Gly Tyr Asn His His Lys Lys Ala
       530                     535                     540
   Gly Ala Met Asn Ala Leu Val Arg Val Ser Ala Val Leu Ser Asn Ala
   545                     550                     555                     560
   Ala Tyr Leu Leu Asn Leu Asp Cys Asp His Tyr Ile Asn Asn Ser Lys
                       565                     570                     575
   Ala Ile Lys Glu Ala Met Cys Phe Met Met Asp Pro Leu Val Gly Lys
                       580                     585                     590
   Lys Val Cys Tyr Val Gln Phe Pro Gln Arg Phe Asp Gly Ile Asp Lys
               595                     600                     605
   Asn Asp Arg Tyr Ala Asn Arg Asn Val Val Phe Phe Asp Ile Asn Met
       610                     615                     620
   Lys Gly Leu Asp Gly Ile Gln Gly Pro Ile Tyr Val Gly Thr Gly Cys
   625                     630                     635                     640
   Val Phe Arg Arg Gln Ala Leu Tyr Gly Tyr Asp Ala Pro Lys Thr Lys
                       645                     650                     655
   Lys Pro Pro Ser Arg Thr Cys Asn Cys Trp Pro Lys Trp Cys Leu Ser
                       660                     665                     670
   Cys Cys Cys Ser Arg Asn Lys Asn Lys Lys Lys Thr Thr Lys Pro Lys
               675                     680                     685
   Thr Glu Lys Lys Lys Arg Leu Phe Phe Lys Lys Ala Glu Asn Pro Ser
       690                     695                     700
   Pro Ala Tyr Ala Leu Gly Glu Ile Asp Glu Gly Ala Pro Gly Ala Asp
   705                     710                     715                     720
   Ile Glu Lys Ala Gly Ile Val Asn Gln Gln Lys Leu Glu Lys Lys Phe
                       725                     730                     735
   Gly Gln Ser Ser Val Phe Val Ala Ser Thr Leu Leu Glu Asn Gly Gly
                       740                     745                     750
   Thr Leu Lys Ser Ala Ser Pro Ala Ser Leu Leu Lys Glu Ala Ile His
               755                     760                     765
   Val Ile Ser Cys Gly Tyr Glu Asp Lys Thr Asp Trp Gly Lys Glu Ile
       770                     775                     780
   Gly Trp Ile Tyr Gly Ser Ile Thr Glu Asp Ile Leu Thr Gly Phe Lys
   785                 790                 795                 800
   Met His Cys His Gly Trp Arg Ser Ile Tyr Cys Ile Pro Lys Arg Pro
                   805                 810                 815
   Ala Phe Lys Gly Ser Ala Pro Leu Asn Leu Ser Asp Arg Leu His Gln
               820                 825                 830
   Val Leu Arg Trp Ala Leu Gly Ser Val Glu Ile Phe Phe Ser Lys His
           835                 840                 845
   Cys Pro Leu Trp Tyr Gly Tyr Gly Gly Gly Leu Lys Phe Leu Glu Arg
       850                 855                 860
   Phe Ser Tyr Ile Asn Ser Ile Val Tyr Pro Trp Thr Ser Ile Pro Leu
   865                 870                 875                 880
   Leu Ala Tyr Cys Thr Leu Pro Ala Ile Cys Leu Leu Thr Gly Lys Phe
                   885                 890                 895
   Ile Thr Pro Glu Leu Thr Asn Val Ala Ser Ile Trp Phe Met Ala Leu
               900                 905                 910
   Phe Ile Cys Ile Ser Val Thr Gly Ile Leu Glu Met Arg Trp Ser Gly
           915                 920                 925
   Val Ala Ile Asp Asp Trp Trp Arg Asn Glu Gln Phe Trp Val Ile Gly
       930                 935                 940
   Gly Val Ser Ala His Leu Phe Ala Val Phe Gln Gly Leu Leu Lys Val
   945                 950                 955                 960
   Phe Ala Gly Ile Asp Thr Ser Phe Thr Val Thr Ser Lys Ala Gly Asp
                   965                 970                 975
   Asp Glu Glu Phe Ser Glu Leu Tyr Thr Phe Lys Trp Thr Thr Leu Leu
               980                 985                 990
   Ile Pro Pro Thr Thr Leu Leu Leu Leu Asn Phe Ile Gly Val Val Ala
           995                 1000                1005
   Gly Ile Ser Asn Ala Ile Asn Asn Gly Tyr Glu Ser Trp Gly Pro Leu
       1010                1015                1020
   Phe Gly Lys Leu Phe Phe Ala Phe Trp Val Ile Val His Leu Tyr Pro
   1025                1030                1035                1040
   Phe Leu Lys Gly Leu Val Gly Arg Gln Asn Arg Thr Pro Thr Ile Val
                   1045                1050                1055
   Ile Val Trp Ser Ile Leu Leu Ala Ser Ile Phe Ser Leu Leu Trp Val
               1060                1065                1070
   Arg Val Asp Pro Phe Leu Ala Lys Ser Asn Gly Pro Leu Leu Glu Glu
           1075                1080                1085
   Cys Gly Leu Asp Cys Asn
       1090
<210> 27
   <211> 25
   <212> DNA
   <213> Zea mays
<400> 27
   atggaggcta gcgcggggct ggtgg
   25
<210> 28
   <211> 25
   <212> DNA
   <213> Zea mays
<400> 28
   tcagttgcag tccaggccac actcc
   25
<210> 29
   <211> 3746
   <212> DNA
   <213> Zea mays
<220>
   <221> CDS
   <222> (321)...(3549)
<400> 29
   ctaggatcaa aaccgtctcg ccgctgcaat aatcttttgt caattcttaa tccctcgcgt     60
   cgacagcgac agcggaacca actcacgttg ccgcggcttc ctccatcggt gcggtgccct    120
   gtccttttct ctcgtccctc ctccccccgt atagttaagc cccgccccgc tactactact    180
   actagcagca gcagcgctct cgcagcggga gatgcggtgt tgatccgtgc cccgctcgga    240
   tctcgggact ggtgccggct ctgcccaggc cccaggctcc aggccagctc cctcgacgtt    300
   tctcggcgag ctcgcttgcc atg gag ggc gac gcg gac ggc gtg aag tcg ggg    353
                         Met Glu Gly Asp Ala Asp Gly Val Lys Ser Gly
                          1               5                  10
   agg cgc ggt ggc gga cag gtg tgc cag atc tgc ggc gac ggc gtg ggc      401
   Arg Arg Gly Gly Gly Gln Val Cys Gln Ile Cys Gly Asp Gly Val Gly
               15                  20                  25
   acc acg gcg gag ggg gac gtc ttc gcc gcc tgc gac gtc tgc ggg ttt      449
   Thr Thr Ala Glu Gly Asp Val Phe Ala Ala Cys Asp Val Cys Gly Phe
           30                  35                  40
   ccg gtg tgc cgc ccc tgc tac gag tac gag cgc aag gac ggc acg cag      497
   Pro Val Cys Arg Pro Cys Tyr Glu Tyr Glu Arg Lys Asp Gly Thr Gln
       45                  50                  55
   gcg tgc ccc cag tgc aag acc aag tac aag cgc cac aag ggg agc ccg      545
   Ala Cys Pro Gln Cys Lys Thr Lys Tyr Lys Arg His Lys Gly Ser Pro
   60                  65                  70                  75
   gcg atc cgt ggg gag gaa gga gac gac act gat gcc gat agc gac ttc      593
   Ala Ile Arg Gly Glu Glu Gly Asp Asp Thr Asp Ala Asp Ser Asp Phe
                   80                  85                  90
   aat tac ctt gca tct ggc aat gag gac cag aag cag aag att gcc gac      641
   Asn Tyr Leu Ala Ser Gly Asn Glu Asp Gln Lys Gln Lys Ile Ala Asp
               95                  100                 105
   aga atg cgc agc tgg cgc atg aac gtt ggg ggc agc ggg gat gtt ggt      689
   Arg Met Arg Ser Trp Arg Met Asn Val Gly Gly Ser Gly Asp Val Gly
           110                 115                 120
   cgc ccc aag tat gac agt ggc gag atc ggg ctt acc aag tat gac agt      737
   Arg Pro Lys Tyr Asp Ser Gly Glu Ile Gly Leu Thr Lys Tyr Asp Ser
       125                 130                 135
   ggc gag att cct cgg gga tac atc cca tca gtc act aac agc cag atc      785
   Gly Glu Ile Pro Arg Gly Tyr Ile Pro Ser Val Thr Asn Ser Gln Ile
   140                 145                 150                 155
   tca gga gaa atc cct ggt gct tcc cct gac cat cat atg atg tcc cca      833
   Ser Gly Glu Ile Pro Gly Ala Ser Pro Asp His His Met Met Ser Pro
                   160                 165                 170
   act ggg aac att ggc aag cgt gct cca ttt ccc tat gtg aac cat tcg      881
   Thr Gly Asn Ile Gly Lys Arg Ala Pro Phe Pro Tyr Val Asn His Ser
                   175                 180                 185
   cca aat ccg tca agg gag ttc tct ggt agc att ggg aat gtt gcc tgg      929
   Pro Asn Pro Ser Arg Glu Phe Ser Gly Ser Ile Gly Asn Val Ala Trp
               190                 195                 200
   aaa gag agg gtt gat ggc tgg aaa atg aag cag gac aag ggg acg att      977
   Lys Glu Arg Val Asp Gly Trp Lys Met Lys Gln Asp Lys Gly Thr Ile
           205                 210                 215
   ccc atg acg aat ggc aca agc att gct ccc tct gag ggt cgg ggt gtt     1025
   Pro Met Thr Asn Gly Thr Ser Ile Ala Pro Ser Glu Gly Arg Gly Val
   220                 225                 230                 235
   ggt gat att gat gca tca act gat tac aac atg gaa gat gcc tta ttg     1073
   Gly Asp Ile Asp Ala Ser Thr Asp Tyr Asn Met Glu Asp Ala Leu Leu
                   240                 245                 250
   aac gac gaa act cga cag cct cta tct agg aaa gtt cca ctt cct tcc     1121
   Asn Asp Glu Thr Arg Gln Pro Leu Ser Arg Lys Val Pro Leu Pro Ser
                   255                 260                 265
   tcc agg ata aat cca tac agg atg gtc att gtg ctg cga ttg att gtt     1169
   Ser Arg Ile Asn Pro Tyr Arg Met Val Ile Val Leu Arg Leu Ile Val
               270                 275                 280
   cta agc atc ttc ttg cac tac cgt atc aca aat cct gtg cgc aat gca     1217
   Leu Ser Ile Phe Leu His Tyr Arg Ile Thr Asn Pro Val Arg Asn Ala
           285                 290                 295
   tac cca tta tgg ctt cta tct gtt ata tgt gag atc tgg ttt gct ctt     1265
   Tyr Pro Leu Trp Leu Leu Ser Val Ile Cys Glu Ile Trp Phe Ala Leu
   300                 305                 310                 315
   tcg tgg ata ttg gat cag ttc cct aag tgg ttt cca atc aac cgg gag     1313
   Ser Trp Ile Leu Asp Gln Phe Pro Lys Trp Phe Pro Ile Asn Arg Glu
                   320                 325                 330
   acg tac ctt gat agg ctg gca tta agg tat gac cgg gaa ggt gag cca     1361
   Thr Tyr Leu Asp Arg Leu Ala Leu Arg Tyr Asp Arg Glu Gly Glu Pro
                   335                 340                 345
   tct cag ttg gct gct gtt gac att ttc gtc agt aca gtc gac cca atg     1409
   Ser Gln Leu Ala Ala Val Asp Ile Phe Val Ser Thr Val Asp Pro Met
               350                 355                 360
   aag gag cct cct ctt gtc act gcc aat acc gtg cta tcc att ctt gct     1457
   Lys Glu Pro Pro Leu Val Thr Ala Asn Thr Val Leu Ser Ile Leu Ala
           365                 370                 375
   gtg gat tac cct gtg gat aag gtc tct tgc tat gta tct gat gat gga     1505
   Val Asp Tyr Pro Val Asp Lys Val Ser Cys Tyr Val Ser Asp Asp Gly
   380                 385                 390                 395
   gct gcg atg ctg aca ttt gat gca cta gct gag act tca gag ttt gct     1553
   Ala Ala Met Leu Thr Phe Asp Ala Leu Ala Glu Thr Ser Glu Phe Ala
                   400                 405                 410
   aga aaa tgg gta cca ttt gtt aag aag tac aac att gaa cct aga gct      1601
   Arg Lys Trp Val Pro Phe Val Lys Lys Tyr Asn Ile Glu Pro Arg Ala
               415                 420                 425
   cct gaa tgg tac ttc tcc cag aaa att gat tac ttg aag gac aaa gtg      1649
   Pro Glu Trp Tyr Phe Ser Gln Lys Ile Asp Tyr Leu Lys Asp Lys Val
           430                 435                 440
   cac cct tca ttt gtt aaa gac cgc cgg gcc atg aag aga gaa tat gaa      1697
   His Pro Ser Phe Val Lys Asp Arg Arg Ala Met Lys Arg Glu Tyr Glu
       445                 450                 455
   gaa ttc aaa gtt agg gta aat ggc ctt gtt gct aag gca cag aaa gtt      1745
   Glu Phe Lys Val Arg Val Asn Gly Leu Val Ala Lys Ala Gln Lys Val
   460                 465                 470                 475
   cct gag gaa gga tgg atc atg caa gat ggc aca cca tgg cca gga aac      1793
   Pro Glu Glu Gly Trp Ile Met Gln Asp Gly Thr Pro Trp Pro Gly Asn
                   480                 485                 490
   aat acc mgg gac cat cct gga atg att cag gtt ttc ctt ggt cac agt      1841
   Asn Thr Xaa Asp His Pro Gly Met Ile Gln Val Phe Leu Gly His Ser
               495                 500                 505
   ggt ggc ctt gat act gag ggc aat gag cta ccc cgt ttg gtc tat gtt      1889
   Gly Gly Leu Asp Thr Glu Gly Asn Glu Leu Pro Arg Leu Val Tyr Val
           510                 515                 520
   tct cgt gaa aag cgt cct gga ttc cag cat cac aag aaa gct ggt gcc      1937
   Ser Arg Glu Lys Arg Pro Gly Phe Gln His His Lys Lys Ala Gly Ala
       525                 530                 535
   atg aat gct ctt gtt cgt gtc tca gct gtg ctt acc aat gga caa tac      1985
   Met Asn Ala Leu Val Arg Val Ser Ala Val Leu Thr Asn Gly Gln Tyr
   540                 545                 550                 555
   atg ttg aat ctt gat tgt gat cac tac att aac aac agt aag gct ctc      2033
   Met Leu Asn Leu Asp Cys Asp His Tyr Ile Asn Asn Ser Lys Ala Leu
                   560                 565                 570
   agg gaa gct atg tgc ttc ctt atg gac cct aac cta gga agg agt gtc      2081
   Arg Glu Ala Met Cys Phe Leu Met Asp Pro Asn Leu Gly Arg Ser Val
               575                 580                 585
   tgc tac gtc cag ttt ccc cag aga ttc gat ggc att gac agg aat gat      2129
   Cys Tyr Val Gln Phe Pro Gln Arg Phe Asp Gly Ile Asp Arg Asn Asp
           590                 595                 600
   cga tat gcc aac agg aac acc gtg ttt ttc gat att aac ttg aga ggt      2177
   Arg Tyr Ala Asn Arg Asn Thr Val Phe Phe Asp Ile Asn Leu Arg Gly
       605                 610                 615
   ctt gat ggc atc caa gga cca gtt tat gtc gga act ggc tgt gtt ttc      2225
   Leu Asp Gly Ile Gln Gly Pro Val Tyr Val Gly Thr Gly Cys Val Phe
   620                 625                 630                 635
   aac cga aca gct cta tat ggt tat gag ccc cca att aag cag aag aag      2273
   Asn Arg Thr Ala Leu Tyr Gly Tyr Glu Pro Pro Ile Lys Gln Lys Lys
                   640                 645                 650
   ggt ggt ttc ttg tca tca cta tgt ggc ggt agg aag aag gca agc aaa      2321
   Gly Gly Phe Leu Ser Ser Leu Cys Gly Gly Arg Lys Lys Ala Ser Lys
               655                 660                 665
   tca aag aag ggc tcg gac aag aag aag tcg cag aag cat gtg gac agt      2369
   Ser Lys Lys Gly Ser Asp Lys Lys Lys Ser Gln Lys His Val Asp Ser
           670                 675                 680
   tct gtg cca gta ttc aac ctt gaa gat ata gag gag gga gtt gaa ggc      2417
   Ser Val Pro Val Phe Asn Leu Glu Asp Ile Glu Glu Gly Val Glu Gly
       685                 690                 695
   gct gga ttt gac gac gag aaa tca ctt ctt atg tct caa atg agc ctg      2465
   Ala Gly Phe Asp Asp Glu Lys Ser Leu Leu Met Ser Gln Met Ser Leu
   700                 705                 710                 715
   gag aag aga ttt ggc cag tcc gca gcg ttt gtt gcc tcc act ctg atg      2513
   Glu Lys Arg Phe Gly Gln Ser Ala Ala Phe Val Ala Ser Thr Leu Met
                   720                 725                 730
   gag tat ggt ggt gtt cct cag tcc gca act ccg gag tct ctt ctg aaa      2561
   Glu Tyr Gly Gly Val Pro Gln Ser Ala Thr Pro Glu Ser Leu Leu Lys
               735                 740                 745
   gaa gct atc cat gtt ata agc tgt ggc tat gag gac aag act gaa tgg      2609
   Glu Ala Ile His Val Ile Ser Cys Gly Tyr Glu Asp Lys Thr Glu Trp
           750                 755                 760
   gga act gag atc ggg tgg atc tac ggt tct gtg aca gaa gac att ctc      2657
   Gly Thr Glu Ile Gly Trp Ile Tyr Gly Ser Val Thr Glu Asp Ile Leu
       765                 770                 775
   acc gga ttc aag atg cac gcg cga ggc tgg cgg tcg atc tac tgc atg      2705
   Thr Gly Phe Lys Met His Ala Arg Gly Trp Arg Ser Ile Tyr Cys Met
   780                 785                 790                 795
   ccc aag cgg cca gct ttc aag ggg tct gcc ccc atc aat ctt tcg gac      2753
   Pro Lys Arg Pro Ala Phe Lys Gly Ser Ala Pro Ile Asn Leu Ser Asp
                   800                 805                 810
   cgt ctg aac cag gtg ctc cgg tgg gct ctt ggg tcc gtg gag atc ctc      2801
   Arg Leu Asn Gln Val Leu Arg Trp Ala Leu Gly Ser Val Glu Ile Leu
               815                 820                 825
   ttc agc cgg cac tgc ccc ctg tgg tac ggc tac gga ggg cgg ctc aag      2849
   Phe Ser Arg His Cys Pro Leu Trp Tyr Gly Tyr Gly Gly Arg Leu Lys
           830                 835                 840
   ttc ctg gag aga ttc gcg tac atc aac acc acc atc tac ccg ctc acg      2897
   Phe Leu Glu Arg Phe Ala Tyr Ile Asn Thr Thr Ile Tyr Pro Leu Thr
       845                 850                 855
   tcc atc ccg ctt ctc atc tac tgc atc ctg ccc gcc atc tgt ctg ctc      2945
   Ser Ile Pro Leu Leu Ile Tyr Cys Ile Leu Pro Ala Ile Cys Leu Leu
   860                 865                 870                 875
   acc gga aag ttc atc att cca gag atc agc aac ttc gcc agc atc tgg      2993
   Thr Gly Lys Phe Ile Ile Pro Glu Ile Ser Asn Phe Ala Ser Ile Trp
                   880                 885                 890
   ttc atc tcc ctc ttc atc tcg atc ttc gcc acg ggc atc ctg gag atg      3041
   Phe Ile Ser Leu Phe Ile Ser Ile Phe Ala Thr Gly Ile Leu Glu Met
               895                 900                 905
   agg tgg agc ggg gtg ggc atc gac gag tgg tgg agg aac gag cag ttc      3089
   Arg Trp Ser Gly Val Gly Ile Asp Glu Trp Trp Arg Asn Glu Gln Phe
           910                 915                 920
   tgg gtg atc ggg ggc atc tcc gcg cac ctc ttc gcc gtg ttc cag ggc      3137
   Trp Val Ile Gly Gly Ile Ser Ala His Leu Phe Ala Val Phe Gln Gly
       925                 930                 935
   ctg ctc aag gtg ctg gcc ggc atc gac acc aac ttc acc gtc acc tcc      3185
   Leu Leu Lys Val Leu Ala Gly Ile Asp Thr Asn Phe Thr Val Thr Ser
   940                 945                 950                 955
   aag gcc tcg gac gag gac ggc gac ttc gcg gag ctg tac atg ttc aag      3233
   Lys Ala Ser Asp Glu Asp Gly Asp Phe Ala Glu Leu Tyr Met Phe Lys
                   960                 965                 970
   tgg acg acg ctc ctg atc ccg ccc acc acc atc ctg atc atc aac ctg      3281
   Trp Thr Thr Leu Leu Ile Pro Pro Thr Thr Ile Leu Ile Ile Asn Leu
               975                 980                 985
   gtc ggc gtc gtc gcc ggc atc tcc tac gcc atc aac agc gga tac cag      3329
   Val Gly Val Val Ala Gly Ile Ser Tyr Ala Ile Asn Ser Gly Tyr Gln
           990                 995                 1000
   tcg tgg ggc ccg ctc ttc ggc aag ctc ttc ttc gcc ttc tgg gtc atc      3377
   Ser Trp Gly Pro Leu Phe Gly Lys Leu Phe Phe Ala Phe Trp Val Ile
       1005                1010                1015
   gtc cac ctg tac ccg ttc ctc aag ggc ctc atg ggc agg cag aac cgc      3425
   Val His Leu Tyr Pro Phe Leu Lys Gly Leu Met Gly Arg Gln Asn Arg
   1020                1025                1030                1035
   acc ccg acc atc gtc gtc gtc tgg gcc atc ctg ctg gcg tcc atc ttc      3473
   Thr Pro Thr Ile Val Val Val Trp Ala Ile Leu Leu Ala Ser Ile Phe
                   1040                1045                1050
   tcc ttg ctg tgg gtt cgc atc gac ccc ttc acc acc cgc gtc act ggc      3521
   Ser Leu Leu Trp Val Arg Ile Asp Pro Phe Thr Thr Arg Val Thr Gly
               1055                1060                1065
   ccg gat acc cag acg tgt ggc atc aac t gctagggaag tggaaggttt          3569
   Pro Asp Thr Gln Thr Cys Gly Ile Asn
           1070                1075
   gtactttgta gaaacggagg aataccacgt gccatctgtt gtctgttaag ttatatatat    3629
   ataagcagca agtggcgtta tttacagcta cgtacagacc agtggatatt gtttaccaca    3689
   aagttttact tgtgttaata tgcattcttt tgttgatata aaaaaaaaaa aaaaaaa        3746
<210> 30
   <211> 1077
   <212> PRT
   <213> Zea mays
<400> 30
   Met Glu Gly Asp Ala Asp Gly Val Lys Ser Gly Arg Arg Gly Gly Gly
   1               5                   10                  15
   Gln Val Cys Gln Ile Cys Gly Asp Gly Val Gly Thr Thr Ala Glu Gly
               20                  25                  30
   Asp Val Phe Ala Ala Cys Asp Val Cys Gly Phe Pro Val Cys Arg Pro
           35                  40                  45
   Cys Tyr Glu Tyr Glu Arg Lys Asp Gly Thr Gln Ala Cys Pro Gln Cys
       50                  55                  60
   Lys Thr Lys Tyr Lys Arg His Lys Gly Ser Pro Ala Ile Arg Gly Glu
   65                  70                  75                  80
   Glu Gly Asp Asp Thr Asp Ala Asp Ser Asp Phe Asn Tyr Leu Ala Ser
                   85                  90                  95
   Gly Asn Glu Asp Gln Lys Gln Lys Ile Ala Asp Arg Met Arg Ser Trp
               100                 105                 110
   Arg Met Asn Val Gly Gly Ser Gly Asp Val Gly Arg Pro Lys Tyr Asp
           115                 120                 125
   Ser Gly Glu Ile Gly Leu Thr Lys Tyr Asp Ser Gly Glu Ile Pro Arg
       130                 135                 140
   Gly Tyr Ile Pro Ser Val Thr Asn Ser Gln Ile Ser Gly Glu Ile Pro
   145                 150                 155                 160
   Gly Ala Ser Pro Asp His His Met Met Ser Pro Thr Gly Asn Ile Gly
                   165                 170                 175
   Lys Arg Ala Pro Phe Pro Tyr Val Asn His Ser Pro Asn Pro Ser Arg
               180                 185                 190
   Glu Phe Ser Gly Ser Ile Gly Asn Val Ala Trp Lys Glu Arg Val Asp
           195                 200                 205
   Gly Trp Lys Met Lys Gln Asp Lys Gly Thr Ile Pro Met Thr Asn Gly
       210                 215                 220
   Thr Ser Ile Ala Pro Ser Glu Gly Arg Gly Val Gly Asp Ile Asp Ala
   225                 230                 235                 240
   Ser Thr Asp Tyr Asn Met Glu Asp Ala Leu Leu Asn Asp Glu Thr Arg
                   245                 250                 255
   Gln Pro Leu Ser Arg Lys Val Pro Leu Pro Ser Ser Arg Ile Asn Pro
               260                 265                 270
   Tyr Arg Met Val Ile Val Leu Arg Leu Ile Val Leu Ser Ile Phe Leu
           275                 280                 285
   His Tyr Arg Ile Thr Asn Pro Val Arg Asn Ala Tyr Pro Leu Trp Leu
       290                 295                 300
   Leu Ser Val Ile Cys Glu Ile Trp Phe Ala Leu Ser Trp Ile Leu Asp
   305                 310                 315                 320
   Gln Phe Pro Lys Trp Phe Pro Ile Asn Arg Glu Thr Tyr Leu Asp Arg
                   325                 330                 335
   Leu Ala Leu Arg Tyr Asp Arg Glu Gly Glu Pro Ser Gln Leu Ala Ala
               340                 345                 350
   Val Asp Ile Phe Val Ser Thr Val Asp Pro Met Lys Glu Pro Pro Leu
           355                 360                 365
   Val Thr Ala Asn Thr Val Leu Ser Ile Leu Ala Val Asp Tyr Pro Val
       370                 375                 380
   Asp Lys Val Ser Cys Tyr Val Ser Asp Asp Gly Ala Ala Met Leu Thr
   385                 390                 395                 400
   Phe Asp Ala Leu Ala Glu Thr Ser Glu Phe Ala Arg Lys Trp Val Pro
                           405                     410                     415
   Phe Val Lys Lys Tyr Asn Ile Glu Pro Arg Ala Pro Glu Trp Tyr Phe
                   420                     425                     430
   Ser Gln Lys Ile Asp Tyr Leu Lys Asp Lys Val His Pro Ser Phe Val
               435                     440                     445
   Lys Asp Arg Arg Ala Met Lys Arg Glu Tyr Glu Glu Phe Lys Val Arg
       450                     455                     460
   Val Asn Gly Leu Val Ala Lys Ala Gln Lys Val Pro Glu Glu Gly Trp
   465                     470                     475                     480
   Ile Met Gln Asp Gly Thr Pro Trp Pro Gly Asn Asn Thr Xaa Asp His
                           485                     490                     495
   Pro Gly Met Ile Gln Val Phe Leu Gly His Ser Gly Gly Leu Asp Thr
                   500                     505                     510
   Glu Gly Asn Glu Leu Pro Arg Leu Val Tyr Val Ser Arg Glu Lys Arg
               515                     520                     525
   Pro Gly Phe Gln His His Lys Lys Ala Gly Ala Met Asn Ala Leu Val
       530                     535                     540
   Arg Val Ser Ala Val Leu Thr Asn Gly Gln Tyr Met Leu Asn Leu Asp
   545                     550                     555                     560
   Cys Asp His Tyr Ile Asn Asn Ser Lys Ala Leu Arg Glu Ala Met Cys
                           565                     570                     575
   Phe Leu Met Asp Pro Asn Leu Gly Arg Ser Val Cys Tyr Val Gln Phe
                   580                     585                     590
   Pro Gln Arg Phe Asp Gly Ile Asp Arg Asn Asp Arg Tyr Ala Asn Arg
               595                     600                     605
   Asn Thr Val Phe Phe Asp Ile Asn Leu Arg Gly Leu Asp Gly Ile Gln
       610                     615                     620
   Gly Pro Val Tyr Val Gly Thr Gly Cys Val Phe Asn Arg Thr Ala Leu
   625                     630                     635                     640
   Tyr Gly Tyr Glu Pro Pro Ile Lys Gln Lys Lys Gly Gly Phe Leu Ser
                           645                     650                     655
   Ser Leu Cys Gly Gly Arg Lys Lys Ala Ser Lys Ser Lys Lys Gly Ser
                   660                     665                     670
   Asp Lys Lys Lys Ser Gln Lys His Val Asp Ser Ser Val Pro Val Phe
               675                     680                     685
   Asn Leu Glu Asp Ile Glu Glu Gly Val Glu Gly Ala Gly Phe Asp Asp
       690                     695                     700
   Glu Lys Ser Leu Leu Met Ser Gln Met Ser Leu Glu Lys Arg Phe Gly
   705                     710                     715                     720
   Gln Ser Ala Ala Phe Val Ala Ser Thr Leu Met Glu Tyr Gly Gly Val
                           725                     730                     735
   Pro Gln Ser Ala Thr Pro Glu Ser Leu Leu Lys Glu Ala Ile His Val
                   740                     745                     750
   Ile Ser Cys Gly Tyr Glu Asp Lys Thr Glu Trp Gly Thr Glu Ile Gly
               755                     760                     765
   Trp Ile Tyr Gly Ser Val Thr Glu Asp Ile Leu Thr Gly Phe Lys Met
       770                     775                     780
   His Ala Arg Gly Trp Arg Ser Ile Tyr Cys Met Pro Lys Arg Pro Ala
   785                     790                     795                     800
   Phe Lys Gly Ser Ala Pro Ile Asn Leu Ser Asp Arg Leu Asn Gln Val
                           805                     810                     815
   Leu Arg Trp Ala Leu Gly Ser Val Glu Ile Leu Phe Ser Arg His Cys
                   820                     825                     830
   Pro Leu Trp Tyr Gly Tyr Gly Gly Arg Leu Lys Phe Leu Glu Arg Phe
               835                     840                     845
   Ala Tyr Ile Asn Thr Thr Ile Tyr Pro Leu Thr Ser Ile Pro Leu Leu
       850                     855                     860
   Ile Tyr Cys Ile Leu Pro Ala Ile Cys Leu Leu Thr Gly Lys Phe Ile
   865                 870                 875                 880
   Ile Pro Glu Ile Ser Asn Phe Ala Ser Ile Trp Phe Ile Ser Leu Phe
                   885                 890                 895
   Ile Ser Ile Phe Ala Thr Gly Ile Leu Glu Met Arg Trp Ser Gly Val
               900                 905                 910
   Gly Ile Asp Glu Trp Trp Arg Asn Glu Gln Phe Trp Val Ile Gly Gly
           915                 920                 925
   Ile Ser Ala His Leu Phe Ala Val Phe Gln Gly Leu Leu Lys Val Leu
       930                 935                 940
   Ala Gly Ile Asp Thr Asn Phe Thr Val Thr Ser Lys Ala Ser Asp Glu
   945                 950                 955                 960
   Asp Gly Asp Phe Ala Glu Leu Tyr Met Phe Lys Trp Thr Thr Leu Leu
                   965                 970                 975
   Ile Pro Pro Thr Thr Ile Leu Ile Ile Asn Leu Val Gly Val Val Ala
               980                 985                 990
   Gly Ile Ser Tyr Ala Ile Asn Ser Gly Tyr Gln Ser Trp Gly Pro Leu
           995                 1000                1005
   Phe Gly Lys Leu Phe Phe Ala Phe Trp Val Ile Val His Leu Tyr Pro
       1010                1015                1020
   Phe Leu Lys Gly Leu Met Gly Arg Gln Asn Arg Thr Pro Thr Ile Val
   1025                1030                1035                1040
   Val Val Trp Ala Ile Leu Leu Ala Ser Ile Phe Ser Leu Leu Trp Val
                   1045                1050                1055
   Arg Ile Asp Pro Phe Thr Thr Arg Val Thr Gly Pro Asp Thr Gln Thr
               1060                1065                1070
   Cys Gly Ile Asn Cys
           1075
<210> 31
   <211> 25
   <212> DNA
   <213> Zea mays
<400> 31
   atggagggcg acgcggacgg cgtga
   25
<210> 32
   <211> 25
   <212> DNA
   <213> Zea mays
<400> 32
   ctagcagttg atgccacacg tctgg
   25
<210> 33
   <211> 3753
   <212> DNA
   <213> Zea mays
<220>
   <221> CDS
   <222> (184)... (3406)
<400> 33
   cagcagcaga agcactgcgc ggcattgcag cgatcgagcg ggaggaattt ggggcatggt 60
   ggtcgccaac gccgctcgga tctagaggcc cgcacgggcc gattggtctc cgcccgcctc 120
   gtcggtgttg gtgtcgttgg cgtgtggagc cgtctcggtg ggagcagcgg ggagggagcg     180
   gag atg gcg gcc aac aag ggg atg gtg gcg ggc tcg cac aac cgc aac       228
       Met Ala Ala Asn Lys Gly Met Val Ala Gly Ser His Asn Arg Asn
       1               5                   10                  15
   gag ttc gtc atg atc cgc cac gac ggc gat gtg ccg ggc tcg gct aag       276
   Glu Phe Val Met Ile Arg His Asp Gly Asp Val Pro Gly Ser Ala Lys
                   20                  25                  30
   ccc aca aag agt gcg aat gga cag gtc tgc cag att tgc ggt gac tct       324
   Pro Thr Lys Ser Ala Asn Gly Gln Val Cys Gln Ile Cys Gly Asp Ser
               35                  40                  45
   gtg ggt gtt tca gcc act ggt gat gtc ttt gtt gcc tgc aat gag tgt       372
   Val Gly Val Ser Ala Thr Gly Asp Val Phe Val Ala Cys Asn Glu Cys
           50                  55                  60
   gcc ttc cct gtc tgc cgc cca tgc tat gag tat gag cgc aag gag ggg       420
   Ala Phe Pro Val Cys Arg Pro Cys Tyr Glu Tyr Glu Arg Lys Glu Gly
       65                  70                  75
   aac caa tgc tgc ccc cag tgc aag act aga tac aag aga cag aaa ggt       468
   Asn Gln Cys Cys Pro Gln Cys Lys Thr Arg Tyr Lys Arg Gln Lys Gly
   80                  85                  90                  95
   agc cct cga gtt cat ggt gat gag gat gag gaa gat gtt gat gac cta       516
   Ser Pro Arg Val His Gly Asp Glu Asp Glu Glu Asp Val Asp Asp Leu
                   100                 105                 110
   gac aat gaa ttc aac tac aag caa ggc agt ggg aaa ggc cca gag tgg       564
   Asp Asn Glu Phe Asn Tyr Lys Gln Gly Ser Gly Lys Gly Pro Glu Trp
               115                 120                 125
   caa ctg caa gga gat gat gct gat ctg tct tca tct gct cgc cat gag       612
   Gln Leu Gln Gly Asp Asp Ala Asp Leu Ser Ser Ser Ala Arg His Glu
           130                 135                 140
   cca cat cat cgg att cca cgc ctg aca agc ggt caa cag ata tct gga       660
   Pro His His Arg Ile Pro Arg Leu Thr Ser Gly Gln Gln Ile Ser Gly
       145                 150                 155
   gag att cct gat gct tcc cct gac cgt cat tct atc cgc agt cca aca       708
   Glu Ile Pro Asp Ala Ser Pro Asp Arg His Ser Ile Arg Ser Pro Thr
   160                 165                 170                 175
   tcg agc tat gtt gat cca agc gtc cca gtt cct gtg agg att gtg gac       756
   Ser Ser Tyr Val Asp Pro Ser Val Pro Val Pro Val Arg Ile Val Asp
                   180                 185                 190
   ccc tcg aag gac ttg aat tcc tat ggg ctt aat agt gtt gac tgg aag       804
   Pro Ser Lys Asp Leu Asn Ser Tyr Gly Leu Asn Ser Val Asp Trp Lys
               195                 200                 205
   gaa aga gtt gag agc tgg agg gtt aaa cag gac aaa aat atg atg caa       852
   Glu Arg Val Glu Ser Trp Arg Val Lys Gln Asp Lys Asn Met Met Gln
           210                 215                 220
   gtg act aat aaa tat cca gag gct aga gga gga gac atg gag ggg act       900
   Val Thr Asn Lys Tyr Pro Glu Ala Arg Gly Gly Asp Met Glu Gly Thr
       225                 230                 235
   ggc tca aat gga gaa nat atg caa atg gtt gat gat gca cgg cta cct      948
   Gly Ser Asn Gly Glu Xaa Met Gln Met Val Asp Asp Ala Arg Leu Pro
   240                 245                 250                 255
   ttg agc cgt atc gtg cca att tcc tca aac cag ctc aac ctt tac cgg      996
   Leu Ser Arg Ile Val Pro Ile Ser Ser Asn Gln Leu Asn Leu Tyr Arg
                   260                 265                 270
   gta gtg atc att ctc cgt ctt atc atc ctg tgc ttc ttc ttc cag tat     1044
   Val Val Ile Ile Leu Arg Leu Ile Ile Leu Cys Phe Phe Phe Gln Tyr
               275                 280                 285
   cgt gtc agt cat cca gtg cgt gat gct tat gga tta tgg cta gta tct     1092
   Arg Val Ser His Pro Val Arg Asp Ala Tyr Gly Leu Trp Leu Val Ser
           290                 295                 300
   gtt atc tgc gag gtc tgg ttt gcc ttg tct tgg ctt cta gat cag ttc     1140
   Val Ile Cys Glu Val Trp Phe Ala Leu Ser Trp Leu Leu Asp Gln Phe
       305                 310                 315
   cca aaa tgg tat cca atc aac cgt gag aca tat ctt gac agg ctt gca     1188
   Pro Lys Trp Tyr Pro Ile Asn Arg Glu Thr Tyr Leu Asp Arg Leu Ala
   320                 325                 330                 335
   ttg agg tat gat aga gag gga gag cca tca cag ctg gct ccc att gat     1236
   Leu Arg Tyr Asp Arg Glu Gly Glu Pro Ser Gln Leu Ala Pro Ile Asp
                   340                 345                 350
   gtc ttc gtc agt aca gtg gat cca ttg aag gaa cct cca ctg atc aca     1284
   Val Phe Val Ser Thr Val Asp Pro Leu Lys Glu Pro Pro Leu Ile Thr
               355                 360                 365
   gcc aac act gtt ttg tcc att ctt tct gtg gat tac cct gtt gac aaa     1332
   Ala Asn Thr Val Leu Ser Ile Leu Ser Val Asp Tyr Pro Val Asp Lys
           370                 375                 380
   gtg tca tgc tat gtt tct gat gat ggt tca gct atg ctg act ttt gag     1380
   Val Ser Cys Tyr Val Ser Asp Asp Gly Ser Ala Met Leu Thr Phe Glu
       385                 390                 395
   tct ctc tca gaa acc gca gaa ttt gct aga aag tgg gtt ccc ttt tgt     1428
   Ser Leu Ser Glu Thr Ala Glu Phe Ala Arg Lys Trp Val Pro Phe Cys
   400                 405                 410                 415
   aag aag cac aat att gaa cca aga gct cca gaa ttt tac ttt gct caa     1476
   Lys Lys His Asn Ile Glu Pro Arg Ala Pro Glu Phe Tyr Phe Ala Gln
                   420                 425                 430
   aaa ata gat tac ctg aag gac aaa att caa cct tca ttt gtt aag gaa     1524
   Lys Ile Asp Tyr Leu Lys Asp Lys Ile Gln Pro Ser Phe Val Lys Glu
               435                 440                 445
   aga cgc gca atg aag agg gag tat gaa gaa ttc aaa gta aga atc aat     1572
   Arg Arg Ala Met Lys Arg Glu Tyr Glu Glu Phe Lys Val Arg Ile Asn
           450                 455                 460
   gcc ctt gtt gcc aaa gca cag aaa gtg cct gaa gag ggg tgg acc atg      1620
   Ala Leu Val Ala Lys Ala Gln Lys Val Pro Glu Glu Gly Trp Thr Met
       465                 470                 475
   gct gat gga act gca tgg cct ggg aat aat cct agg gac cat cct ggc      1668
   Ala Asp Gly Thr Ala Trp Pro Gly Asn Asn Pro Arg Asp His Pro Gly
   480                 485                 490                 495
   atg att cag gtt ttc ttg ggg cac agt ggt ggg ctc gac act gat gga      1716
   Met Ile Gln Val Phe Leu Gly His Ser Gly Gly Leu Asp Thr Asp Gly
                   500                 505                 510
   aat gag tta cca cgt ctt gtc tat gtc tct cgt gaa aag aga cca ggc      1764
   Asn Glu Leu Pro Arg Leu Val Tyr Val Ser Arg Glu Lys Arg Pro Gly
               515                 520                 525
   ttt cag cat cac aag aag gct ggt gca atg aat gcg ctg att cgt gta      1812
   Phe Gln His His Lys Lys Ala Gly Ala Met Asn Ala Leu Ile Arg Val
           530                 535                 540
   tct gct gtg ctg aca aat ggt gcc tat ctt ctc aat gtg gat tgc gac      1860
   Ser Ala Val Leu Thr Asn Gly Ala Tyr Leu Leu Asn Val Asp Cys Asp
       545                 550                 555
   cat tac ttc aat agc agc aaa gct ctt aga gaa gca atg tgc ttc atg      1908
   His Tyr Phe Asn Ser Ser Lys Ala Leu Arg Glu Ala Met Cys Phe Met
   560                 565                 570                 575
   atg gat ccg gct cta gga agg aaa act tgt tat gta caa ttt cca cag      1956
   Met Asp Pro Ala Leu Gly Arg Lys Thr Cys Tyr Val Gln Phe Pro Gln
                   580                 585                 590
   aga ttt gat ggc att gac ttg cac gat cga tat gct aat cgg aac ata      2004
   Arg Phe Asp Gly Ile Asp Leu His Asp Arg Tyr Ala Asn Arg Asn Ile
               595                 600                 605
   gtt ttc ttt gat atc aac atg aaa ggt ctg gat ggc att cag ggt cca      2052
   Val Phe Phe Asp Ile Asn Met Lys Gly Leu Asp Gly Ile Gln Gly Pro
           610                 615                 620
   gtt tac gtg gga aca gga tgc tgt ttc aat aga cag gct ttg tat gga      2100
   Val Tyr Val Gly Thr Gly Cys Cys Phe Asn Arg Gln Ala Leu Tyr Gly
       625                 630                 635
   tac gat cct gtt ttg act gaa gct gat ctg gag cca aac att gtt att      2148
   Tyr Asp Pro Val Leu Thr Glu Ala Asp Leu Glu Pro Asn Ile Val Ile
   640                 645                 650                 655
   aag agc tgc tgt ggt aga agg aag aaa aag aac aag agt tat atg gat      2196
   Lys Ser Cys Cys Gly Arg Arg Lys Lys Lys Asn Lys Ser Tyr Met Asp
                   660                 665                 670
   agt caa agc cgt att atg aag aga aca gaa tct tca gct ccc atc ttc      2244
   Ser Gln Ser Arg Ile Met Lys Arg Thr Glu Ser Ser Ala Pro Ile Phe
               675                 680                 685
   aat atg gaa gac atc gaa gag ggt att gaa ggt tac gag gat gaa agg      2292
   Asn Met Glu Asp Ile Glu Glu Gly Ile Glu Gly Tyr Glu Asp Glu Arg
           690                 695                 700
   tca gtg ctt atg tcc cag agg aaa ttg gag aaa cgc ttt ggt cag tct      2340
   Ser Val Leu Met Ser Gln Arg Lys Leu Glu Lys Arg Phe Gly Gln Ser
       705                 710                 715
   cct att ttc att gca tcc acc ttt atg aca caa ggt ggc ata cca cct      2388
   Pro Ile Phe Ile Ala Ser Thr Phe Met Thr Gln Gly Gly Ile Pro Pro
   720                 725                 730                 735
   tca aca aac cca gct tct cta cta aag gaa gct atc cat gtc atc agt      2436
   Ser Thr Asn Pro Ala Ser Leu Leu Lys Glu Ala Ile His Val Ile Ser
                   740                 745                 750
   tgt gga tat gag gac aaa act gaa tgg gga aaa gag att ggc tgg atc      2484
   Cys Gly Tyr Glu Asp Lys Thr Glu Trp Gly Lys Glu Ile Gly Trp Ile
               755                 760                 765
   tat ggt tca gta acg gag gat att ctg act ggg ttt aaa atg cat gca      2532
   Tyr Gly Ser Val Thr Glu Asp Ile Leu Thr Gly Phe Lys Met His Ala
           770                 775                 780
   agg ggc tgg caa tca atc tac tgc atg cca cca cga cct tgt ttc aag      2580
   Arg Gly Trp Gln Ser Ile Tyr Cys Met Pro Pro Arg Pro Cys Phe Lys
       785                 790                 795
   ggt tct gca cca atc aat ctt tcc gat cgt ctt aat cag gtg ctc cgt      2628
   Gly Ser Ala Pro Ile Asn Leu Ser Asp Arg Leu Asn Gln Val Leu Arg
   800                 805                 810                 815
   tgg gct ctt ggg tca gtg gaa att ctg ctt agt aga cat tgt cct atc      2676
   Trp Ala Leu Gly Ser Val Glu Ile Leu Leu Ser Arg His Cys Pro Ile
                   820                 825                 830
   tgg tat ggt tac aat gga cga ttg aag ctt ttg gag agg ctg gct tac      2724
   Trp Tyr Gly Tyr Asn Gly Arg Leu Lys Leu Leu Glu Arg Leu Ala Tyr
               835                 840                 845
   atc aac act att gta tat cca atc aca tcc att ccg ctt att gcc tat      2772
   Ile Asn Thr Ile Val Tyr Pro Ile Thr Ser Ile Pro Leu Ile Ala Tyr
           850                 855                 860
   tgt gtg ctt ccc gct atc tgc ctc ctt acc aat aaa ttt atc att cct      2820
   Cys Val Leu Pro Ala Ile Cys Leu Leu Thr Asn Lys Phe Ile Ile Pro
       865                 870                 875
   gag att agc aat tat gct ggg atg ttc ttc att ctt ctt ttc gcc tcc      2868
   Glu Ile Ser Asn Tyr Ala Gly Met Phe Phe Ile Leu Leu Phe Ala Ser
   880                 885                 890                 895
   att ttt gcc act ggt ata ttg gag ctt aga tgg agt ggt gtt ggc att      2916
   Ile Phe Ala Thr Gly Ile Leu Glu Leu Arg Trp Ser Gly Val Gly Ile
                   900                 905                 910
   gaa gat tgg tgg aga aat gag cag ttt tgg gtt att ggt ggc acc tct      2964
   Glu Asp Trp Trp Arg Asn Glu Gln Phe Trp Val Ile Gly Gly Thr Ser
               915                 920                 925
   gcc cat ctc ttc gca gtg ttc cag ggt ctg ctg aaa gtg ttg gct ggg     3012
   Ala His Leu Phe Ala Val Phe Gln Gly Leu Leu Lys Val Leu Ala Gly
           930                 935                 940
   att gat acc aac ttc aca gtt acc tca aag gca tct gat gag gat ggc     3060
   Ile Asp Thr Asn Phe Thr Val Thr Ser Lys Ala Ser Asp Glu Asp Gly
       945                 950                 955
   gac ttt gct gag cta tat gtg ttc aag tgg acc agt ttg ctc att cct     3108
   Asp Phe Ala Glu Leu Tyr Val Phe Lys Trp Thr Ser Leu Leu Ile Pro
   960                 965                 970                 975
   ccg acc act gtt ctt gtc att aac ctg gtc gga atg gtg gca gga att     3156
   Pro Thr Thr Val Leu Val Ile Asn Leu Val Gly Met Val Ala Gly Ile
                   980                 985                 990
   tct tat gcc att aac agt ggc tac caa tcc tgg ggt ccg ctc ttt gga     3204
   Ser Tyr Ala Ile Asn Ser Gly Tyr Gln Ser Trp Gly Pro Leu Phe Gly
               995                 1000                1005
   aag ctg ttc ttc tcg atc tgg gtg atc ctc cat ctc tac ccc ttc ctc     3252
   Lys Leu Phe Phe Ser Ile Trp Val Ile Leu His Leu Tyr Pro Phe Leu
           1010                1015                1020
   aag ggt ctc atg gga agg cag aac cgc aca cca aca atc gtc att gtc     3300
   Lys Gly Leu Met Gly Arg Gln Asn Arg Thr Pro Thr Ile Val Ile Val
       1025                1030                1035
   tgg tcc atc ctt ctt gca tct atc ttc tcc ttg ctg tgg gtg aag atc     3348
   Trp Ser Ile Leu Leu Ala Ser Ile Phe Ser Leu Leu Trp Val Lys Ile
   1040                1045                1050                1055
   gat cct ttc atc tcc ccg aca cag aaa gct gct gcc ttg ggg caa tgt     3396
   Asp Pro Phe Ile Ser Pro Thr Gln Lys Ala Ala Ala Leu Gly Gln Cys
                   1060                1065                1070
   ggc gtc aac t gctgatcgag acagtgactc ttatttgaag aggctcaatc          3446
   Gly Val Asn
   aagatctgcc ccctcgtgta aatacctgag gaggctagat gggaattcct tttgttgtag   3506
   gtgaggatgg atttgcatct aagttatgcc tctgttcatt agcttcttcc gtgccggtgc   3566
   tgctgcggac taagaatcac ggagcctttc taccttccat gtagcgccag ccagcagcgt   3626
   aagatgtgaa ttttgaagtt ttgttatgcg tgcagtttat tgttttagag taaattatca   3686
   tttgtttgtg ggaactgttc acacgagctt ataatggcaa tgctgttatt taaaaaaaaa   3746
   aaaaaaa                                                              3753
<210> 34
   <211> 1075
   <212> PRT
   <213> Zea mays
<400> 34
   Met Ala Ala Asn Lys Gly Met Val Ala Gly Ser His Asn Arg Asn Glu
   1               5                   10                  15
   Phe Val Met Ile Arg His Asp Gly Asp Val Pro Gly Ser Ala Lys Pro
               20                  25                  30
   Thr Lys Ser Ala Asn Gly Gln Val Cys Gln Ile Cys Gly Asp Ser Val
           35                  40                  45
   Gly Val Ser Ala Thr Gly Asp Val Phe Val Ala Cys Asn Glu Cys Ala
       50                  55                  60
   Phe Pro Val Cys Arg Pro Cys Tyr Glu Tyr Glu Arg Lys Glu Gly Asn
   65                  70                  75                  80
   Gln Cys Cys Pro Gln Cys Lys Thr Arg Tyr Lys Arg Gln Lys Gly Ser
                   85                  90                  95
   Pro Arg Val His Gly Asp Glu Asp Glu Glu Asp Val Asp Asp Leu Asp
               100                 105                 110
   Asn Glu Phe Asn Tyr Lys Gln Gly Ser Gly Lys Gly Pro Glu Trp Gln
           115                 120                 125
   Leu Gln Gly Asp Asp Ala Asp Leu Ser Ser Ser Ala Arg His Glu Pro
       130                 135                 140
   His His Arg Ile Pro Arg Leu Thr Ser Gly Gln Gln Ile Ser Gly Glu
   145                 150                 155                 160
   Ile Pro Asp Ala Ser Pro Asp Arg His Ser Ile Arg Ser Pro Thr Ser
                   165                 170                 175
   Ser Tyr Val Asp Pro Ser Val Pro Val Pro Val Arg Ile Val Asp Pro
               180                 185                 190
   Ser Lys Asp Leu Asn Ser Tyr Gly Leu Asn Ser Val Asp Trp Lys Glu
           195                 200                 205
   Arg Val Glu Ser Trp Arg Val Lys Gln Asp Lys Asn Met Met Gln Val
       210                 215                 220
   Thr Asn Lys Tyr Pro Glu Ala Arg Gly Gly Asp Met Glu Gly Thr Gly
   225                 230                 235                 240
   Ser Asn Gly Glu Xaa Met Gln Met Val Asp Asp Ala Arg Leu Pro Leu
                   245                 250                 255
   Ser Arg Ile Val Pro Ile Ser Ser Asn Gln Leu Asn Leu Tyr Arg Val
               260                 265                 270
   Val Ile Ile Leu Arg Leu Ile Ile Leu Cys Phe Phe Phe Gln Tyr Arg
           275                 280                 285
   Val Ser His Pro Val Arg Asp Ala Tyr Gly Leu Trp Leu Val Ser Val
       290                 295                 300
   Ile Cys Glu Val Trp Phe Ala Leu Ser Trp Leu Leu Asp Gln Phe Pro
   305                 310                 315                 320
   Lys Trp Tyr Pro Ile Asn Arg Glu Thr Tyr Leu Asp Arg Leu Ala Leu
                   325                 330                 335
   Arg Tyr Asp Arg Glu Gly Glu Pro Ser Gln Leu Ala Pro Ile Asp Val
               340                 345                 350
   Phe Val Ser Thr Val Asp Pro Leu Lys Glu Pro Pro Leu Ile Thr Ala
           355                 360                 365
   Asn Thr Val Leu Ser Ile Leu Ser Val Asp Tyr Pro Val Asp Lys Val
       370                 375                 380
   Ser Cys Tyr Val Ser Asp Asp Gly Ser Ala Met Leu Thr Phe Glu Ser
   385                 390                 395                 400
   Leu Ser Glu Thr Ala Glu Phe Ala Arg Lys Trp Val Pro Phe Cys Lys
                   405                 410                 415
   Lys His Asn Ile Glu Pro Arg Ala Pro Glu Phe Tyr Phe Ala Gln Lys
               420                 425                 430
   Ile Asp Tyr Leu Lys Asp Lys Ile Gln Pro Ser Phe Val Lys Glu Arg
           435                 440                 445
   Arg Ala Met Lys Arg Glu Tyr Glu Glu Phe Lys Val Arg Ile Asn Ala
       450                 455                 460
   Leu Val Ala Lys Ala Gln Lys Val Pro Glu Glu Gly Trp Thr Met Ala
   465                 470                 475                 480
   Asp Gly Thr Ala Trp Pro Gly Asn Asn Pro Arg Asp His Pro Gly Met
                   485                 490                 495
   Ile Gln Val Phe Leu Gly His Ser Gly Gly Leu Asp Thr Asp Gly Asn
               500                 505                 510
   Glu Leu Pro Arg Leu Val Tyr Val Ser Arg Glu Lys Arg Pro Gly Phe
           515                 520                 525
   Gln His His Lys Lys Ala Gly Ala Met Asn Ala Leu Ile Arg Val Ser
       530                 535                 540
   Ala Val Leu Thr Asn Gly Ala Tyr Leu Leu Asn Val Asp Cys Asp His
   545                 550                 555                 560
   Tyr Phe Asn Ser Ser Lys Ala Leu Arg Glu Ala Met Cys Phe Met Met
                   565                 570                 575
   Asp Pro Ala Leu Gly Arg Lys Thr Cys Tyr Val Gln Phe Pro Gln Arg
               580                 585                 590
   Phe Asp Gly Ile Asp Leu His Asp Arg Tyr Ala Asn Arg Asn Ile Val
           595                 600                 605
   Phe Phe Asp Ile Asn Met Lys Gly Leu Asp Gly Ile Gln Gly Pro Val
       610                 615                 620
   Tyr Val Gly Thr Gly Cys Cys Phe Asn Arg Gln Ala Leu Tyr Gly Tyr
   625                 630                 635                 640
   Asp Pro Val Leu Thr Glu Ala Asp Leu Glu Pro Asn Ile Val Ile Lys
                   645                 650                 655
   Ser Cys Cys Gly Arg Arg Lys Lys Lys Asn Lys Ser Tyr Met Asp Ser
               660                 665                 670
   Gln Ser Arg Ile Met Lys Arg Thr Glu Ser Ser Ala Pro Ile Phe Asn
           675                 680                 685
   Met Glu Asp Ile Glu Glu Gly Ile Glu Gly Tyr Glu Asp Glu Arg Ser
       690                 695                 700
   Val Leu Met Ser Gln Arg Lys Leu Glu Lys Arg Phe Gly Gln Ser Pro
   705                 710                 715                 720
   Ile Phe Ile Ala Ser Thr Phe Met Thr Gln Gly Gly Ile Pro Pro Ser
                   725                 730                 735
   Thr Asn Pro Ala Ser Leu Leu Lys Glu Ala Ile His Val Ile Ser Cys
               740                 745                 750
   Gly Tyr Glu Asp Lys Thr Glu Trp Gly Lys Glu Ile Gly Trp Ile Tyr
           755                 760                 765
   Gly Ser Val Thr Glu Asp Ile Leu Thr Gly Phe Lys Met His Ala Arg
       770                 775                 780
   Gly Trp Gln Ser Ile Tyr Cys Met Pro Pro Arg Pro Cys Phe Lys Gly
   785                 790                 795                 800
   Ser Ala Pro Ile Asn Leu Ser Asp Arg Leu Asn Gln Val Leu Arg Trp
                   805                 810                 815
   Ala Leu Gly Ser Val Glu Ile Leu Leu Ser Arg His Cys Pro Ile Trp
               820                 825                 830
   Tyr Gly Tyr Asn Gly Arg Leu Lys Leu Leu Glu Arg Leu Ala Tyr Ile
           835                 840                 845
   Asn Thr Ile Val Tyr Pro Ile Thr Ser Ile Pro Leu Ile Ala Tyr Cys
       850                 855                 860
   Val Leu Pro Ala Ile Cys Leu Leu Thr Asn Lys Phe Ile Ile Pro Glu
   865                 870                 875                 880
   Ile Ser Asn Tyr Ala Gly Met Phe Phe Ile Leu Leu Phe Ala Ser Ile
                   885                 890                 895
   Phe Ala Thr Gly Ile Leu Glu Leu Arg Trp Ser Gly Val Gly Ile Glu
               900                 905                 910
   Asp Trp Trp Arg Asn Glu Gln Phe Trp Val Ile Gly Gly Thr Ser Ala
           915                 920                 925
   His Leu Phe Ala Val Phe Gln Gly Leu Leu Lys Val Leu Ala Gly Ile
       930                 935                 940
   Asp Thr Asn Phe Thr Val Thr Ser Lys Ala Ser Asp Glu Asp Gly Asp
   945                 950                 955                 960
   Phe Ala Glu Leu Tyr Val Phe Lys Trp Thr Ser Leu Leu Ile Pro Pro
   965 970 975
   Thr Thr Val Leu Val Ile Asn Leu Val Gly Met Val Ala Gly Ile Ser
   980 985 990
   Tyr Ala Ile Asn Ser Gly Tyr Gln Ser Trp Gly Pro Leu Phe Gly Lys
   995 1000 1005
   Leu Phe Phe Ser Ile Trp Val Ile Leu His Leu Tyr Pro Phe Leu Lys
   1010 1015 1020
   Gly Leu Met Gly Arg Gln Asn Arg Thr Pro Thr Ile Val Ile Val Trp
   1025 1030 1035 1040
   Ser Ile Leu Leu Ala Ser Ile Phe Ser Leu Leu Trp Val Lys Ile Asp
   1045 1050 1055
   Pro Phe Ile Ser Pro Thr Gln Lys Ala Ala Ala Leu Gly Gln Cys Gly
   1060 1065 1070
   Val Asn Cys
   1075
<210> 35
   <211> 25
   <212> DNA
   <213> Zea mays
<400> 35
   atggcggcca acaaggggat ggtgg
   25
<210> 36
   <211> 25
   <212> DNA
   <213> Zea mays
<400> 36
   tcagcagttg acgccacatt gcccc
   25
<210> 37
   <211> 3969
   <212> DNA
   <213> Zea mays
<220>
   <221> CDS
   <222> (144)...(3399)
<400> 37
   cttctccctc gtcggtgcgg cgtggcgcgg ctcggcgttc ggtgagaaac cactcggggg    60
   atgaggatct gctgctagag tgagaggagc tacggtcagt atcctctgcc ttcgtcggcg   120
   gcggaagtgg aggggaggaa gcg atg gag gcg agc gcc ggg ctg gtg gcc ggc   173
                             Met Glu Ala Ser Ala Gly Leu Val Ala Gly
                             1               5                   10
   tcc cac aac cgc aac gag ctc gtc gtc atc cgc cgc gac ggc gat ccc     221
   Ser His Asn Arg Asn Glu Leu Val Val Ile Arg Arg Asp Gly Asp Pro
               15                  20                  25
   ggg ccg aag ccg ccg cgg gag cag aac ggg cag gtg tgc cag att tgc     269
   Gly Pro Lys Pro Pro Arg Glu Gln Asn Gly Gln Val Cys Gln Ile Cys
           30                  35                  40
   ggc gac gac gtc ggc ctt gcc ccc ggc ggg gac ccc ttc gtg gcg tgc      317
   Gly Asp Asp Val Gly Leu Ala Pro Gly Gly Asp Pro Phe Val Ala Cys
           45                  50                  55
   aac gag tgc gcc ttc ccc gtc tgc cgg gac tgc tac gaa tac gag cgc      365
   Asn Glu Cys Ala Phe Pro Val Cys Arg Asp Cys Tyr Glu Tyr Glu Arg
       60                  65                  70
   cgg gag ggc acg cag aac tgc ccc cag tgc aag act cga tac aag cgc      413
   Arg Glu Gly Thr Gln Asn Cys Pro Gln Cys Lys Thr Arg Tyr Lys Arg
   75                  80                  85                  90
   ctc aag ggc tgc caa cgt gtg acc ggt gac gag gag gag gac ggc gtc      461
   Leu Lys Gly Cys Gln Arg Val Thr Gly Asp Glu Glu Glu Asp Gly Val
                   95                  100                 105
   gat gac ctg gac aac gag ttc aac tgg gac ggc cat gac tcg cag tct      509
   Asp Asp Leu Asp Asn Glu Phe Asn Trp Asp Gly His Asp Ser Gln Ser
               110                 115                 120
   gtg gcc gag tcc atg ctc tac ggc cac atg agc tac ggc cgt gga ggt      557
   Val Ala Glu Ser Met Leu Tyr Gly His Met Ser Tyr Gly Arg Gly Gly
           125                 130                 135
   gac cct aat ggc gcg cca caa gct ttc cag ctc aac ccc aat gtt cca      605
   Asp Pro Asn Gly Ala Pro Gln Ala Phe Gln Leu Asn Pro Asn Val Pro
       140                 145                 150
   ctc ctc acc aac ggg caa atg gtg gat gac atc cca ccg gag cag cac      653
   Leu Leu Thr Asn Gly Gln Met Val Asp Asp Ile Pro Pro Glu Gln His
   155                 160                 165                 170
   gcg ctg gtg cct tct ttc atg ggt ggt ggg gga aag agg ata cat ccc      701
   Ala Leu Val Pro Ser Phe Met Gly Gly Gly Gly Lys Arg Ile His Pro
                   175                 180                 185
   ctt cct tat gcg gat ccc agc tta cct gtg caa ccc agg tct atg gac      749
   Leu Pro Tyr Ala Asp Pro Ser Leu Pro Val Gln Pro Arg Ser Met Asp
               190                 195                 200
   cca tcc aag gat ctt gct gca tat ggg tat ggt agt gtt gct tgg aag      797
   Pro Ser Lys Asp Leu Ala Ala Tyr Gly Tyr Gly Ser Val Ala Trp Lys
           205                 210                 215
   gaa cgg atg gag aat tgg aag cag aga caa gag agg atg cac cag acg      845
   Glu Arg Met Glu Asn Trp Lys Gln Arg Gln Glu Arg Met His Gln Thr
       220                 225                 230
   ggg aat gat ggt ggt ggt gat gat ggt gac gat gct gat cta cca cta      893
   Gly Asn Asp Gly Gly Gly Asp Asp Gly Asp Asp Ala Asp Leu Pro Leu
   235                 240                 245                 250
   atg gat gaa gca aga caa caa ctg tcc agg aaa att cca ctt cca tca      941
   Met Asp Glu Ala Arg Gln Gln Leu Ser Arg Lys Ile Pro Leu Pro Ser
                   255                 260                 265
   agc cag att aat cca tat agg atg att atc att att cgg ctt gtg gtt      989
   Ser Gln Ile Asn Pro Tyr Arg Met Ile Ile Ile Ile Arg Leu Val Val
               270                 275                 280
   ttg ggg ttc ttc ttc cac tac cga gtg atg cat ccg gtg aat gat gca      1037
   Leu Gly Phe Phe Phe His Tyr Arg Val Met His Pro Val Asn Asp Ala
           285                 290                 295
   ttt gct ttg tgg ctc ata tct gtt atc tgt gaa atc tgg ttt gcc atg      1085
   Phe Ala Leu Trp Leu Ile Ser Val Ile Cys Glu Ile Trp Phe Ala Met
       300                 305                 310
   tct tgg att ctt gat caa ttc cca aag tgg ttc cct att gag aga gag      1133
   Ser Trp Ile Leu Asp Gln Phe Pro Lys Trp Phe Pro Ile Glu Arg Glu
   315                 320                 325                 330
   act tac cta gac cgg ctg tca ctg agg ttc gac aag gaa ggc cag cca      1181
   Thr Tyr Leu Asp Arg Leu Ser Leu Arg Phe Asp Lys Glu Gly Gln Pro
                   335                 340                 345
   tct caa ctt gct cca att gat ttc ttt gtc agt acg gtt gat ccc tta      1229
   Ser Gln Leu Ala Pro Ile Asp Phe Phe Val Ser Thr Val Asp Pro Leu
               350                 355                 360
   aag gaa cct cct ttg gtc aca aca aat act gtt cta tct atc ctt tcg      1277
   Lys Glu Pro Pro Leu Val Thr Thr Asn Thr Val Leu Ser Ile Leu Ser
           365                 370                 375
   gtg gat tat cct gtt gat aag gtt tct tgc tat gtt tct gat gat ggt      1325
   Val Asp Tyr Pro Val Asp Lys Val Ser Cys Tyr Val Ser Asp Asp Gly
       380                 385                 390
   gct gca atg cta acg ttt gaa gca tta tct gaa aca tct gaa ttt gca      1373
   Ala Ala Met Leu Thr Phe Glu Ala Leu Ser Glu Thr Ser Glu Phe Ala
   395                 400                 405                 410
   aag aaa tgg gtt cct ttc tgc aaa cgg tac aat att gaa cct cgc gct      1421
   Lys Lys Trp Val Pro Phe Cys Lys Arg Tyr Asn Ile Glu Pro Arg Ala
                   415                 420                 425
   cca gag tgg tac ttc caa cag aag ata gac tac ttg aaa gac aag gtg      1469
   Pro Glu Trp Tyr Phe Gln Gln Lys Ile Asp Tyr Leu Lys Asp Lys Val
               430                 435                 440
   gca gca aac ttt gtt agg gag agg aga gca atg aag aga gag tat gag      1517
   Ala Ala Asn Phe Val Arg Glu Arg Arg Ala Met Lys Arg Glu Tyr Glu
           445                 450                 455
   gaa ttc aag gtg aga atc aat gcc tta gtt gcc aaa gcc cag aaa gtt      1565
   Glu Phe Lys Val Arg Ile Asn Ala Leu Val Ala Lys Ala Gln Lys Val
       460                 465                 470
   cct gaa gaa gga tgg aca atg caa gat gga acc ccc tgg cct gga aac      1613
   Pro Glu Glu Gly Trp Thr Met Gln Asp Gly Thr Pro Trp Pro Gly Asn
   475                 480                 485                 490
   aat gtt cgt gat cat cct gga atg att cag gtc ttc ctt ggc caa agc      1661
   Asn Val Arg Asp His Pro Gly Met Ile Gln Val Phe Leu Gly Gln Ser
                   495                 500                 505
   gga ggc ctt gac tgt gag gga aat gaa ctg cca cga ttg gtt tat gtt      1709
   Gly Gly Leu Asp Cys Glu Gly Asn Glu Leu Pro Arg Leu Val Tyr Val
               510                 515                 520
   tct aga gag aaa cga cca ggc tat aac cat cat aag aaa gct ggt gct      1757
   Ser Arg Glu Lys Arg Pro Gly Tyr Asn His His Lys Lys Ala Gly Ala
           525                 530                 535
   atg aat gca ttg gtc cga gtc tct gct gta cta aca aat gct cca tat      1805
   Met Asn Ala Leu Val Arg Val Ser Ala Val Leu Thr Asn Ala Pro Tyr
       540                 545                 550
   ttg tta aac ttg gat tgt gat cac tac atc aac aac agc aag gct ata      1853
   Leu Leu Asn Leu Asp Cys Asp His Tyr Ile Asn Asn Ser Lys Ala Ile
   555                 560                 565                 570
   aag gaa gca atg tgt ttt atg atg gac cct tta cta gga aag aag gtt      1901
   Lys Glu Ala Met Cys Phe Met Met Asp Pro Leu Leu Gly Lys Lys Val
                   575                 580                 585
   tgc tat gta cag ttc cct caa aga ttt gat ggg att gat cgc cat gac      1949
   Cys Tyr Val Gln Phe Pro Gln Arg Phe Asp Gly Ile Asp Arg His Asp
               590                 595                 600
   cga tat gct aac cgg aat gtt gtc ttt ttt gat atc aac atg aaa ggt      1997
   Arg Tyr Ala Asn Arg Asn Val Val Phe Phe Asp Ile Asn Met Lys Gly
           605                 610                 615
   ttg gat ggt att cag ggt cca att tat gtt ggt act gga tgt gta ttt      2045
   Leu Asp Gly Ile Gln Gly Pro Ile Tyr Val Gly Thr Gly Cys Val Phe
       620                 625                 630
   aga agg cag gca tta tat ggt tat gat gcc ccc aaa aca aag aag cca      2093
   Arg Arg Gln Ala Leu Tyr Gly Tyr Asp Ala Pro Lys Thr Lys Lys Pro
   635                 640                 645                 650
   cca tca agg act tgc aac tgc tgg ccc aag tgg tgc ttt tgc tgt tgc      2141
   Pro Ser Arg Thr Cys Asn Cys Trp Pro Lys Trp Cys Phe Cys Cys Cys
                   655                 660                 665
   tgc ttt ggc aat agg aag caa aag aag act acc aaa ccc aaa aca gag      2189
   Cys Phe Gly Asn Arg Lys Gln Lys Lys Thr Thr Lys Pro Lys Thr Glu
               670                 675                 680
   aag aaa aag tta tta ttt ttc aag aaa gaa gag aac caa tcc cct gca      2237
   Lys Lys Lys Leu Leu Phe Phe Lys Lys Glu Glu Asn Gln Ser Pro Ala
           685                 690                 695
   tat gct ctt ggt gaa att gac gaa gct gct cca gga gct gag aat gaa      2285
   Tyr Ala Leu Gly Glu Ile Asp Glu Ala Ala Pro Gly Ala Glu Asn Glu
       700                 705                 710
   aag gcc ggt att gta aat caa caa aaa tta gaa aag aaa ttt ggc caa      2333
   Lys Ala Gly Ile Val Asn Gln Gln Lys Leu Glu Lys Lys Phe Gly Gln
   715                 720                 725                 730
   tct tct gtt ttt gtt aca tcc aca ctt ctc gag aat ggt gga acc ttg      2381
   Ser Ser Val Phe Val Thr Ser Thr Leu Leu Glu Asn Gly Gly Thr Leu
                   735                 740                 745
   aag agt gca agt cct gct tct ctt ttg aaa gaa gct ata cat gtc att      2429
   Lys Ser Ala Ser Pro Ala Ser Leu Leu Lys Glu Ala Ile His Val Ile
               750                 755                 760
   agt tgt ggt tat gaa gac aag aca gac tgg gga aaa gag att ggc tgg      2477
   Ser Cys Gly Tyr Glu Asp Lys Thr Asp Trp Gly Lys Glu Ile Gly Trp
           765                 770                 775
   atc tat gga tca gtt aca gaa gat att cta act ggt ttc aag atg cat      2525
   Ile Tyr Gly Ser Val Thr Glu Asp Ile Leu Thr Gly Phe Lys Met His
       780                 785                 790
   tgt cat ggt tgg cgg tca att tac tgc ata cct aaa cgg gtt gca ttc      2573
   Cys His Gly Trp Arg Ser Ile Tyr Cys Ile Pro Lys Arg Val Ala Phe
   795                 800                 805                 810
   aaa ggt tct gca cct ctg aat ctt tca gat cgt ctt cac cag gtg ctt      2621
   Lys Gly Ser Ala Pro Leu Asn Leu Ser Asp Arg Leu His Gln Val Leu
                   815                 820                 825
   cgg tgg gct ctt ggg tct att gag atc ttc ttc agc aat cat tgc cct      2669
   Arg Trp Ala Leu Gly Ser Ile Glu Ile Phe Phe Ser Asn His Cys Pro
               830                 835                 840
   ctt tgg tat ggg tat ggt ggc ggt ctg aaa ttt ttg gaa aga ttt tcc      2717
   Leu Trp Tyr Gly Tyr Gly Gly Gly Leu Lys Phe Leu Glu Arg Phe Ser
           845                 850                 855
   tac atc aac tcc atc gtg tat cct tgg aca tct att ccc ctc ttg gct      2765
   Tyr Ile Asn Ser Ile Val Tyr Pro Trp Thr Ser Ile Pro Leu Leu Ala
       860                 865                 870
   tac tgt aca ttg cct gcc atc tgt tta ttg aca ggg aaa ttt atc act      2813
   Tyr Cys Thr Leu Pro Ala Ile Cys Leu Leu Thr Gly Lys Phe Ile Thr
   875                 880                 885                 890
   cca gag ctg aat aat gtt gcc agc ctg tgg ttc atg tca ctt ttt atc      2861
   Pro Glu Leu Asn Asn Val Ala Ser Leu Trp Phe Met Ser Leu Phe Ile
                   895                 900                 905
   tgc att ttt gct acg agc atc cta gaa atg aga tgg agt ggt gtt gga      2909
   Cys Ile Phe Ala Thr Ser Ile Leu Glu Met Arg Trp Ser Gly Val Gly
               910                 915                 920
   att gat gac tgg tgg agg aat gag cag ttc tgg gtc att gga ggt gtg      2957
   Ile Asp Asp Trp Trp Arg Asn Glu Gln Phe Trp Val Ile Gly Gly Val
           925                 930                 935
   tcc tca cac ctc ttt gct gtg ttc cag gga ctt ctc aag gtc ata gct      3005
   Ser Ser His Leu Phe Ala Val Phe Gln Gly Leu Leu Lys Val Ile Ala
       940                 945                 950
   ggt gtt gat aca agc ttc acc gtg aca tca aag ggt gga gat gat gag      3053
   Gly Val Asp Thr Ser Phe Thr Val Thr Ser Lys Gly Gly Asp Asp Glu
   955                 960                 965                 970
   gag ttc tca gag cta tat aca ttc aaa tgg act acc tta ttg ata cct      3101
   Glu Phe Ser Glu Leu Tyr Thr Phe Lys Trp Thr Thr Leu Leu Ile Pro
                   975                 980                 985
   cct acc acc ttg ctt cta ttg aac ttc att ggt gtg gtc gct ggc gtt      3149
   Pro Thr Thr Leu Leu Leu Leu Asn Phe Ile Gly Val Val Ala Gly Val
               990                 995                 1000
   tca aat gcg atc aat aac gga tat gag tca tgg ggc ccc ctc ttt ggg      3197
   Ser Asn Ala Ile Asn Asn Gly Tyr Glu Ser Trp Gly Pro Leu Phe Gly
           1005                1010                1015
   aag cta ttc ttt gca ttt tgg gtg att gtc cat ctt tat ccc ttt ctc      3245
   Lys Leu Phe Phe Ala Phe Trp Val Ile Val His Leu Tyr Pro Phe Leu
       1020                1025                1030
   aaa ggt ttg gtt gga agg caa aac agg aca cca acg att gtc atc gtc      3293
   Lys Gly Leu Val Gly Arg Gln Asn Arg Thr Pro Thr Ile Val Ile Val
   1035                1040                1045                1050
   tgg tcc att ctg ctg gct tca atc ttc tcg ctc ctt tgg gtt cgg att      3341
   Trp Ser Ile Leu Leu Ala Ser Ile Phe Ser Leu Leu Trp Val Arg Ile
                   1055                1060                1065
   gat cct ttc ctt gcg aag gat gat ggt ccg ctt ctt gag gag tgt ggt      3389
   Asp Pro Phe Leu Ala Lys Asp Asp Gly Pro Leu Leu Glu Glu Cys Gly
               1070                1075                1080
   ttg gat tgc a actaggatgt cagtgcatca gctcccccaa tctgcatatg            3439
   Leu Asp Cys
           1085
   cttgaagtat attttctggt gtttgtcccc atattcagtg tctgtagata agagacatga   3499
   aatgtcccaa gtttcttttg atccatggtg aacctactta atatctgaga gatatactgg   3559
   gggaaaatgg aggctgcggc aatccttgtg cagttgggcc gtggaataca gcatatgcaa   3619
   gtgtttgatt gtgcagcatt ctttattact tggtcgcaat atagatgggc tgagccgaac   3679
   agcaaggtat tttgattctg cactgctccc gtgtacaaac ttggttctca ataaggcagg   3739
   caggaatgca tctgccagtg gaacagagca acctgcacat tatttatgta tgcctgttca   3799
   ttggagggct tgttcattac atgttcgtct atactagaaa aaacagaata ttagcattaa   3859
   tctatagtta attaaagtat gtaaatgcgc ctgttttttg ttgtgtactg taatcatctg   3919
   agttggtttt gtgaaaaaaa aaaaaaaaaa aaaaaaaaaa aaaaaaaaaa              3969
<210> 38
   <211> 1086
   <212> PRT
   <213> Zea mays
<400> 38
   Met Glu Ala Ser Ala Gly Leu Val Ala Gly Ser His Asn Arg Asn Glu
   1               5                   10                  15
   Leu Val Val Ile Arg Arg Asp Gly Asp Pro Gly Pro Lys Pro Pro Arg
               20                  25                  30
   Glu Gln Asn Gly Gln Val Cys Gln Ile Cys Gly Asp Asp Val Gly Leu
           35                  40                  45
   Ala Pro Gly Gly Asp Pro Phe Val Ala Cys Asn Glu Cys Ala Phe Pro
       50                  55                  60
   Val Cys Arg Asp Cys Tyr Glu Tyr Glu Arg Arg Glu Gly Thr Gln Asn
   65                  70                  75                  80
   Cys Pro Gln Cys Lys Thr Arg Tyr Lys Arg Leu Lys Gly Cys Gln Arg
                   85                  90                  95
   Val Thr Gly Asp Glu Glu Glu Asp Gly Val Asp Asp Leu Asp Asn Glu
               100                 105                 110
   Phe Asn Trp Asp Gly His Asp Ser Gln Ser Val Ala Glu Ser Met Leu
           115                 120                 125
   Tyr Gly His Met Ser Tyr Gly Arg Gly Gly Asp Pro Asn Gly Ala Pro
       130                 135                 140
   Gln Ala Phe Gln Leu Asn Pro Asn Val Pro Leu Leu Thr Asn Gly Gln
   145                 150                 155                 160
   Met Val Asp Asp Ile Pro Pro Glu Gln His Ala Leu Val Pro Ser Phe
                   165                 170                 175
   Met Gly Gly Gly Gly Lys Arg Ile His Pro Leu Pro Tyr Ala Asp Pro
               180                 185                 190
   Ser Leu Pro Val Gln Pro Arg Ser Met Asp Pro Ser Lys Asp Leu Ala
           195                 200                 205
   Ala Tyr Gly Tyr Gly Ser Val Ala Trp Lys Glu Arg Met Glu Asn Trp
       210                 215                 220
   Lys Gln Arg Gln Glu Arg Met His Gln Thr Gly Asn Asp Gly Gly Gly
   225                 230                 235                 240
   Asp Asp Gly Asp Asp Ala Asp Leu Pro Leu Met Asp Glu Ala Arg Gln
                   245                 250                 255
   Gln Leu Ser Arg Lys Ile Pro Leu Pro Ser Ser Gln Ile Asn Pro Tyr
               260                 265                 270
   Arg Met Ile Ile Ile Ile Arg Leu Val Val Leu Gly Phe Phe Phe His
           275                 280                 285
   Tyr Arg Val Met His Pro Val Asn Asp Ala Phe Ala Leu Trp Leu Ile
       290                 295                 300
   Ser Val Ile Cys Glu Ile Trp Phe Ala Met Ser Trp Ile Leu Asp Gln
   305                 310                 315                 320
   Phe Pro Lys Trp Phe Pro Ile Glu Arg Glu Thr Tyr Leu Asp Arg Leu
                   325                 330                 335
   Ser Leu Arg Phe Asp Lys Glu Gly Gln Pro Ser Gln Leu Ala Pro Ile
               340                 345                 350
   Asp Phe Phe Val Ser Thr Val Asp Pro Leu Lys Glu Pro Pro Leu Val
           355                 360                 365
   Thr Thr Asn Thr Val Leu Ser Ile Leu Ser Val Asp Tyr Pro Val Asp
       370                 375                 380
   Lys Val Ser Cys Tyr Val Ser Asp Asp Gly Ala Ala Met Leu Thr Phe
   385                 390                 395                 400
   Glu Ala Leu Ser Glu Thr Ser Glu Phe Ala Lys Lys Trp Val Pro Phe
                   405                 410                 415
   Cys Lys Arg Tyr Asn Ile Glu Pro Arg Ala Pro Glu Trp Tyr Phe Gln
               420                 425                 430
   Gln Lys Ile Asp Tyr Leu Lys Asp Lys Val Ala Ala Asn Phe Val Arg
           435                 440                 445
   Glu Arg Arg Ala Met Lys Arg Glu Tyr Glu Glu Phe Lys Val Arg Ile
       450                 455                 460
   Asn Ala Leu Val Ala Lys Ala Gln Lys Val Pro Glu Glu Gly Trp Thr
   465                 470                 475                 480
   Met Gln Asp Gly Thr Pro Trp Pro Gly Asn Asn Val Arg Asp His Pro
                   485                 490                 495
   Gly Met Ile Gln Val Phe Leu Gly Gln Ser Gly Gly Leu Asp Cys Glu
               500                 505                 510
   Gly Asn Glu Leu Pro Arg Leu Val Tyr Val Ser Arg Glu Lys Arg Pro
           515                 520                 525
   Gly Tyr Asn His His Lys Lys Ala Gly Ala Met Asn Ala Leu Val Arg
           530                 535                 540
   Val Ser Ala Val Leu Thr Asn Ala Pro Tyr Leu Leu Asn Leu Asp Cys
   545                 550                 555                 560
   Asp His Tyr Ile Asn Asn Ser Lys Ala Ile Lys Glu Ala Met Cys Phe
                   565                 570                 575
   Met Met Asp Pro Leu Leu Gly Lys Lys Val Cys Tyr Val Gln Phe Pro
               580                 585                 590
   Gln Arg Phe Asp Gly Ile Asp Arg His Asp Arg Tyr Ala Asn Arg Asn
           595                 600                 605
   Val Val Phe Phe Asp Ile Asn Met Lys Gly Leu Asp Gly Ile Gln Gly
       610                 615                 620
   Pro Ile Tyr Val Gly Thr Gly Cys Val Phe Arg Arg Gln Ala Leu Tyr
   625                 630                 635                 640
   Gly Tyr Asp Ala Pro Lys Thr Lys Lys Pro Pro Ser Arg Thr Cys Asn
                   645                 650                 655
   Cys Trp Pro Lys Trp Cys Phe Cys Cys Cys Cys Phe Gly Asn Arg Lys
               660                 665                 670
   Gln Lys Lys Thr Thr Lys Pro Lys Thr Glu Lys Lys Lys Leu Leu Phe
           675                 680                 685
   Phe Lys Lys Glu Glu Asn Gln Ser Pro Ala Tyr Ala Leu Gly Glu Ile
       690                 695                 700
   Asp Glu Ala Ala Pro Gly Ala Glu Asn Glu Lys Ala Gly Ile Val Asn
   705                 710                 715                 720
   Gln Gln Lys Leu Glu Lys Lys Phe Gly Gln Ser Ser Val Phe Val Thr
                   725                 730                 735
   Ser Thr Leu Leu Glu Asn Gly Gly Thr Leu Lys Ser Ala Ser Pro Ala
               740                 745                 750
   Ser Leu Leu Lys Glu Ala Ile His Val Ile Ser Cys Gly Tyr Glu Asp
           755                 760                 765
   Lys Thr Asp Trp Gly Lys Glu Ile Gly Trp Ile Tyr Gly Ser Val Thr
       770                 775                 780
   Glu Asp Ile Leu Thr Gly Phe Lys Met His Cys His Gly Trp Arg Ser
   785                 790                 795                 800
   Ile Tyr Cys Ile Pro Lys Arg Val Ala Phe Lys Gly Ser Ala Pro Leu
                   805                 810                 815
   Asn Leu Ser Asp Arg Leu His Gln Val Leu Arg Trp Ala Leu Gly Ser
               820                 825                 830
   Ile Glu Ile Phe Phe Ser Asn His Cys Pro Leu Trp Tyr Gly Tyr Gly
           835                 840                 845
   Gly Gly Leu Lys Phe Leu Glu Arg Phe Ser Tyr Ile Asn Ser Ile Val
       850                 855                 860
   Tyr Pro Trp Thr Ser Ile Pro Leu Leu Ala Tyr Cys Thr Leu Pro Ala
   865                 870                 875                 880
   Ile Cys Leu Leu Thr Gly Lys Phe Ile Thr Pro Glu Leu Asn Asn Val
                   885                 890                 895
   Ala Ser Leu Trp Phe Met Ser Leu Phe Ile Cys Ile Phe Ala Thr Ser
               900                 905                 910
   Ile Leu Glu Met Arg Trp Ser Gly Val Gly Ile Asp Asp Trp Trp Arg
           915                 920                 925
   Asn Glu Gln Phe Trp Val Ile Gly Gly Val Ser Ser His Leu Phe Ala
       930                 935                 940
   Val Phe Gln Gly Leu Leu Lys Val Ile Ala Gly Val Asp Thr Ser Phe
   945                 950                 955                 960
   Thr Val Thr Ser Lys Gly Gly Asp Asp Glu Glu Phe Ser Glu Leu Tyr
                   965                 970                 975
   Thr Phe Lys Trp Thr Thr Leu Leu Ile Pro Pro Thr Thr Leu Leu Leu
               980                 985                 990
   Leu Asn Phe Ile Gly Val Val Ala Gly Val Ser Asn Ala Ile Asn Asn
   995 1000 1005
   Gly Tyr Glu Ser Trp Gly Pro Leu Phe Gly Lys Leu Phe Phe Ala Phe
   1010 1015 1020
   Trp Val Ile Val His Leu Tyr Pro Phe Leu Lys Gly Leu Val Gly Arg
   1025 1030 1035 1040
   Gln Asn Arg Thr Pro Thr Ile Val Ile Val Trp Ser Ile Leu Leu Ala
   1045 1050 1055
   Ser Ile Phe Ser Leu Leu Trp Val Arg Ile Asp Pro Phe Leu Ala Lys
   1060 1065 1070
   Asp Asp Gly Pro Leu Leu Glu Glu Cys Gly Leu Asp Cys Asn
   1075 1080 1085
<210> 39
   <211> 25
   <212> DNA
   <213> Zea mays
<400> 39
   atggaggcga gcgccgggct ggtgg
   25
<210> 40
   <211> 25
   <212> DNA
   <213> Zea mays
<400> 40
   ctagttgcaa tccaaaccac actcc
   25
<210> 41
   <211> 3725
   <212> DMA
   <213> Zea mays
<220>
   <221> CDS
   <222> (179)...(3398)
<400> 41
   gcagcagcag caccaccact gcgcggcatt gcagcgagca agcgggaggg atctggggca     60
   tggtggcggt cgctgccgct gccgctcgga tctagagggc cgcacgggct gattgccctc    120
   cgccggcctc gtcggtgtcg gtggagtgtg aatcggtgtg tgtaggagga gcgcggag      178
   atg gcg gcc aac aag ggg atg gtg gca ggc tct cac aac cgc aac gag      226
   Met Ala Ala Asn Lys Gly Met Val Ala Gly Ser His Asn Arg Asn Glu
    1               5                  10                  15
   ttc gtc atg atc cgc cac gac ggc gac gcg cct gtc ccg gct aag ccc      274
   Phe Val Met Ile Arg His Asp Gly Asp Ala Pro Val Pro Ala Lys Pro
               20                  25                  30
   acg aag agt gcg aat ggg cag gtc tgc cag att tgt ggc gac act gtt      322
   Thr Lys Ser Ala Asn Gly Gln Val Cys Gln Ile Cys Gly Asp Thr Val
           35                  40                  45
   ggc gtt tca gcc act ggt gat gtc ttt gtt gcc tgc aat gag tgt gcc      370
   Gly Val Ser Ala Thr Gly Asp Val Phe Val Ala Cys Asn Glu Cys Ala
       50                  55                  60
   ttc cct gtc tgc cgc cct tgc tat gag tac gag cgc aag gaa ggg aac     418
   Phe Pro Val Cys Arg Pro Cys Tyr Glu Tyr Glu Arg Lys Glu Gly Asn
    65                 70                  75                  80
   caa tgc tgc cct cag tgc aag act aga tac aag aga cag aaa ggt agc     466
   Gln Cys Cys Pro Gln Cys Lys Thr Arg Tyr Lys Arg Gln Lys Gly Ser
                   85                  90                  95
   cct cga gtt cat ggt gat gat gag gag gaa gat gtt gat gac ctg gac     514
   Pro Arg Val His Gly Asp Asp Glu Glu Glu Asp Val Asp Asp Leu Asp
               100                 105                 110
   aat gaa ttc aac tat aag caa ggc aat ggg aag ggc cca gag tgg cag     562
   Asn Glu Phe Asn Tyr Lys Gln Gly Asn Gly Lys Gly Pro Glu Trp Gln
           115                 120                 125
   ctt caa gga gat gac gct gat ctg tct tca tct gct cgc cat gac cca     610
   Leu Gln Gly Asp Asp Ala Asp Leu Ser Ser Ser Ala Arg His Asp Pro
       130                 135                 140
   cac cat cgg att cca cgc ctt aca agt gga caa cag ata tct gga gag     658
   His His Arg Ile Pro Arg Leu Thr Ser Gly Gln Gln Ile Ser Gly Glu
   145                 150                 155                 160
   atc cct gat gca tcc cct gac cgt cat tct atc cgc agt cca aca tcg     706
   Ile Pro Asp Ala Ser Pro Asp Arg His Ser Ile Arg Ser Pro Thr Ser
                   165                 170                 175
   agc tat gtt gat cca agc gtt cca gtt cct gtg agg att gtg gac ccc     754
   Ser Tyr Val Asp Pro Ser Val Pro Val Pro Val Arg Ile Val Asp Pro
               180                 185                 190
   tcg aag gac ttg aat tcc tat ggg ctt aat agt gtt gac tgg aag gaa     802
   Ser Lys Asp Leu Asn Ser Tyr Gly Leu Asn Ser Val Asp Trp Lys Glu
           195                 200                 205
   aga gtt gag agc tgg agg gtt aaa cag gac aaa aat atg ttg caa gtg     850
   Arg Val Glu Ser Trp Arg Val Lys Gln Asp Lys Asn Met Leu Gln Val
       210                 215                 220
   act aat aaa tat cca gag gct aga gga gac atg gag ggg act ggc tca     898
   Thr Asn Lys Tyr Pro Glu Ala Arg Gly Asp Met Glu Gly Thr Gly Ser
   225                 230                 235                 240
   aat gga gaa gat atg caa atg gtt gat gat gca cgc cta cct ttg agc     946
   Asn Gly Glu Asp Met Gln Met Val Asp Asp Ala Arg Leu Pro Leu Ser
                   245                 250                 255
   cgc att gtg cca att tcc tca aac cag ctc aac ctt tac cgg ata gta     994
   Arg Ile Val Pro Ile Ser Ser Asn Gln Leu Asn Leu Tyr Arg Ile Val
               260                 265                 270
   atc att ctc cgt ctt atc atc ctg tgc ttc ttc ttc caa tat cgt atc    1042
   Ile Ile Leu Arg Leu Ile Ile Leu Cys Phe Phe Phe Gln Tyr Arg Ile
           275                 280                 285
   agt cat cca gtg cgt aat gct tat gga ttg tgg cta gta tct gtt atc    1090
   Ser His Pro Val Arg Asn Ala Tyr Gly Leu Trp Leu Val Ser Val Ile
       290                 295                 300
   tgt gag gtc tgg ttt gcc ttg tcc tgg ctt cta gat cag ttc cca aaa      1138
   Cys Glu Val Trp Phe Ala Leu Ser Trp Leu Leu Asp Gln Phe Pro Lys
   305                 310                 315                 320
   tgg tat cca atc aac cgt gag aca tat ctc gac agg ctt gca ttg agg      1186
   Trp Tyr Pro Ile Asn Arg Glu Thr Tyr Leu Asp Arg Leu Ala Leu Arg
                   325                 330                 335
   tat gat aga gag gga gag cca tca cag ctg gct ccc att gat gtc ttt      1234
   Tyr Asp Arg Glu Gly Glu Pro Ser Gln Leu Ala Pro Ile Asp Val Phe
               340                 345                 350
   gtc agt aca gtg gat cca ttg aag gaa cct cca ctg atc aca gcc aac      1282
   Val Ser Thr Val Asp Pro Leu Lys Glu Pro Pro Leu Ile Thr Ala Asn
           355                 360                 365
   act gtt ttg tcc att ctt gct gtg gat tac cct gtt gac aaa gtg tca      1330
   Thr Val Leu Ser Ile Leu Ala Val Asp Tyr Pro Val Asp Lys Val Ser
       370                 375                 380
   tgc tat gtt tct gat gat ggc tca gct atg ctg act ttt gag tct ctc      1378
   Cys Tyr Val Ser Asp Asp Gly Ser Ala Met Leu Thr Phe Glu Ser Leu
   385                 390                 395                 400
   tct gaa act gcc gaa ttt gct aga aag tgg gtt ccc ttt tgt aag aag      1426
   Ser Glu Thr Ala Glu Phe Ala Arg Lys Trp Val Pro Phe Cys Lys Lys
                   405                 410                 415
   cac aat att gaa cca aga gct cca gaa ttt tac ttt gct caa aaa ata      1474
   His Asn Ile Glu Pro Arg Ala Pro Glu Phe Tyr Phe Ala Gln Lys Ile
               420                 425                 430
   gat tac ctg aag gac aaa att caa cct tca ttt gtt aag gaa aga cga      1522
   Asp Tyr Leu Lys Asp Lys Ile Gln Pro Ser Phe Val Lys Glu Arg Arg
           435                 440                 445
   gca atg aag aga gag tat gaa gaa ttc aaa ata aga atc aat gcc ctt      1570
   Ala Met Lys Arg Glu Tyr Glu Glu Phe Lys Ile Arg Ile Asn Ala Leu
       450                 455                 460
   gtt gcc aaa gca cag aaa gtg cct gaa gag ggg tgg acc atg gct gat      1618
   Val Ala Lys Ala Gln Lys Val Pro Glu Glu Gly Trp Thr Met Ala Asp
   465                 470                 475                 480
   gga act gct tgg cct ggg aat aac cct agg gac cat cct ggc atg att      1666
   Gly Thr Ala Trp Pro Gly Asn Asn Pro Arg Asp His Pro Gly Met Ile
                   485                 490                 495
   cag gtg ttc ttg ggg cac agt ggt ggg ctt gac act gat gga aat gaa      1714
   Gln Val Phe Leu Gly His Ser Gly Gly Leu Asp Thr Asp Gly Asn Glu
               500                 505                 510
   tta cca cgt ctt gtc tat gtc tct cgt gaa aag aga cca ggc ttt cag      1762
   Leu Pro Arg Leu Val Tyr Val Ser Arg Glu Lys Arg Pro Gly Phe Gln
           515                 520                 525
   cat cac aag aag gct ggt gca atg aat gca ctg att cgt gta tct gct      1810
   His His Lys Lys Ala Gly Ala Met Asn Ala Leu Ile Arg Val Ser Ala
       530                 535                 540
   gtg ctg aca aat ggt gcc tat ctt ctc aat gtg gat tgt gac cat tac      1858
   Val Leu Thr Asn Gly Ala Tyr Leu Leu Asn Val Asp Cys Asp His Tyr
   545                 550                 555                 560
   ttc aat agc agc aaa gct ctt aga gaa gca atg tgc ttc atg atg gat      1906
   Phe Asn Ser Ser Lys Ala Leu Arg Glu Ala Met Cys Phe Met Met Asp
                   565                 570                 575
   cca gct cta gga agg aaa act tgt tat gta caa ttt cca caa aga ttt      1954
   Pro Ala Leu Gly Arg Lys Thr Cys Tyr Val Gln Phe Pro Gln Arg Phe
               580                 585                 590
   gat ggc att gac ttg cac gat cga tat gct aat agg aac ata gtc ttc      2002
   Asp Gly Ile Asp Leu His Asp Arg Tyr Ala Asn Arg Asn Ile Val Phe
           595                 600                 605
   ttt gat atc aac atg aaa ggt cta gat ggc att cag ggt cca gtc tat      2050
   Phe Asp Ile Asn Met Lys Gly Leu Asp Gly Ile Gln Gly Pro Val Tyr
       610                 615                 620
   gtg gga aca gga tgc tgt ttc aat agg cag gct ttg tat gga tat gat      2098
   Val Gly Thr Gly Cys Cys Phe Asn Arg Gln Ala Leu Tyr Gly Tyr Asp
   625                 630                 635                 640
   cct gtt ttg act gaa gct gat ctg gaa cct aac att gtt gtt aag agc      2146
   Pro Val Leu Thr Glu Ala Asp Leu Glu Pro Asn Ile Val Val Lys Ser
                   645                 650                 655
   tgc tgt ggt aga agg aag aga aag aac aag agt tat atg gat agt caa      2194
   Cys Cys Gly Arg Arg Lys Arg Lys Asn Lys Ser Tyr Met Asp Ser Gln
               660                 665                 670
   agc cgt att atg aag aga aca gaa tct tca gct ccc atc ttt aac atg      2242
   Ser Arg Ile Met Lys Arg Thr Glu Ser Ser Ala Pro Ile Phe Asn Met
           675                 680                 685
   gaa gac atc gag gag ggt att gaa ggt tat gag gat gaa agg tca gtg      2290
   Glu Asp Ile Glu Glu Gly Ile Glu Gly Tyr Glu Asp Glu Arg Ser Val
       690                 695                 700
   ctt atg tcc cag agg aaa ttg gag aaa cgc ttt ggt cag tct cca atc      2338
   Leu Met Ser Gln Arg Lys Leu Glu Lys Arg Phe Gly Gln Ser Pro Ile
   705                 710                 715                 720
   ttc att gca tcc acc ttt atg act caa ggt ggc ata cca cct tca aca      2386
   Phe Ile Ala Ser Thr Phe Met Thr Gln Gly Gly Ile Pro Pro Ser Thr
                   725                 730                 735
   aac cca gct tct cta ctg aag gaa gct atc cat gtt atc agc tgt ggg      2434
   Asn Pro Ala Ser Leu Leu Lys Glu Ala Ile His Val Ile Ser Cys Gly
               740                 745                 750
   tac gag gac aaa act gaa tgg gga aaa gag att ggc tgg atc tat ggt      2482
   Tyr Glu Asp Lys Thr Glu Trp Gly Lys Glu Ile Gly Trp Ile Tyr Gly
           755                 760                 765
   tca gtt aca gag gat att ctg act ggg ttt aaa atg cat gca aga ggc      2530
   Ser Val Thr Glu Asp Ile Leu Thr Gly Phe Lys Met His Ala Arg Gly
       770                 775                 780
   tgg caa tca atc tac tgc atg cca cca cga cct tgt ttc aag ggt tct      2578
   Trp Gln Ser Ile Tyr Cys Met Pro Pro Arg Pro Cys Phe Lys Gly Ser
   785                 790                 795                 800
   gca cca atc aat ctt tct gat cgt ctt aat cag gtg ctc cgt tgg gct      2626
   Ala Pro Ile Asn Leu Ser Asp Arg Leu Asn Gln Val Leu Arg Trp Ala
                   805                 810                 815
   ctt ggg tca gtg gaa att ctg ctt agc aga cat tgt cct ata tgg tat      2674
   Leu Gly Ser Val Glu Ile Leu Leu Ser Arg His Cys Pro Ile Trp Tyr
               820                 825                 830
   ggc tac aat ggg cga ttg aag ctt ttg gag agg ctg gct tac att aac      2722
   Gly Tyr Asn Gly Arg Leu Lys Leu Leu Glu Arg Leu Ala Tyr Ile Asn
           835                 840                 845
   acc att gtt tat cca atc aca tct gtt ccg ctt atc gcc tat tgt gtg      2770
   Thr Ile Val Tyr Pro Ile Thr Ser Val Pro Leu Ile Ala Tyr Cys Val
       850                 855                 860
   ctt cct gct atc tgt ctt ctt acc aat aaa ttt atc att cct gag att      2818
   Leu Pro Ala Ile Cys Leu Leu Thr Asn Lys Phe Ile Ile Pro Glu Ile
   865                 870                 875                 880
   agt aat tat gct gga atg ttc ttc att ctt ctt ttt gcc tcc att ttc      2866
   Ser Asn Tyr Ala Gly Met Phe Phe Ile Leu Leu Phe Ala Ser Ile Phe
                   885                 890                 895
   gca act ggt ata ttg gag ctc aga tgg agt ggt gtt ggc att gaa gat      2914
   Ala Thr Gly Ile Leu Glu Leu Arg Trp Ser Gly Val Gly Ile Glu Asp
               900                 905                 910
   tgg tgg aga aat gag cag ttt tgg gtt att ggt ggc acc tct gcc cat      2962
   Trp Trp Arg Asn Glu Gln Phe Trp Val Ile Gly Gly Thr Ser Ala His
           915                 920                 925
   ctc ttc gcg gtg ttc cag ggt ctg ctg aaa gtg ttg gct ggg att gat      3010
   Leu Phe Ala Val Phe Gln Gly Leu Leu Lys Val Leu Ala Gly Ile Asp
       930                 935                 940
   acc aac ttc aca gtt acc tca aag gca tct gat gag gat ggc gac ttt      3058
   Thr Asn Phe Thr Val Thr Ser Lys Ala Ser Asp Glu Asp Gly Asp Phe
   945                 950                 955                 960
   gct gag cta tat gtg ttc aag tgg acc agt ttg ctc atc cct ccg acc      3106
   Ala Glu Leu Tyr Val Phe Lys Trp Thr Ser Leu Leu Ile Pro Pro Thr
                   965                 970                 975
   act gtt ctt gtc att aac ctg gtc gga atg gtg gca gga att tcg tat      3154
   Thr Val Leu Val Ile Asn Leu Val Gly Met Val Ala Gly Ile Ser Tyr
               980                 985                 990
   gcc att aac agc ggc tac caa tcc tgg ggt ccg ctc ttt gga aag ctg      3202
   Ala Ile Asn Ser Gly Tyr Gln Ser Trp Gly Pro Leu Phe Gly Lys Leu
           995                 1000                1005
   ttc ttc tcg atc tgg gtg atc ctc cat ctc tac ccc ttc ctc aag ggt      3250
   Phe Phe Ser Ile Trp Val Ile Leu His Leu Tyr Pro Phe Leu Lys Gly
       1010                1015                1020
   ctc atg ggc agg cag aac cgc acg cca aca atc gtc atc gtt tgg tcc      3298
   Leu Met Gly Arg Gln Asn Arg Thr Pro Thr Ile Val Ile Val Trp Ser
   1025                1030                1035                1040
   atc ctc ctt gcg tct atc ttc tcc ttg ctg tgg gtg aag atc gat cct      3346
   Ile Leu Leu Ala Ser Ile Phe Ser Leu Leu Trp Val Lys Ile Asp Pro
                   1045                1050                1055
   ttc atc tcc ccg aca cag aaa gct gcc gcc ttg ggg caa tgt ggt gtg      3394
   Phe Ile Ser Pro Thr Gln Lys Ala Ala Ala Leu Gly Gln Cys Gly Val
               1060                1065                1070
   aac t gctgatccag attgtgactc ttatctgaag aggctcagcc aaagatctgc         3448
   Asn
   cccctcgtgt aaatacctga gggggctaga tgggaatttt ttgttgtaga tgaggatgga   3508
   tctgcatcca agttatgcct ctgtttatta gcttcttcgg tgccggtgct gctgcagaca   3568
   atcatggagc ctttctacct tgcttgtagt gctggccagc agcgtaaatt gtgaattctg   3628
   cattttttta tacgtggtgt ttattgtttt agagtaaatt atcatttgtt tgaggtaact  3688
   attcacacga actatatggc aatgctgtta tttaaaa                            3725
<210> 42
   <211> 1074
   <212> PRT
   <213> Zea mays
<400> 42
   Met Ala Ala Asn Lys Gly Met Val Ala Gly Ser His Asn Arg Asn Glu
    1               5                  10                  15
   Phe Val Met Ile Arg His Asp Gly Asp Ala Pro Val Pro Ala Lys Pro
               20                  25                  30
   Thr Lys Ser Ala Asn Gly Gln Val Cys Gln Ile Cys Gly Asp Thr Val
           35                  40                  45
   Gly Val Ser Ala Thr Gly Asp Val Phe Val Ala Cys Asn Glu Cys Ala
       50                  55                  60
   Phe Pro Val Cys Arg Pro Cys Tyr Glu Tyr Glu Arg Lys Glu Gly Asn
   65                  70                  75                  80
   Gln Cys Cys Pro Gln Cys Lys Thr Arg Tyr Lys Arg Gln Lys Gly Ser
                   85                  90                  95
   Pro Arg Val His Gly Asp Asp Glu Glu Glu Asp Val Asp Asp Leu Asp
               100                 105                 110
   Asn Glu Phe Asn Tyr Lys Gln Gly Asn Gly Lys Gly Pro Glu Trp Gln
           115                 120                 125
   Leu Gln Gly Asp Asp Ala Asp Leu Ser Ser Ser Ala Arg His Asp Pro
       130                 135                 140
   His His Arg Ile Pro Arg Leu Thr Ser Gly Gln Gln Ile Ser Gly Glu
   145                 150                 155                 160
   Ile Pro Asp Ala Ser Pro Asp Arg His Ser Ile Arg Ser Pro Thr Ser
                   165                 170                 175
   Ser Tyr Val Asp Pro Ser Val Pro Val Pro Val Arg Ile Val Asp Pro
               180                 185                 190
   Ser Lys Asp Leu Asn Ser Tyr Gly Leu Asn Ser Val Asp Trp Lys Glu
           195                 200                 205
   Arg Val Glu Ser Trp Arg Val Lys Gln Asp Lys Asn Met Leu Gln Val
       210                 215                 220
   Thr Asn Lys Tyr Pro Glu Ala Arg Gly Asp Met Glu Gly Thr Gly Ser
   225                 230                 235                 240
   Asn Gly Glu Asp Met Gln Met Val Asp Asp Ala Arg Leu Pro Leu Ser
                   245                 250                 255
   Arg Ile Val Pro Ile Ser Ser Asn Gln Leu Asn Leu Tyr Arg Ile Val
               260                 265                 270
   Ile Ile Leu Arg Leu Ile Ile Leu Cys Phe Phe Phe Gln Tyr Arg Ile
           275                 280                 285
   Ser His Pro Val Arg Asn Ala Tyr Gly Leu Trp Leu Val Ser Val Ile
       290                 295                 300
   Cys Glu Val Trp Phe Ala Leu Ser Trp Leu Leu Asp Gln Phe Pro Lys
   305                 310                 315                 320
   Trp Tyr Pro Ile Asn Arg Glu Thr Tyr Leu Asp Arg Leu Ala Leu Arg
                   325                 330                 335
   Tyr Asp Arg Glu Gly Glu Pro Ser Gln Leu Ala Pro Ile Asp Val Phe
               340                 345                 350
   Val Ser Thr Val Asp Pro Leu Lys Glu Pro Pro Leu Ile Thr Ala Asn
           355                 360                 365
   Thr Val Leu Ser Ile Leu Ala Val Asp Tyr Pro Val Asp Lys Val Ser
       370                 375                 380
   Cys Tyr Val Ser Asp Asp Gly Ser Ala Met Leu Thr Phe Glu Ser Leu
   385                 390                 395                 400
   Ser Glu Thr Ala Glu Phe Ala Arg Lys Trp Val Pro Phe Cys Lys Lys
                   405                 410                 415
   His Asn Ile Glu Pro Arg Ala Pro Glu Phe Tyr Phe Ala Gln Lys Ile
               420                 425                 430
   Asp Tyr Leu Lys Asp Lys Ile Gln Pro Ser Phe Val Lys Glu Arg Arg
           435                 440                 445
   Ala Met Lys Arg Glu Tyr Glu Glu Phe Lys Ile Arg Ile Asn Ala Leu
       450                 455                 460
   Val Ala Lys Ala Gln Lys Val Pro Glu Glu Gly Trp Thr Met Ala Asp
   465                 470                 475                 480
   Gly Thr Ala Trp Pro Gly Asn Asn Pro Arg Asp His Pro Gly Met Ile
                   485                 490                 495
   Gln Val Phe Leu Gly His Ser Gly Gly Leu Asp Thr Asp Gly Asn Glu
               500                 505                 510
   Leu Pro Arg Leu Val Tyr Val Ser Arg Glu Lys Arg Pro Gly Phe Gln
           515                 520                 525
   His His Lys Lys Ala Gly Ala Met Asn Ala Leu Ile Arg Val Ser Ala
       530                 535                 540
   Val Leu Thr Asn Gly Ala Tyr Leu Leu Asn Val Asp Cys Asp His Tyr
   545                 550                 555                 560
   Phe Asn Ser Ser Lys Ala Leu Arg Glu Ala Met Cys Phe Met Met Asp
                   565                 570                 575
   Pro Ala Leu Gly Arg Lys Thr Cys Tyr Val Gln Phe Pro Gln Arg Phe
               580                 585                 590
   Asp Gly Ile Asp Leu His Asp Arg Tyr Ala Asn Arg Asn Ile Val Phe
           595                 600                 605
   Phe Asp Ile Asn Met Lys Gly Leu Asp Gly Ile Gln Gly Pro Val Tyr
       610                 615                 620
   Val Gly Thr Gly Cys Cys Phe Asn Arg Gln Ala Leu Tyr Gly Tyr Asp
   625 630 635 640
   Pro Val Leu Thr Glu Ala Asp Leu Glu Pro Asn Ile Val Val Lys Ser
   645 650 655
   Cys Cys Gly Arg Arg Lys Arg Lys Asn Lys Ser Tyr Met Asp Ser Gln
   660 665 670
   Ser Arg Ile Met Lys Arg Thr Glu Ser Ser Ala Pro Ile Phe Asn Met
   675 680 685
   Glu Asp Ile Glu Glu Gly Ile Glu Gly Tyr Glu Asp Glu Arg Ser Val
   690 695 700
   Leu Met Ser Gln Arg Lys Leu Glu Lys Arg Phe Gly Gln Ser Pro Ile
   705 710 715 720
   Phe Ile Ala Ser Thr Phe Met Thr Gln Gly Gly Ile Pro Pro Ser Thr
   725 730 735
   Asn Pro Ala Ser Leu Leu Lys Glu Ala Ile His Val Ile Ser Cys Gly
   740 745 750
   Tyr Glu Asp Lys Thr Glu Trp Gly Lys Glu Ile Gly Trp Ile Tyr Gly
   755 760 765
   Ser Val Thr Glu Asp Ile Leu Thr Gly Phe Lys Met His Ala Arg Gly
   770 775 780
   Trp Gln Ser Ile Tyr Cys Met Pro Pro Arg Pro Cys Phe Lys Gly Ser
   785 790 795 800
   Ala Pro Ile Asn Leu Ser Asp Arg Leu Asn Gln Val Leu Arg Trp Ala
   805 810 815
   Leu Gly Ser Val Glu Ile Leu Leu Ser Arg His Cys Pro Ile Trp Tyr
   820 825 830
   Gly Tyr Asn Gly Arg Leu Lys Leu Leu Glu Arg Leu Ala Tyr Ile Asn
   835 840 845
   Thr Ile Val Tyr Pro Ile Thr Ser Val Pro Leu Ile Ala Tyr Cys Val
   850 855 860
   Leu Pro Ala Ile Cys Leu Leu Thr Asn Lys Phe Ile Ile Pro Glu Ile
   865 870 875 880
   Ser Asn Tyr Ala Gly Met Phe Phe Ile Leu Leu Phe Ala Ser Ile Phe
   885 890 895
   Ala Thr Gly Ile Leu Glu Leu Arg Trp Ser Gly Val Gly Ile Glu Asp
   900 905 910
   Trp Trp Arg Asn Glu Gln Phe Trp Val Ile Gly Gly Thr Ser Ala His
   915 920 925
   Leu Phe Ala Val Phe Gln Gly Leu Leu Lys Val Leu Ala Gly Ile Asp
   930 935 940
   Thr Asn Phe Thr Val Thr Ser Lys Ala Ser Asp Glu Asp Gly Asp Phe
   945 950 955 960
   Ala Glu Leu Tyr Val Phe Lys Trp Thr Ser Leu Leu Ile Pro Pro Thr
   965 970 975
   Thr Val Leu Val Ile Asn Leu Val Gly Met Val Ala Gly Ile Ser Tyr
   980 985 990
   Ala Ile Asn Ser Gly Tyr Gln Ser Trp Gly Pro Leu Phe Gly Lys Leu
   995 1000 1005
   Phe Phe Ser Ile Trp Val Ile Leu His Leu Tyr Pro Phe Leu Lys Gly
   1010 1015 1020
   Leu Met Gly Arg Gln Asn Arg Thr Pro Thr Ile Val Ile Val Trp Ser
   1025 1030 1035 1040
   Ile Leu Leu Ala Ser Ile Phe Ser Leu Leu Trp Val Lys Ile Asp Pro
   1045 1050 1055
   Phe Ile Ser Pro Thr Gln Lys Ala Ala Ala Leu Gly Gln Cys Gly Val
   1060 1065 1070
   Asn Cys
<210> 43
   <211> 25
   <212> DNA
   <213> Zea mays
<400> 43
   atggcggcca acaaggggat ggtgg
   25
<210> 44
   <211> 25
   <212> DNA
   <213> Zea mays
<400> 44
   tcagcagttc acaccacatt gcccc
   25
<210> 45
   <211> 3813
   <212> DNA
   <213> Zea mays
<220>
   <221> CDS
   <222> (215)...(3494)
<400> 45
   ccacagctca tataccaaga gccggagcag cttagcgcag cccagagcgg cgccgcgcca      60
   agcacaaccc ccacccgcca cagccgcgtg cgcatgtgag cggtcgccgc ggccgggaga     120
   ccagaggagg ggaggactac gtgcatttcg ctgtgccgcc gccgcggggt tcgtgcgcga     180
   gcgagatccg gcggggcggg gcgggggccc tgag atg gag gct agc gcg ggg ctg     235
                                         Met Glu Ala Ser Ala Gly Leu
                                          1               5
   gtg gcc ggc tcg cat aac cgg aac gag ctg gtg gtg atc cgc cgc gac       283
   Val Ala Gly Ser His Asn Arg Asn Glu Leu Val Val Ile Arg Arg Asp
           10                  15                  20
   cgc gag tcg gga gcc gcg ggc ggc ggc gcg gcg cgc cgg gcg gag gcg       331
   Arg Glu Ser Gly Ala Ala Gly Gly Gly Ala Ala Arg Arg Ala Glu Ala
       25                  30                  35
   ccg tgc cag ata tgc ggc gac gag gtc ggg gtg ggc ttc gac ggg gag       379
   Pro Cys Gln Ile Cys Gly Asp Glu Val Gly Val Gly Phe Asp Gly Glu
   40                  45                  50                  55
   ccc ttc gtg gcg tgc aac gag tgc gcc ttc ccc gtc tgc cgc gcc tgc       427
   Pro Phe Val Ala Cys Asn Glu Cys Ala Phe Pro Val Cys Arg Ala Cys
                   60                  65                  70
   tac gag tac gag cgc cgc gag ggc tcg caa gcg tgc ccg cag tgc agg       475
   Tyr Glu Tyr Glu Arg Arg Glu Gly Ser Gln Ala Cys Pro Gln Cys Arg
               75                  80                  85
   acc cgc tac aag cgc ctc aag ggc tgc ccg cgg gtg gcc ggc gac gag       523
   Thr Arg Tyr Lys Arg Leu Lys Gly Cys Pro Arg Val Ala Gly Asp Glu
           90                  95                  100
   gag gag gac ggc gtc gac gac ctg gag ggc gag ttc ggc ctg cag gac 571
   Glu Glu Asp Gly Val Asp Asp Leu Glu Gly Glu Phe Gly Leu Gln Asp
   105 110 115
   ggc gcc gcc cac gag gac gac ccg cag tac gtc gcc gag tcc atg ctc 619
   Gly Ala Ala His Glu Asp Asp Pro Gln Tyr Val Ala Glu Ser Met Leu
   120 125 130 135
   agg gcg cag atg agc tac ggc cgc ggc ggc gac gcg cac ccc ggc ttc 667
   Arg Ala Gln Met Ser Tyr Gly Arg Gly Gly Asp Ala His Pro Gly Phe
   140 145 150
   agc ccc gtc ccc aac gtg ccg ctc ctc acc aac ggc cag atg gtt gat 715
   Ser Pro Val Pro Asn Val Pro Leu Leu Thr Asn Gly Gln Met Val Asp
   155 160 165
   gac atc ccg ccg gag cag cac gcg ctc gtg ccg tcc tac atg agc ggc 763
   Asp Ile Pro Pro Glu Gln His Ala Leu Val Pro Ser Tyr Met Ser Gly
   170 175 180
   ggc ggc ggc ggg ggc aag agg atc cac ccg ctc cct ttc gca gat ccc 811
   Gly Gly Gly Gly Gly Lys Arg Ile His Pro Leu Pro Phe Ala Asp Pro
   185 190 195
   aac ctt cca gtg caa ccg aga tcc atg gac ccg tcc aag gat ctg gcc 859
   Asn Leu Pro Val Gln Pro Arg Ser Met Asp Pro Ser Lys Asp Leu Ala
   200 205 210 215
   gcc tac gga tat ggc agc gtg gcc tgg aag gag aga atg gag ggc tgg 907
   Ala Tyr Gly Tyr Gly Ser Val Ala Trp Lys Glu Arg Met Glu Gly Trp
   220 225 230
   aag cag aag cag gag cgc ctg cag cat gtc agg agc gag ggt ggc ggt 955
   Lys Gln Lys Gln Glu Arg Leu Gln His Val Arg Ser Glu Gly Gly Gly
   235 240 245
   gat tgg gat ggc gac gat gca gat ctg cca cta atg gat gaa gct agg 1003
   Asp Trp Asp Gly Asp Asp Ala Asp Leu Pro Leu Met Asp Glu Ala Arg
   250 255 260
   cag cca ttg tcc aga aaa gtc cct ata tca tca agc cga att aat ccc 1051
   Gln Pro Leu Ser Arg Lys Val Pro Ile Ser Ser Ser Arg Ile Asn Pro
   265 270 275
   tac agg atg att atc gtt atc cgg ttg gtg gtt ttg ggt ttc ttc ttc 1099
   Tyr Arg Met Ile Ile Val Ile Arg Leu Val Val Leu Gly Phe Phe Phe
   280 285 290 295
   cac tac cga gtg atg cat ccg gcg aaa gat gca ttt gca ttg tgg ctc 1147
   His Tyr Arg Val Met His Pro Ala Lys Asp Ala Phe Ala Leu Trp Leu
   300 305 310
   ata tct gta atc tgt gaa atc tgg ttt gcg atg tcc tgg att ctt gat 1195
   Ile Ser Val Ile Cys Glu Ile Trp Phe Ala Met Ser Trp Ile Leu Asp
   315 320 325
   cag ttc cca aag tgg ctt cca atc gag aga gag act tac ctg gac cgt 1243
   Gln Phe Pro Lys Trp Leu Pro Ile Glu Arg Glu Thr Tyr Leu Asp Arg
           330                 335                 340
   ttg tca cta agg ttt gac aag gaa ggt caa ccc tct cag ctt gct cca      1291
   Leu Ser Leu Arg Phe Asp Lys Glu Gly Gln Pro Ser Gln Leu Ala Pro
       345                 350                 355
   atc gac ttc ttt gtc agt acg gtt gat ccc aca aag gaa cct ccc ttg      1339
   Ile Asp Phe Phe Val Ser Thr Val Asp Pro Thr Lys Glu Pro Pro Leu
   360                 365                 370                 375
   gtc aca gcg aac act gtc ctt tcc atc ctt tct gtg gat tat ccg gtt      1387
   Val Thr Ala Asn Thr Val Leu Ser Ile Leu Ser Val Asp Tyr Pro Val
                   380                 385                 390
   gag aag gtc tcc tgc tat gtt tct gat gat ggt gct gca atg ctt acg      1435
   Glu Lys Val Ser Cys Tyr Val Ser Asp Asp Gly Ala Ala Met Leu Thr
               395                 400                 405
   ttt gaa gca ttg tct gaa aca tct gaa ttt gca aag aaa tgg gtt cct      1483
   Phe Glu Ala Leu Ser Glu Thr Ser Glu Phe Ala Lys Lys Trp Val Pro
           410                 415                 420
   ttc agc aaa aag ttt aat atc gag cct cgt gct cct gag tgg tac ttc      1531
   Phe Ser Lys Lys Phe Asn Ile Glu Pro Arg Ala Pro Glu Trp Tyr Phe
       425                 430                 435
   caa cag aag ata gac tac ctg aaa gac aag gtt gct gct tca ttt gtt      1579
   Gln Gln Lys Ile Asp Tyr Leu Lys Asp Lys Val Ala Ala Ser Phe Val
   440                 445                 450                 455
   agg gag agg agg gcg atg aag aga gaa tac gag gaa ttc aag gta agg      1627
   Arg Glu Arg Arg Ala Met Lys Arg Glu Tyr Glu Glu Phe Lys Val Arg
                   460                 465                 470
   atc aat gcc ttg gtt gca aaa gcc caa aag gtt cct gag gaa gga tgg      1675
   Ile Asn Ala Leu Val Ala Lys Ala Gln Lys Val Pro Glu Glu Gly Trp
               475                 480                 485
   aca atg caa gat gga agc ccc tgg cct gga aac aac gta cgc gat cat      1723
   Thr Met Gln Asp Gly Ser Pro Trp Pro Gly Asn Asn Val Arg Asp His
           490                 495                 500
   cct gga atg att cag gta ttc ctt ggc caa agt ggc ggt cgt gat gtg      1771
   Pro Gly Met Ile Gln Val Phe Leu Gly Gln Ser Gly Gly Arg Asp Val
       505                 510                 515
   gaa gga aat gag ttg cct cgc ctg gtt tat gtc tcg aga gaa aag agg      1819
   Glu Gly Asn Glu Leu Pro Arg Leu Val Tyr Val Ser Arg Glu Lys Arg
   520                 525                 530                 535
   cca ggt tat aac cat cac aag aag gct ggt gcc atg aat gca ctg gtc      1867
   Pro Gly Tyr Asn His His Lys Lys Ala Gly Ala Met Asn Ala Leu Val
                   540                 545                 550
   cgt gtc tct gct gtc tta tca aat gct gca tac cta ttg aac ttg gac      1915
   Arg Val Ser Ala Val Leu Ser Asn Ala Ala Tyr Leu Leu Asn Leu Asp
               555                 560                 565
   tgt gat cac tac atc aac aat agc aag gcc ata aaa gag gct atg tgt      1963
   Cys Asp His Tyr Ile Asn Asn Ser Lys Ala Ile Lys Glu Ala Met Cys
           570                 575                 580
   ttc atg atg gat cct ttg gtg ggg aag aaa gtg tgc tat gta cag ttc      2011
   Phe Met Met Asp Pro Leu Val Gly Lys Lys Val Cys Tyr Val Gln Phe
       585                 590                 595
   cct cag agg ttt gat ggt att gac aaa aat gat cga tac gct aac agg      2059
   Pro Gln Arg Phe Asp Gly Ile Asp Lys Asn Asp Arg Tyr Ala Asn Arg
   600                 605                 610                 615
   aac gtt gtc ttt ttt gac atc aac atg aaa ggt ttg gac ggt att caa      2107
   Asn Val Val Phe Phe Asp Ile Asn Met Lys Gly Leu Asp Gly Ile Gln
                   620                 625                 630
   gga ccc att tat gtg ggt act gga tgt gtt ttc aga cgg cag gca ctg      2155
   Gly Pro Ile Tyr Val Gly Thr Gly Cys Val Phe Arg Arg Gln Ala Leu
               635                 640                 645
   tat ggt tat gat gct cct aaa acg aag aag cca cca tca aga act tgc      2203
   Tyr Gly Tyr Asp Ala Pro Lys Thr Lys Lys Pro Pro Ser Arg Thr Cys
           650                 655                 660
   aac tgc tgg ccc aag tgg tgc ctc tct tgc tgc tgc agc agg aac aag      2251
   Asn Cys Trp Pro Lys Trp Cys Leu Ser Cys Cys Cys Ser Arg Asn Lys
       665                 670                 675
   aat aaa aag aag act aca aaa cca aag acg gag aag aag aaa aga tta      2299
   Asn Lys Lys Lys Thr Thr Lys Pro Lys Thr Glu Lys Lys Lys Arg Leu
   680                 685                 690                 695
   ttt ttc aag aaa gca gaa aac cca tct cct gca tat gct ttg ggt gaa      2347
   Phe Phe Lys Lys Ala Glu Asn Pro Ser Pro Ala Tyr Ala Leu Gly Glu
                   700                 705                 710
   att gat gaa ggt gct cca ggt gct gat atc gag aag gcc gga atc gta      2395
   Ile Asp Glu Gly Ala Pro Gly Ala Asp Ile Glu Lys Ala Gly Ile Val
               715                 720                 725
   aat caa cag aaa cta gag aag aaa ttt ggg cag tct tct gtt ttt gtc      2443
   Asn Gln Gln Lys Leu Glu Lys Lys Phe Gly Gln Ser Ser Val Phe Val
           730                 735                 740
   gca tca aca ctt ctt gag aac gga ggg acc ctg aag agc gca agt cca      2491
   Ala Ser Thr Leu Leu Glu Asn Gly Gly Thr Leu Lys Ser Ala Ser Pro
       745                 750                 755
   gct tct ctt ctg aag gaa gct ata cat gtt atc agc tgc ggc tac gaa      2539
   Ala Ser Leu Leu Lys Glu Ala Ile His Val Ile Ser Cys Gly Tyr Glu
   760                 765                 770                 775
   gac aag acc gac tgg gga aaa gag att ggc tgg att tac gga tcg atc      2587
   Asp Lys Thr Asp Trp Gly Lys Glu Ile Gly Trp Ile Tyr Gly Ser Ile
                   780                 785                 790
   aca gag gat atc ttg act gga ttt aag atg cac tgc cat ggc tgg cgg      2635
   Thr Glu Asp Ile Leu Thr Gly Phe Lys Met His Cys His Gly Trp Arg
               795                 800                 805
   tct att tac tgc atc ccg aag cgg cct gca ttc aaa ggt tct gcg cct      2683
   Ser Ile Tyr Cys Ile Pro Lys Arg Pro Ala Phe Lys Gly Ser Ala Pro
           810                 815                 820
   ctg aac ctt tcc gac cgt ctt cac cag gtc ctt cgc tgg gcc ctt ggg      2731
   Leu Asn Leu Ser Asp Arg Leu His Gln Val Leu Arg Trp Ala Leu Gly
       825                 830                 835
   tcc gtc gaa att ttc ttc agc aag cac tgc cca ctt tgg tac gga tac      2779
   Ser Val Glu Ile Phe Phe Ser Lys His Cys Pro Leu Trp Tyr Gly Tyr
   840                 845                 850                 855
   ggc ggc ggg cta aaa ttc ctg gaa agg ttt tct tat atc aac tcc atc      2827
   Gly Gly Gly Leu Lys Phe Leu Glu Arg Phe Ser Tyr Ile Asn Ser Ile
                   860                 865                 870
   gtt tat ccc tgg acg tcc att cct ctc ctg gct tac tgt acc ttg cct      2875
   Val Tyr Pro Trp Thr Ser Ile Pro Leu Leu Ala Tyr Cys Thr Leu Pro
               875                 880                 885
   gcc atc tgc ctg ctc acg ggg aag ttt atc aca cca gag ctt acc aat      2923
   Ala Ile Cys Leu Leu Thr Gly Lys Phe Ile Thr Pro Glu Leu Thr Asn
           890                 895                 900
   gtc gcc agt atc tgg ttc atg gca ctt ttc atc tgc atc tcc gtg acc      2971
   Val Ala Ser Ile Trp Phe Met Ala Leu Phe Ile Cys Ile Ser Val Thr
       905                 910                 915
   ggc atc ctg gaa atg agg tgg agt ggc gtg gcc atc gac gac tgg tgg      3019
   Gly Ile Leu Glu Met Arg Trp Ser Gly Val Ala Ile Asp Asp Trp Trp
   920                 925                 930                 935
   agg aac gag cag ttc tgg gtc atc gga ggc gtt tcg gcg cat ctg ttc      3067
   Arg Asn Glu Gln Phe Trp Val Ile Gly Gly Val Ser Ala His Leu Phe
                   940                 945                 950
   gcg gtg ttc cag ggc ctg ctg aag gtg ttc gcc ggc atc gac acg agc      3115
   Ala Val Phe Gln Gly Leu Leu Lys Val Phe Ala Gly Ile Asp Thr Ser
               955                 960                 965
   ttc acc gtg acg tcg aag gcc ggg gac gac gag gag ttc tcg gag ctg      3163
   Phe Thr Val Thr Ser Lys Ala Gly Asp Asp Glu Glu Phe Ser Glu Leu
           970                 975                 980
   tac acg ttc aag tgg acc acc ctg ctg ata ccc ccg acc acg ctc ctc      3211
   Tyr Thr Phe Lys Trp Thr Thr Leu Leu Ile Pro Pro Thr Thr Leu Leu
       985                 990                 995
   ctg ctg aac ttc atc ggg gtg gtg gcc ggg atc tcg aac gcg atc aac      3259
   Leu Leu Asn Phe Ile Gly Val Val Ala Gly Ile Ser Asn Ala Ile Asn
   1000                1005                1010                1015
   aac ggg tac gag tcg tgg ggc ccc ctg ttc ggg aag ctc ttc ttc gcc      3307
   Asn Gly Tyr Glu Ser Trp Gly Pro Leu Phe Gly Lys Leu Phe Phe Ala
                   1020                1025                1030
   ttc tgg gtg atc gtc cac ctg tac ccg ttc ctc aag ggt ctg gtg ggg      3355
   Phe Trp Val Ile Val His Leu Tyr Pro Phe Leu Lys Gly Leu Val Gly
               1035                1040                1045
   agg cag aac agg acg ccg acg atc gtc atc gtc tgg tcc atc ctg ctg      3403
   Arg Gln Asn Arg Thr Pro Thr Ile Val Ile Val Trp Ser Ile Leu Leu
           1050                1055                1060
   gcc tcg atc ttc tcg ctc ctg tgg gtc cgc gtc gac ccg ttc ctc gcc      3451
   Ala Ser Ile Phe Ser Leu Leu Trp Val Arg Val Asp Pro Phe Leu Ala
       1065                1070                1075
   aag agc aac ggc ccg ctc ctg gag gag tgt ggc ctg gac tgc a            3494
   Lys Ser Asn Gly Pro Leu Leu Glu Glu Cys Gly Leu Asp Cys
   1080                1085                1090
   actgaagtgg gggccccctg tcactcgaag ttctgtcacg ggcgaattac gcctgatttt    3554
   ttgttgttgt tgttgttgga attctttgct gtagatagaa accacatgtc cacggcatct    3614
   ctgctgtgtc cattggagca ggagagaggt gcctgctgct gtttgttgag taaattaaaa    3674
   gttttaaagt tatacagtga tgcacattcc agtgcccagt gtattccctt tttacagtct    3734
   gtatattagc gacaaaggac atattggtta ggagtttgat tcttttgtaa aaaaaaaaaa    3794
   aaaaaaaaaa aaaaaaaaa                                                 3813
<210> 46
   <211> 1094
   <212> PRT
   <213> Zea mays
<400> 46
   Met Glu Ala Ser Ala Gly Leu Val Ala Gly Ser His Asn Arg Asn Glu
   1               5                   10                  15
   Leu Val Val Ile Arg Arg Asp Arg Glu Ser Gly Ala Ala Gly Gly Gly
               20                  25                  30
   Ala Ala Arg Arg Ala Glu Ala Pro Cys Gln Ile Cys Gly Asp Glu Val
           35                  40                  45
   Gly Val Gly Phe Asp Gly Glu Pro Phe Val Ala Cys Asn Glu Cys Ala
       50                  55                  60
   Phe Pro Val Cys Arg Ala Cys Tyr Glu Tyr Glu Arg Arg Glu Gly Ser
   65                  70                  75                  80
   Gln Ala Cys Pro Gln Cys Arg Thr Arg Tyr Lys Arg Leu Lys Gly Cys
                   85                  90                  95
   Pro Arg Val Ala Gly Asp Glu Glu Glu Asp Gly Val Asp Asp Leu Glu
               100                 105                 110
   Gly Glu Phe Gly Leu Gln Asp Gly Ala Ala His Glu Asp Asp Pro Gln
           115                 120                 125
   Tyr Val Ala Glu Ser Met Leu Arg Ala Gln Met Ser Tyr Gly Arg Gly
       130                 135                 140
   Gly Asp Ala His Pro Gly Phe Ser Pro Val Pro Asn Val Pro Leu Leu
   145                 150                 155                 160
   Thr Asn Gly Gln Met Val Asp Asp Ile Pro Pro Glu Gln His Ala Leu
                   165                 170                 175
   Val Pro Ser Tyr Met Ser Gly Gly Gly Gly Gly Gly Lys Arg Ile His
               180                 185                 190
   Pro Leu Pro Phe Ala Asp Pro Asn Leu Pro Val Gln Pro Arg Ser Met
           195                 200                 205
   Asp Pro Ser Lys Asp Leu Ala Ala Tyr Gly Tyr Gly Ser Val Ala Trp
       210                 215                 220
   Lys Glu Arg Met Glu Gly Trp Lys Gln Lys Gln Glu Arg Leu Gln His
   225 230 235 240
   Val Arg Ser Glu Gly Gly Gly Asp Trp Asp Gly Asp Asp Ala Asp Leu
   245 250 255
   Pro Leu Met Asp Glu Ala Arg Gln Pro Leu Ser Arg Lys Val Pro Ile
   260 265 270
   Ser Ser Ser Arg Ile Asn Pro Tyr Arg Met Ile Ile Val Ile Arg Leu
   275 280 285
   Val Val Leu Gly Phe Phe Phe His Tyr Arg Val Met His Pro Ala Lys
   290 295 300
   Asp Ala Phe Ala Leu Trp Leu Ile Ser Val Ile Cys Glu Ile Trp Phe
   305 310 315 320
   Ala Met Ser Trp Ile Leu Asp Gln Phe Pro Lys Trp Leu Pro Ile Glu
   325 330 335
   Arg Glu Thr Tyr Leu Asp Arg Leu Ser Leu Arg Phe Asp Lys Glu Gly
   340 345 350
   Gln Pro Ser Gln Leu Ala Pro Ile Asp Phe Phe Val Ser Thr Val Asp
   355 360 365
   Pro Thr Lys Glu Pro Pro Leu Val Thr Ala Asn Thr Val Leu Ser Ile
   370 375 380
   Leu Ser Val Asp Tyr Pro Val Glu Lys Val Ser Cys Tyr Val Ser Asp
   385 390 395 400
   Asp Gly Ala Ala Met Leu Thr Phe Glu Ala Leu Ser Glu Thr Ser Glu
   405 410 415
   Phe Ala Lys Lys Trp Val Pro Phe Ser Lys Lys Phe Asn Ile Glu Pro
   420 425 430
   Arg Ala Pro Glu Trp Tyr Phe Gln Gln Lys Ile Asp Tyr Leu Lys Asp
   435 440 445
   Lys Val Ala Ala Ser Phe Val Arg Glu Arg Arg Ala Met Lys Arg Glu
   450 455 460
   Tyr Glu Glu Phe Lys Val Arg Ile Asn Ala Leu Val Ala Lys Ala Gln
   465 470 475 480
   Lys Val Pro Glu Glu Gly Trp Thr Met Gln Asp Gly Ser Pro Trp Pro
   485 490 495
   Gly Asn Asn Val Arg Asp His Pro Gly Met Ile Gln Val Phe Leu Gly
   500 505 510
   Gln Ser Gly Gly Arg Asp Val Glu Gly Asn Glu Leu Pro Arg Leu Val
   515 520 525
   Tyr Val Ser Arg Glu Lys Arg Pro Gly Tyr Asn His His Lys Lys Ala
   530 535 540
   Gly Ala Met Asn Ala Leu Val Arg Val Ser Ala Val Leu Ser Asn Ala
   545 550 555 560
   Ala Tyr Leu Leu Asn Leu Asp Cys Asp His Tyr Ile Asn Asn Ser Lys
   565 570 575
   Ala Ile Lys Glu Ala Met Cys Phe Met Met Asp Pro Leu Val Gly Lys
   580 585 590
   Lys Val Cys Tyr Val Gln Phe Pro Gln Arg Phe Asp Gly Ile Asp Lys
   595 600 605
   Asn Asp Arg Tyr Ala Asn Arg Asn Val Val Phe Phe Asp Ile Asn Met
   610 615 620
   Lys Gly Leu Asp Gly Ile Gln Gly Pro Ile Tyr Val Gly Thr Gly Cys
   625 630 635 640
   Val Phe Arg Arg Gln Ala Leu Tyr Gly Tyr Asp Ala Pro Lys Thr Lys
   645 650 655
   Lys Pro Pro Ser Arg Thr Cys Asn Cys Trp Pro Lys Trp Cys Leu Ser
   660 665 670
   Cys Cys Cys Ser Arg Asn Lys Asn Lys Lys Lys Thr Thr Lys Pro Lys
   675 680 685
   Thr Glu Lys Lys Lys Arg Leu Phe Phe Lys Lys Ala Glu Asn Pro Ser
   690 695 700
   Pro Ala Tyr Ala Leu Gly Glu Ile Asp Glu Gly Ala Pro Gly Ala Asp
   705 710 715 720
   Ile Glu Lys Ala Gly Ile Val Asn Gln Gln Lys Leu Glu Lys Lys Phe
   725 730 735
   Gly Gln Ser Ser Val Phe Val Ala Ser Thr Leu Leu Glu Asn Gly Gly
   740 745 750
   Thr Leu Lys Ser Ala Ser Pro Ala Ser Leu Leu Lys Glu Ala Ile His
   755 760 765
   Val Ile Ser Cys Gly Tyr Glu Asp Lys Thr Asp Trp Gly Lys Glu Ile
   770 775 780
   Gly Trp Ile Tyr Gly Ser Ile Thr Glu Asp Ile Leu Thr Gly Phe Lys
   785 790 795 800
   Met His Cys His Gly Trp Arg Ser Ile Tyr Cys Ile Pro Lys Arg Pro
   805 810 815
   Ala Phe Lys Gly Ser Ala Pro Leu Asn Leu Ser Asp Arg Leu His Gln
   820 825 830
   Val Leu Arg Trp Ala Leu Gly Ser Val Glu Ile Phe Phe Ser Lys His
   835 840 845
   Cys Pro Leu Trp Tyr Gly Tyr Gly Gly Gly Leu Lys Phe Leu Glu Arg
   850 855 860
   Phe Ser Tyr Ile Asn Ser Ile Val Tyr Pro Trp Thr Ser Ile Pro Leu
   865 870 875 880
   Leu Ala Tyr Cys Thr Leu Pro Ala Ile Cys Leu Leu Thr Gly Lys Phe
   885 890 895
   Ile Thr Pro Glu Leu Thr Asn Val Ala Ser Ile Trp Phe Met Ala Leu
   900 905 910
   Phe Ile Cys Ile Ser Val Thr Gly Ile Leu Glu Met Arg Trp Ser Gly
   915 920 925
   Val Ala Ile Asp Asp Trp Trp Arg Asn Glu Gln Phe Trp Val Ile Gly
   930 935 940
   Gly Val Ser Ala His Leu Phe Ala Val Phe Gln Gly Leu Leu Lys Val
   945 950 955 960
   Phe Ala Gly Ile Asp Thr Ser Phe Thr Val Thr Ser Lys Ala Gly Asp
   965 970 975
   Asp Glu Glu Phe Ser Glu Leu Tyr Thr Phe Lys Trp Thr Thr Leu Leu
   980 985 990
   Ile Pro Pro Thr Thr Leu Leu Leu Leu Asn Phe Ile Gly Val Val Ala
   995 1000 1005
   Gly Ile Ser Asn Ala Ile Asn Asn Gly Tyr Glu Ser Trp Gly Pro Leu
   1010 1015 1020
   Phe Gly Lys Leu Phe Phe Ala Phe Trp Val Ile Val His Leu Tyr Pro
   1025 1030 1035 1040
   Phe Leu Lys Gly Leu Val Gly Arg Gln Asn Arg Thr Pro Thr Ile Val
   1045 1050 1055
   Ile Val Trp Ser Ile Leu Leu Ala Ser Ile Phe Ser Leu Leu Trp Val
   1060 1065 1070
   Arg Val Asp Pro Phe Leu Ala Lys Ser Asn Gly Pro Leu Leu Glu Glu
   1075 1080 1085
   Cys Gly Leu Asp Cys Asn
   1090
<210> 47
   <211> 25
   <212> DNA
   <213> Zea mays
<400> 47
   atggaggcta gcgcggggct ggtgg
   25
<210> 48
   <211> 25
   <212> DNA
   <213> Zea mays
<400> 48
   tcagttgcag tccaggccac actcc
   25
<210> 49
   <211> 3746
   <212> DNA
   <213> Zea mays
<220>
   <221> CDS
   <222> (321)...(3449)
<400> 49
   ctaggatcaa aaccgtctcg ccgctgcaat aatcttttgt caattcttaa tccctcgcgt     60
   cgacagcgac agcggaacca actcacgttg ccgcggcttc ctccatcggt gcggtgccct    120
   gtccttttct ctcgtccctc ctccccccgt atagttaagc cccgccccgc tactactact    180
   actagcagca gcagcgctct cgcagcggga gatgcggtgt tgatccgtgc cccgctcgga    240
   tctcgggact ggtgccggct ctgcccaggc cccaggctcc aggccagctc cctcgacgtt    300
   tctcggcgag ctcgcttgcc atg gag ggc gac gcg gac ggc gtg aag tcg ggg    353
                         Met Glu Gly Asp Ala Asp Gly Val Lys Ser Gly
                         1               5                   10
   agg cgc ggt ggc gga cag gtg tgc cag atc tgc ggc gac ggc gtg ggc    401
   Arg Arg Gly Gly Gly Gln Val Cys Gln Ile Cys Gly Asp Gly Val Gly
               15                  20                  25
   acc acg gcg gag ggg gac gtc ttc gcc gcc tgc gac gtc tgc ggg ttt    449
   Thr Thr Ala Glu Gly Asp Val Phe Ala Ala Cys Asp Val Cys Gly Phe
           30                  35                  40
   ccg gtg tgc cgc ccc tgc tac gag tac gag cgc aag gac ggc acg cag    497
   Pro Val Cys Arg Pro Cys Tyr Glu Tyr Glu Arg Lys Asp Gly Thr Gln
       45                  50                  55
   gcg tgc ccc cag tgc aag acc aag tac aag cgc cac aag ggg agc ccg    545
   Ala Cys Pro Gln Cys Lys Thr Lys Tyr Lys Arg His Lys Gly Ser Pro
   60                  65                  70                  75
   gcg atc cgt ggg gag gaa gga gac gac act gat gcc gat agc gac ttc    593
   Ala Ile Arg Gly Glu Glu Gly Asp Asp Thr Asp Ala Asp Ser Asp Phe
                   80                  85                  90
   aat tac ctt gca tct ggc aat gag gac cag aag cag aag att gcc gac    641
   Asn Tyr Leu Ala Ser Gly Asn Glu Asp Gln Lys Gln Lys Ile Ala Asp
               95                  100                 105
   aga atg cgc agc tgg cgc atg aac gtt ggg ggc agc ggg gat gtt ggt    689
   Arg Met Arg Ser Trp Arg Met Asn Val Gly Gly Ser Gly Asp Val Gly
           110                 115                 120
   cgc ccc aag tat gac agt ggc gag atc ggg ctt acc aag tat gac agt       737
   Arg Pro Lys Tyr Asp Ser Gly Glu Ile Gly Leu Thr Lys Tyr Asp Ser
       125                 130                 135
   ggc gag att cct cgg gga tac atc cca tca gtc act aac agc cag atc       785
   Gly Glu Ile Pro Arg Gly Tyr Ile Pro Ser Val Thr Asn Ser Gln Ile
   140                 145                 150                 155
   tca gga gaa atc cct ggt gct tcc cct gac cat cat atg atg tcc cca       833
   Ser Gly Glu Ile Pro Gly Ala Ser Pro Asp His His Met Met Ser Pro
                   160                 165                 170
   act ggg aac att ggc aag cgt gct cca ttt ccc tat gtg aac cat tcg       881
   Thr Gly Asn Ile Gly Lys Arg Ala Pro Phe Pro Tyr Val Asn His Ser
               175                 180                 185
   cca aat ccg tca agg gag ttc tct ggt agc att ggg aat gtt gcc tgg       929
   Pro Asn Pro Ser Arg Glu Phe Ser Gly Ser Ile Gly Asn Val Ala Trp
           190                 195                 200
   aaa gag agg gtt gat ggc tgg aaa atg aag cag gac aag ggg acg att       977
   Lys Glu Arg Val Asp Gly Trp Lys Met Lys Gln Asp Lys Gly Thr Ile
       205                 210                 215
   ccc atg acg aat ggc aca agc att gct ccc tct gag ggt cgg ggt gtt      1025
   Pro Met Thr Asn Gly Thr Ser Ile Ala Pro Ser Glu Gly Arg Gly Val
   220                 225                 230                 235
   ggt gat att gat gca tca act gat tac aac atg gaa gat gcc tta ttg      1073
   Gly Asp Ile Asp Ala Ser Thr Asp Tyr Asn Met Glu Asp Ala Leu Leu
                   240                 245                 250
   aac gac gaa act cga cag cct cta tct agg aaa gtt cca ctt cct tcc      1121
   Asn Asp Glu Thr Arg Gln Pro Leu Ser Arg Lys Val Pro Leu Pro Ser
               255                 260                 265
   tcc agg ata aat cca tac agg atg gtc att gtg ctg cga ttg att gtt      1169
   Ser Arg Ile Asn Pro Tyr Arg Met Val Ile Val Leu Arg Leu Ile Val
           270                 275                 280
   cta agc atc ttc ttg cac tac cgt atc aca aat cct gtg cgc aat gca      1217
   Leu Ser Ile Phe Leu His Tyr Arg Ile Thr Asn Pro Val Arg Asn Ala
       285                 290                 295
   tac cca tta tgg ctt cta tct gtt ata tgt gag atc tgg ttt gct ctt      1265
   Tyr Pro Leu Trp Leu Leu Ser Val Ile Cys Glu Ile Trp Phe Ala Leu
   300                 305                 310                 315
   tcg tgg ata ttg gat cag ttc cct aag tgg ttt cca atc aac cgg gag      1313
   Ser Trp Ile Leu Asp Gln Phe Pro Lys Trp Phe Pro Ile Asn Arg Glu
                   320                 325                 330
   acg tac ctt gat agg ctg gca tta agg tat gac cgg gaa ggt gag cca      1361
   Thr Tyr Leu Asp Arg Leu Ala Leu Arg Tyr Asp Arg Glu Gly Glu Pro
               335                 340                 345
   tct cag ttg gct gct gtt gac att ttc gtc agt aca gtc gac cca atg      1409
   Ser Gln Leu Ala Ala Val Asp Ile Phe Val Ser Thr Val Asp Pro Met
               350                 355                 360
   aag gag cct cct ctt gtc act gcc aat acc gtg cta tcc att ctt gct      1457
   Lys Glu Pro Pro Leu Val Thr Ala Asn Thr Val Leu Ser Ile Leu Ala
           365                 370                 375
   gtg gat tac cct gtg gat aag gtc tct tgc tat gta tct gat gat gga      1505
   Val Asp Tyr Pro Val Asp Lys Val Ser Cys Tyr Val Ser Asp Asp Gly
   380                 385                 390                 395
   gct gcg atg ctg aca ttt gat gca cta gct gag act tca gag ttt gct      1553
   Ala Ala Met Leu Thr Phe Asp Ala Leu Ala Glu Thr Ser Glu Phe Ala
                   400                 405                 410
   aga aaa tgg gta cca ttt gtt aag aag tac aac att gaa cct aga gct      1601
   Arg Lys Trp Val Pro Phe Val Lys Lys Tyr Asn Ile Glu Pro Arg Ala
               415                 420                 425
   cct gaa tgg tac ttc tcc cag aaa att gat tac ttg aag gac aaa gtg      1649
   Pro Glu Trp Tyr Phe Ser Gln Lys Ile Asp Tyr Leu Lys Asp Lys Val
           430                 435                 440
   cac cct tca ttt gtt aaa gac cgc cgg gcc atg aag aga gaa tat gaa      1697
   His Pro Ser Phe Val Lys Asp Arg Arg Ala Met Lys Arg Glu Tyr Glu
       445                 450                 455
   gaa ttc aaa gtt agg gta aat ggc ctt gtt gct aag gca cag aaa gtt      1745
   Glu Phe Lys Val Arg Val Asn Gly Leu Val Ala Lys Ala Gln Lys Val
   460                 465                 470                 475
   cct gag gaa gga tgg atc atg caa gat ggc aca cca tgg cca gga aac      1793
   Pro Glu Glu Gly Trp Ile Met Gln Asp Gly Thr Pro Trp Pro Gly Asn
                   480                 485                 490
   aat acc mgg gac cat cct gga atg att cag gtt ttc ctt ggt cac agt      1841
   Asn Thr Xaa Asp His Pro Gly Met Ile Gln Val Phe Leu Gly His Ser
               495                 500                 505
   ggt ggc ctt gat act gag ggc aat gag cta ccc cgt ttg gtc tat gtt      1889
   Gly Gly Leu Asp Thr Glu Gly Asn Glu Leu Pro Arg Leu Val Tyr Val
           510                 515                 520
   tct cgt gaa aag cgt cct gga ttc cag cat cac aag aaa gct ggt gcc      1937
   Ser Arg Glu Lys Arg Pro Gly Phe Gln His His Lys Lys Ala Gly Ala
       525                 530                 535
   atg aat gct ctt gtt cgt gtc tca gct gtg ctt acc aat gga caa tac      1985
   Met Asn Ala Leu Val Arg Val Ser Ala Val Leu Thr Asn Gly Gln Tyr
   540                 545                 550                 555
   atg ttg aat ctt gat tgt gat cac tac att aac aac agt aag gct ctc      2033
   Met Leu Asn Leu Asp Cys Asp His Tyr Ile Asn Asn Ser Lys Ala Leu
                   560                 565                 570
   agg gaa gct atg tgc ttc ctt atg gac cct aac cta gga agg agt gtc      2081
   Arg Glu Ala Met Cys Phe Leu Met Asp Pro Asn Leu Gly Arg Ser Val
                   575                 580                 585
   tgc tac gtc cag ttt ccc cag aga ttc gat ggc att gac agg aat gat      2129
   Cys Tyr Val Gln Phe Pro Gln Arg Phe Asp Gly Ile Asp Arg Asn Asp
           590                 595                 600
   cga tat gcc aac agg aac acc gtg ttt ttc gat att aac ttg aga ggt      2177
   Arg Tyr Ala Asn Arg Asn Thr Val Phe Phe Asp Ile Asn Leu Arg Gly
       605                 610                 615
   ctt gat ggc atc caa gga cca gtt tat gtc gga act ggc tgt gtt ttc      2225
   Leu Asp Gly Ile Gln Gly Pro Val Tyr Val Gly Thr Gly Cys Val Phe
   620                 625                 630                 635
   aac cga aca gct cta tat ggt tat gag ccc cca att aag cag aag aag      2273
   Asn Arg Thr Ala Leu Tyr Gly Tyr Glu Pro Pro Ile Lys Gln Lys Lys
                   640                 645                 650
   ggt ggt ttc ttg tca tca cta tgt ggc ggt agg aag aag gca agc aaa      2321
   Gly Gly Phe Leu Ser Ser Leu Cys Gly Gly Arg Lys Lys Ala Ser Lys
               655                 660                 665
   tca aag aag ggc tcg gac aag aag aag tcg cag aag cat gtg gac agt      2369
   Ser Lys Lys Gly Ser Asp Lys Lys Lys Ser Gln Lys His Val Asp Ser
           670                 675                 680
   tct gtg cca gta ttc aac ctt gaa gat ata gag gag gga gtt gaa ggc      2417
   Ser Val Pro Val Phe Asn Leu Glu Asp Ile Glu Glu Gly Val Glu Gly
       685                 690                 695
   gct gga ttt gac gac gag aaa tca ctt ctt atg tct caa atg agc ctg      2465
   Ala Gly Phe Asp Asp Glu Lys Ser Leu Leu Met Ser Gln Met Ser Leu
   700                 705                 710                 715
   gag aag aga ttt ggc cag tcc gca gcg ttt gtt gcc tcc act ctg atg      2513
   Glu Lys Arg Phe Gly Gln Ser Ala Ala Phe Val Ala Ser Thr Leu Met
                   720                 725                 730
   gag tat ggt ggt gtt cct cag tcc gca act ccg gag tct ctt ctg aaa      2561
   Glu Tyr Gly Gly Val Pro Gln Ser Ala Thr Pro Glu Ser Leu Leu Lys
               735                 740                 745
   gaa gct atc cat gtt ata agc tgt ggc tat gag gac aag act gaa tgg      2609
   Glu Ala Ile His Val Ile Ser Cys Gly Tyr Glu Asp Lys Thr Glu Trp
           750                 755                 760
   gga act gag atc ggg tgg atc tac ggt tct gtg aca gaa gac att ctc      2657
   Gly Thr Glu Ile Gly Trp Ile Tyr Gly Ser Val Thr Glu Asp Ile Leu
       765                 770                 775
   acc gga ttc aag atg cac gcg cga ggc tgg cgg tcg atc tac tgc atg      2705
   Thr Gly Phe Lys Met His Ala Arg Gly Trp Arg Ser Ile Tyr Cys Met
   780                 785                 790                 795
   ccc aag cgg cca gct ttc aag ggg tct gcc ccc atc aat ctt tcg gac      2753
   Pro Lys Arg Pro Ala Phe Lys Gly Ser Ala Pro Ile Asn Leu Ser Asp
                   800                 805                 810
   cgt ctg aac cag gtg ctc cgg tgg gct ctt ggg tcc gtg gag atc ctc      2801
   Arg Leu Asn Gln Val Leu Arg Trp Ala Leu Gly Ser Val Glu Ile Leu
               815                 820                 825
   ttc agc cgg cac tgc ccc ctg tgg tac ggc tac gga ggg cgg ctc aag      2849
   Phe Ser Arg His Cys Pro Leu Trp Tyr Gly Tyr Gly Gly Arg Leu Lys
           830                 835                 840
   ttc ctg gag aga ttc gcg tac atc aac acc acc atc tac ccg ctc acg      2897
   Phe Leu Glu Arg Phe Ala Tyr Ile Asn Thr Thr Ile Tyr Pro Leu Thr
       845                 850                 855
   tcc atc ccg ctt ctc atc tac tgc atc ctg ccc gcc atc tgt ctg ctc      2945
   Ser Ile Pro Leu Leu Ile Tyr Cys Ile Leu Pro Ala Ile Cys Leu Leu
   860                 865                 870                 875
   acc gga aag ttc atc att cca gag atc agc aac ttc gcc agc atc tgg      2993
   Thr Gly Lys Phe Ile Ile Pro Glu Ile Ser Asn Phe Ala Ser Ile Trp
                   880                 885                 890
   ttc atc tcc ctc ttc atc tcg atc ttc gcc acg ggc atc ctg gag atg      3041
   Phe Ile Ser Leu Phe Ile Ser Ile Phe Ala Thr Gly Ile Leu Glu Met
               895                 900                 905
   agg tgg agc ggg gtg ggc atc gac gag tgg tgg agg aac gag cag ttc      3089
   Arg Trp Ser Gly Val Gly Ile Asp Glu Trp Trp Arg Asn Glu Gln Phe
           910                 915                 920
   tgg gtg atc ggg ggc atc tcc gcg cac ctc ttc gcc gtg ttc cag ggc      3137
   Trp Val Ile Gly Gly Ile Ser Ala His Leu Phe Ala Val Phe Gln Gly
       925                 930                 935
   ctg ctc aag gtg ctg gcc ggc atc gac acc aac ttc acc gtc acc tcc      3185
   Leu Leu Lys Val Leu Ala Gly Ile Asp Thr Asn Phe Thr Val Thr Ser
   940                 945                 950                 955
   aag gcc tcg gac gag gac ggc gac ttc gcg gag ctg tac atg ttc aag      3233
   Lys Ala Ser Asp Glu Asp Gly Asp Phe Ala Glu Leu Tyr Met Phe Lys
                   960                 965                 970
   tgg acg acg ctc ctg atc ccg ccc acc acc atc ctg atc atc aac ctg      3281
   Trp Thr Thr Leu Leu Ile Pro Pro Thr Thr Ile Leu Ile Ile Asn Leu
               975                 980                 985
   gtc ggc gtc gtc gcc ggc atc tcc tac gcc atc aac agc gga tac cag      3329
   Val Gly Val Val Ala Gly Ile Ser Tyr Ala Ile Asn Ser Gly Tyr Gln
           990                 995                 1000
   tcg tgg ggc ccg ctc ttc ggc aag ctc ttc ttc gcc ttc tgg gtc atc      3377
   Ser Trp Gly Pro Leu Phe Gly Lys Leu Phe Phe Ala Phe Trp Val Ile
       1005                1010                1015
   gtc cac ctg tac ccg ttc ctc aag ggc ctc atg ggc agg cag aac cgc      3425
   Val His Leu Tyr Pro Phe Leu Lys Gly Leu Met Gly Arg Gln Asn Arg
   1020                1025                1030                1035
   acc ccg acc atc gtc gtc gtc tgg gccatcctgc tggcgtccat cttctccttg     3479
   Thr Pro Thr Ile Val Val Val Trp
   1040
   ctgtgggttc gcatcgaccc cttcaccacc cgcgtcactg gcccggatac ccagacgtgt   3539
   ggcatcaact gctagggaag tggaaggttt gtactttgta gaaacggagg aataccacgt   3599
   gccatctgtt gtctgttaag ttatatatat ataagcagca agtggcgtta tttacagcta   3659
   cgtacagacc agtggatatt gtttaccaca aagttttact tgtgttaata tgcattcttt   3719
   tgttgatata aaaaaaaaaa aaaaaaa                                       3746
<210> 50
   <211> 1043
   <212> PRT
   <213> Zea mays
<400> 50
   Met Glu Gly Asp Ala Asp Gly Val Lys Ser Gly Arg Arg Gly Gly Gly
   1               5                   10                  15
   Gln Val Cys Gln Ile Cys Gly Asp Gly Val Gly Thr Thr Ala Glu Gly
               20                  25                  30
   Asp Val Phe Ala Ala Cys Asp Val Cys Gly Phe Pro Val Cys Arg Pro
           35                  40                  45
   Cys Tyr Glu Tyr Glu Arg Lys Asp Gly Thr Gln Ala Cys Pro Gln Cys
       50                  55                  60
   Lys Thr Lys Tyr Lys Arg His Lys Gly Ser Pro Ala Ile Arg Gly Glu
   65                  70                  75                  80
   Glu Gly Asp Asp Thr Asp Ala Asp Ser Asp Phe Asn Tyr Leu Ala Ser
                   85                  90                  95
   Gly Asn Glu Asp Gln Lys Gln Lys Ile Ala Asp Arg Met Arg Ser Trp
               100                 105                 110
   Arg Met Asn Val Gly Gly Ser Gly Asp Val Gly Arg Pro Lys Tyr Asp
           115                 120                 125
   Ser Gly Glu Ile Gly Leu Thr Lys Tyr Asp Ser Gly Glu Ile Pro Arg
       130                 135                 140
   Gly Tyr Ile Pro Ser Val Thr Asn Ser Gln Ile Ser Gly Glu Ile Pro
   145                 150                 155                 160
   Gly Ala Ser Pro Asp His His Met Met Ser Pro Thr Gly Asn Ile Gly
                   165                 170                 175
   Lys Arg Ala Pro Phe Pro Tyr Val Asn His Ser Pro Asn Pro Ser Arg
               180                 185                 190
   Glu Phe Ser Gly Ser Ile Gly Asn Val Ala Trp Lys Glu Arg Val Asp
           195                 200                 205
   Gly Trp Lys Met Lys Gln Asp Lys Gly Thr Ile Pro Met Thr Asn Gly
       210                 215                 220
   Thr Ser Ile Ala Pro Ser Glu Gly Arg Gly Val Gly Asp Ile Asp Ala
   225                 230                 235                 240
   Ser Thr Asp Tyr Asn Met Glu Asp Ala Leu Leu Asn Asp Glu Thr Arg
                   245                 250                 255
   Gln Pro Leu Ser Arg Lys Val Pro Leu Pro Ser Ser Arg Ile Asn Pro
               260                 265                 270
   Tyr Arg Met Val Ile Val Leu Arg Leu Ile Val Leu Ser Ile Phe Leu
           275                 280                 285
   His Tyr Arg Ile Thr Asn Pro Val Arg Asn Ala Tyr Pro Leu Trp Leu
       290                 295                 300
   Leu Ser Val Ile Cys Glu Ile Trp Phe Ala Leu Ser Trp Ile Leu Asp
   305                 310                 315                 320
   Gln Phe Pro Lys Trp Phe Pro Ile Asn Arg Glu Thr Tyr Leu Asp Arg
                   325                 330                 335
   Leu Ala Leu Arg Tyr Asp Arg Glu Gly Glu Pro Ser Gln Leu Ala Ala
               340                 345                 350
   Val Asp Ile Phe Val Ser Thr Val Asp Pro Met Lys Glu Pro Pro Leu
           355                 360                 365
   Val Thr Ala Asn Thr Val Leu Ser Ile Leu Ala Val Asp Tyr Pro Val
       370                 375                 380
   Asp Lys Val Ser Cys Tyr Val Ser Asp Asp Gly Ala Ala Met Leu Thr
   385                 390                 395                 400
   Phe Asp Ala Leu Ala Glu Thr Ser Glu Phe Ala Arg Lys Trp Val Pro
                   405                 410                 415
   Phe Val Lys Lys Tyr Asn Ile Glu Pro Arg Ala Pro Glu Trp Tyr Phe
               420                 425                 430
   Ser Gln Lys Ile Asp Tyr Leu Lys Asp Lys Val His Pro Ser Phe Val
           435                 440                 445
   Lys Asp Arg Arg Ala Met Lys Arg Glu Tyr Glu Glu Phe Lys Val Arg
       450                 455                 460
   Val Asn Gly Leu Val Ala Lys Ala Gln Lys Val Pro Glu Glu Gly Trp
   465                 470                 475                 480
   Ile Met Gln Asp Gly Thr Pro Trp Pro Gly Asn Asn Thr Xaa Asp His
                   485                 490                 495
   Pro Gly Met Ile Gln Val Phe Leu Gly His Ser Gly Gly Leu Asp Thr
               500                 505                 510
   Glu Gly Asn Glu Leu Pro Arg Leu Val Tyr Val Ser Arg Glu Lys Arg
           515                 520                 525
   Pro Gly Phe Gln His His Lys Lys Ala Gly Ala Met Asn Ala Leu Val
       530                 535                 540
   Arg Val Ser Ala Val Leu Thr Asn Gly Gln Tyr Met Leu Asn Leu Asp
   545                 550                 555                 560
   Cys Asp His Tyr Ile Asn Asn Ser Lys Ala Leu Arg Glu Ala Met Cys
                   565                 570                 575
   Phe Leu Met Asp Pro Asn Leu Gly Arg Ser Val Cys Tyr Val Gln Phe
               580                 585                 590
   Pro Gln Arg Phe Asp Gly Ile Asp Arg Asn Asp Arg Tyr Ala Asn Arg
           595                 600                 605
   Asn Thr Val Phe Phe Asp Ile Asn Leu Arg Gly Leu Asp Gly Ile Gln
       610                 615                 620
   Gly Pro Val Tyr Val Gly Thr Gly Cys Val Phe Asn Arg Thr Ala Leu
   625                 630                 635                 640
   Tyr Gly Tyr Glu Pro Pro Ile Lys Gln Lys Lys Gly Gly Phe Leu Ser
                   645                 650                 655
   Ser Leu Cys Gly Gly Arg Lys Lys Ala Ser Lys Ser Lys Lys Gly Ser
               660                 665                 670
   Asp Lys Lys Lys Ser Gln Lys His Val Asp Ser Ser Val Pro Val Phe
           675                 680                 685
   Asn Leu Glu Asp Ile Glu Glu Gly Val Glu Gly Ala Gly Phe Asp Asp
       690                 695                 700
   Glu Lys Ser Leu Leu Met Ser Gln Met Ser Leu Glu Lys Arg Phe Gly
   705                 710                 715                 720
   Gln Ser Ala Ala Phe Val Ala Ser Thr Leu Met Glu Tyr Gly Gly Val
                   725                 730                 735
   Pro Gln Ser Ala Thr Pro Glu Ser Leu Leu Lys Glu Ala Ile His Val
               740                 745                 750
   Ile Ser Cys Gly Tyr Glu Asp Lys Thr Glu Trp Gly Thr Glu Ile Gly
           755                 760                 765
   Trp Ile Tyr Gly Ser Val Thr Glu Asp Ile Leu Thr Gly Phe Lys Met
       770                 775                 780
   His Ala Arg Gly Trp Arg Ser Ile Tyr Cys Met Pro Lys Arg Pro Ala
   785                 790                 795                 800
   Phe Lys Gly Ser Ala Pro Ile Asn Leu Ser Asp Arg Leu Asn Gln Val
                   805                 810                 815
   Leu Arg Trp Ala Leu Gly Ser Val Glu Ile Leu Phe Ser Arg His Cys
                   820                 825                 830
   Pro Leu Trp Tyr Gly Tyr Gly Gly Arg Leu Lys Phe Leu Glu Arg Phe
               835                 840                 845
   Ala Tyr Ile Asn Thr Thr Ile Tyr Pro Leu Thr Ser Ile Pro Leu Leu
       850                 855                 860
   Ile Tyr Cys Ile Leu Pro Ala Ile Cys Leu Leu Thr Gly Lys Phe Ile
   865                 870                 875                 880
   Ile Pro Glu Ile Ser Asn Phe Ala Ser Ile Trp Phe Ile Ser Leu Phe
                   885                 890                 895
   Ile Ser Ile Phe Ala Thr Gly Ile Leu Glu Met Arg Trp Ser Gly Val
               900                 905                 910
   Gly Ile Asp Glu Trp Trp Arg Asn Glu Gln Phe Trp Val Ile Gly Gly
           915                 920                 925
   Ile Ser Ala His Leu Phe Ala Val Phe Gln Gly Leu Leu Lys Val Leu
       930                 935                 940
   Ala Gly Ile Asp Thr Asn Phe Thr Val Thr Ser Lys Ala Ser Asp Glu
   945                 950                 955                 960
   Asp Gly Asp Phe Ala Glu Leu Tyr Met Phe Lys Trp Thr Thr Leu Leu
                   965                 970                 975
   Ile Pro Pro Thr Thr Ile Leu Ile Ile Asn Leu Val Gly Val Val Ala
               980                 985                 990
   Gly Ile Ser Tyr Ala Ile Asn Ser Gly Tyr Gln Ser Trp Gly Pro Leu
           995                 1000                1005
   Phe Gly Lys Leu Phe Phe Ala Phe Trp Val Ile Val His Leu Tyr Pro
       1010                1015                1020
   Phe Leu Lys Gly Leu Met Gly Arg Gln Asn Arg Thr Pro Thr Ile Val
   1025                1030                1035                1040
   Val Val Trp
<210> 51
   <211> 25
   <212> DNA
   <213> Zea mays
<400> 51
   atggagggcg acgcggacgg cgtga
   25
<210> 52
   <211> 25
   <212> DNA
   <213> Zea mays
<400> S2
<210> 53
   <211> 3753
   <212> DNA
   <213> Zea mays
<220>
   <221> CDS
   <222> (184)...(3406)
<400> 53
   cagcagcaga agcactgcgc ggcattgcag cgatcgagcg ggaggaattt ggggcatggt     60
   ggtcgccaac gccgctcgga tctagaggcc cgcacgggcc gattggtctc cgcccgcctc    120
   gtcggtgttg gtgtcgttgg cgtgtggagc cgtctcggtg ggagcagcgg ggagggagcg    180
   gag atg gcg gcc aac aag ggg atg gtg gcg ggc tcg cac aac cgc aac      228
       Met Ala Ala Asn Lys Gly Met Val Ala Gly Ser His Asn Arg Asn
        1               5                  10                  15
   gag ttc gtc atg atc cgc cac gac ggc gat gtg ccg ggc tcg gct aag      276
   Glu Phe Val Met Ile Arg His Asp Gly Asp Val Pro Gly Ser Ala Lys
                   20                  25                  30
   ccc aca aag agt gcg aat gga cag gtc tgc cag att tgc ggt gac tct      324
   Pro Thr Lys Ser Ala Asn Gly Gln Val Cys Gln Ile Cys Gly Asp Ser
               35                  40                  45
   gtg ggt gtt tca gcc act ggt gat gtc ttt gtt gcc tgc aat gag tgt      372
   Val Gly Val Ser Ala Thr Gly Asp Val Phe Val Ala Cys Asn Glu Cys
           50                  55                  60
   gcc ttc cct gtc tgc cgc cca tgc tat gag tat gag cgc aag gag ggg      420
   Ala Phe Pro Val Cys Arg Pro Cys Tyr Glu Tyr Glu Arg Lys Glu Gly
       65                  70                  75
   aac caa tgc tgc ccc cag tgc aag act aga tac aag aga cag aaa ggt      468
   Asn Gln Cys Cys Pro Gln Cys Lys Thr Arg Tyr Lys Arg Gln Lys Gly
   80                  85                  90                  95
   agc cct cga gtt cat ggt gat gag gat gag gaa gat gtt gat gac cta      516
   Ser Pro Arg Val His Gly Asp Glu Asp Glu Glu Asp Val Asp Asp Leu
                   100                 105                 110
   gac aat gaa ttc aac tac aag caa ggc agt ggg aaa ggc cca gag tgg      564
   Asp Asn Glu Phe Asn Tyr Lys Gln Gly Ser Gly Lys Gly Pro Glu Trp
               115                 120                 125
   caa ctg caa gga gat gat gct gat ctg tct tca tct gct cgc cat gag      612
   Gln Leu Gln Gly Asp Asp Ala Asp Leu Ser Ser Ser Ala Arg His Glu
           130                 135                 140
   cca cat cat cgg att cca cgc ctg aca agc ggt caa cag ata tct gga      660
   Pro His His Arg Ile Pro Arg Leu Thr Ser Gly Gln Gln Ile Ser Gly
       145                 150                 155
   gag att cct gat gct tcc cct gac cgt cat tct atc cgc agt cca aca      708
   Glu Ile Pro Asp Ala Ser Pro Asp Arg His Ser Ile Arg Ser Pro Thr
   160                 165                 170                 175
   tcg agc tat gtt gat cca agc gtc cca gtt cct gtg agg att gtg gac      756
   Ser Ser Tyr Val Asp Pro Ser Val Pro Val Pro Val Arg Ile Val Asp
                   180                 185                 190
   ccc tcg aag gac ttg aat tcc tat ggg ctt aat agt gtt gac tgg aag      804
   Pro Ser Lys Asp Leu Asn Ser Tyr Gly Leu Asn Ser Val Asp Trp Lys
               195                 200                 205
   gaa aga gtt gag agc tgg agg gtt aaa cag gac aaa aat atg atg caa      852
   Glu Arg Val Glu Ser Trp Arg Val Lys Gln Asp Lys Asn Met Met Gln
               210                      215                      220
   gtg act aat aaa tat cca gag gct aga gga gga gac atg gag ggg act      900
   Val Thr Asn Lys Tyr Pro Glu Ala Arg Gly Gly Asp Met Glu Gly Thr
       225                      230                      235
   ggc tca aat gga gaa nat atg caa atg gtt gat gat gca cgg cta cct      948
   Gly Ser Asn Gly Glu Xaa Met Gln Met Val Asp Asp Ala Arg Leu Pro
   240                      245                      250                 255
   ttg agc cgt atc gtg cca att tcc tca aac cag ctc aac ctt tac cgg      996
   Leu Ser Arg Ile Val Pro Ile Ser Ser Asn Gln Leu Asn Leu Tyr Arg
                   260                      265                      270
   gta gtg atc att ctc cgt ctt atc atc ctg tgc ttc ttc ttc cag tat     1044
   Val Val Ile Ile Leu Arg Leu Ile Ile Leu Cys Phe Phe Phe Gln Tyr
               275                      280                      285
   cgt gtc agt cat cca gtg cgt gat gct tat gga tta tgg cta gta tct     1092
   Arg Val Ser His Pro Val Arg Asp Ala Tyr Gly Leu Trp Leu Val Ser
           290                      295                      300
   gtt atc tgc gag gtc tgg ttt gcc ttg tct tgg ctt cta gat cag ttc     1140
   Val Ile Cys Glu Val Trp Phe Ala Leu Ser Trp Leu Leu Asp Gln Phe
       305                      310                      315
   cca aaa tgg tat cca atc aac cgt gag aca tat ctt gac agg ctt gca     1188
   Pro Lys Trp Tyr Pro Ile Asn Arg Glu Thr Tyr Leu Asp Arg Leu Ala
   320                      325                      330                 335
   ttg agg tat gat aga gag gga gag cca tca cag ctg gct ccc att gat     1236
   Leu Arg Tyr Asp Arg Glu Gly Glu Pro Ser Gln Leu Ala Pro Ile Asp
                   340                      345                      350
   gtc ttc gtc agt aca gtg gat cca ttg aag gaa cct cca ctg atc aca     1284
   Val Phe Val Ser Thr Val Asp Pro Leu Lys Glu Pro Pro Leu Ile Thr
               355                      360                      365
   gcc aac act gtt ttg tcc att ctt tct gtg gat tac cct gtt gac aaa     1332
   Ala Asn Thr Val Leu Ser Ile Leu Ser Val Asp Tyr Pro Val Asp Lys
           370                      375                      380
   gtg tca tgc tat gtt tct gat gat ggt tca gct atg ctg act ttt gag     1380
   Val Ser Cys Tyr Val Ser Asp Asp Gly Ser Ala Met Leu Thr Phe Glu
       385                      390                      395
   tct ctc tca gaa acc gca gaa ttt gct aga aag tgg gtt ccc ttt tgt     1428
   Ser Leu Ser Glu Thr Ala Glu Phe Ala Arg Lys Trp Val Pro Phe Cys
   400                      405                      410                 415
   aag aag cac aat att gaa cca aga gct cca gaa ttt tac ttt gct caa     1476
   Lys Lys His Asn Ile Glu Pro Arg Ala Pro Glu Phe Tyr Phe Ala Gln
                   420                      425                      430
   aaa ata gat tac ctg aag gac aaa att caa cct tca ttt gtt aag gaa     1524
   Lys Ile Asp Tyr Leu Lys Asp Lys Ile Gln Pro Ser Phe Val Lys Glu
               435                      440                      445
   aga cgc gca atg aag agg gag tat gaa gaa ttc aaa gta aga atc aat      1572
   Arg Arg Ala Met Lys Arg Glu Tyr Glu Glu Phe Lys Val Arg Ile Asn
           450                 455                 460
   gcc ctt gtt gcc aaa gca cag aaa gtg cct gaa gag ggg tgg acc atg      1620
   Ala Leu Val Ala Lys Ala Gln Lys Val Pro Glu Glu Gly Trp Thr Met
       465                 470                 475
   gct gat gga act gca tgg cct ggg aat aat cct agg gac cat cct ggc      1668
   Ala Asp Gly Thr Ala Trp Pro Gly Asn Asn Pro Arg Asp His Pro Gly
   480                 485                 490                 495
   atg att cag gtt ttc ttg ggg cac agt ggt ggg ctc gac act gat gga      1716
   Met Ile Gln Val Phe Leu Gly His Ser Gly Gly Leu Asp Thr Asp Gly
                   500                 505                 510
   aat gag tta cca cgt ctt gtc tat gtc tct cgt gaa aag aga cca ggc      1764
   Asn Glu Leu Pro Arg Leu Val Tyr Val Ser Arg Glu Lys Arg Pro Gly
               515                 520                 525
   ttt cag cat cac aag aag gct ggt gca atg aat gcg ctg att cgt gta      1812
   Phe Gln His His Lys Lys Ala Gly Ala Met Asn Ala Leu Ile Arg Val
           530                 535                 540
   tct gct gtg ctg aca aat ggt gcc tat ctt ctc aat gtg gat tgc gac      1860
   Ser Ala Val Leu Thr Asn Gly Ala Tyr Leu Leu Asn Val Asp Cys Asp
       545                 550                 555
   cat tac ttc aat agc agc aaa gct ctt aga gaa gca atg tgc ttc atg      1908
   His Tyr Phe Asn Ser Ser Lys Ala Leu Arg Glu Ala Met Cys Phe Met
   560                 565                 570                 575
   atg gat ccg gct cta gga agg aaa act tgt tat gta caa ttt cca cag      1956
   Met Asp Pro Ala Leu Gly Arg Lys Thr Cys Tyr Val Gln Phe Pro Gln
                   580                 585                 590
   aga ttt gat ggc att gac ttg cac gat cga tat gct aat cgg aac ata      2004
   Arg Phe Asp Gly Ile Asp Leu His Asp Arg Tyr Ala Asn Arg Asn Ile
               595                 600                 605
   gtt ttc ttt gat atc aac atg aaa ggt ctg gat ggc att cag ggt cca      2052
   Val Phe Phe Asp Ile Asn Met Lys Gly Leu Asp Gly Ile Gln Gly Pro
           610                 615                 620
   gtt tac gtg gga aca gga tgc tgt ttc aat aga cag gct ttg tat gga      2100
   Val Tyr Val Gly Thr Gly Cys Cys Phe Asn Arg Gln Ala Leu Tyr Gly
       625                 630                 635
   tac gat cct gtt ttg act gaa gct gat ctg gag cca aac att gtt att      2148
   Tyr Asp Pro Val Leu Thr Glu Ala Asp Leu Glu Pro Asn Ile Val Ile
   640                 645                 650                 655
   aag agc tgc tgt ggt aga agg aag aaa aag aac aag agt tat atg gat      2196
   Lys Ser Cys Cys Gly Arg Arg Lys Lys Lys Asn Lys Ser Tyr Met Asp
                   660                 665                 670
   agt caa agc cgt att atg aag aga aca gaa tct tca gct ccc atc ttc      2244
   Ser Gln Ser Arg Ile Met Lys Arg Thr Glu Ser Ser Ala Pro Ile Phe
   675 680 685
   aat atg gaa gac atc gaa gag ggt att gaa ggt tac gag gat gaa agg 2292
   Asn Met Glu Asp Ile Glu Glu Gly Ile Glu Gly Tyr Glu Asp Glu Arg
   690 695 700
   tca gtg ctt atg tcc cag agg aaa ttg gag aaa cgc ttt ggt cag tct 2340
   Ser Val Leu Met Ser Gln Arg Lys Leu Glu Lys Arg Phe Gly Gln Ser
   705 710 715
   cct att ttc att gca tcc acc ttt atg aca caa ggt ggc ata cca cct 2388
   Pro Ile Phe Ile Ala Ser Thr Phe Met Thr Gln Gly Gly Ile Pro Pro
   720 725 730 735
   tca aca aac cca gct tct cta cta aag gaa gct atc cat gtc atc agt 2436
   Ser Thr Asn Pro Ala Ser Leu Leu Lys Glu Ala Ile His Val Ile Ser
   740 745 750
   tgt gga tat gag gac aaa act gaa tgg gga aaa gag att ggc tgg atc 2484
   Cys Gly Tyr Glu Asp Lys Thr Glu Trp Gly Lys Glu Ile Gly Trp Ile
   755 760 765
   tat ggt tca gta acg gag gat att ctg act ggg ttt aaa atg cat gca 2532
   Tyr Gly Ser Val Thr Glu Asp Ile Leu Thr Gly Phe Lys Met His Ala
   770 775 780
   agg ggc tgg caa tca atc tac tgc atg cca cca cga cct tgt ttc aag 2580
   Arg Gly Trp Gln Ser Ile Tyr Cys Met Pro Pro Arg Pro Cys Phe Lys
   785 790 795
   ggt tct gca cca atc aat ctt tcc gat cgt ctt aat cag gtg ctc cgt 2628
   Gly Ser Ala Pro Ile Asn Leu Ser Asp Arg Leu Asn Gln Val Leu Arg
   800 805 810 815
   tgg gct ctt ggg tca gtg gaa att ctg ctt agt aga cat tgt cct atc 2676
   Trp Ala Leu Gly Ser Val Glu Ile Leu Leu Ser Arg His Cys Pro Ile
   820 825 830
   tgg tat ggt tac aat gga cga ttg aag ctt ttg gag agg ctg gct tac 2724
   Trp Tyr Gly Tyr Asn Gly Arg Leu Lys Leu Leu Glu Arg Leu Ala Tyr
   835 840 845
   atc aac act att gta tat cca atc aca tcc att ccg ctt att gcc tat 2772
   Ile Asn Thr Ile Val Tyr Pro Ile Thr Ser Ile Pro Leu Ile Ala Tyr
   850 855 860
   tgt gtg ctt ccc gct atc tgc ctc ctt acc aat aaa ttt atc att cct 2820
   Cys Val Leu Pro Ala Ile Cys Leu Leu Thr Asn Lys Phe Ile Ile Pro
   865 870 875
   gag att agc aat tat gct ggg atg ttc ttc att ctt ctt ttc gcc tcc 2868
   Glu Ile Ser Asn Tyr Ala Gly Met Phe Phe Ile Leu Leu Phe Ala Ser
   880 885 890 895
   att ttt gcc act ggt ata ttg gag ctt aga tgg agt ggt gtt ggc att 2916
   Ile Phe Ala Thr Gly Ile Leu Glu Leu Arg Trp Ser Gly Val Gly Ile
   900 905 910
   gaa gat tgg tgg aga aat gag cag ttt tgg gtt att ggt ggc acc tct       2964
   Glu Asp Trp Trp Arg Asn Glu Gln Phe Trp Val Ile Gly Gly Thr Ser
               915                 920                 925
   gcc cat ctc ttc gca gtg ttc cag ggt ctg ctg aaa gtg ttg gct ggg       3012
   Ala His Leu Phe Ala Val Phe Gln Gly Leu Leu Lys Val Leu Ala Gly
           930                 935                 940
   att gat acc aac ttc aca gtt acc tca aag gca tct gat gag gat ggc       3060
   Ile Asp Thr Asn Phe Thr Val Thr Ser Lys Ala Ser Asp Glu Asp Gly
       945                 950                 955
   gac ttt gct gag cta tat gtg ttc aag tgg acc agt ttg ctc att cct       3108
   Asp Phe Ala Glu Leu Tyr Val Phe Lys Trp Thr Ser Leu Leu Ile Pro
   960                 965                 970                 975
   ccg acc act gtt ctt gtc att aac ctg gtc gga atg gtg gca gga att       3156
   Pro Thr Thr Val Leu Val Ile Asn Leu Val Gly Met Val Ala Gly Ile
                   980                 985                 990
   tct tat gcc att aac agt ggc tac caa tcc tgg ggt ccg ctc ttt gga       3204
   Ser Tyr Ala Ile Asn Ser Gly Tyr Gln Ser Trp Gly Pro Leu Phe Gly
               995                 1000                1005
   aag ctg ttc ttc tcg atc tgg gtg atc ctc cat ctc tac ccc ttc ctc       3252
   Lys Leu Phe Phe Ser Ile Trp Val Ile Leu His Leu Tyr Pro Phe Leu
           1010                1015                1020
   aag ggt ctc atg gga agg cag aac cgc aca cca aca atc gtc att gtc       3300
   Lys Gly Leu Met Gly Arg Gln Asn Arg Thr Pro Thr Ile Val Ile Val
       1025                1030                1035
   tgg tcc atc ctt ctt gca tct atc ttc tcc ttg ctg tgg gtg aag atc       3348
   Trp Ser Ile Leu Leu Ala Ser Ile Phe Ser Leu Leu Trp Val Lys Ile
   1040                1045                1050                1055
   gat cct ttc atc tcc ccg aca cag aaa gct gct gcc ttg ggg caa tgt       3396
   Asp Pro Phe Ile Ser Pro Thr Gln Lys Ala Ala Ala Leu Gly Gln Cys
                   1060                1065                1070
   ggc gtc aac t gctgatcgag acagtgactc ttatttgaag aggctcaatc            3446
   Gly Val Asn
   aagatctgcc ccctcgtgta aatacctgag gaggctagat gggaattcct tttgttgtag   3506
   gtgaggatgg atttgcatct aagttatgcc tctgttcatt agcttcttcc gtgccggtgc   3566
   tgctgcggac taagaatcac ggagcctttc taccttccat gtagcgccag ccagcagcgt   3626
   aagatgtgaa ttttgaagtt ttgttatgcg tgcagtttat tgttttagag taaattatca   3686
   tttgtttgtg ggaactgttc acacgagctt ataatggcaa tgctgttatt taaaaaaaaa   3746
   aaaaaaa                                                             3753
<210> 54
   <211> 1075
   <212> PRT
   <213> Zea mays
<400> 54
   Met Ala Ala Asn Lys Gly Met Val Ala Gly Ser His Asn Arg Asn Glu
   1               5                   10                  15
   Phe Val Met Ile Arg His Asp Gly Asp Val Pro Gly Ser Ala Lys Pro
               20                  25                  30
   Thr Lys Ser Ala Asn Gly Gln Val Cys Gln Ile Cys Gly Asp Ser Val
           35                  40                  45
   Gly Val Ser Ala Thr Gly Asp Val Phe Val Ala Cys Asn Glu Cys Ala
       50                  55                  60
   Phe Pro Val Cys Arg Pro Cys Tyr Glu Tyr Glu Arg Lys Glu Gly Asn
   65                  70                  75                  80
   Gln Cys Cys Pro Gln Cys Lys Thr Arg Tyr Lys Arg Gln Lys Gly Ser
                   85                  90                  95
   Pro Arg Val His Gly Asp Glu Asp Glu Glu Asp Val Asp Asp Leu Asp
               100                 105                 110
   Asn Glu Phe Asn Tyr Lys Gln Gly Ser Gly Lys Gly Pro Glu Trp Gln
           115                 120                 125
   Leu Gln Gly Asp Asp Ala Asp Leu Ser Ser Ser Ala Arg His Glu Pro
       130                 135                 140
   His His Arg Ile Pro Arg Leu Thr Ser Gly Gln Gln Ile Ser Gly Glu
   145                 150                 155                 160
   Ile Pro Asp Ala Ser Pro Asp Arg His Ser Ile Arg Ser Pro Thr Ser
                   165                 170                 175
   Ser Tyr Val Asp Pro Ser Val Pro Val Pro Val Arg Ile Val Asp Pro
               180                 185                 190
   Ser Lys Asp Leu Asn Ser Tyr Gly Leu Asn Ser Val Asp Trp Lys Glu
           195                 200                 205
   Arg Val Glu Ser Trp Arg Val Lys Gln Asp Lys Asn Met Met Gln Val
       210                 215                 220
   Thr Asn Lys Tyr Pro Glu Ala Arg Gly Gly Asp Met Glu Gly Thr Gly
   225                 230                 235                 240
   Ser Asn Gly Glu Xaa Met Gln Met Val Asp Asp Ala Arg Leu Pro Leu
                   245                 250                 255
   Ser Arg Ile Val Pro Ile Ser Ser Asn Gln Leu Asn Leu Tyr Arg Val
               260                 265                 270
   Val Ile Ile Leu Arg Leu Ile Ile Leu Cys Phe Phe Phe Gln Tyr Arg
           275                 280                 285
   Val Ser His Pro Val Arg Asp Ala Tyr Gly Leu Trp Leu Val Ser Val
       290                 295                 300
   Ile Cys Glu Val Trp Phe Ala Leu Ser Trp Leu Leu Asp Gln Phe Pro
   305                 310                 315                 320
   Lys Trp Tyr Pro Ile Asn Arg Glu Thr Tyr Leu Asp Arg Leu Ala Leu
                   325                 330                 335
   Arg Tyr Asp Arg Glu Gly Glu Pro Ser Gln Leu Ala Pro Ile Asp Val
               340                 345                 350
   Phe Val Ser Thr Val Asp Pro Leu Lys Glu Pro Pro Leu Ile Thr Ala
           355                 360                 365
   Asn Thr Val Leu Ser Ile Leu Ser Val Asp Tyr Pro Val Asp Lys Val
       370                 375                 380
   Ser Cys Tyr Val Ser Asp Asp Gly Ser Ala Met Leu Thr Phe Glu Ser
   385                 390                 395                 400
   Leu Ser Glu Thr Ala Glu Phe Ala Arg Lys Trp Val Pro Phe Cys Lys
                   405                 410                 415
   Lys His Asn Ile Glu Pro Arg Ala Pro Glu Phe Tyr Phe Ala Gln Lys
               420                 425                 430
   Ile Asp Tyr Leu Lys Asp Lys Ile Gln Pro Ser Phe Val Lys Glu Arg
           435                 440                 445
   Arg Ala Met Lys Arg Glu Tyr Glu Glu Phe Lys Val Arg Ile Asn Ala
       450                 455                 460
   Leu Val Ala Lys Ala Gln Lys Val Pro Glu Glu Gly Trp Thr Met Ala
   465 470 475 480
   Asp Gly Thr Ala Trp Pro Gly Asn Asn Pro Arg Asp His Pro Gly Met
   485 490 495
   Ile Gln Val Phe Leu Gly His Ser Gly Gly Leu Asp Thr Asp Gly Asn
   500 505 510
   Glu Leu Pro Arg Leu Val Tyr Val Ser Arg Glu Lys Arg Pro Gly Phe
   515 520 525
   Gln His His Lys Lys Ala Gly Ala Met Asn Ala Leu Ile Arg Val Ser
   530 535 540
   Ala Val Leu Thr Asn Gly Ala Tyr Leu Leu Asn Val Asp Cys Asp His
   545 550 555 560
   Tyr Phe Asn Ser Ser Lys Ala Leu Arg Glu Ala Met Cys Phe Met Met
   565 570 575
   Asp Pro Ala Leu Gly Arg Lys Thr Cys Tyr Val Gln Phe Pro Gln Arg
   580 585 590
   Phe Asp Gly Ile Asp Leu His Asp Arg Tyr Ala Asn Arg Asn Ile Val
   595 600 605
   Phe Phe Asp Ile Asn Met Lys Gly Leu Asp Gly Ile Gln Gly Pro Val
   610 615 620
   Tyr Val Gly Thr Gly Cys Cys Phe Asn Arg Gln Ala Leu Tyr Gly Tyr
   625 630 635 640
   Asp Pro Val Leu Thr Glu Ala Asp Leu Glu Pro Asn Ile Val Ile Lys
   645 650 655
   Ser Cys Cys Gly Arg Arg Lys Lys Lys Asn Lys Ser Tyr Met Asp Ser
   660 665 670
   Gln Ser Arg Ile Met Lys Arg Thr Glu Ser Ser Ala Pro Ile Phe Asn
   675 680 685
   Met Glu Asp Ile Glu Glu Gly Ile Glu Gly Tyr Glu Asp Glu Arg Ser
   690 695 700
   Val Leu Met Ser Gln Arg Lys Leu Glu Lys Arg Phe Gly Gln Ser Pro
   705 710 715 720
   Ile Phe Ile Ala Ser Thr Phe Met Thr Gln Gly Gly Ile Pro Pro Ser
   725 730 735
   Thr Asn Pro Ala Ser Leu Leu Lys Glu Ala Ile His Val Ile Ser Cys
   740 745 750
   Gly Tyr Glu Asp Lys Thr Glu Trp Gly Lys Glu Ile Gly Trp Ile Tyr
   755 760 765
   Gly Ser Val Thr Glu Asp Ile Leu Thr Gly Phe Lys Met His Ala Arg
   770 775 780
   Gly Trp Gln Ser Ile Tyr Cys Met Pro Pro Arg Pro Cys Phe Lys Gly
   785 790 795 800
   Ser Ala Pro Ile Asn Leu Ser Asp Arg Leu Asn Gln Val Leu Arg Trp
   805 810 815
   Ala Leu Gly Ser Val Glu Ile Leu Leu Ser Arg His Cys Pro Ile Trp
   820 825 830
   Tyr Gly Tyr Asn Gly Arg Leu Lys Leu Leu Glu Arg Leu Ala Tyr Ile
   835 840 845
   Asn Thr Ile Val Tyr Pro Ile Thr Ser Ile Pro Leu Ile Ala Tyr Cys
   850 855 860
   Val Leu Pro Ala Ile Cys Leu Leu Thr Asn Lys Phe Ile Ile Pro Glu
   865 870 875 880
   Ile Ser Asn Tyr Ala Gly Met Phe Phe Ile Leu Leu Phe Ala Ser Ile
   885 890 895
   Phe Ala Thr Gly Ile Leu Glu Leu Arg Trp Ser Gly Val Gly Ile Glu
   900 905 910
   Asp Trp Trp Arg Asn Glu Gln Phe Trp Val Ile Gly Gly Thr Ser Ala
   915 920 925
   His Leu Phe Ala Val Phe Gln Gly Leu Leu Lys Val Leu Ala Gly Ile
       930                 935                 940
   Asp Thr Asn Phe Thr Val Thr Ser Lys Ala Ser Asp Glu Asp Gly Asp
   945                 950                 955                 960
   Phe Ala Glu Leu Tyr Val Phe Lys Trp Thr Ser Leu Leu Ile Pro Pro
                   965                 970                 975
   Thr Thr Val Leu Val Ile Asn Leu Val Gly Met Val Ala Gly Ile Ser
               980                 985                 990
   Tyr Ala Ile Asn Ser Gly Tyr Gln Ser Trp Gly Pro Leu Phe Gly Lys
           995                 1000                1005
   Leu Phe Phe Ser Ile Trp Val Ile Leu His Leu Tyr Pro Phe Leu Lys
       1010                1015                1020
   Gly Leu Met Gly Arg Gln Asn Arg Thr Pro Thr Ile Val Ile Val Trp
   1025                1030                1035                1040
   Ser Ile Leu Leu Ala Ser Ile Phe Ser Leu Leu Trp Val Lys Ile Asp
                   1045                1050                1055
   Pro Phe Ile Ser Pro Thr Gln Lys Ala Ala Ala Leu Gly Gln Cys Gly
               1060                1065                1070
   Val Asn Cys
           1075
<210> 55
   <211> 25
   <212> DNA
   <213> Zea mays
<400> 55
   atggcggcca acaaggggat ggtgg
   25
<210> 56
   <211> 25
   <212> DNA
   <213> Zea mays
<400> 56
   tcagcagttg acgccacatt gcccc
   25
<210> 57
   <211> 3704
   <212> DNA
   <213> Zea mays
<220>
   <221> CDS
   <222> (272)...(3497)
<400> 57
   gtcgacccac gcttccggtc ggttccgcgt cccttttccc ctcccccctc cgtcgccgcc    60
   tcgagcgagc tccaccactt gctcctgcgc gaggtgaaca ctgggttagg gccactgcca    120
   ccgctgggct gcctctgctt ctgcctctcc cgccagcgcg cgagcccggg ggcgattcgg     180
   cgccggcacg cgggagggga agccgaggaa tgcggtgagt cggcgggggt ccggcgtttg    240
   tgaactcgtg gagggctcgg attggtgcgc c atg gac ggc ggc gac gcc acg       292
                                      Met Asp Gly Gly Asp Ala Thr
                                      1               5
   aat tcg ggg aag cat gtg gcc ggg cag gtg tgc cag atc tgc ggc gac      340
   Asn Ser Gly Lys His Val Ala Gly Gln Val Cys Gln Ile Cys Gly Asp
           10                  15                  20
   ggc gtg ggc acc gcg gcg gac ggc gac ctc ttc acc gcc tgc gac gtc      388
   Gly Val Gly Thr Ala Ala Asp Gly Asp Leu Phe Thr Ala Cys Asp Val
       25                  30                  35
   tgc ggc ttc ccc gtg tgc cgc cca tgc tac gag tac gag cgc aag gac      436
   Cys Gly Phe Pro Val Cys Arg Pro Cys Tyr Glu Tyr Glu Arg Lys Asp
   40                  45                  50                  55
   ggc acc cag gcg tgc ccg cag tgc aag act aag tac aag cgc cac aaa      484
   Gly Thr Gln Ala Cys Pro Gln Cys Lys Thr Lys Tyr Lys Arg His Lys
                   60                  65                  70
   ggg agc cca cca gta cac ggt gag gaa aat gag gat gtg gat gct gac      532
   Gly Ser Pro Pro Val His Gly Glu Glu Asn Glu Asp Val Asp Ala Asp
               75                  80                  85
   gat gtg agt gac tac aac tac caa gca tct ggc aac cag gat cag aag      580
   Asp Val Ser Asp Tyr Asn Tyr Gln Ala Ser Gly Asn Gln Asp Gln Lys
           90                  95                  100
   caa aag att gct gag aga atg ctc act tgg cgg aca aac tca cgt ggc      628
   Gln Lys Ile Ala Glu Arg Met Leu Thr Trp Arg Thr Asn Ser Arg Gly
       105                 110                 115
   agt gat att ggc ctg gct aag tat gac agc ggt gaa att ggg cat ggg      676
   Ser Asp Ile Gly Leu Ala Lys Tyr Asp Ser Gly Glu Ile Gly His Gly
   120                 125                 130                 135
   aag tat gac agt ggt gag atc cct cgt gga tat atc ccg tca cta act      724
   Lys Tyr Asp Ser Gly Glu Ile Pro Arg Gly Tyr Ile Pro Ser Leu Thr
                   140                 145                 150
   cat agc cag atc tca gga gag att cct gga gct tcc cct gat cat atg      772
   His Ser Gln Ile Ser Gly Glu Ile Pro Gly Ala Ser Pro Asp His Met
               155                 160                 165
   atg tct cct gtt ggg aac att ggc agg cgt gga cat caa ttt cct tat      820
   Met Ser Pro Val Gly Asn Ile Gly Arg Arg Gly His Gln Phe Pro Tyr
           170                 175                 180
   gta aat cat tct cca aac cca tcg agg gag ttc tcc ggt agc ctt ggc      868
   Val Asn His Ser Pro Asn Pro Ser Arg Glu Phe Ser Gly Ser Leu Gly
       185                 190                 195
   aat gtt gca tgg aaa gag agg gtg gat gga tgg aaa atg aag gat aaa      916
   Asn Val Ala Trp Lys Glu Arg Val Asp Gly Trp Lys Met Lys Asp Lys
   200                 205                 210                 215
   ggt gca att cct atg acc aat gga aca agc att gct cca tca gaa ggg      964
   Gly Ala Ile Pro Met Thr Asn Gly Thr Ser Ile Ala Pro Ser Glu Gly
                   220                 225                 230
   cgt gga gtt gct gat att gat gct tct act gat tat aac atg gaa gat     1012
   Arg Gly Val Ala Asp Ile Asp Ala Ser Thr Asp Tyr Asn Met Glu Asp
               235                 240                 245
   gcc tta ctg aat gat gaa act cgg caa cct cta tct aga aaa gtg cca      1060
   Ala Leu Leu Asn Asp Glu Thr Arg Gln Pro Leu Ser Arg Lys Val Pro
           250                 255                 260
   att cct tca tcc aga ata aat ccg tac aga atg gtc att gtg cta cgt      1108
   Ile Pro Ser Ser Arg Ile Asn Pro Tyr Arg Met Val Ile Val Leu Arg
       265                 270                 275
   ttg gct gtt cta tgc ata ttc ttg cgc tac cgt atc aca cat cct gtg      1156
   Leu Ala Val Leu Cys Ile Phe Leu Arg Tyr Arg Ile Thr His Pro Val
   280                 285                 290                 295
   aac aat gca tat cca ctg tgg ctt tta tcc gtc ata tgt gag atc tgg      1204
   Asn Asn Ala Tyr Pro Leu Trp Leu Leu Ser Val Ile Cys Glu Ile Trp
                   300                 305                 310
   ttt gct ttg tcc tgg att ttg gat cag ttc cca aag tgg tcc cca atc      1252
   Phe Ala Leu Ser Trp Ile Leu Asp Gln Phe Pro Lys Trp Ser Pro Ile
               315                 320                 325
   aac cgt gaa aca tac ctt gat aga ctg gct tta agg tat gac cga gaa      1300
   Asn Arg Glu Thr Tyr Leu Asp Arg Leu Ala Leu Arg Tyr Asp Arg Glu
           330                 335                 340
   ggt gaa cca tct caa tta gct cct gtt gat att ttt gtc agt act gtg      1348
   Gly Glu Pro Ser Gln Leu Ala Pro Val Asp Ile Phe Val Ser Thr Val
       345                 350                 355
   gat cca atg aag gag cct cct ctt gtc act gca aat act gtg ctt tcc      1396
   Asp Pro Met Lys Glu Pro Pro Leu Val Thr Ala Asn Thr Val Leu Ser
   360                 365                 370                 375
   atc ctt gct gtc gat tat ccg gtt gac aag gta tct tgc tat gtt tcg      1444
   Ile Leu Ala Val Asp Tyr Pro Val Asp Lys Val Ser Cys Tyr Val Ser
                   380                 385                 390
   gat gat gga gct gct atg ctg act ttt gat gct ctc tct gaa act tca      1492
   Asp Asp Gly Ala Ala Met Leu Thr Phe Asp Ala Leu Ser Glu Thr Ser
               395                 400                 405
   gag ttt gct aga aaa tgg gtt ccg ttc tgt aag aag tac aac ata gag      1540
   Glu Phe Ala Arg Lys Trp Val Pro Phe Cys Lys Lys Tyr Asn Ile Glu
           410                 415                 420
   cct ang gcc ccg gaa tgg tac ttt gct cag aaa att gat tac ttg aaa      1588
   Pro Xaa Ala Pro Glu Trp Tyr Phe Ala Gln Lys Ile Asp Tyr Leu Lys
       425                 430                 435
   gac aaa gtt caa acc tca ttt gtg aaa gaa cgc cgg gcc atg aag aga      1636
   Asp Lys Val Gln Thr Ser Phe Val Lys Glu Arg Arg Ala Met Lys Arg
   440                 445                 450                 455
   gaa tat gaa gaa ttc aaa gtt cgt atc aat ggt ctt gta gcc aag gca      1684
   Glu Tyr Glu Glu Phe Lys Val Arg Ile Asn Gly Leu Val Ala Lys Ala
                   460                 465                 470
   caa aaa gtt ccc gag gag gga tgg atc atg caa gat ggt aca cct tgg      1732
   Gln Lys Val Pro Glu Glu Gly Trp Ile Met Gln Asp Gly Thr Pro Trp
               475                 480                 485
   cct ggg aac aat act agg gac cat cct gga atg att cag gtt ttc ctg     1780
   Pro Gly Asn Asn Thr Arg Asp His Pro Gly Met Ile Gln Val Phe Leu
           490                 495                 500
   ggt cac agt gga ggg ctt gac gtt gaa ggc aat gaa ctt cct cgt ttg     1828
   Gly His Ser Gly Gly Leu Asp Val Glu Gly Asn Glu Leu Pro Arg Leu
       505                 510                 515
   gtt tat gtg tct cgt gaa aaa cgt cct gga ttc caa cat cac aag aag     1876
   Val Tyr Val Ser Arg Glu Lys Arg Pro Gly Phe Gln His His Lys Lys
   520                 525                 530                 535
   gct ggt gcc atg aat gca ctt gtt cgt gta tca gct gtc ctt act aat     1924
   Ala Gly Ala Met Asn Ala Leu Val Arg Val Ser Ala Val Leu Thr Asn
                   540                 545                 550
   ggg caa tac atg ttg aat ctt gat tgt gac cac tac atc aat aat agc     1972
   Gly Gln Tyr Met Leu Asn Leu Asp Cys Asp His Tyr Ile Asn Asn Ser
               555                 560                 565
   aag gct ctt cga gaa gct atg tgc ttc ctt atg gac cca aac cta gga     2020
   Lys Ala Leu Arg Glu Ala Met Cys Phe Leu Met Asp Pro Asn Leu Gly
           570                 575                 580
   agg aat gtc tgt tat gtc caa ttt cct cag agg ttt gat ggt att gat     2068
   Arg Asn Val Cys Tyr Val Gln Phe Pro Gln Arg Phe Asp Gly Ile Asp
       585                 590                 595
   agg aat gac cga tat gca aac agg aac act gtg ttt ttc gat att aac     2116
   Arg Asn Asp Arg Tyr Ala Asn Arg Asn Thr Val Phe Phe Asp Ile Asn
   600                 605                 610                 615
   ttg aga ggt ctt gac ggc att caa ggg cca gtt tat gtg gga act ggt     2164
   Leu Arg Gly Leu Asp Gly Ile Gln Gly Pro Val Tyr Val Gly Thr Gly
                   620                 625                 630
   tgt gtg ttt aac aga acg gcc tta tat ggt tat gag cct cca gtc aag     2212
   Cys Val Phe Asn Arg Thr Ala Leu Tyr Gly Tyr Glu Pro Pro Val Lys
               635                 640                 645
   aaa aaa aag cca ggc ttc ttc tct tcg ctt tgt ggg gga agg aaa aag     2260
   Lys Lys Lys Pro Gly Phe Phe Ser Ser Leu Cys Gly Gly Arg Lys Lys
           650                 655                 660
   acg tca aaa tct aag aag agc tcg gaa aag aag aag tca cat aga cac     2308
   Thr Ser Lys Ser Lys Lys Ser Ser Glu Lys Lys Lys Ser His Arg His
       665                 670                 675
   gca gac agt tct gta cca gta ttt aat ctc gaa gat ata gag gaa ggg     2356
   Ala Asp Ser Ser Val Pro Val Phe Asn Leu Glu Asp Ile Glu Glu Gly
   680                 685                 690                 695
   att gaa ggt tct cag ttt gat gat gag aaa tcg ctg att atg tct caa     2404
   Ile Glu Gly Ser Gln Phe Asp Asp Glu Lys Ser Leu Ile Met Ser Gln
                   700                 705                 710
   atg agc ttg gag aag aga ttt ggc cag tcc agt gtt ttt gta gcc tct      2452
   Met Ser Leu Glu Lys Arg Phe Gly Gln Ser Ser Val Phe Val Ala Ser
               715                 720                 725
   act ctg atg gaa tat ggt ggt gtt cca caa tct gca act cca gag tct      2500
   Thr Leu Met Glu Tyr Gly Gly Val Pro Gln Ser Ala Thr Pro Glu Ser
           730                 735                 740
   ctt ctg aaa gaa gct att cat gtc atc agc tgt ggc tat gag gac aaa      2548
   Leu Leu Lys Glu Ala Ile His Val Ile Ser Cys Gly Tyr Glu Asp Lys
       745                 750                 755
   act gac tgg gga act gag att ggg tgg atc tat ggt tct gtt aca gaa      2596
   Thr Asp Trp Gly Thr Glu Ile Gly Trp Ile Tyr Gly Ser Val Thr Glu
   760                 765                 770                 775
   gac att ctc acc gga ttc aag atg cat gct cga ggc tgg cga tca atc      2644
   Asp Ile Leu Thr Gly Phe Lys Met His Ala Arg Gly Trp Arg Ser Ile
                   780                 785                 790
   tac tgc atg cct aag cga cca gct ttc aag gga tct gct cct atc aac      2692
   Tyr Cys Met Pro Lys Arg Pro Ala Phe Lys Gly Ser Ala Pro Ile Asn
               795                 800                 805
   ctt tcg gat cgt ttg aat caa gtg ctt cgg tgg gct ctt ggt tcc att      2740
   Leu Ser Asp Arg Leu Asn Gln Val Leu Arg Trp Ala Leu Gly Ser Ile
           810                 815                 820
   gaa att ctt ttc agc agg cat tgt ccc ata tgg tat ggc tat gga ggc      2788
   Glu Ile Leu Phe Ser Arg His Cys Pro Ile Trp Tyr Gly Tyr Gly Gly
       825                 830                 835
   cgg ctt aaa ttc ctg gag aga ttt gct tat atc aac aca aca att tat      2836
   Arg Leu Lys Phe Leu Glu Arg Phe Ala Tyr Ile Asn Thr Thr Ile Tyr
   840                 845                 850                 855
   cca ctc aca tca atc ccg ctc ctc ctg tac tgc ata ttg cca gca gtt      2884
   Pro Leu Thr Ser Ile Pro Leu Leu Leu Tyr Cys Ile Leu Pro Ala Val
                   860                 865                 870
   tgt ctt ctc act ggg aag ttc atc atc cca aag att agt aac cta gag      2932
   Cys Leu Leu Thr Gly Lys Phe Ile Ile Pro Lys Ile Ser Asn Leu Glu
               875                 880                 885
   agt gtt tgg ttt ata tcg ctc ttt atc tca atc ttt gcc act ggt atc      2980
   Ser Val Trp Phe Ile Ser Leu Phe Ile Ser Ile Phe Ala Thr Gly Ile
           890                 895                 900
   ctt gag atg agg tgg agt ggt gtt ggc att gat gaa tgg tgg agg aac      3028
   Leu Glu Met Arg Trp Ser Gly Val Gly Ile Asp Glu Trp Trp Arg Asn
       905                 910                 915
   gag cag ttc tgg gtc att ggt ggt att tct gcg cat tta ttt gcc gtc      3076
   Glu Gln Phe Trp Val Ile Gly Gly Ile Ser Ala His Leu Phe Ala Val
   920                 925                 930                 935
   ttc cag ggt ctc ctg aag gtg ctt gct ggt atc gac acg agc ttc act      3124
   Phe Gln Gly Leu Leu Lys Val Leu Ala Gly Ile Asp Thr Ser Phe Thr
                       940                     945                     950
   gtc acc tct aag gcc act gac gaa gaa ggt gat ttt gcc gag ctc tac      3172
   Val Thr Ser Lys Ala Thr Asp Glu Glu Gly Asp Phe Ala Glu Leu Tyr
               955                     960                     965
   atg ttc aag tgg aca acg ctt ctg atc cca cca acc act att ttg atc      3220
   Met Phe Lys Trp Thr Thr Leu Leu Ile Pro Pro Thr Thr Ile Leu Ile
           970                     975                     980
   atc aac ctg gtc ggc gtg gtc gct ggc att tcc tac gca atc aat agc      3268
   Ile Asn Leu Val Gly Val Val Ala Gly Ile Ser Tyr Ala Ile Asn Ser
       985                     990                     995
   ggt tac cag tca tgg gga cct ctt ttc ggg aag ctc ttc ttt gcg ttc      3316
   Gly Tyr Gln Ser Trp Gly Pro Leu Phe Gly Lys Leu Phe Phe Ala Phe
   1000                1005                    1010                    1015
   tgg gtg att gtc cac ctg tac ccc ttc ctc aag ggc ctc atg ggg aag      3364
   Trp Val Ile Val His Leu Tyr Pro Phe Leu Lys Gly Leu Met Gly Lys
                   1020                    1025                    1030
   cag aac cgc acg ccg acc att gtc gtt gtc tgg gct atc ctc ctt gcg      3412
   Gln Asn Arg Thr Pro Thr Ile Val Val Val Trp Ala Ile Leu Leu Ala
               1035                    1040                    1045
   tcg atc ttt tcc ctg atg tgg gtt cgt atc gat cca ttc acc acc cgg      3460
   Ser Ile Phe Ser Leu Met Trp Val Arg Ile Asp Pro Phe Thr Thr Arg
           1050                    1055                    1060
   gtc act ggc cct gat atc gcg aaa tgt ggc atc aac t gctaggatga         3507
   Val Thr Gly Pro Asp Ile Ala Lys Cys Gly Ile Asn
       1065                    1070                    1075
   gctgaagata gttaaagagt ggaactagac gcattgtgca tcgtaagtta tcagtgggtg   3567
   gctcttttta tagtatggta ggaacttggt cgggagacgt taattacata tgctatatgt   3627
   acctccgctg gtctttatcc gtaagttaat atatatactg ctttgagaat taaaaaaaaa   3687
   aaaaaaaggg cggccgc                                                   3704
<210> 58
   <211> 1076
   <212> PRT
   <213> Zea mays
<400> 58
   Met Asp Gly Gly Asp Ala Thr Asn Ser Gly Lys His Val Ala Gly Gln
   1               5                   10                  15
   Val Cys Gln Ile Cys Gly Asp Gly Val Gly Thr Ala Ala Asp Gly Asp
               20                  25                  30
   Leu Phe Thr Ala Cys Asp Val Cys Gly Phe Pro Val Cys Arg Pro Cys
           35                  40                  45
   Tyr Glu Tyr Glu Arg Lys Asp Gly Thr Gln Ala Cys Pro Gln Cys Lys
       50                  55                  60
   Thr Lys Tyr Lys Arg His Lys Gly Ser Pro Pro Val His Gly Glu Glu
   65                  70                  75                  80
   Asn Glu Asp Val Asp Ala Asp Asp Val Ser Asp Tyr Asn Tyr Gln Ala
                   85                  90                  95
   Ser Gly Asn Gln Asp Gln Lys Gln Lys Ile Ala Glu Arg Met Leu Thr
                   100                 105                 110
   Trp Arg Thr Asn Ser Arg Gly Ser Asp Ile Gly Leu Ala Lys Tyr Asp
               115                 120                 125
   Ser Gly Glu Ile Gly His Gly Lys Tyr Asp Ser Gly Glu Ile Pro Arg
           130                 135                 140
   Gly Tyr Ile Pro Ser Leu Thr His Ser Gln Ile Ser Gly Glu Ile Pro
   145                 150                 155                 160
   Gly Ala Ser Pro Asp His Met Met Ser Pro Val Gly Asn Ile Gly Arg
                   165                 170                 175
   Arg Gly His Gln Phe Pro Tyr Val Asn His Ser Pro Asn Pro Ser Arg
               180                 185                 190
   Glu Phe Ser Gly Ser Leu Gly Asn Val Ala Trp Lys Glu Arg Val Asp
           195                 200                 205
   Gly Trp Lys Met Lys Asp Lys Gly Ala Ile Pro Met Thr Asn Gly Thr
       210                 215                 220
   Ser Ile Ala Pro Ser Glu Gly Arg Gly Val Ala Asp Ile Asp Ala Ser
   225                 230                 235                 240
   Thr Asp Tyr Asn Met Glu Asp Ala Leu Leu Asn Asp Glu Thr Arg Gln
                   245                 250                 255
   Pro Leu Ser Arg Lys Val Pro Ile Pro Ser Ser Arg Ile Asn Pro Tyr
               260                 265                 270
   Arg Met Val Ile Val Leu Arg Leu Ala Val Leu Cys Ile Phe Leu Arg
           275                 280                 285
   Tyr Arg Ile Thr His Pro Val Asn Asn Ala Tyr Pro Leu Trp Leu Leu
       290                 295                 300
   Ser Val Ile Cys Glu Ile Trp Phe Ala Leu Ser Trp Ile Leu Asp Gln
   305                 310                 315                 320
   Phe Pro Lys Trp Ser Pro Ile Asn Arg Glu Thr Tyr Leu Asp Arg Leu
                   325                 330                 335
   Ala Leu Arg Tyr Asp Arg Glu Gly Glu Pro Ser Gln Leu Ala Pro Val
               340                 345                 350
   Asp Ile Phe Val Ser Thr Val Asp Pro Met Lys Glu Pro Pro Leu Val
           355                 360                 365
   Thr Ala Asn Thr Val Leu Ser Ile Leu Ala Val Asp Tyr Pro Val Asp
       370                 375                 380
   Lys Val Ser Cys Tyr Val Ser Asp Asp Gly Ala Ala Met Leu Thr Phe
   385                 390                 395                 400
   Asp Ala Leu Ser Glu Thr Ser Glu Phe Ala Arg Lys Trp Val Pro Phe
                   405                 410                 415
   Cys Lys Lys Tyr Asn Ile Glu Pro Xaa Ala Pro Glu Trp Tyr Phe Ala
               420                 425                 430
   Gln Lys Ile Asp Tyr Leu Lys Asp Lys Val Gln Thr Ser Phe Val Lys
           435                 440                 445
   Glu Arg Arg Ala Met Lys Arg Glu Tyr Glu Glu Phe Lys Val Arg Ile
       450                 455                 460
   Asn Gly Leu Val Ala Lys Ala Gln Lys Val Pro Glu Glu Gly Trp Ile
   465                 470                 475                 480
   Met Gln Asp Gly Thr Pro Trp Pro Gly Asn Asn Thr Arg Asp His Pro
                   485                 490                 495
   Gly Met Ile Gln Val Phe Leu Gly His Ser Gly Gly Leu Asp Val Glu
               500                 505                 510
   Gly Asn Glu Leu Pro Arg Leu Val Tyr Val Ser Arg Glu Lys Arg Pro
           515                 520                 525
   Gly Phe Gln His His Lys Lys Ala Gly Ala Met Asn Ala Leu Val Arg
       530                 535                 540
   Val Ser Ala Val Leu Thr Asn Gly Gln Tyr Met Leu Asn Leu Asp Cys
   545                 550                 555                 560
   Asp His Tyr Ile Asn Asn Ser Lys Ala Leu Arg Glu Ala Met Cys Phe
                   565                 570                 575
   Leu Met Asp Pro Asn Leu Gly Arg Asn Val Cys Tyr Val Gln Phe Pro
               580                 585                 590
   Gln Arg Phe Asp Gly Ile Asp Arg Asn Asp Arg Tyr Ala Asn Arg Asn
           595                 600                 605
   Thr Val Phe Phe Asp Ile Asn Leu Arg Gly Leu Asp Gly Ile Gln Gly
       610                 615                 620
   Pro Val Tyr Val Gly Thr Gly Cys Val Phe Asn Arg Thr Ala Leu Tyr
   625                 630                 635                 640
   Gly Tyr Glu Pro Pro Val Lys Lys Lys Lys Pro Gly Phe Phe Ser Ser
                   645                 650                 655
   Leu Cys Gly Gly Arg Lys Lys Thr Ser Lys Ser Lys Lys Ser Ser Glu
               660                 665                 670
   Lys Lys Lys Ser His Arg His Ala Asp Ser Ser Val Pro Val Phe Asn
           675                 680                 685
   Leu Glu Asp Ile Glu Glu Gly Ile Glu Gly Ser Gln Phe Asp Asp Glu
       690                 695                 700
   Lys Ser Leu Ile Met Ser Gln Met Ser Leu Glu Lys Arg Phe Gly Gln
   705                 710                 715                 720
   Ser Ser Val Phe Val Ala Ser Thr Leu Met Glu Tyr Gly Gly Val Pro
                   725                 730                 735
   Gln Ser Ala Thr Pro Glu Ser Leu Leu Lys Glu Ala Ile His Val Ile
               740                 745                 750
   Ser Cys Gly Tyr Glu Asp Lys Thr Asp Trp Gly Thr Glu Ile Gly Trp
           755                 760                 765
   Ile Tyr Gly Ser Val Thr Glu Asp Ile Leu Thr Gly Phe Lys Met His
       770                 775                 780
   Ala Arg Gly Trp Arg Ser Ile Tyr Cys Met Pro Lys Arg Pro Ala Phe
   785                 790                 795                 800
   Lys Gly Ser Ala Pro Ile Asn Leu Ser Asp Arg Leu Asn Gln Val Leu
                   805                 810                 815
   Arg Trp Ala Leu Gly Ser Ile Glu Ile Leu Phe Ser Arg His Cys Pro
               820                 825                 830
   Ile Trp Tyr Gly Tyr Gly Gly Arg Leu Lys Phe Leu Glu Arg Phe Ala
           835                 840                 845
   Tyr Ile Asn Thr Thr Ile Tyr Pro Leu Thr Ser Ile Pro Leu Leu Leu
       850                 855                 860
   Tyr Cys Ile Leu Pro Ala Val Cys Leu Leu Thr Gly Lys Phe Ile Ile
   865                 870                 875                 880
   Pro Lys Ile Ser Asn Leu Glu Ser Val Trp Phe Ile Ser Leu Phe Ile
                   885                 890                 895
   Ser Ile Phe Ala Thr Gly Ile Leu Glu Met Arg Trp Ser Gly Val Gly
               900                 905                 910
   Ile Asp Glu Trp Trp Arg Asn Glu Gln Phe Trp Val Ile Gly Gly Ile
           915                 920                 925
   Ser Ala His Leu Phe Ala Val Phe Gln Gly Leu Leu Lys Val Leu Ala
       930                 935                 940
   Gly Ile Asp Thr Ser Phe Thr Val Thr Ser Lys Ala Thr Asp Glu Glu
   945                 950                 955                 960
   Gly Asp Phe Ala Glu Leu Tyr Met Phe Lys Trp Thr Thr Leu Leu Ile
                   965                 970                 975
   Pro Pro Thr Thr Ile Leu Ile Ile Asn Leu Val Gly Val Val Ala Gly
               980                 985                 990
   Ile Ser Tyr Ala Ile Asn Ser Gly Tyr Gln Ser Trp Gly Pro Leu Phe
           995                 1000                1005
   Gly Lys Leu Phe Phe Ala Phe Trp Val Ile Val His Leu Tyr Pro Phe
       1010                1015                1020
   Leu Lys Gly Leu Met Gly Lys Gln Asn Arg Thr Pro Thr Ile Val Val
   1025                1030                1035                1040
   Val Trp Ala Ile Leu Leu Ala Ser Ile Phe Ser Leu Met Trp Val Arg
                   1045                1050                1055
   Ile Asp Pro Phe Thr Thr Arg Val Thr Gly Pro Asp Ile Ala Lys Cys
               1060                1065                1070
   Gly Ile Asn Cys
           1075
<210> S9
   <211> 25
   <212> DNA
   <213> Zea mays
<400> 59
<210> 60
   <211> 25
   <212> DNA
   <213> Zea mays
<400> 60

## Claims

1. An isolated cellulose synthase nucleic acid encoding a polypeptide having cellulose synthase activity and comprising:
(a) a polynucleotide of SEQ ID NO: 57; or
(b) a polynucleotide encoding a polypeptide of SEQ ID NO: 58.

2. A recombinant expression cassette, comprising a nucleic acid of claim 1 operably linked to a promoter.

3. The expression cassette of claim 2 wherein said promoter is a tissue-preferred promoter.

4. A host cell comprising a heterologous expression cassette of claim 2 or 3.

5. The host cell of claim 4 which is a plant cell.

6. A transgenic plant comprising a heterologous expression cassette of claim 2 or 3.

7. The transgenic plant of claim 6, wherein the plant is a monocot.

8. The transgenic plant of claim 6, wherein the plant is selected from maize, soybean, sunflower, sorghum, canola, wheat, alfalfa, cotton, rice, barley, and millet.

9. A transgenic seed from the transgenic plant of claim 6, 7 or 8, which seed comprises a heterologous expression cassette of claim 2 or 3.

10. A method of modulating the level of cellulose synthase in a plant cell capable of plant regeneration, comprising:
(a) transforming the plant cell with an expression cassette comprising a cellulose synthase nucleic acid of claim 1 operably linked to a promoter;
(b) culturing the transformed plant cell; and
(c) expressing said polynucleotide for a time sufficient to modulate the level of cellulose synthase in said transformed plant cell.

11. A method of producing a transgenic plant in which the level of cellulose synthase is modulated by a cellulose synthase nucleic acid of claim 1, comprising transforming a plant cell with an expression cassette comprising said nucleic acid operably linked to a promoter and regenerating from said transformed cell a plant comprising said expression cassette.

12. The method of claim 10 or 11 wherein the plant is selected from maize, soybean, sunflower, sorghum, canola, wheat, alfalfa, cotton, rice, barley and millet.

13. The method of claim 10, 11 or 12, wherein the promoter is a tissue-preferred promoter.

14. The method of claim 10, 11, 12 or 13 wherein the level of cellulose synthase is increased relative to that in the plant cell prior to transformation.

15. A plant of claim 6, 7 or 8 which is homozygous for the nucleic acid of claim 1 contained in the heterologous expression cassette.

16. An isolated protein having cellulose synthase activity and selected from:
(a) a polypeptide of SEQ ID NO: 58; and
(b) a polypeptide encoded by a nucleic acid of claim 1.

## Patentansprüche

1. Isolierte Cellulosesynthase-Nucleinsäure, die ein Polypeptid mit Cellulosesynthaseaktivität codiert und umfasst:
(a) ein Polynucleotid von SEQ ID NO: 57; oder
(b) ein Polynucleotid, das ein Polypeptid von SEQ ID NO: 58 codiert.

2. Rekombinante Expressionskassette, umfassend eine Nucleinsäure nach Anspruch 1, funktionell verknüpft mit einem Promotor.

3. Expressionskassette nach Anspruch 2, wobei der Promotor ein Promotor mit Gewebebevorzugung ist.

4. Wirtszelle, umfassend eine heterologe Expressionskassette nach Anspruch 2 oder 3.

5. Wirtszelle nach Anspruch 4, die eine Pflanzenzelle ist.

6. Transgene Pflanze, umfassend eine heterologe Expressionskassette nach Anspruch 2 oder 3.

7. Transgene Pflanze nach Anspruch 6, wobei die Pflanze eine Monokotyle ist.

8. Transgene Pflanze nach Anspruch 6, wobei die Pflanze ausgewählt ist aus Mais, Sojabohne, Sonnenblume, Sorghum, Canola-Raps, Weizen, Alfalfa, Baumwolle, Reis, Gerste und Hirse.

9. Transgener Samen aus der transgenen Pflanze nach Anspruch 6, 7 oder 8, wobei der Samen eine heterologe Expressionskassette nach Anspruch 2 oder 3 umfasst.

10. Verfahren zum Modulieren des Levels der Cellulosesynthase in einer Pflanzenzelle, die zur Pflanzenneubildung befähigt ist, umfassend:
(a) Transformieren der Pflanzenzelle mit einer Expressionskassette, umfassend eine Cellulosesynthase-Nucleinsäure nach Anspruch 1, funktionell verknüpft mit einem Promotor;
(b) Kultivieren der transformierten Pflanzenzelle;
(c) Exprimierten des Polynucleotids für eine Zeit, die ausreichend ist, um. den Level der Cellulosesynthase in der transformierten Pflanzenzelle zu modulieren.

11. Verfahren zum Produzieren einer transgenen Pflanze, in der der Level der Cellulosesynthase durch eine Cellulosesynthase-Nucleinsäure nach Anspruch moduliert ist, umfassend das Transformieren einer Pflanzenzelle mit einer Expressionskassette, umfassend die Nukleinsäure, funktionell verknüpft mit einem Promotor, und das Neubilden einer Pflanze, umfassend die Expressionskassette, aus der transformierten Zelle.

12. Verfahren nach Anspruch 10 oder 11, wobei die Pflanze ausgewählt ist aus Mais, Sojabohne, Sonnenblume, Canola-Raps, Weizen, Alfalfa, Baumwolle, Reis, Gerste und Hirse.

13. Verfahren nach Anspruch 10, 11 oder 12, wobei der Promotor ein Promotor mit Gewebebevorzugung ist.

14. Verfahren nach Anspruch 10, 11, 12, oder 13, in dem der Level der Cellulosesynthase in Relation zu demjenigen in der Pflanzenzelle vor der Transformation erhöht wird.

15. Pflanze nach Anspruch 6, 7 oder 8, die für die Nucleinsäure nach Anspruch 1, enthalten in der heterologen Expressionskassette, homozygot ist.

16. Isoliertes Protein mit Cellulosesynthaseaktivität und ausgewählt aus:
(a) einem Polypeptid von SEQ ID NO: 58; und
(b) einem Polypeptid, das durch eine Nucleinsäure nach Anspruch 1 codiert wird.

## Revendications

1. Acide nucléique de cellulose synthase isolé codant un polypeptide ayant une activité cellulose synthase et comprenant
(a) un polynucléotide de SEQ ID NO: 57; ou
(b) un polynucléotide codant un polypeptide de SEQ ID NO: 58.

2. Cassette d'expression recombinante, comprenant un acide nucléique de la revendication 1 lié de façon opérationnelle à un promoteur.

3. Cassette d'expression de la revendication 2, dans laquelle ledit promoteur est un promoteur tissu-spécifique.

4. Cellule hôte comprenant une cassette d'expression hétérologue de la revendication 2 ou 3.

5. Cellule hôte de la revendication 4 qui est une cellule végétale

6. Plante transgénique comprenant une cassette d'expression hétérologue de la revendication 2 ou 3.

7. Plante transgénique de la revendication 6, laquelle plante est une monocotylédone.

8. Plante transgénique de la revendication 6, laquelle plante est choisie parmi le maïs, le soja, le tournesol, le sorgho, le canola, le blé, la luzerne, le coton, le riz, l'orge et le mille.

9. Graines transgéniques issues de la plante transgénique de la revendication 6, 7 ou 8, cette graine comprenant une cassette d'expression hétérologue de la revendication 2 ou 3.

10. Procédé de modulation du niveau de cellulose synthase dans une cellule végétale capable de régénération de plante, comprenant :
(a) la transformation de la cellule végétale par une cassette d'expression comprenant un acide nucléique de cellulose synthase de la revendication 1 lié de façon opérationnelle à un promoteur ;
(b) la culture de la cellule végétale transformée ; et
(c) l'expression dudit polynucléotide pendant une durée suffisante pour moduler le niveau de cellulose synthase dans ladite cellule végétale transformée.

11. Procédé de production d'une plante transgénique dans laquelle le niveau de cellulose synthase est modulé par un acide nucléique de cellulose synthase de la revendication 1, comprenant la transformation d'une cellule végétale par une cassette d'expression comprenant ledit acide nucléique lié de façon opérationnelle à un promoteur et la régénération à partir de ladite cellule transformée d'une plante comprenant ladite cassette d'expression.

12. Procédé de la revendication 10 ou 11, dans lequel la plante est choisie parmi le maïs, le soja, le tournesol, le canola, le blé, la luzerne, le coton, le riz, l'orge et le millet.

13. Procédé de la revendication 10, 11 ou 12, dans lequel le promoteur est un promoteur tissu-spécifique.

14. Procédé de la revendication 10, 11, 12 ou 13, dans lequel le niveau de cellulose synthase est accru par rapports à celui présent dans la cellule végétale avant transformation.

15. Plante de la revendication 6, 7 ou 8 qui est homozygote pour l'acide nucléique de la revendication 1 contenue dans la cassette d'expression hétérologue.

16. Protéine isolée ayant une activité cellulose synthase et choisie parmi:
(a) un polypeptide de SEQ ID NO: 58; et
(b) un polypeptide codé par un acide nucléique de la revendication 1.
